# EUROPEAN PATENT APPLICATION

(11) **EP 4 393 920 A1**
(43) Date of publication of application: **03.07.2024**
(21) Application number: 22860623.2
(22) Date of filing: 26.08.2022
(51) Int. Cl.: C07D 413/12, C07D 413/14, C07D 403/02, C07D 261/16, C07D 401/12, A61K 31/422, A61P 9/12, A61P 13/12, A61P 3/10, A61P 35/00

(54) **AROMATIC RING-CONTAINING BIOLOGICAL ANTAGONIST, AND PREPARATION METHOD THEREFOR AND USE THEREOF**

(30) Priority: 26.08.2021 CN 202110988568; 06.06.2022 CN 202210633028
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: XIAO, Hualing, Shanghai 201203 (CN); DONG, Jiaqiang, Shanghai 201203 (CN); LU, Xingyun, Shanghai 201203 (CN); LIU, Qiang, Shanghai 201203 (CN)
(74) Representative: EP&C
(86) International application number: PCT/CN2022/115068
(87) International publication number: WO 2023/025277

(57) **Abstract**

Provided are an aromatic ring-containing biological antagonist as of general formula (I) or a stereoisomer thereof, a preparation method therefor, a pharmaceutical composition containing same, and the use thereof in the preparation of a drug for treating diabetes, kidney diseases or hypertension.

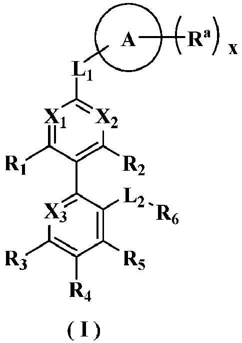

## Description

### FIELD OF THE INVENTION

The present invention belongs to the field of biomedicine, and specifically relates to an aromatic ring-containing biological antagonist, and a preparation method therefor and the application thereof.

### BACKGROUND OF THE INVENTION

Focal segmental glomerulosclerosis (FSGS) is one of the manifestations of nephrotic syndrome and the main cause of end-stage kidney disease. Its pathogenesis is complex and has not yet been fully understood. Current drug treatments, mainly glucocorticoids and immunosuppressants, have poor responses, cannot ideally control the occurrence and progression of FSGS, and have obvious side effects. There is currently no approved treatment for FSGS. The complete response rate of FSGS treatment is less than 30%. One-third of patients progress to chronic renal failure after five years and require long-term dialysis or kidney transplantation to maintain life, which brings heavy economic burden to families and society, and exploring new treatment options has become a focus.

In addition to FSGS, other kidney diseases or conditions characterized by glomerular damage include IgA nephropathy and idiopathic membranous nephropathy. IgA nephropathy, also called Berger's disease, is caused by the accumulation of immunoglobulin A (IgA) in the kidneys. The presence of IgA in the kidney may lead to inflammation, damage to the glomeruli of the kidney, and impaired kidney function, including proteinuria. In some cases, people with IgA nephropathy progress to ESRD. IgA nephropathy is the most common form of glomerulonephritis in the world. In approximately 30% of patients, a decrease in glomerular filtration rate of approximately 50% over 10 years is observed. Patients with IgA nephropathy develop IgG autoantibodies against galactose-deficient IgA1 antibodies. This results in the deposition of these antibodies in the mesangium and activation of complement. Basic treatment for patients with IgA nephropathy involves eliminating risk factors, particularly hypertension, by blocking the renin-angiotensin-aldosterone system (RAAS). Immunosuppression has also been studied in various studies, but no clear advantage was observed. Common side effects of hormone therapy include increased blood sugar, osteoporosis, infection, *etc.* Therefore, there remains a need for compositions and methods for treating various kidney diseases or conditions, such as FSGS, IgA nephropathy, and IMN.

The endogenous vasoactive peptide angiotensin II (AngII) and endothelin-1 (ET-1) are two powerful vasoconstrictors and are thought to play a role in the control of vascular tone and pathological tissue remodeling associated with a variety of diseases, including diabetic nephropathy, heart failure, and chronic or persistently elevated blood pressure. The renin-angiotensin-aldosterone system (RAAS) regulates blood pressure, fluid and sodium balance. Excessive activation of RAAS can promote systemic and regional glomerular capillary hypertension, cause glomerular hemodynamic damage, and lead to kidney damage and kidney fibrosis via profibrotic and proinflammatory pathways. Drugs for RAAS system such as angiotensin receptor blockers (ARBs) have been used to treat diabetic nephropathy, heart failure, and chronic or persistently elevated blood pressure. In addition, accumulating data demonstrate the potential therapeutic benefits of ETA receptor antagonists (ERAs) in hypertension and diabetic nephropathy.

Studies have shown that the combination of ARB and ERA produces a synergistic effect, with AngII and ET-1 working together in blood pressure control and pathological tissue remodeling. Increased Ang II levels promote ET-1 synthesis and vasoconstriction. Blocking ET receptors with ETA can reduce AngII-induced vasoconstriction and reduce plasma aldosterone. ARB not only blocks the effect of AngII on its AT1 receptor, but also limits the production of ET-1. Therefore, blocking AngII and ET-1 activity simultaneously may provide better efficacy than blocking either substance alone. In addition, although ARB is the standard treatment for patients with diabetic nephropathy, dual antagonists (ARB and ERA) have been reported in phase II clinical development to improve proteinuria changes in patients with FSGS. Therefore, drugs with AT1/ETA dual-target antagonistic mechanism have the potential to treat kidney diseases and are of great significance for drug development.

International application WO 2018071784 reports that Sparsentan, an AT1/ETA dual-target antagonist developed by Retrophin, has good anti-glomerular fibrosis effects in preclinical studies, and has been proven in clinical phase II to improve proteinuria levels in FSGS patients, and Phase III clinical trial is launched for the treatment of FSGS and IgA nephropathy. This project aims to develop an AT1/ETA dual-target antagonist to better treat nephrotic syndrome (including FSGS, IgA nephropathy, diabetic nephropathy, etc.).

### SUMMARY OF THE INVENTION

The object of the present invention is to provide a compound of general formula (I), a stereoisomer or a pharmaceutically acceptable salt thereof, the compound has a structure as following: 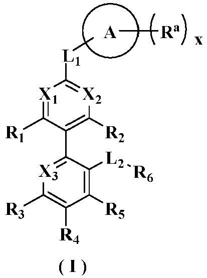 wherein:
X₁ is N or CR¹;
X₂ is N or CR²;
X₃ is N or CR³;
R¹, R² and R³ are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-;
L₂ is selected from the group consisting of -(CH₂)ₙ₂-, -(CH₂)ₙ₂NR_{c}-, - (CH₂)ₙ₂C(O)NR_{c}-, -(CH₂)ₙ₂C(O)NR_{c}S(O)ₘ₂-, -(CH₂)ₙ₂NR_{c}C(O)-, -(CH₂)ₙ₂S(O)ₘ₂-, -(CH₂)ₙ₂S(O)ₘ₂NR_{c}-, -(CH₂)ₙ₂S(O)ₘ₂NR_{c}C(O)-, -(CH₂)ₙ₂S(O)ₘ₂NR_{c}C(O)NR_{d}-, - (CH₂)ₙ₂S(O)ₘ₂NR_{c}C(O)O(CH₂)ₙ₃-, -(CH₂)ₙ₂NR_{c}S(O)ₘ₂- and -(CH₂)ₙ₂NR_{c}S(O)ₘ₂N R_{d}C(O)-;
ring A is selected from the group consisting of cycloalkyl, heterocyclyl, aryl or heteroaryl, and the cycloalkyl, heterocyclyl, aryl and heteroaryl may be optionally further substituted;
R^{a} is independently selected from the group consisting of hydrogen, deuterium, halogen, amino, nitro, hydroxy, cyano, oxo, thio, alkyl, deuteroalkyl, haloalkyl, hydroxyalkyl, alkoxy, haloalkoxy, alkenyl, alkynyl, cycloalkyl, heterocyclyl, aryl, heteroaryl, -(CH₂)ₙ₄C(O)R_{A1}, -(CH₂)ₙ₄C(O)OR_{A1}, - (CH₂)ₙ₄C(O)NR_{A1}R_{B1} and -(CH₂)ₙ₄C(=S) NR_{A1}R_{B1}, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
R₁ is selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl, heteroaryl, - (CH₂)ₙ₅R_{A2}-, -(CH₂)ₙ₅O(CH₂)ₙ₆R_{A2}-, -(CH₂)ₙ₅C(O)R_{A2}, -(CH₂)ₙ₅NR_{A2}C(O)R_{B2}, - (CH₂)ₙ₅C(O)NR_{A2}R_{B2}, -(CH₂)ₙ₅OC(O)N R_{A2}R_{B2} and -(CH₂)ₙ₅NR_{A2}C(O)OR_{B2}, wherein the amino, alkyl, deuteroalkyl, haloalkyl, hydroxyalkyl, alkoxy, haloalkoxy, alkenyl, alkynyl, cycloalkyl, heterocyclyl, aryl and heteroaryl may be optionally further substituted;
or, R₁ and R^{a}, together with the atoms to which they are attached, form cycloalkyl, heterocyclyl, aryl or heteroaryl, the cycloalkyl, heterocyclyl, aryl and heteroaryl may be optionally further substituted;
R₂, R₃, R₄ and R₅ are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
R₆ is selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
Rₐ, R_{b}, R_{c}, R_{d}, R_{A1}, R_{A2}, R_{B1} and R_{B2} are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
x is 0, 1, 2, 3, 4 or 5;
n1-n6 are 0, 1, 2, 3, 4 or 5; and
m1 and m2 are 0, 1 or 2.

In one embodiment of the present invention, R₂ and R₅, together with the atoms to which they are attached, form a heterocyclyl, the heterocyclyl may be optionally further substituted.

In a preferred embodiment of the present invention, the ring A is selected from the group consisting of C₃₋₁₂ cycloalkyl, 3- to 12-membered heterocyclyl, C₆₋₁₄ aryl and 5- to 14-membered heteroaryl, wherein the C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl.

In a further preferred embodiment of the present invention, the ring A is selected from the group consisting of a 5- to 10-membered heterocyclyl and a 5- to 10-membered heteroaryl.

In a further preferred embodiment of the present invention, the ring A is selected from the group consisting of a 5- to 6-membered nitrogen-containing monoheterocyclyl, a 6- to 10-membered nitrogen-containing spiroheterocyclyl, and a 5- to 6-membered nitrogen-containing heteroaryl.

In a further preferred embodiment of the present invention, the ring A is selected from the group consisting of pyridyl, pyrazinyl, pyrimidinyl, pyridazinyl, pyrrolyl, imidazolyl, pyrazolyl, 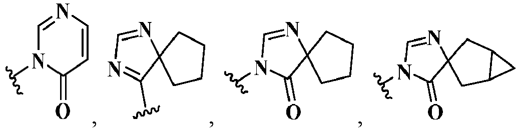 and 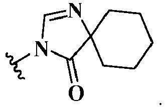
R^{a} is independently selected from the group consisting of hydrogen, deuterium, halogen, amino, nitro, hydroxy, cyano, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 12-membered heteroaryl, -(CH₂)ₙ₄C(O)R_{A1}, -(CH₂)ₙ₄C(O)OR_{A1}, - (CH₂)ₙ₄C(O)NR_{A1}R_{B1} and -(CH₂)ₙ₄C(=S)NR_{A1}R_{B1}, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 12-membered heteroaryl may be optionally further substituted;
R_{A1} and R_{B1} are each independently selected from the group consisting of hydrogen, deuterium, halogen, amino, nitro, hydroxy, cyano, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 12-membered heteroaryl, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 12-membered heteroaryl may be optionally further substituted;
preferably, R^{a} is independently selected from the group consisting of amino, nitro, hydroxy, cyano, oxo, C₁₋₆ alkyl, C₁₋₆ hydroxyalkyl, -(CH₂)ₙ₄C(O)OR_{A1}, and (CH₂)ₙ₄C(=S)NR_{A1}R_{B1}; R_{A1} and R_{B1} are each independently selected from the group consisting of hydrogen and C₁₋₆ alkyl;
more preferably, R^{a} is independently selected from the group consisting of oxo, C₁₋₄ alkyl, C₁₋₃ hydroxyalkyl, -(CH₂)ₙ₄C(O)OR_{A1}, and (CH₂)ₙ₄C(=S)NR_{A1}R_{B1}; R_{A1} and R_{B1} are each independently selected from the group consisting of hydrogen and C₁₋₆ alkyl;
and most preferably, R^{a} is independently selected from the group consisting of oxo, n-propyl, n-butyl, carboxyl, ethyl, -CH₂OH, -C(O)NH₂, -C(O)NHCH₃,

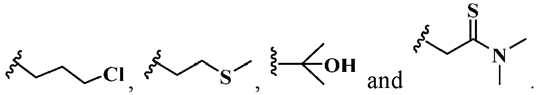

In a further preferred embodiment of the present invention, the compound, or the stereoisomer or the pharmaceutically acceptable salt thereof is further of general formula (II): 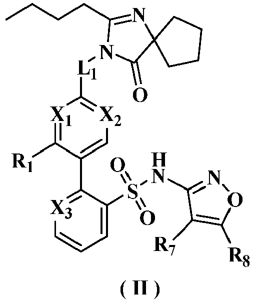
X₁ is N or CR¹;
X₂ is N or CR²;
X₃ is N or CR³;
R¹, R² and R³ are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}-, -(CH₂)ₙ₅O(CH₂)ₙ₆R_{A2}-, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R₇ and R₈ are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
or, R₇ and R₈ are bond to form a C₃₋₈ cycloalkyl, 5- to 8-membered heterocyclyl, C₆₋₁₄ aryl or 5- to 14-membered heteroaryl, wherein the C₃₋₈ cycloalkyl, 5- to 8-membered heterocyclyl, C₆₋₁₄ aryl or 5- to 14-membered heteroaryl may be optionally further substituted;
Rₐ and R_{b} are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R_{A2} and R_{B2} are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
n1, n5 and n6 are 0, 1, 2 or 3; and
m1 is 0, 1 or 2.

In a further preferred embodiment of the present invention, the compound is further a compound of general formula (VIII-1) or general formula (VIII-2), or the stereoisomer or the pharmaceutically acceptable salt thereof: 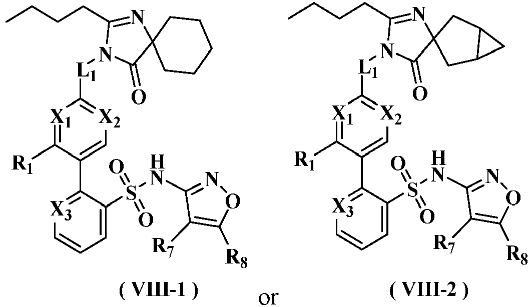
wherein L₁, X₁, X₂, X₃, R₇ and R₈ are as defined in claim 2;
for general formula (VIII-1), when L₁ is CH₂, R₇ and R₈ are both methyl, and R₁ is 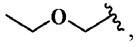 , at least one of X₁, X₂ and X₃ is not CH.

In a preferred embodiment of the present invention, the L₁ is selected from the group consisting of -CRₐR_{b}-, -CRₐR_{b}O-, -OCRₐR_{b}-, -CRₐR_{b}S- and -SCRₐR_{b}-.

In a further preferred embodiment of the present invention, the L₁ is selected from the group consisting of -CH₂-, -CD₂- and -CH₂O-;
Rₐ and R_{b} are each independently selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, wherein the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl is optionally further substituted with one or more substituents selected from the group consisting of deuterium, halogen, amino, hydroxy, cyano, oxo, thioxo, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl.

In a preferred embodiment of the present invention, the R₁ and R₇ are bond to form a 8- to 20-membered heterocyclyl, the heterocyclyl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl.

In a further preferred embodiment of the present invention, the R₁ and R₇ are bond to form a 8- to 14-membered heterocyclyl.

In a further preferred embodiment of the present invention, the R₁ and R₇ are bond to form a 8- to 14-membered oxygen-containing heterocyclyl.

In a preferred embodiment of the present invention, X₁, X₂ and X₃ are all CH;
L₁ is selected from the group consisting of -CH₂- and -CD₂-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
preferably, the R₁ is selected from the group consisting of H, -CH₃, -CH₂CH₃, 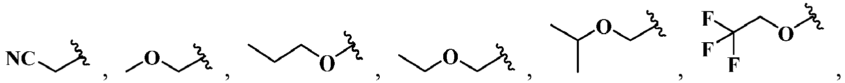 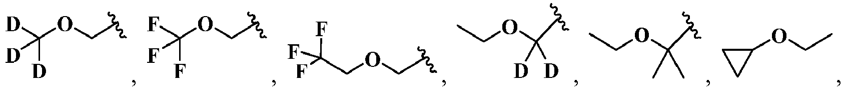 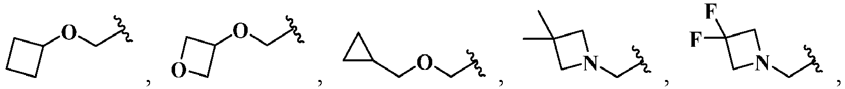 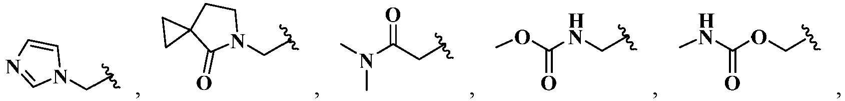 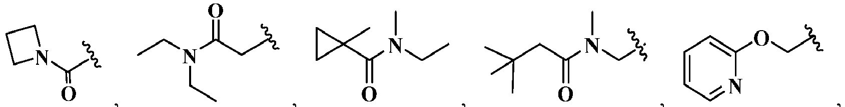 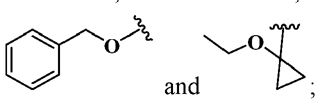
R₇ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl;
preferably, the R₇ is selected from the group consisting of deuterium, fluorine, chlorine, bromine and methyl;
the R₈ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl;
preferably, the R₈ is selected from the group consisting of methyl, ethyl and cyclopropyl.

In a further preferred embodiment of the present invention, the compound, or the stereoisomer or the pharmaceutically acceptable salt thereof is further a compound of general formula (II-1): 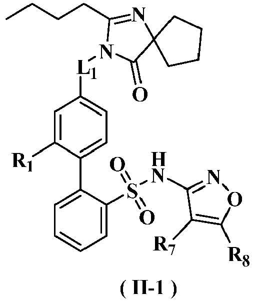
L₁ is selected from the group consisting of -CH₂- and -CD₂-;
R₁ is selected from the group consisting of deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
Further, R₁ is preferably selected from the group consisting of -CH₃, - CH₂CH₃, 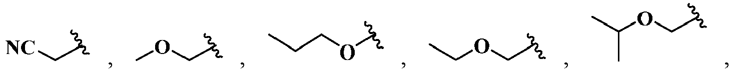 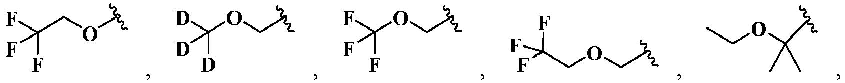 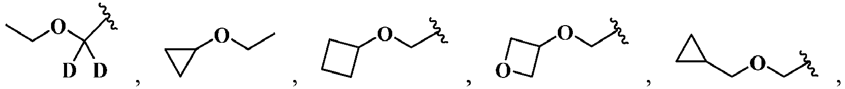 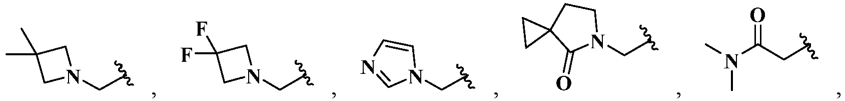 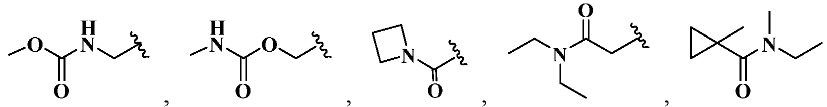 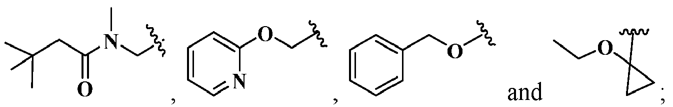 more preferably 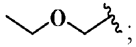
R₇ is selected from the group consisting of halogen, preferably fluorine, chlorine, and bromine, more preferably chlorine;
R₈ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, preferably methyl, ethyl and cyclopropyl, and more preferably methyl.

In a further preferred embodiment of the present invention, in the general formula (II-1),
L₁ is selected from the group consisting of -CH₂- and -CD₂-;
R₁ is -(CH₂)ₙ₅O(CH₂)ₙ₆R_{A2}, n5 is 1 or 2, n6 is 0, R_{A2} is selected from the group consisting of C₁₋₃ alkyl, C₁₋₃ deuteroalkyl and C₁₋₃ haloalkyl;
R₇ is selected from the group consisting of fluorine, chlorine, and bromine, preferably chlorine;
R₈ is methyl, ethyl or cyclopropyl, preferably methyl.

In a further preferred embodiment of the present invention, in the general formula (II-1),
L₁ is selected from the group consisting of -CH₂- and -CD₂-;
R₁ is 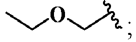
R₇ is selected from the group consisting of fluorine, chlorine, and bromine, preferably chlorine;
and R₈ is methyl.

In a further preferred embodiment of the present invention, the compound, or the stereoisomer or the pharmaceutically acceptable salt thereof is further a compound of general formula (III): 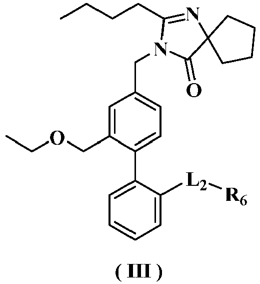 wherein:
L₂ is selected from the group consisting of -(CH₂)ₙ₂C(O)NR_{c}-, - (CH₂)ₙ₂C(O)NR_{c}S(O)ₘ₂-, -(CH₂)ₙ₂NR_{c}C(O)-, -(CH₂)ₙ₂S(O)ₘ₂-, - (CH₂)ₙ₂S(O)ₘ₂NR_{c}-, -(CH₂)ₙ₂S(O)ₘ₂NR_{c}C (O)-, -(CH₂)ₙ₂S(O)ₘ₂NR_{c}C(O)NR_{d}-, - (CH₂)ₙ₂S(O)ₘ₂NR_{c}C(O)O(CH₂)ₙ₃-, -(CH₂)ₙ₂NR_{c}S(O)ₘ₂- and-(CH₂)ₙ₂NR_{c}S(O)ₘ₂NR_{d}C(O)-;
R₆ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R_{c} and R_{d} are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
n2 and n3 are 0, 1, 2 or 3;
m2 is 0, 1 or 2.

In a preferred embodiment of the present invention, the L₂ is selected from the group consisting of C(O)NR_{c}-, -C(O)NR_{c}S(O)₂-, -NR_{c}C(O)-, -S(O)₂-, -S(O)₂NR_{c}-, -S(O)₂NR_{c}C(O)-, -S(O)₂NR_{c}C(O)NR_{d}-, -S(O)₂NR_{c}C(O)OCH₂-, -NR_{c}S(O)₂- and - NR_{c}S(O)₂NR_{d}C(O)-;

In a further preferred embodiment of the present invention, the L₂ is selected from the group consisting of -C(O)NH-, -C(O)NHS(O)₂-, -S(O)₂NH-, - S(O)₂NHC(O)-, -S(O)₂NHC(O)NH-, -S(O)₂NHC(O)O-, -S(O)₂NHC(O)OCH₂-, - NHS(O)₂- and -NHS(O)₂NHC(O)-;
the R_{c} and R_{d} are each independently selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl is optionally further substituted with one or more substituents selected from the group consisting of deuterium, halogen, amino, hydroxy, cyano, oxo, thioxo, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl.

In a preferred embodiment of the present invention, the R₆ is selected from the group consisting of amino, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, wherein the amino, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl is optionally further substituted with one or more substituents selected from the group consisting of deuterium, halogen, amino, hydroxy, cyano, oxo, thioxo, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3-to 8-membered heterocyclyl, C₆₋₁₀ aryl and 5- to 10-membered heteroaryl;
In a further preferred embodiment of the present invention, the R₆ is selected from the group consisting of amino, methyl, ethyl, propyl, isopropyl, cyclopropyl, oxazolyl, isoxazolyl, triazolyl, phenyl, pyridyl, pyrazinyl, tetrazolyl, dihydrotetrazolyl, 1,2,4-oxadiazol-5(2H)-one and 5,6-dihydro-4H-cyclopenta[d]isoxazolyl, wherein the amino, methyl, ethyl, propyl, isopropyl, cyclopropyl, oxazolyl, isoxazolyl, triazolyl, phenyl, pyridyl, pyrazinyl, tetrazolyl, dihydrotetrazolyl, 1,2,4-oxadiazol-5(2H)-one or 5,6-dihydro-4H-cyclopenta[d]isoxazolyl is optionally further substituted with one or more substituents selected from the group consisting of oxo, methyl, ethyl, propyl and cyclopropyl.

In a further preferred embodiment of the present invention, the compound, or the stereoisomer or the pharmaceutically acceptable salt thereof is characterized in that, the compound is further a compound of general formula (IV): 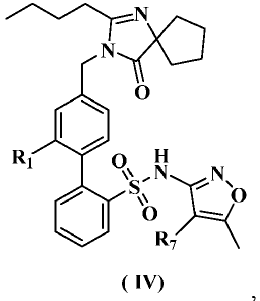
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)N R_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl,
Preferably hydrogen, -CH₃, -CH₂CH₃, 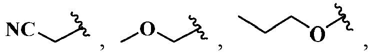 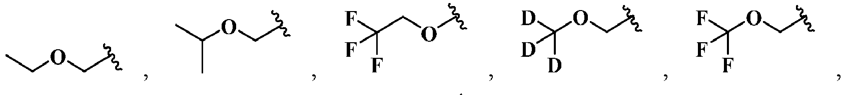 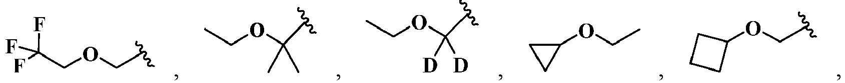 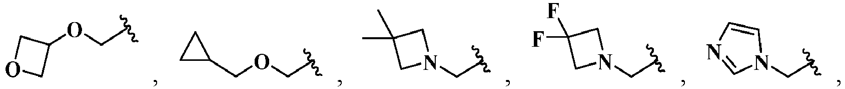 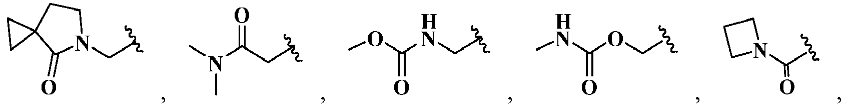 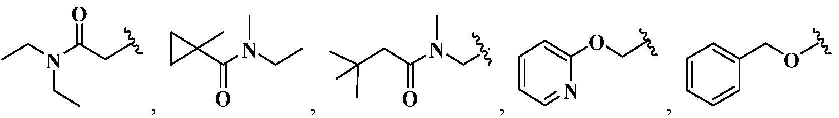 and 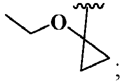
R₇ is a halogen, preferably fluorine, chlorine, or bromine.

In a further preferred embodiment of the present invention, the compound, or the stereoisomer or the pharmaceutically acceptable salt thereof is characterized in that, the compound is further a compound of general formula (V): 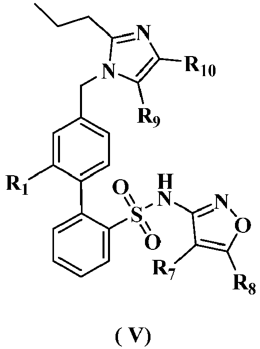
R₉ and R₁₀ are each independently selected from the group consisting of hydrogen, deuterium, halogen, amino, nitro, hydroxy, cyano, oxo, thio, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 12-membered heteroaryl, -(CH₂)ₙ₄C(O)R_{A1}, -(CH₂)ₙ₄C(O)OR_{A1}, - (CH₂)ₙ₄C(O)NR_{A1}R_{B1} and -(CH₂)ₙ₄C(=S)NR_{A1}R_{B1}, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 12-membered heteroaryl may be optionally further substituted; R_{A1} and R_{B1} are each independently selected from the group consisting of hydrogen, deuterium, halogen, amino, nitro, hydroxy, cyano, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, and 5- to 12-membered heteroaryl, wherein the amino, C₁₋₆ alkyl, C₁₋₆ deuteroalkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, or 5- to 12-membered heteroaryl may be optionally further substituted,
preferably, selected from the group consisting of amino, nitro, hydroxy, cyano, oxo, C₁₋₆ alkyl, C₁₋₆ hydroxyalkyl, -(CH₂)ₙ₄C(O)OR_{A1}, and (CH₂)ₙ₄C(=S)NR_{A1}R_{B1}; R_{A1} and R_{B1} are each independently selected from the group consisting of hydrogen and C₁₋₆ alkyl,
more preferably, selected from the group consisting of oxo, C₁₋₄ alkyl, C₁₋₃ hydroxyalkyl, -(CH₂)ₙ₄C(O)OR_{A1}, and (CH₂)ₙ₄C(=S )NR_{A1}R_{B1}; R_{A1} and R_{B1} are each independently selected from the group consisting of hydrogen and C₁₋₆ alkyl,
and most preferably, selected from the group consisting of methyl, ethyl, propyl, carboxyl, 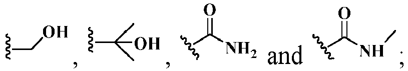
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thio, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl,
Preferably hydrogen, -CH₃, -CH₂CH₃, 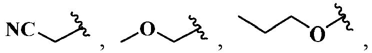 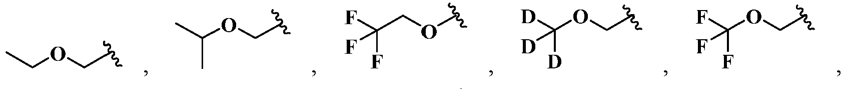 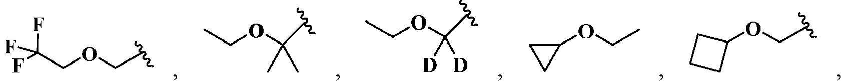 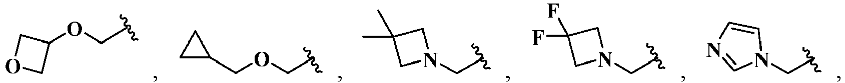 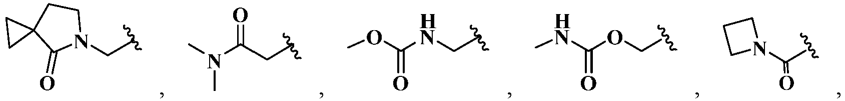 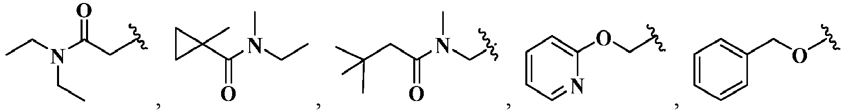 and 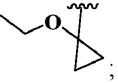
R₇ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
Preferably deuterium, fluorine, chlorine, bromine and methyl;
R₈ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
and preferably methyl, ethyl and cyclopropyl.

The present invention further provides a compound of general formula (VI), a stereoisomer or a pharmaceutically acceptable salt thereof: 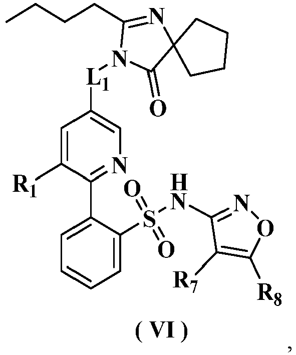
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-,
Preferably -CRaRb-, -CRaRbO-, -OCRaRb-, -CRaRbS- and -SCRaRb-, more preferably -CH2- and -CD2-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thio, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl,
Preferably hydrogen, -CH₃, -CH₂CH₃, 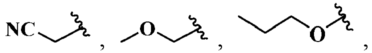 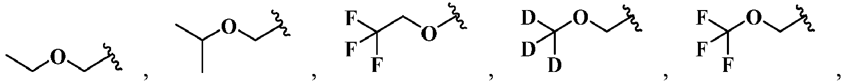 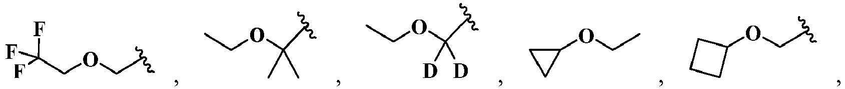 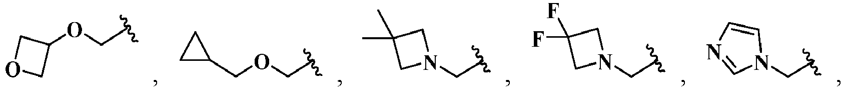 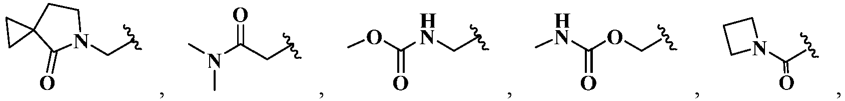 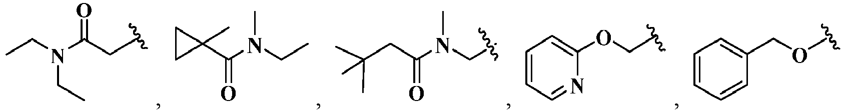 and 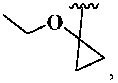
more preferably, hydrogen, -CH₃, -CH₂CH₃ and •
R₇ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
Preferably deuterium, fluorine, chlorine, bromine or methyl;
R₈ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
and preferably methyl, ethyl and cyclopropyl.

The present invention further provides a compound of general formula (VII), a stereoisomer or a pharmaceutically acceptable salt thereof: 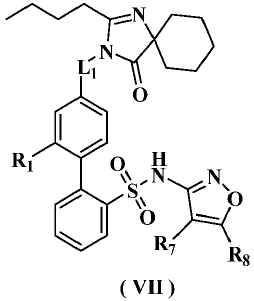
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-,
Preferably -CRₐR_{b}-, -CRₐR_{b}O-, -OCRₐR_{b}-, -CRₐR_{b}S- and -SCRₐR_{b}-,
more preferably -CH₂- and -CD₂-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)N R_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl,
Preferably hydrogen, -CH₃, -CH₂CH₃, 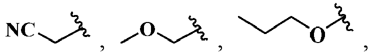 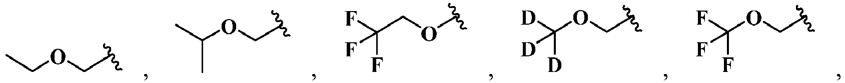 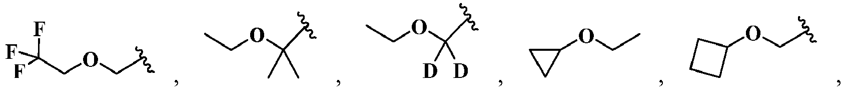 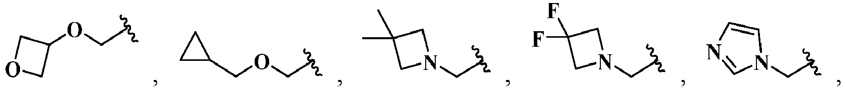 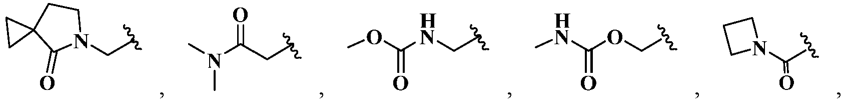 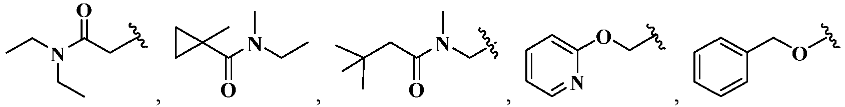 and 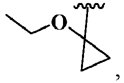
more preferably, hydrogen, -CH₃, -CH₂CH₃ and 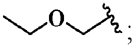
R₇ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
Preferably deuterium, fluorine, chlorine, bromine and methyl;
Rs is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl,
and preferably methyl, ethyl and cyclopropyl.

In certain embodiments of the present invention, in the compound, or the stereoisomer or the pharmaceutically acceptable salt thereof, when R₇ and R₈ are both methyl and R₁ is not 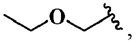 R₁ is selected from the group consisting of 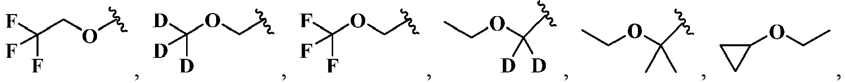 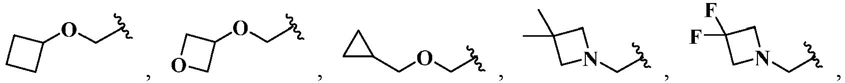 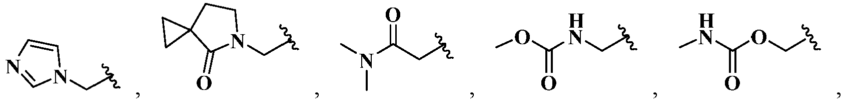 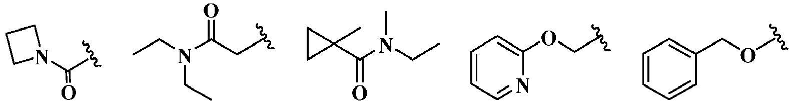 and 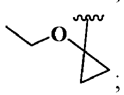 when R₇ and R₈ are both methyl and R₁ is 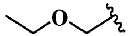 , L₁ contains deuterium or at least one of X₁, X₂ or X₃ is N or R³ is not hydrogen.

In a further preferred embodiment of the present invention, the compound, or the stereoisomer or the pharmaceutically acceptable salt thereof is selected from the group consisting of the following compounds:

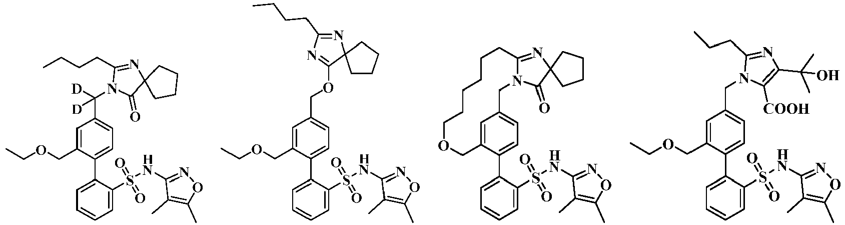

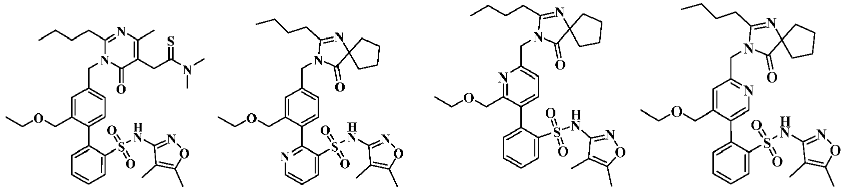

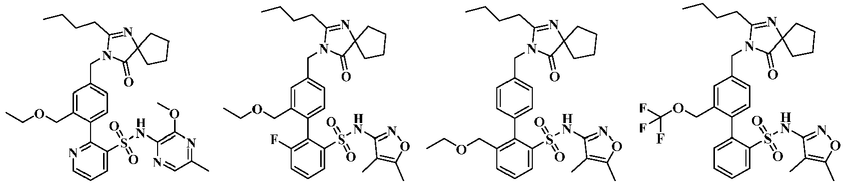

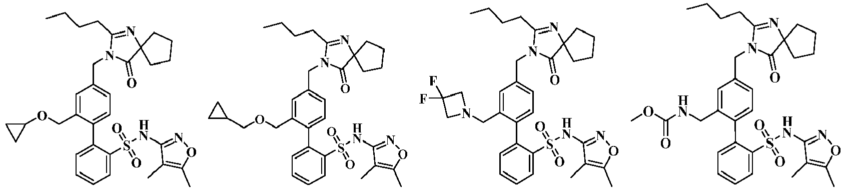

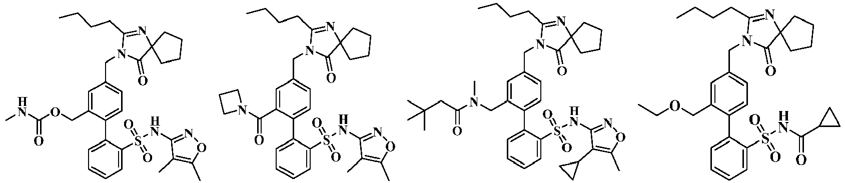

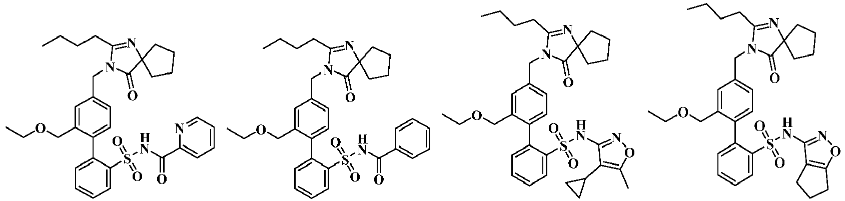

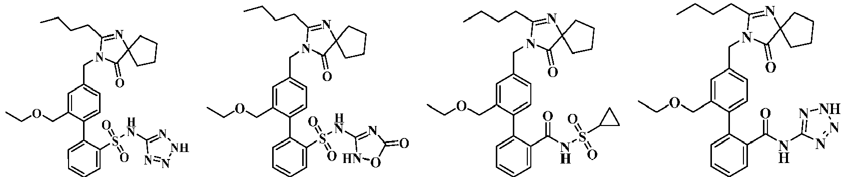

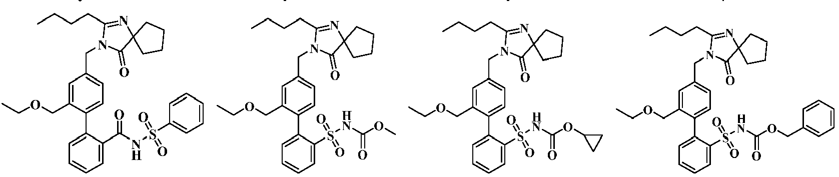

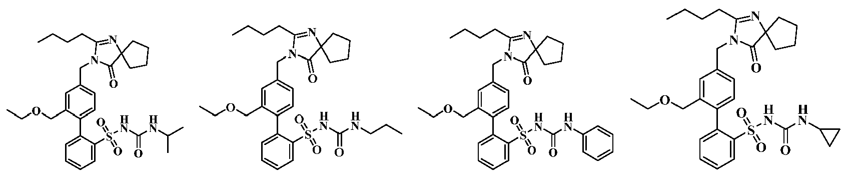

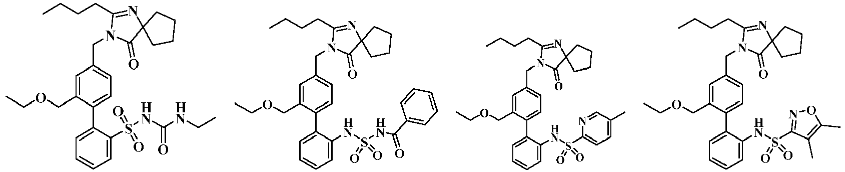

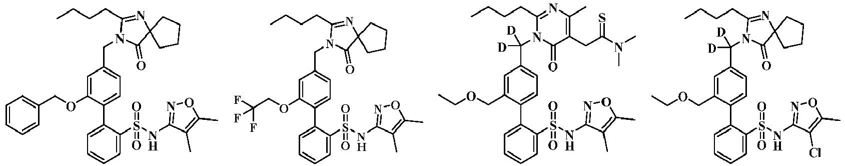

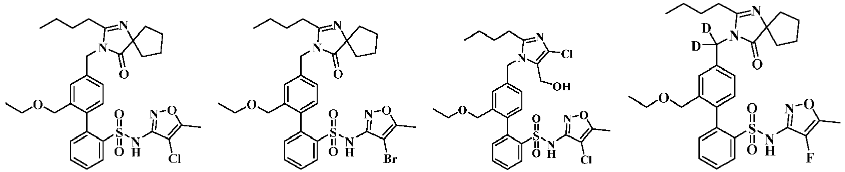

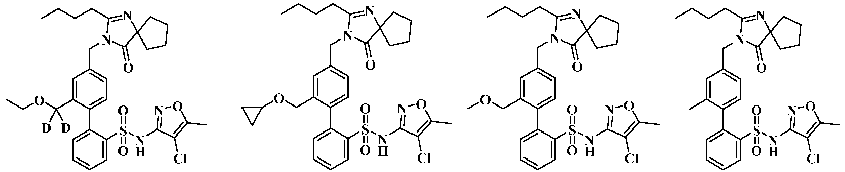

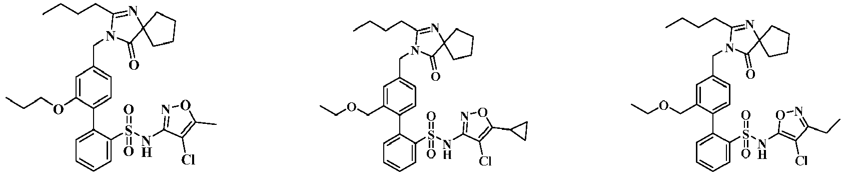

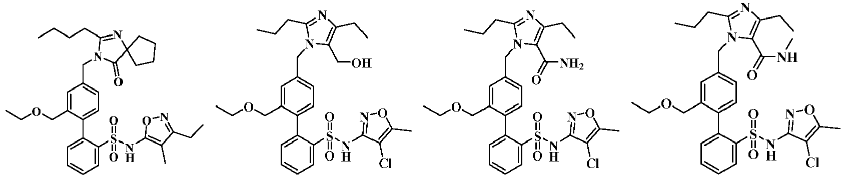

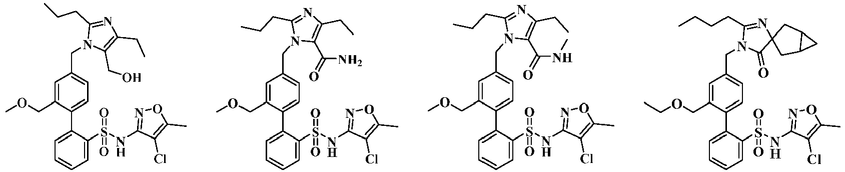

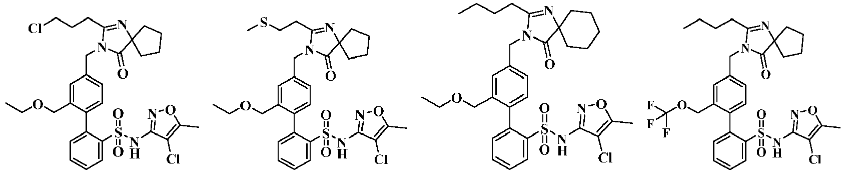

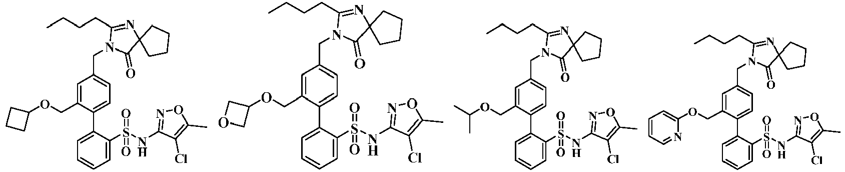

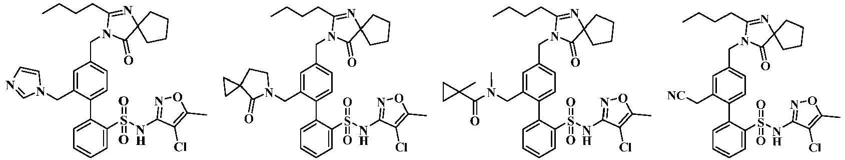

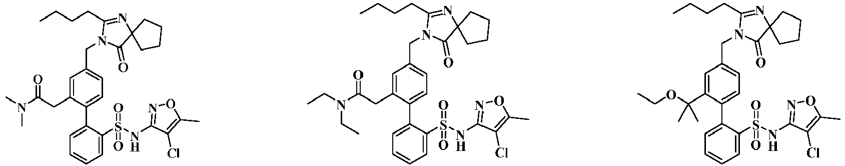

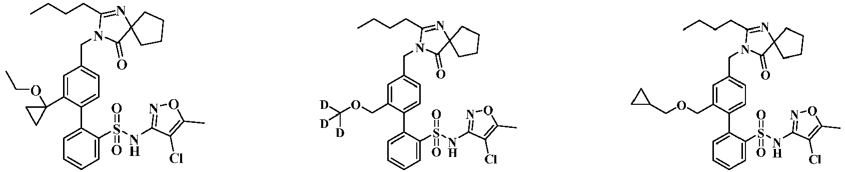

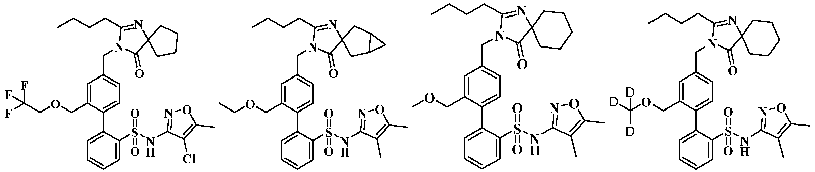

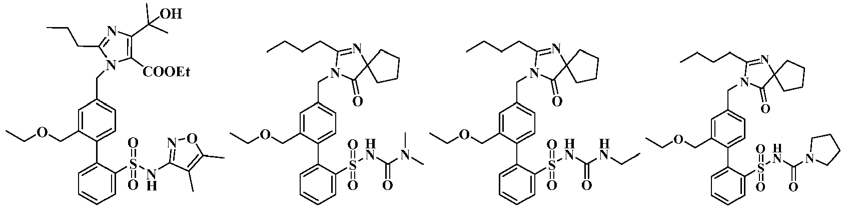

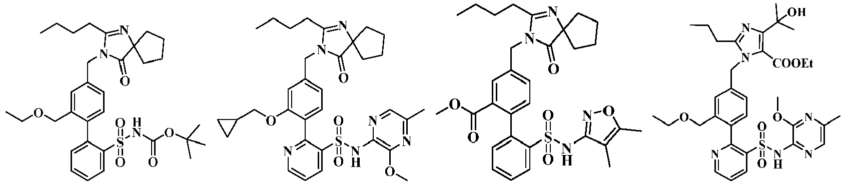

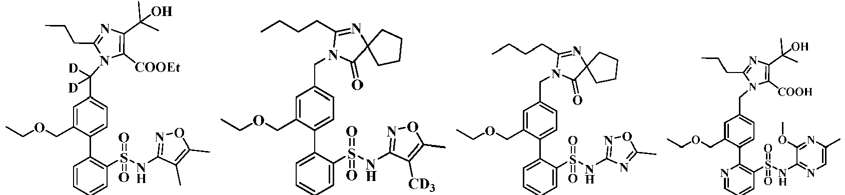

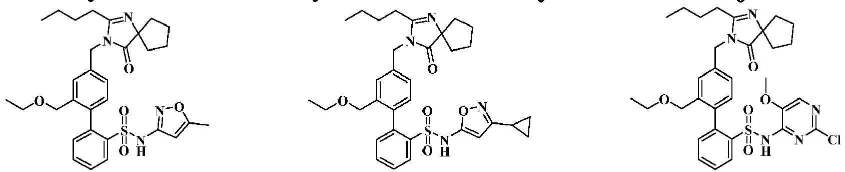

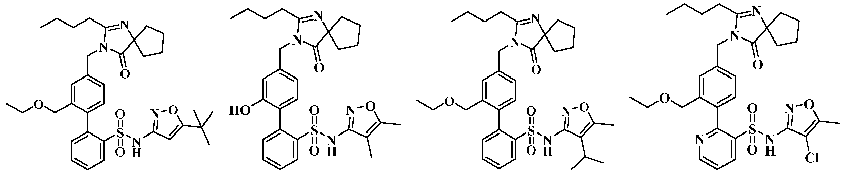

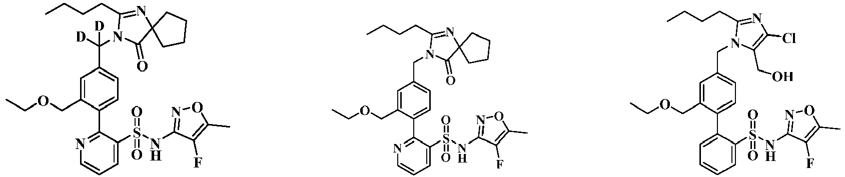

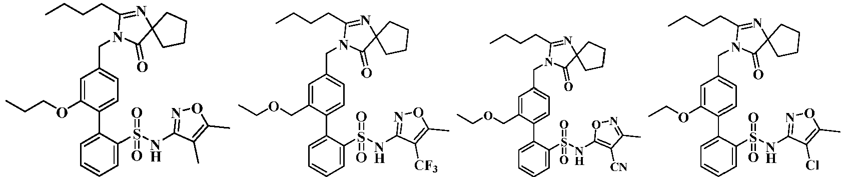

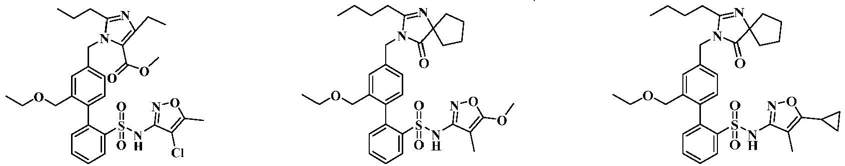

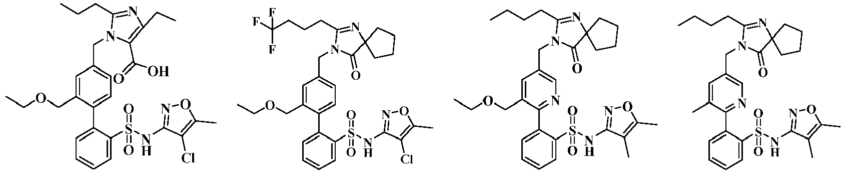

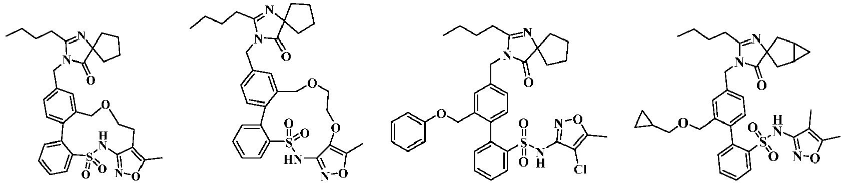

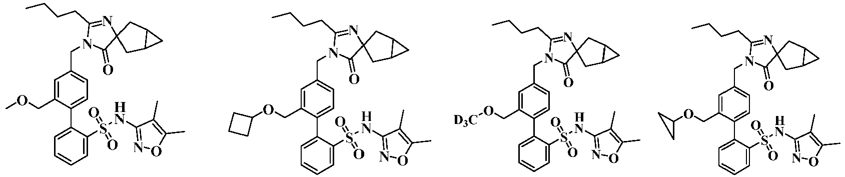

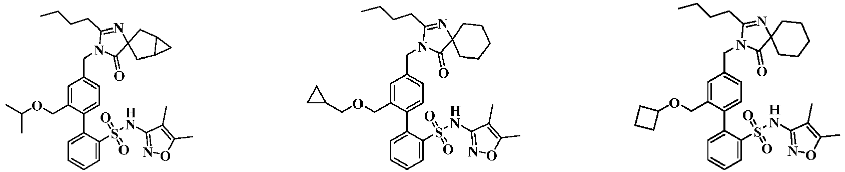

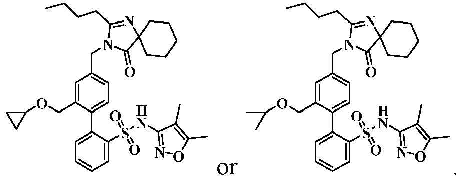

The present invention further provides a compound of general formula (M-1) or (M-2), a stereoisomer or a pharmaceutically acceptable salt thereof: 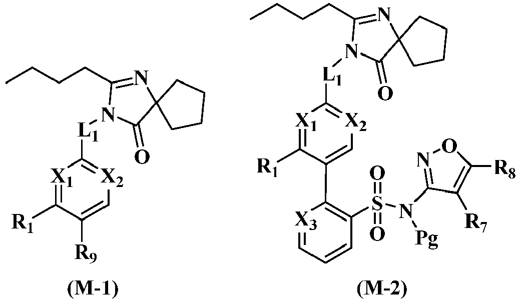
the L₁, X₁, X₂, X₃, R₁, R₇, and R₈ are as defined above;
R₉ is selected from the group consisting of halogen and 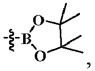 preferably bromine, chlorine or 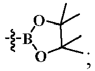
Pg is an amino protecting group; preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl; more preferably (trimethylsilyl)ethoxymethyl or methoxymethylether.

In one embodiment of the present invention, L₁ is selected from the group consisting of -CH₂- and -CD₂-; R₁ is 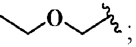
R₉ is 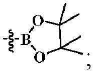
Pg is selected from the group consisting of (trimethylsilyl)ethoxymethyl and methoxymethylether.

The present invention further provides a method for preparing the aforementioned compound of general formula (II) or the stereoisomer and the pharmaceutically acceptable salt thereof, the method comprises the following steps: 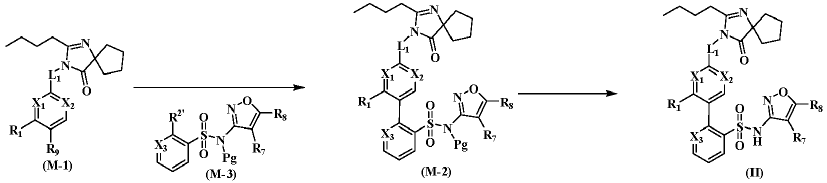
a compound of general formula (M-1) is reacted with a compound of general formula (M-3) to obtain a compound of general formula (M-2), and the compound of general formula (M-2) is deprotected to obtain a compound of general formula (II);
the L₁, X₁, X₂, X₃, R₁, R₇, and R₈ are as defined above;
R^{2'} is selected from the group consisting of 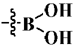 and halogen, preferably 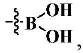 chlorine or bromine;
R₉ is selected from the group consisting of 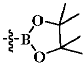 and halogen, preferably 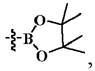 chlorine or bromine;
Pg is an amino protecting group; preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl; more preferably (trimethylsilyl)ethoxymethyl or methoxymethylether.

The present invention further provides a method for preparing the aforementioned compound of general formula (II) or the stereoisomer and pharmaceutically acceptable salt thereof, the method comprises the following steps: 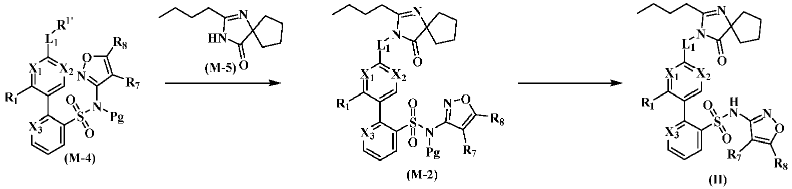
a compound of general formula (M-4) is reacted with a compound of general formula (M-5) to obtain a compound of general formula (M-2), and the compound of general formula (M-2) is deprotected to obtain a compound of general formula (II);
the L₁, X₁, X₂, X₃, R₁, R₇, and R₈ are as defined above;
R^{1'} is selected from the group consisting of methanesulfonyloxy and halogen, preferably methanesulfonyloxy or bromine;
Pg is an amino protecting group; preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl; more preferably (trimethylsilyl)ethoxymethyl or methoxymethylether.

The present invention further relates to a pharmaceutical composition comprising a therapeutically effective dose of a compound of general formula (I), a stereoisomer or a pharmaceutically acceptable salt thereof and one or more pharmaceutically acceptable carriers, diluents or excipients.

On the other hand, the object of the present invention is to provide a use of a compound of general formula (I), a stereoisomer or a pharmaceutically acceptable salt thereof, or a pharmaceutical composition thereof in the preparation of treatment and/or prevention angiotensin II (AT) dependent.

On the other hand, the object of the present invention is to provide use of a compound of general formula (I), a stereoisomer or a pharmaceutically acceptable salt thereof, or a pharmaceutical composition thereof in the preparation of a drug for treating and/or preventing an endothelin (ET)-dependent disease.

On the other hand, the object of the present invention is to provide use of a compound of general formula (I), a stereoisomer or a pharmaceutically acceptable salt thereof, or a pharmaceutical composition thereof in the preparation of a drug for treating and/or preventing a dual-acting angiotensin-dependent and endothelin-dependent (DARA)-dependent disease.

On the other hand, the object of the present invention is to provide use of a compound of general formula (I), a stereoisomer or a pharmaceutically acceptable salt thereof, or a pharmaceutical composition thereof in the preparation of a drug for treating and/or preventing pain, sexual dysfunction, hypoxia and an ischemic disease, dementia, a neurological disease, a liver disease, a cancer, hypertension, diabetes, a kidney disease and related diseases.

The present invention further relates to a method for treating and/or preventing pain, sexual dysfunction, hypoxia and an ischemic disease, dementia, a neurological disease, a liver disease, a cancer, hypertension, diabetes, a kidney disease and related diseases.

On the other hand, the object of the present invention is to provide use of a compound of general formula (I), a stereoisomer or a pharmaceutically acceptable salt thereof, or a pharmaceutical composition thereof for treating and/or preventing pain, sexual dysfunction, hypoxia and an ischemic disease, dementia, a neurological disease, a liver disease, a cancer, hypertension, diabetes, a kidney disease and related diseases.

In the above technical solutions, the kidney-related diseases are diseases or conditions related to kidney, glomerular or glomerular mesangial cell function, more preferably focal segmental glomerulosclerosis or IgA nephropathy.

### Detailed Description of The Present Invention

Unless stated to the contrary, the terms used in the specification and claims have the following meanings.

The term "alkyl" refers to a saturated aliphatic hydrocarbon group, which is a straight or branched chain group containing 1 to 20 carbon atoms, preferably an alkyl group containing 1 to 12 carbon atoms, more preferably an alkyl group containing 1 to 8 carbon atoms, further preferably an alkyl group containing 1 to 6 carbon atoms, and most preferably an alkyl group containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 4-heptyl, 1-propylbutyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, their respective branched chain isomers, *etc.* More preferably is a lower alkyl group containing 1 to 6 carbon atoms, and non-limiting examples thereof include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, 4-heptyl, 1-propylbutyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *etc.* Alkyl group may be substituted or unsubstituted, and when substituted, the substituents may be at any available point of attachment. The substituents are preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, heterocycloalkoxy, cycloalkylthio, heterocycloalkylthio, oxo, carboxyl and carboxylate group, and methyl, ethyl, isopropyl, tert-butyl, haloalkyl, deuteroalkyl, alkoxy-substituted alkyl and hydroxy-substituted alkyl are preferred in the present invention.

The term "cycloalkyl" refers to a saturated or partially unsaturated monocyclic or polycyclic cyclic hydrocarbon substituent. The cycloalkyl ring contains 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl includes spiro, fused and bridged cycloalkyl, preferably cyclopropyl, cyclobutyl, cyclohexyl, cyclopentyl and cycloheptyl.

The cycloalkyl ring can be fused to an aryl, heteroaryl or heterocycloalkyl ring, where the ring bound to the parent structure is cycloalkyl, non-limiting examples include indanyl, tetrahydronaphthyl, benzocycloheptyl, and the like. Cycloalkyl may be optionally substituted or unsubstituted, and when substituted, the substituent is preferably one or more of the following groups, which are independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "heterocyclyl" refers to a saturated or partially unsaturated monocyclic or polycyclic hydrocarbon group, which comprises 3 to 20 ring atoms, wherein one or more ring atoms are heteroatoms selected from nitrogen, oxygen, C(O) or S(O)ₘ (wherein m is an integer of 0 to 2), but excluding ring moieties of - O-O-, -O-S- or -S-S-, and the remaining ring atoms are carbon. Preferably it contains 3 to 12 ring atoms, of which 1-4 are heteroatoms; more preferably it contains 3 to 8 ring atoms; most preferably it contains 3 to 8 ring atoms; further preferably, it is a 3- to 8-membered heterocyclyl containing 1-3 nitrogen atoms, optionally substituted with 1-2 oxygen atoms, sulfur atoms, or oxo, including nitrogen-containing monocyclic heterocyclyl, nitrogen-containing spirocyclic heterocyclyl or nitrogen-containing fused heterocyclyl.

Non-limiting examples of monocyclic heterocyclyl include oxetanyl, azetidinyl, thietanyl, pyrrolidinyl, imidazolidinyl, tetrahydrofuryl, tetrahydrothienyl, tetrahydropyranyl, dihydroimidazolyl, dihydrofuryl, dihydropyrazolyl, dihydropyrrolyl, piperidinyl, piperazinyl, morpholinyl, thiomorpholinyl, homopiperazinyl, azepanyl, 1,4-diazacycloheptyl, pyranyl or tetrahydrothiopyranyl dioxide group, *etc*.; preferably, oxetanyl, azetidinyl, thietanyl, tetrahydrofuryl, tetrahydropyranyl, tetrahydrothienyl, tetrahydrothiopyranyl, tetrahydrothiopyranyl dioxide group, pyrrolidinyl, morpholinyl, piperidinyl, piperazinyl, hexahydropyrazinyl, hexahydropyrimidinyl, azepanyl, 1,4-diazacycloheptyl and piperazinyl; more preferably, piperidinyl, piperazinyl, pyrrolidinyl, morpholinyl, azetidinyl, dihydrotetrazolyl, pyrimidin-4(3H)-one, 1,2,4-oxadiazol-5(2H)-one or 5,6-dihydro-4H-cyclopenta[d]isoxazole. Polycyclic heterocyclyl includes spiro, fused and bridged heterocyclyl; the involved spiro, fused and bridged heterocyclyl are optionally bound to other groups through a single bond, or further fused to other cycloalkyl, heterocyclyl, aryl and heteroaryl through any two or more atoms on the ring.

Heterocyclyl may be optionally substituted or unsubstituted, and when substituted, the substituent is preferably one or more of the following groups, which are independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, oxo, carboxyl and carboxylate group.

The term "aryl" refers to a 6- to 14-membered all-carbon monocyclic or fused polycyclic (i.e., rings sharing adjacent pairs of carbon atoms) group having a conjugated π electron system, preferably 6- to 12-membered, such as phenyl and naphthyl, more preferably phenyl.

Aryl may be substituted or unsubstituted, and when substituted, the substituent is preferably one or more of the following groups, which are independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl and carboxylate group.

The term "heteroaryl" refers to a heteroaromatic system containing 1 to 4 heteroatoms and 5 to 14 ring atoms, where the heteroatoms are selected from oxygen, sulfur and nitrogen. The heteroaryl is preferably 5- to 12-membered, more preferably 5-membered or 6-membered, such as imidazolyl, furyl, thienyl, thiazolyl, pyrazolyl, oxazolyl, pyrrolyl, triazolyl, tetrazolyl , pyridyl, pyrimidinyl, thiadiazolyl, pyrazinyl, *etc.,* preferably pyridyl, pyrazinyl, oxadiazolyl, triazolyl, tetrazolyl, thienyl, imidazolyl, pyrazolyl, oxazolyl, thiazolyl, pyrimidinyl or thiazolyl; more preferred pyridyl, oxadiazolyl, pyrazolyl, pyrazinyl, isoxazolyl, triazolyl, tetrazolyl, pyrrolyl, thiazolyl and oxazolyl.

Heteroaryl may be optionally substituted or unsubstituted, and when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.

The term "alkoxy" refers to -O-(alkyl) and -O-(unsubstituted cycloalkyl), where alkyl is as defined above. Non-limiting examples of alkoxy include: methoxy, ethoxy, propoxy, butoxy, cyclopropoxy, cyclobutoxy, cyclopentyloxy or cyclohexyloxy; Alkoxy may be optionally substituted or unsubstituted, and when substituted, the substituent is preferably one or more of the following groups, which are independently selected from alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio, carboxyl or carboxylate group.
"Haloalkyl" refers to an alkyl substituted with one or more halogens, where alkyl is as defined above.
"Haloalkoxy" refers to an alkoxy substituted with one or more halogens, where alkoxy is as defined above.
"Hydroxyalkyl" refers to an alkyl substituted with one or more hydroxy, where alkyl is as defined above.
"Hydroxyl" refers to the -OH group.
"Halogen" refers to fluorine, chlorine, bromine or iodine.
"Amino" refers to -NH₂.
"Cyano" refers to -CN.
"Nitro" refers to -NO₂.
"Carbonyl" refers to -C(O)-.
"Carboxy" refers to -C(O)OH.
"THF" refers to tetrahydrofuran.
"Ethyl acetate" refers to ethyl acetate.
"MeOH" refers to methanol.
"DMF" refers to N,N-dimethylformamide.
"DIPEA" refers to diisopropylethylamine.
"TFA" refers to trifluoroacetic acid.
"TEA" refers to triethylamine.
"MeCN" refers to acetonitrile.
"DMA" refers to N,N-dimethylacetamide.
"Et₂O" refers to diethyl ether.
"DCM" refers to dichloromethane.
"DMAP" refers to 4-dimethylaminopyridine.
"DCC" refers to dicyclohexylcarbodiimide.
"DCE" refers to 1,2 dichloroethane.
"DIPEA" refers to N,N-diisopropylethylamine.
"NBS" refers to N-bromosuccinimide.
"NIS" refers to N-iodosuccinimide.
"Cbz-Cl" refers to benzyl chloroformate.
"Pd₂(dba)₃" refers to tris(dibenzylideneacetone)dipalladium. "Dppf' refers to 1,1'-bisdiphenylphosphine ferrocene.
"HATU" refers to 2-(7-azabenzotriazol-1-yl)-N,N,N',N'-tetramethyluronium hexafluorophosphate.
"KHMDS" refers to potassium bis(trimethylsilyl)amide.
"LiHMDS" refers to lithium hexamethyldisilazide.
"MeLi" refers to methyllithium.
"n-BuLi" refers to n-butyllithium.
"NaBH(OAc)₃" refers to sodium triacetoxyborohydride.
"SEM" refers to (trimethylsilyl)ethoxymethyl.
"MOM" refers to methyloxymethylether.
"OMs" refers to methylsulfonyloxy.

Different terms such as "X is selected from A, B, or C", "X is selected from A, B and C", "X is A, B or C", "X is A, B and C" all express the same meaning, *i.e.,* X can be any one or more of A, B, and C.

The hydrogen atoms described in the present invention can be replaced by its isotope deuterium, and any hydrogen atom in the example compounds involved in the present invention can also be replaced by deuterium atom.

"Optional" or "optionally" means that the event or circumstance subsequently described may but need not to occur, and the description includes the occasions where the events or circumstances occur or do not occur. For example, "heterocyclic group optionally substituted with alkyl" means the alkyl may but need not be present, the description includes the case where the heterocyclic group is substituted with alkyl and the case where the heterocyclic group is not substituted with alkyl.

"Substituted" refers to one or more hydrogen atoms in the group, preferably at most 5, more preferably 1-3 hydrogen atoms each independently substituted with a corresponding number of substituents. It goes without saying, the substituents may be only in their possible chemical positions, a person skilled in the art can determine the possible or impossible substitutions (by experiment or theory) without paying too much effort. For example, the amino group having a free hydrogen or a hydroxy group may be unstable when combined the carbon atoms having an unsaturated (e.g., olefinic) bond.

"Pharmaceutical composition" denotes a mixture containing one or more of the compounds as stated herein or physiologically/pharmaceutically acceptable salts or prodrug thereof and other chemical components, as well as other components, such as a physiologically/pharmaceutically acceptable carrier and an excipient. The purpose of pharmaceutical compositions is to facilitate administration to living organisms and facilitate the absorption of active ingredients to exert biological activity.

"Pharmaceutically acceptable salt" refers to a salt of the compound of the present invention, which are safe and effective when used in mammals, and have appropriate biological activity.

### DETAILED DESCRIPTION OF THE INVENTION

The present invention will be further described below with reference to examples, but these examples do not limit the scope of the present invention.

### Example

The structure of the compound of the present invention was determined by nuclear magnetic resonance (NMR) or/and liquid mass spectrometry (LC-MS). NMR chemical shift (δ) was given in parts per million (ppm) unit. NMR was determined using a Bruker AVANCE-400 nuclear magnetic instrument. The solvents for determination were deuterated dimethyl sulfoxide (DMSO-d6), deuterated methanol (CD₃OD) and deuterated chloroform (CDCl₃), and the internal standard was tetramethylsilane (TMS).

Agilent 1200 Infinity Series mass spectrometer was used for Liquid chromatography-mass spectrometry LC-MS determination. HPLC determination used Agilent 1200DAD high-pressure liquid chromatograph instrument (Sunfire C18 150 × 4.6 mm chromatographic column) and Waters 2695-2996 high-pressure liquid chromatograph instrument (Gimini C₁₈ 150 × 4.6 mm chromatographic column).

Yantai Huanghai HSGF254 or Qingdao GF254 silica gel plate was used as a thin layer chromatography silica plate, and the specification used for the TLC was 0.15 mm - 0.20 mm, and the specification when separating and purifying a product by thin layer chromatography is 0.4 mm - 0.5 mm. For the column chromatography, Yantai Huanghai silica gel of 200-300 mesh silica gel was generally used as a carrier.

The starting materials in the examples of the present invention were known and can be purchased on the market, or can be synthesized using or according to methods known in the art.

Unless otherwise specified, all reactions of the present invention were carried out under continuous magnetic stirring in a dry nitrogen or argon atmosphere, the solvent was a dry solvent, and the reaction temperature unit was degrees Celsius.

### Intermediate 1

### 2-bromo-N-(4-chloro-5-methylisoxazol-3-yl)-N-((2-(trimethylsilyl)ethoxy)methyl) benzsulfamide

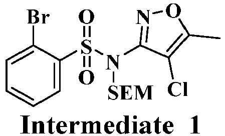

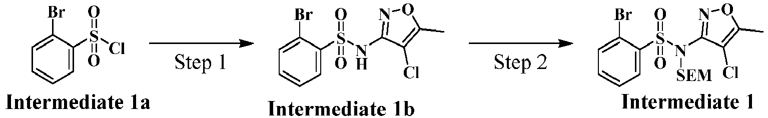

### Step 1

### Preparation of 2-bromo-N-(4-chloro-5-methylisoxazol-3-yl)benzsulfamide

4-chloro-5-methylisoxazole-3-amine (5.0 g, 37.8 mmol) was dissolved in tetrahydrofuran (50 mL), the reaction liquid was cooled to -78°C, and then potassium tert-butoxide (8.43 g, 75.3 mmol) was added to the reaction liquid, and the reaction liquid was reacted at -78°C for 0.5 h under stirring. Intermediate **1a** (10.0 g, 39.4 mmol) was added to the reaction liquid, and the reaction liquid was stirred at room temperature for 1 h. Water and dichloromethane (3 × 20 mL) were added for extraction. The organic layers were combined, dried over anhydrous sodium sulfate, filtered, and concentrated to afford the target product intermediate 1b (12.5 g, yield: 88.5%).

MS m/z (ESI): 351.2 [M+1]⁺.

### Step 2

### Preparation of 2-bromo-N-(4-chloro-5-methylisoxazol-3-yl)-N-((2-(trimethylsilyl) ethoxy)methyl)benzsulfamide

Intermediate **1b** (12.5 g, 35.7 mmol) and potassium carbonate (9.8 g, 71.4 mmol) were dissolved in N,N-dimethylformamide (20 mL), then 2-(trimethylsilyl)ethyloxymethyl chloride (8.9 g, 53.6 mmol) was added to the reaction liquid, and the reaction was stirred at room temperature for 16 h. Water and dichloromethane (3 × 20 mL) were added for extraction. The organic layers were combined, dried over anhydrous sodium sulfate, filtered, concentrated, and purified with column (petroleum ether/ethyl acetate system) to afford the target product intermediate **1** (17.2 g, yield: 98.5%).

### Example 1

### 4'-((2-butyl-4-oxo-1,3-diazospiro[4.4]non-1-en-3-yl)methyl-d2)-N-(4,5-dimethylisoxazol-3-yl)- 2'-(ethoxymethyl)-[1,1'-biphenyl] -2-sulfonamide

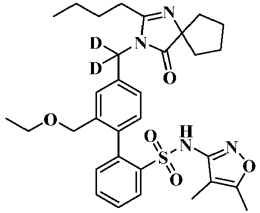

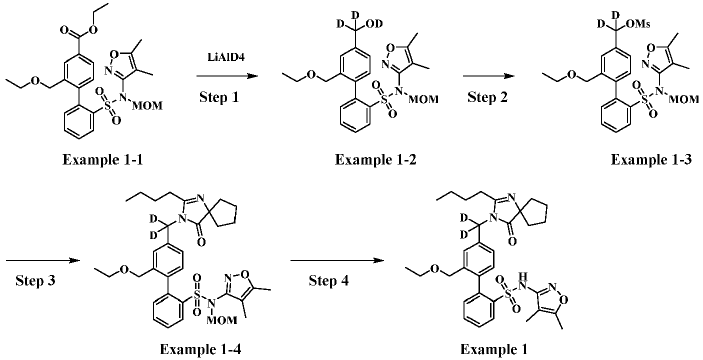

### Step 1

### Preparation of N-(4,5-dimethylisoxazol-3-yl)-2'-(ethoxymethyl)-4'-((hydroxy-d)-methyl-d2)-N-(methoxymethyl)-[1,1'-biphenyl]- 2-sulfonamide

Example **1-1** (80 mg, 0.17 mmol) (referring to WO 2010114801 A1 for the preparation method) and deuterated lithium aluminum tetrahydrogen (11 mg, 0.26 mmol) were dissolved in tetrahydrofuran (5 mL), and the reaction liquid was cooled to 0°C and reacted under stirring for 2 h. Saturated brine (10 mL) was added to the reaction liquid, and the mixture was extracted with ethyl acetate (2 × 10 mL). The organic phases were combined, dried over anhydrous sodium sulfate, concentrated, and purified with silica gel chromatography column (petroleum ether/ethyl acetate system) to afford Example **1-2** (50 mg, 70%).

MS m/z (ESI): 464.2 [M+1] ⁺.

### Step 2

### Preparation of (2'-(N-(4,5-dimethylisoxazol-3-yl)-N-(methoxymethyl)sulfamoyl)-2-(ethoxymethyl)-[1,1'-biphenyl]-4-yl)methyl-d2 methanesulfonic acid

Under ice bath conditions, methylsulfonyl chloride (14.8 mg, 0.13 mmol) and diisopropylethylamine (41.8 mg, 0.32 mmol) were added to a solution of Example **1-2** (50 mg, 0.11 mmol) in dichloromethane (4 mL), and the reaction liquid was warmed to room temperature and stirred for 1 h. The reaction liquid was concentrated to afford crude product Example **1-3** (60 mg, 98%), which was directly used in the next reaction.

MS m/z (ESI): 541.2 [M+1] ⁺.

### Step 3

### Preparation of 4'-((2-butyl-4-oxo-1,3-diazaspiro[4.4]non-1-en-3-yl)methyl-d2)-N-(4,5-dimethylisoxazol-3-yl)- 2'-(ethoxymethyl)-N-(methoxymethyl)-[1,1'-biphenyl] -2-sulfonamide

Example **1-3** (60 mg, 0.11 mmol) was dissolved in DMF (4 mL), potassium carbonate (30.7 mg, 0.24 mmol) and 2-butyl-1,3-diazaspiro-[4,4]non-1-en-4one (25.8 mg, 0.13 mmol) were added under ice bath conditions, and the reaction liquid was stirred at room temperature for 2 h. The reaction liquid was concentrated, and the crude product was purified by HPLC to afford Example **1-4** (42 mg, 72%).

MS m/z (ESI): 639.3 [M+1] ⁺.

### Step 4

### Preparation of 4'-((2-butyl-4-oxo-1,3-diazaspiro[4.4]non-1-en-3-yl)methyl-d2)-N-(4,5-dimethylisoxazol-3-yl)- 2'-(ethoxymethyl)-[1,1'-biphenyl] -2-sulfonamide

Example **1-4** (42 mg, 0.07 mmol) was dissolved in ethanol (2 mL), 6N hydrochloric acid was added, the mixture was heated to reflux for 1 h, the pH was adjusted to 8 with sodium carbonate, and then the pH was adjusted to 5. The mixture was extracted with ethyl acetate (2 × 10 mL). The organic phases were combined, dried over anhydrous sodium sulfate, concentrated, and purified by reverse HPLC to afford Example **1** (10 mg, 26%).

MS m/z (ESI): 595.3 [M+1] ⁺.

### Example 2

### 4'-((((2-butyl-1,3-diazaspiro[4,4]non-1,3-dien-4-yl)oxy]methyl)-N-(4,5-dimethylisoxazol-3-yl)-2'-(ethoxymethyl)-[1,1'-biphenyl]-2-sulfonamide

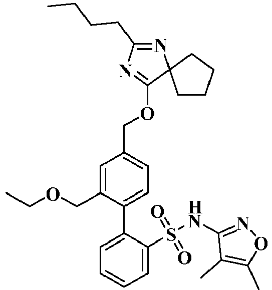

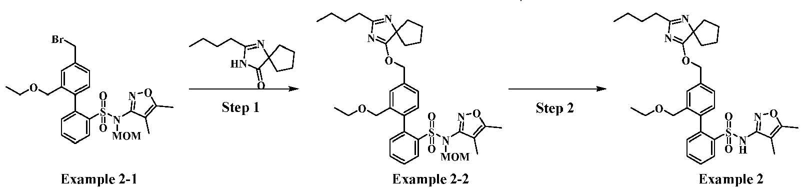

### Step 1

### Preparation of 4'-((((2-butyl-1,3-diazaspiro[4.4]non-1,3-dien-4-yl]oxy)methyl)-N-(4,5-dimethylisoxazol-3-yl)-2'-(ethoxymethyl)-N-(methoxymethyl)-[1,1'-biphenyl] -2-sulfonamide

Example **2-1** (100 mg, 0.19 mmol) (referring to WO 2010114801 A1 for the preparation method ) was dissolved in chloroform (4 mL), silver oxide (47.3 mg, 0.38 mmol) and 2-butyl -1,3-diazaspiro-[4,4]non-1-en-4one (44.5 mg, 0.23 mmol) were added, and the mixture was heated to reflux for 12 h. The reaction liquid was concentrated, and the crude product was subjected to reverse phase HPLC to afford Example 2-2 (56 mg, 46%).

MS m/z (ESI): 637.3 [M+1] ⁺.

### Step 2

### Preparation of 4'-((((2-butyl-1,3-diazaspiro[4.4]non-1,3-dien-4-yl]oxy)methyl)-N-(4,5-dimethylisoxazol-3-yl)-2 '-(ethoxymethyl)-[1,1'-biphenyl] -2-sulfonamide

Synthesis method of Example **2** referred to the synthesis method of Example **1**, Example **2-1** was used as raw material to afford Example **2** (30 mg, 57%).

MS m/z (ESI): 593.3 [M+1] ⁺.

### Example 4

### 1-((2'-(N-(4,5-dimethylisoxazol-3-yl)sulfamoyl)-2-(ethoxymethyl)-[1,1'-biphenyl]-4-yl)methyl)-4-(2 -hydroxypropan-2-yl)-2-propyl-1H-imidazole-5-carboxylic acid

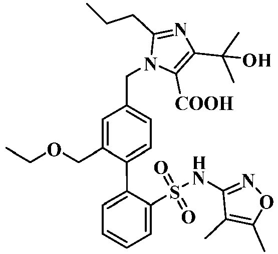

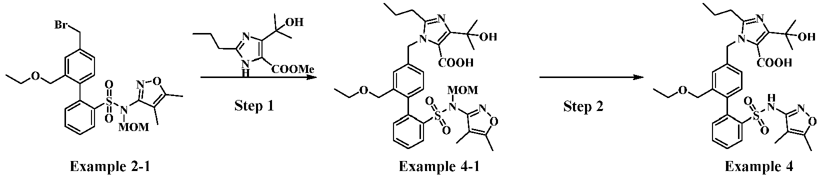

### Step 1

### Preparation of methyl 1-((2'-(N-(4,5-dimethylisoxazol-3-yl)-N-(methoxymethyl)sulfamoyl)-2-(ethoxymethyl)-[1,1'-biphenyl] -4-yl)methyl)-4-(2-hydroxyprop-2-yl)-2-propyl-1H-imidazole-5-carboxylate

Example **2-1** (100 mg, 0.19 mmol) (referring to WO 2010114801 A1 for the preparation method) was dissolved in acetonitrile (4 mL), methyl 4-(2-hydroxyprop-2-yl)-2-propyl-1H-imidazole-5-carboxylate (51.9 mg, 0.23 mmol) and potassium carbonate (52.8 mg, 0.38 mmol) were added, the reaction liquid was heated to reflux for 6 h. The reaction liquid was concentrated, and the crude product was subjected to reverse phase HPLC to afford Example **4-1** (86 mg, 67%).

MS m/z (ESI): 655.3 [M+1] ⁺.

### Step 2

### Preparation of 1-((2'-(N-(4,5-dimethylisoxazol-3-yl)sulfamoyl)-2-(ethoxymethyl)-[1,1'-biphenyl] -4-yl)methyl)-4-(2-hydroxypropan-2-yl)-2-propyl-1H-imidazole-5-carboxylic acid

Synthesis method of Example **4** referred to the synthesis method of Example **1**, Example 4-**1** was used as raw material to afford Example 4 (31 mg, 40%).

MS m/z (ESI): 611.2 [M+1] ⁺.

### Example 5

### 2-(2-bulyl-1-((2'-(N-(4,5-dimethylisoxazol-3-yl)sulfamoyl)-2-(ethoxymethyl)-[1,1'-biphenyl]-4-yl)methyl)-4-methyl-6-oxo-1,6-dihydropyrimidin-5-yl)-N,N-dimethylethylthioamide

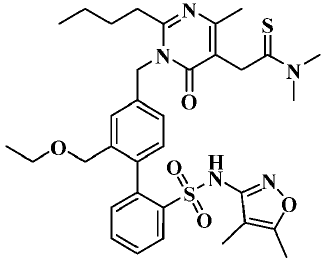

Synthesis method of Example **5** referred to the synthesis method of Example **4.** 2-(2-butyl-4-methyl-6-oxo-1,6-dihydropyrimidin-5-yl)-N,N-dimethylethylthioamide was used instead of methyl 4-(2-hydroxyprop-2-yl)-2-propyl-1H-imidazole-5-carboxylate to afford Example 5 (21 mg, 56%).

MS m/z (ESI): 666.3 [M+1] ⁺.

### Example 6

### 2-(4-((2-butyl-4-oxo-1,3-diazaspiro[4.4]non-1-en-3-yl)methyl)-2-(ethoxymethyl)phenyl)-N-(4,5-dimethylisoxazol-3-yl)pyridine-3-sulfanilamide

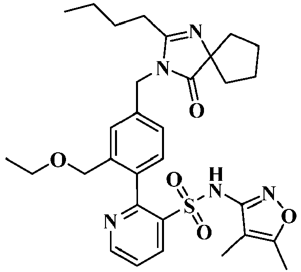

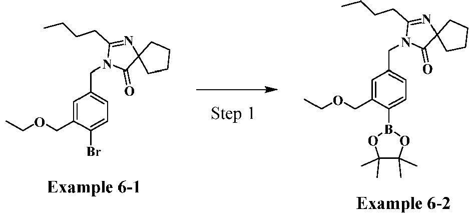

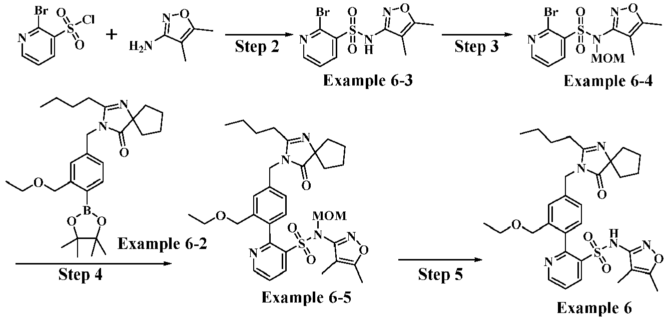

### Step 1

### Preparation of 3-(4-borate-3-(ethoxymethyl)benzyl)-2-butyl-1,3-diazaspiro[4.4]non-1-en-4-one

Potassium acetate (3.4 g, 35 mmol) and Pd(dppf)₂Cl₂ (0.95 g, 1.1 mmol) were added to a solution of Example **6-1** (4.8 g, 11.6 mmol) (referring to WO 2010135350 A2 for the synthesis method) and bis(pinacol)diboron (4.4 g, 17.5 mmol) in dioxane (100 mL). The mixture was subjected to nitrogen replacement and heated at 85°C overnight. The reaction mixture was concentrated under reduced pressure to afford a crude product, which was purified by column chromatography (petroleum ether/ethyl acetate, 15% v/v) to afford Example **6-2** (4.3 g, 80%).

MS m/z (ESI): 469.3 [M+1] ⁺.

### Step 2

### Preparation of 2-bromo-N-(4,5-dimethylisoxazol-3-yl)pyridine-3-sulfanilamide

3-amino-4,5-dimethylisoxazole (264 mg, 2.35 mmol) was dissolved in 10 mL of dichloromethane, triethylamine (594 mg, 5.88 mmol) and 2-bromopyridine-3-sulfonyl chloride (500 mg, 1.96 mmol) were added, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, the filtrate was concentrated under reduced pressure, and the residue was purified with silica gel column chromatography (petroleum ether/ethyl acetate system) to afford Example **6-3** (350 mg, 53.8%).

MS m/z (ESI): 332.0 [M+1] ⁺.

### Step 3

### Preparation of 2-bromo-N-(4,5-dimethylisoxazol-3-yl)-N-(methoxymethyl)pyridine-3-sulfanilamide

Example **6-3** (350 mg, 1.05 mmol) was dissolved in dichloromethane (10 mL), triethylamine (319 mg, 3.16 mmol), 4-dimethylaminopyridine (129 mg, 1.05 mmol) and bromomethyl methyl ether (158 mg, 1.26 mmol) were added in sequence, and the mixture was stirred at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **6-4** (280 mg, 71.1%).

MS m/z (ESI): 376.0 [M+1] ⁺.

### Step 4

### Preparation of 2-(4-((2-butyl-4-oxo-1,3-diazaspiro[4.4]non-1-en-3-yl)methyl)-2-(ethoxymethyl)phenyl)-N-(4,5-dimethylisoxazol-3-yl)-N-(methoxymethyl)pyridine-3-sulfanilamide

Example **6-4** (50 mg, 0.133 mmol) was dissolved in 1,4-dioxane (2 mL) and water (0.5 mL), and Example **6-2** (63 mg, 0.133 mmol), [1,1'-bis(diphenylphosphino)ferrocene]palladium dichloride (10 mg, 0.0133 mmol), and cesium carbonate (65 mg, 0.200 mmol) were added, nitrogen replacement was performed three times, and the mixture was reacted under microwave at 100°C for 1 h. The reaction liquid was cooled to room temperature, 30 mL of water was added, and the mixture was extracted with ethyl acetate (30 mL × 2). The organic phases were combined, washed with water (30 mL) and saturated sodium chloride solution (30 mL) in sequence, dried over anhydrous sodium sulfate and filtered, and the filtrate was concentrated under reduced pressure to afford the title product crude Example **6-5** (75 mg), which was used directly in the next reaction.

MS m/z (ESI): 638.3 [M+1] ⁺.

### Step 5

### Preparation of 2-(4-((2-butyl-4-oxo-1,3-diazaspiro[4.4]non-1-en-3-yl)methyl)-2-(ethoxymethyl)phenyl)-N-(4,5-dimethylisoxazol-3-yl)pyridine-3-sulfanilamide

Example 6-5 (75 mg, 0.118 mmol) was dissolved in ethanol (3 mL), hydrochloric acid (6 M, 1 mL) was added, and the mixture was reacted at 80°C for 3 h. The reaction liquid was cooled to room temperature, concentrated under reduced pressure, and the residue was prepared with reverse HPLC to afford the title product 2-(4-((2-butyl-4-oxo-1,3-diazaspiro[4.4]non-1-en-3-yl)methyl)-2-(ethoxymethyl) phenyl)-N-(4,5-dimethylisoxazol-3-yl)pyridine-3-sulfanilamide 6 (30 mg, 42.8%).

MS m/z (ESI): 594.3 [M+1] ⁺.

### Example 7

### 2-(6-((2-butyl-4-oxo-1,3-diazaspiro[4.4]non-1-en-3-yl)methyl)-2-(ethoxymethyl)pyridin-3-yl)-N-(4,5-dimethylisoxazol-3-yl)benzsulfamide

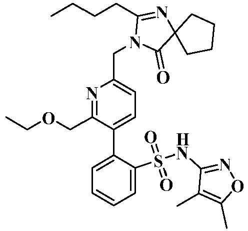

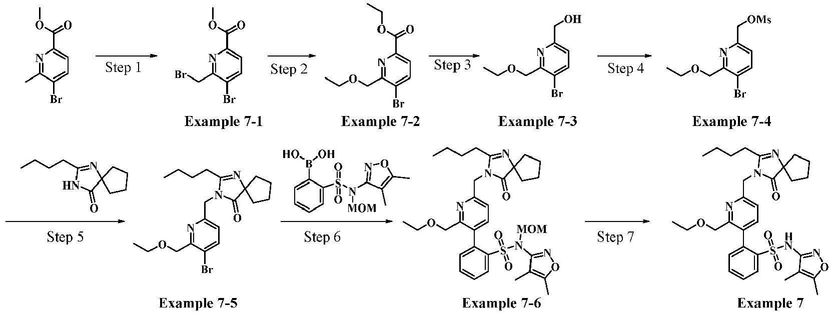

### Step 1

### Preparation of methyl 5-bromo-6-(bromomethyl)picolinate

Methyl 5-Bromo-6-methylpicolinate (1.0 g, 4.35 mmol) was dissolved in carbon tetrachloride (30 mL), N-bromosuccinimide (851 mg, 4.78 mmol) and azobisisobutyronitrile (71 mg, 0.435 mmol) were added, nitrogen replacement was performed three times, and the mixture was reacted at reflux for 5 h. The reaction liquid was cooled to room temperature, concentrated under reduced pressure, and 50 mL of water was added. Ethyl acetate (50 mL×2) was used for extraction. The organic phases were combined, washed with water (50 mL) and saturated sodium chloride solution (50 mL) in sequence, dried over anhydrous sodium sulfate and filtered, and the filtrate was concentrated under reduced pressure, and the residue was purified with silica gel column chromatography to afford Example **7-1** (600 mg, 44.9%).

MS m/z (ESI): 308.9 [M+1] ⁺.

### Step 2

### Preparation of ethyl 5-bromo-6-(ethoxymethyl)picolinate

Example **7-1** (600 mg, 1.95 mmol) was dissolved in ethanol (20 mL), sodium ethoxide (399 mg, 5.86 mmol) was added, and the mixture was heated to reflux for 2 h. The reaction liquid was cooled to room temperature, concentrated under reduced pressure, 50 mL of water was added, and the mixture was extracted with ethyl acetate (50 mL × 2). The organic phases were combined, washed with water (50 mL) and saturated sodium chloride solution (50 mL) in sequence, dried over anhydrous sodium sulfate and filtered, and the filtrate was concentrated under reduced pressure, and the residue was subjected to silica gel column chromatography to afford Example **7-2** (520 mg, 92.7%).

MS m/z (ESI): 288.0 [M+1] ⁺.

### Step 3

### Preparation of (5-bromo-6-(ethoxymethyl)pyridin-2-yl)methanol

Example **7-2** (520 mg, 1.80 mmol) was dissolved in tetrahydrofuran (15 mL), nitrogen replacement was performed three times, the mixture was cooled to -78°C, and a solution of diisobutylaluminum hydride in toluene (1.5 M, 3.6 mL, 5.42 mmol) was added dropwise, then the mixture was warmed to room temperature and reacted for 5 h. The reaction liquid was poured into 100 mL of ice water, and the mixture was extracted with ethyl acetate (80 mL × 2). The organic phases were combined, washed with water (80 mL) and saturated sodium chloride solution (80 mL) in sequence, dried over anhydrous sodium sulfate and filtered, and the filtrate was concentrated under reduced pressure, and the residue was purified with silica gel column chromatography to afford Example 7-3 (260 mg, 58.7%).

MS m/z (ESI): 246.0 [M+1] ⁺.

### Step 4

### Preparation of methyl (5-bromo-6-(ethoxymethyl)pyridin-2-yl)methylsulfonate

Example **7-3** (260 mg, 1.06 mmol) was dissolved in dichloromethane (10 mL), and triethylamine (320 mg, 3.17 mmol) and methylsulfonyl chloride (242 mg, 2.11 mmol) were added, and the mixture was reacted at room temperature for 2 h. The reaction liquid was poured into 50 mL of ice water, and the mixture was extracted with ethyl acetate (50 mL × 2). The organic phases were combined, washed with water (50 mL) and saturated sodium chloride solution (50 mL) in sequence, dried over anhydrous sodium sulfate and filtered, and the filtrate was concentrated under reduced pressure to afford the title product crude Example **7-4** (330 mg), which was used directly in the next reaction.

MS m/z (ESI): 324.0 [M+1] ⁺.

### Step 5

### Preparation of 3-((5-bromo-6-(ethoxymethyl)pyridin-2-yl)methyl)-2-butyl-1,3-diazaspiro[4.4]non-1-en-4-one

Example **7-4** (330 mg, 1.02 mmol) was dissolved in N,N-dimethylformamide (10 mL), and 2-butyl-1,3-diazaspiro-[4,4]non-1-en-4one (237 mg, 1.22 mmol) and potassium carbonate (422 mg, 3.05 mmol) were added, and the mixture was heated to 80°C and reacted for 5 h. The reaction liquid was cooled to room temperature, 50 mL of water was added, and the mixture was extracted with ethyl acetate (50 mL × 2). The organic phases were combined, washed with water (50 mL) and saturated sodium chloride solution (50 mL) in sequence, dried over anhydrous sodium sulfate and filtered, and the filtrate was concentrated under reduced pressure, and the residue was purified with silica gel column chromatography to afford Example **7-5** (180 mg, 41.9%).

MS m/z (ESI): 422.1 [M+1] ⁺.

### Step 6

### Preparation of 2-(6-((2-butyl-4-oxo-1,3-diazaspiro[4.4]non-1-en-3-yl)methyl)-2-(ethoxymethyl)pyridin-3-yl)-N-(4,5-dimethylisoxazol-3-yl)-N-(methoxymethyl)benzsulfamide

Example **7-5** (50 mg, 0.118 mmol) was dissolved in 1,4-dioxane (2 mL) and 0.5 mL of water, (2-(N-(4,5-dimethylisoxazol-3-yl)-N-(methoxymethyl)sulfamoyl) phenyl)boronic acid (48 mg, 0.142 mmol) (referring to WO 2010135350 A2 for the preparation method), [1,1'-bis(diphenylphosphino)ferrocene]palladium dichloride(8.6 mg, 0.0118 mmol) and cesium carbonate (58 mg, 0.177 mmol) were added, nitrogen replacement was performed three times, and the mixture was reacted under microwave at 100°C for 1 h. The reaction liquid was cooled to room temperature, 30 mL of water was added, and the mixture was extracted with ethyl acetate (30 mL × 2). The organic phases were combined, washed with water (30 mL) and saturated sodium chloride solution (30 mL) in sequence, dried over anhydrous sodium sulfate and filtered, and the filtrate was concentrated under reduced pressure, and the residue was purified with silica gel column chromatography to afford Example **7-6** (35 mg), which was used directly in the next reaction.

MS m/z (ESI): 638.3 [M+1] ⁺.

### Step 7

### Preparation of 2-(6-((2-butyl-4-oxo-1,3-diazaspiro[4.4]non-1-en-3-yl)methyl)-2-(ethoxymethyl)pyridin-3-yl)-N-(4,5-dimethylisoxazol-3-yl)benzsulfamide

Referring to the synthesis method of Example **6**, Example **7-6** was subjected to removal of protecting group to afford Example **7** (19 mg, 58.3%).

MS m/z (ESI): 594.3 [M+1] ⁺.

### Example 8

### 2-(6-((2-bulyl-4-oxo-1,3-diazaspiro[4.4]non-1-en-3-yl)methyl)-4-(ethoxymethyl)pyridin-3-yl)-N-(4,5-dimethylisoxazol-3-yl)benzsulfamide

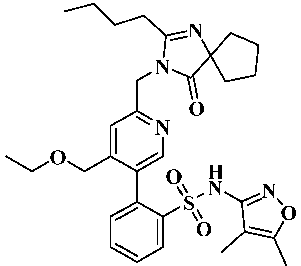

Referring to the route and method of Example **7,** ethyl 5-bromo-4-methylpicolinate was used instead of methyl 5-bromo-6-methylpicolinate as the starting material to afford Example **8** (11 mg, 39.4%).

MS m/z (ESI): 594.3 [M+1] ⁺.

### Example 9

### 2-(4-((2-butyl-4-oxo-1,3-diazaspiro[4.4]non-1-en-3-yl)methyl)-2-(ethoxymethyl)phenyl)-N-(3-methoxy-5-methylpyrazin-2-yl)pyridine-3-sulfonamide

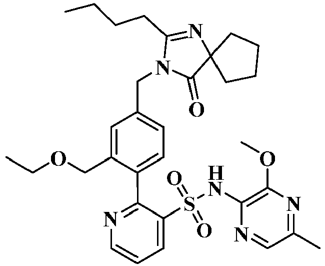

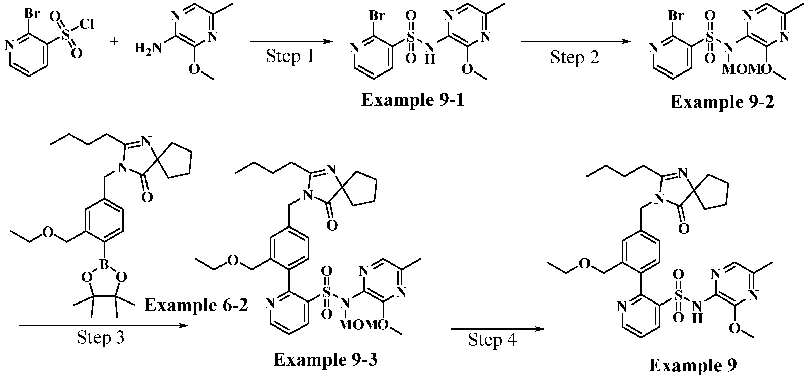

### Step 1

### Preparation of 2-bromo-N-(3-methoxy-5-methylpyrazin-2-yl)pyridine-3-sulfanilamide

3-methoxy-5-methylpyrazin-2-amine (130 mg, 0.938 mmol) was dissolved in dichloromethane (5 mL), triethylamine (237 mg, 2.34 mmol) and 2-bromopyridine-3-sulfonyl chloride (200 mg, 0.781 mmol) were added, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **9-1** (110 mg, 32.7%).

MS m/z (ESI): 359.0 [M+1] ⁺.

### Step 2

### Preparation of 2-bromo-N-(3-methoxy-5-methylpyrazin-2-yl)-N-(methoxymethyl) pyridine-3-sulfanilamide

Example **9-1** (110 mg, 0.306 mmol) was dissolved in dichloromethane (5 mL), triethylamine (93 mg, 0.919 mmol), 4-dimethylaminopyridine (37 mg, 0.306 mmol) and bromomethyl methyl ether (46 mg, 0.368 mmol) were added in sequence, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **9-2** (80 mg, 64.9%).

MS m/z (ESI): 403.0 [M+1] ⁺.

### Step 3

### Preparation of 2-(4-((2-butyl-4-oxo-1,3-diazaspiro[4.4]non-1-en-3-yl)methyl)-2-(ethoxymethyl)phenyl)-N-(3-methoxy-5-methylpyrazin-2-yl-N-(methoxymethyl) pyridine-3-sulfonamide

Using Example **9-2** as raw material and referring to the route and method of Example **6-3**, the Example **9-3** (81 mg, 61%) was obtained.

MS m/z (ESI): 665.3 [M+1] ⁺.

### Step 4

### Preparation of 2-(4-((2-butyl-4-oxo-1,3-diazaspiro[4.4]non-1-en-3-yl)methyl)-2-(ethoxymethyl)phenyl)-N-(3-methoxy-5-methylpyrazin-2-yl)pyridine-3-sulfonamide

Using Example **9-3** as raw material and referring to the route and method of Example **6**, the Example **9** (30 mg, 40%) was obtained.

MS m/z (ESI): 620.3 [M+1] ⁺.

### Example 10

### 4'-((2-butyl-4-oxo-1,3-diazaspiro[4.4]non-1-en-3-yl)methyl)-N-(4,5-dimethylisoxazol-3-yl)-2'-(ethoxymethyl)-6-fluoro-[1,1'-biphenyl]-2-sulfanilamide

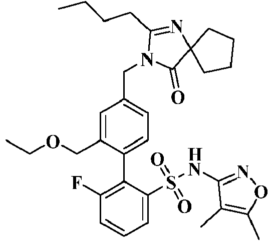

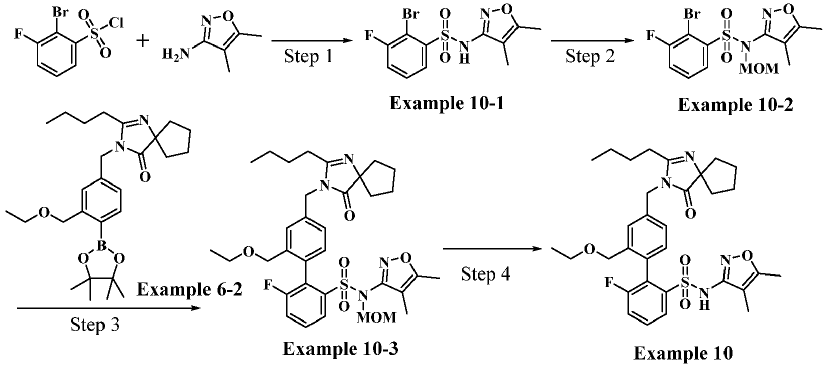

### Step 1

### Preparation of 2-bromo-N-(4,5-dimethylisoxazol-3-yl)-3-fluorobenzsulfamide

3-amino-4,5-dimethylisoxazole (123 mg, 1.10 mmol) was dissolved in dichloromethane (10 mL), triethylamine (222 mg, 2.19 mmol) and 2-bromo-3-fluorobenzenesulfonyl chloride (200 mg, 0.731 mmol) were added, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **10-1** (120 mg, 31.3%).

MS m/z (ESI): 349.0 [M+1] ⁺.

### Step 2

### Preparation of 2-bromo-N-(4,5-dimethylisoxazol-3-yl)-3-fluoro-N-(methoxymethyl)benzsulfamide

Example **10-1** (120 mg, 0.344 mmol) was dissolved in dichloromethane (10 mL), triethylamine (104 mg, 1.03 mmol), 4-dimethylaminopyridine (42 mg, 0.344 mmol) and bromomethyl methyl ether (86 mg, 0.688 mmol) were added in sequence, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **10-2** (90 mg, 66.6%).

MS m/z (ESI): 393.0 [M+1] ⁺.

### Step 3

### Preparation of 4'-(((2-butyl-4-oxo-1,3-diazaspiro[4.4]non-1-en-3-yl)methyl)-N-(4,5-dimethylisoxazol-3-yl)-2' -(ethoxymethyl)-6-fluoro-N-(methoxymethyl)-[1,1'-biphenyl] -2-sulfonamide

Using Example **10-2** as raw material and referring to the route and method of Example **6-3**, the Example **10-3** (51 mg, 56%) was obtained.

MS m/z (ESI): 655.3 [M+1] ⁺.

### Step 4

### Preparation of 4'-((2-butyl-4-oxo-1,3-diazaspiro[4.4]non-1-en-3-yl)methyl)-N-(4,5-dimethylisoxazol-3-yl)-2'-(ethoxymethyl)-6-fluoro-[1,1'-biphenyl]-2-sulfanilamide

Using Example **10-3** as raw material and referring to the routes and methods of the step 3 and step 4 of Example **6**, the Example 10 (18 mg, 55.2%) was obtained.

MS m/z (ESI): 611.3 [M+1] ⁺.

### Example 11

### 4'-((2-bulyl-4-oxo-1,3-diazaspiro[4.4]non-1-en-3-yl)methyl)-N-(4,5-dimethylisoxazol-3-yl)-6-(ethoxymethyl)-[1,1'-biphenyl]-2-sulfonamide

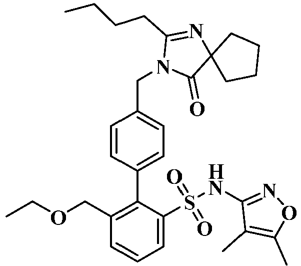

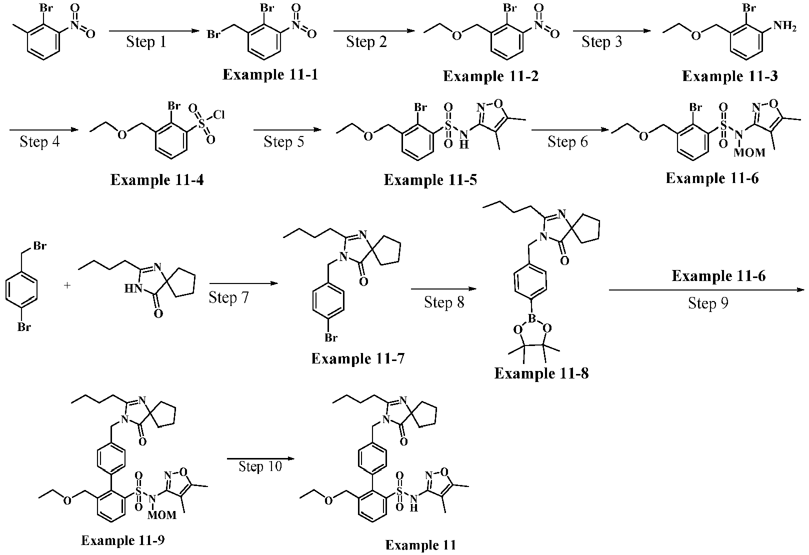

### Step 1

### Preparation of 2-bromo-1-(bromomethyl)-3-nitrobenzene

2-bromo-3-nitrotoluene (1.0 g, 4.63 mmol) was dissolved in carbon tetrachloride (30 mL), N-bromosuccinimide (988 mg, 5.56 mmol) and azobisisobutyronitrile (76 mg, 0.463 mmol) were added, nitrogen replacement was performed three times, and the mixture was reacted at reflux for 5 h. The reaction liquid was cooled to room temperature, concentrated under reduced pressure, and 50 mL of water was added. Ethyl acetate (50 mL×2) was used for extraction. The organic phases were combined, washed with water (50 mL) and saturated sodium chloride solution (50 mL) in sequence, dried over anhydrous sodium sulfate and filtered, and the filtrate was concentrated under reduced pressure, and the residue was purified with silica gel column chromatography to afford Example **11-1** (630 mg, 46.4%).

MS m/z (ESI): 293.9 [M+1] ⁺.

### Step 2

### Preparation of 2-bromo-1-(ethoxymethyl)-3-nitrobenzene

Example **11-1** (630 mg, 2.15 mmol) was dissolved in ethanol (20 mL), sodium ethoxide (419 mg, 6.45 mmol) was added, and the mixture was reacted at 40°C for 2 h. The reaction liquid was cooled to room temperature, concentrated under reduced pressure, 50 mL of water was added, and the mixture was extracted with ethyl acetate (50 mL × 2). The organic phases were combined, washed with water (50 mL) and saturated sodium chloride solution (50 mL) in sequence, dried over anhydrous sodium sulfate and filtered, and the filtrate was concentrated under reduced pressure to afford the crude Example **11-2** (410 mg), which was used directly in the next reaction.

MS m/z (ESI): 260.0 [M+1] ⁺.

### Step 3

### Preparation of 2-bromo-3-(ethoxymethyl)aniline

20 mL of acetic acid was heated to 80°C, iron powder (880 mg, 15.8 mmol) was added, Example **11-2** (410 mg, 1.58 mmol) was slowly added in batches, and the reaction was continued at 80°C for 30 min. The reaction liquid was cooled to room temperature and filtered, the filtrate was concentrated under reduced pressure, 50 mL of water was added, and the mixture was extracted with ethyl acetate (50 mL × 2). The organic phases were combined, washed with saturated sodium carbonate solution (50 mL) and saturated sodium chloride solution (50 mL) in sequence, dried over anhydrous sodium sulfate and filtered, and the filtrate was concentrated under reduced pressure to afford Example **11-3** (300 mg), which was used directly in the next reaction.

MS m/z (ESI): 230.0 [M+1] ⁺.

### Step 4

### Preparation of 2-bromo-3-(ethoxymethyl)benzenesulfonyl chloride

Example **11-3** (300 mg, 1.30 mmol) was dissolved in hydrochloric acid solution (6 M, 5 mL), the mixture was cooled to 0°C, and 1 mL of sodium nitrite (108 mg, 1.57 mmol) solution was slowly added dropwise, the reaction was continued at 0°C for 1 h. 3 mL of acetic acid, copper chloride (6.4 mg, 0.0650 mmol), copper chloride dihydrate (22 mg, 0.130 mmol) were added in sequence, and then thionyl chloride (774 mg, 6.50 mmol) was slowly added dropwise. The mixed solution was continued to react at 0°C for 1 h. 50 mL of water was added and the mixture was extracted with ethyl acetate (40 mL × 2). The organic phases were combined, washed with saturated sodium chloride solution (40 mL), dried over anhydrous sodium sulfate and filtered, and the filtrate was concentrated under reduced pressure to afford the crude Example **11-4** (330 mg), which was used directly in the next reaction.

MS m/z (ESI): 312.9 [M+1] ⁺.

### Step 5

### Preparation of 2-bromo-N-(4,5-dimethylisoxazol-3-yl)-3-(ethoxymethyl) benzsulfamide

3-amino-4,5-dimethylisoxazole (177 mg, 1.58 mmol) was dissolved in 5 mL of dichloromethane, triethylamine (319 mg, 3.16 mmol) and Example **11- 4** (330 mg, 1.05 mmol) were added, and then the mixture was reacted at room temperature for 2 h. 40 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **11-5** (350 mg).

MS m/z (ESI): 389.0 [M+1] ⁺.

### Step 6

### Preparation of 2-bromo-N-(4,5-dimethylisoxazol-3-yl)-3-(ethoxymethyl) benzsulfamide

Example **11-5** (350 mg, 0.900 mmol) was dissolved in dichloromethane (10 mL), triethylamine (273 mg, 2.70 mmol), 4-dimethylaminopyridine (110 mg, 0.900 mmol) and bromomethyl methyl ether (169 mg, 1.35 mmol) were added in sequence, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **11-6** (130 mg, 33.4%).

MS m/z (ESI): 433.0 [M+1] ⁺.

### Step 7

### Preparation of 3-(4-bromobenzoyl)-2-butyl-1,3-diazaspiro[4.4]non-1 -en-4-one

p-Bromobenzyl bromide (200 mg, 0.800 mmol) was dissolved in N,N-dimethylformamide (5 mL), potassium carbonate (221 mg, 1.60 mmol) and 2-butyl-1,3-diazaspiro-[4,4]non-1-en-4one (155 mg, 0.800 mmol) were added, and the mixture was heated to 80°C and reacted for 4 h. The reaction liquid was cooled to room temperature, 50 mL of water was added, and the mixture was extracted with ethyl acetate (40 mL × 2). The organic phases were combined, washed with water (40 mL × 2) and saturated sodium chloride solution (40 mL) in sequence, dried over anhydrous sodium sulfate and filtered, and the filtrate was concentrated under reduced pressure, and purified with silica gel column chromatography to afford Example **11-7** (130 mg, 44.8%).

MS m/z (ESI): 363.1 [M+1] ⁺.

### Step 8

### Preparation of 2-butyl-3-(4-(4,4,5,5-tetramethyl-1,3,2-dioxanaphth-2-yl)benzyl)-1,3-diazaspiro[4.4]non-1-en-4-one

Example **11-7** (130 mg, 0.358 mmol) was dissolved in 1,4-dioxane (5 mL), bis(pinacolato)diboron (182 mg, 0.716 mmol), [1,1'-bis(diphenylphosphino) ferrocene]palladium dichloride (26 mg, 0.0358 mmol) and potassium acetate (70 mg, 0.358 mmol) were added, nitrogen replacement was performed three times, and the mixture was heated to 100°C and reacted for 4 h. The reaction liquid was cooled to room temperature, 40 mL of water was added, and the mixture was extracted with ethyl acetate (40 mL × 2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford Example **11-8** (90 mg, 61.1%).

MS m/z (ESI): 411.3 [M+1] ⁺.

### Step 9

### Preparation of 4'-((2-butyl-4-oxo-1,3-diazaspiro[4.4]non-1-en-3-yl)methyl)-N-(4,5-dimethylisoxazol-3-yl)-6-(ethoxymethyl)-N-(methoxymethyl)-[1,1'-biphenyl]-2-sulfanilamide

Example **11-7** (50 mg, 0.122 mmol) was dissolved in 1,4-dioxane (2 mL) and water (0.5 mL), and Example **11-8** (53 mg, 0.122 mmol), [1,1'-bis(diphenylphosphino)ferrocene]palladium dichloride (8.9 mg, 0.0122 mmol), and cesium carbonate (79 mg, 0.243 mmol) were added, nitrogen replacement was performed three times, and the mixture was reacted under the condition of microwave at 100°C for 1 h. The reaction liquid was cooled to room temperature, 30 mL of water was added, and the mixture was extracted with ethyl acetate (30 mL × 2). The organic phases were combined, washed with water (30 mL) and saturated sodium chloride solution (30 mL) in sequence, dried over anhydrous sodium sulfate and filtered, and the filtrate was concentrated under reduced pressure, and the residue was purified with silica gel column chromatography to afford Example **11-9** (40 mg, 51.5%).

MS m/z (ESI): 637.3 [M+1] ⁺.

### Step 10

### Preparation of 4'-((2-butyl-4-oxo-1,3-diazaspiro[4.4]non-1-en-3-yl)methyl)-N-(4,5-dimethylisoxazol-3-yl)-6-(ethoxymethyl)-[1,1'-biphenyl]-2-sulfonamide

Example **11-9** (40 mg, 0.0628 mmol) was dissolved in 3 mL of ethanol, hydrochloric acid (6 M, 1 mL) was added, and the mixture was heated to 80°C and reacted for 3 h. The reaction liquid was cooled to room temperature, concentrated under reduced pressure, and the residue was subjected to reverse HPLC to afford Example **11** (25 mg, 67.1%).

MS m/z (ESI): 593.3 [M+1] ⁺.

### Intermediate 2

### 3-(3-(bromomethyl)-4-(4,4,5,5-tetramethyl-1,3,2-dioxaborolan-2-yl)benzyl)-2-butyl-1,3-diazaspiro[4.4]non-1-en-4-one

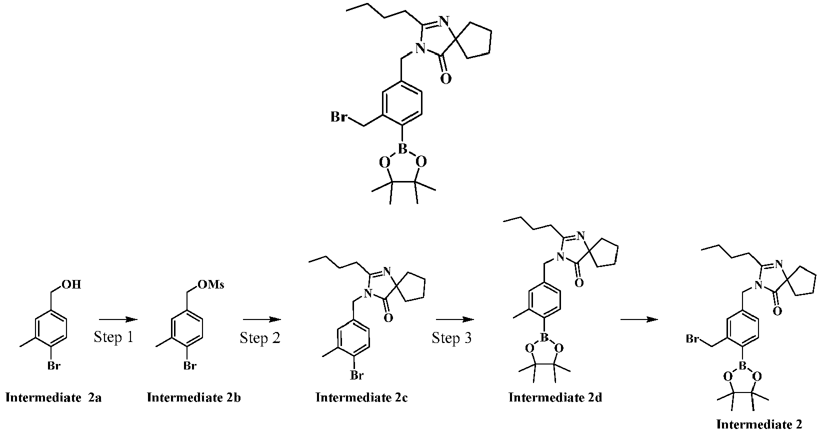

### Step 1

### Preparation of 4-bromo-3-methylbenzylmethanesulfonate

Intermediate 2a (2.0 g, 10.0 mmol) and triethylamine (1.4 g, 11.0 mmol) were dissolved in dichloromethane (5 mL), methylsulfonyl chloride (1.24 g, 11.0 mmol) was added to the reaction liquid under ice bath, and the mixture was reacted at room temperature for 1 h under stirring. Water and dichloromethane (20 mL × 3) were added for extraction. The organic layers were combined, dried over anhydrous sodium sulfate, filtered, concentrated, and purified with column (petroleum ether/ethyl acetate system) to afford the target product 4-bromo-3-methylbenzylmethanesulfonate (2.6 g, yield: 93.8%).

MS m/z (ESI): 278.9 [M+1] ⁺.

### Step 2

### Preparation of 3-(4-bromo-3-methylbenzyl)-2-butyl-1,3-diazaspiro[4.4]non-1-en-4-one

Intermediate **2b** (2.0 g, 7.2 mmol) was dissolved in N,N-dimethylformamide (5 mL), sodium hydride (0.34 g, 8.6 mmol) was added to the reaction liquid under ice bath, and the mixture was reacted under ice bath for 1 h under stirring. 2-butyl-1,3-diazaspiro[4.4]non-1-en-4-one (1.63 g, 8.6 mmol) was added to the reaction liquid, and the mixture was reacted at room temperature for 1 h under stirring. Water and dichloromethane (20 mL × 3) were added for extraction. The organic layers were combined, dried over anhydrous sodium sulfate, filtered, concentrated, and purified with column (petroleum ether/ethyl acetate system) to afford the target product 4-bromo-3-methylbenzylmethanesulfonate (2.3 g, yield: 87.8%).

MS m/z (ESI): 377.1 [M+1] ⁺.

### Step 3

### Preparation of 2-butyl-3-(3-methyl-4-(4,4,5,5-tetramethyl-1,3,2-dioxaborolan-2-yl)benzyl)-1,3 -diazaspiro[4.4]non-1 -en-4-one

Intermediate **2c** (2.0 g, 5.3 mmol), bis(pinacolato)diboron (1.6 g, 6.4 mmol), [1,1'-bis(diphenylphosphino)ferrocene]palladium dichloride dichloromethane complex (42 mg, 0.05 mmol) and potassium acetate (1.1 g, 10.6 mmol) were dissolved in dioxane (5 mL), and the reaction liquid was stirred at 80°C under nitrogen protection for 16 h. Water and dichloromethane (20 mL × 3) were added for extraction. The organic layers were combined, dried over anhydrous sodium sulfate, filtered, concentrated and purified with column (petroleum ether/ethyl acetate system) to afford the target product 2-butyl-3-(3-methyl-4-(4,4,5,5-tetramethyl-1,3,2-dioxaborolan-2-yl)benzyl)-1,3-diazaspiro[4.4]non-1-en-4-one (1.8 g, yield: 52.4%).

MS m/z (ESI): 425.3 [M+1] ⁺.

### Step 4

### Preparation of 3-(3-(bromomethyl)-4-(4,4,5,5-tetramethyl-1,3,2-dioxaborolan-2-yl)benzyl)-2-butyl-1,3-diazaspiro[4.4]non-1-en-4-one

Intermediate **2d** (1.5 g, 3.5 mmol) was dissolved in acetonitrile (5 mL), N-bromosuccinimide (0.75 g, 4.2 mmol) was added to the reaction liquid under ice bath, and the reaction was stirred at room temperature for 1 h. Water and dichloromethane (3 × 20 mL) were added for extraction. The organic layers were combined, dried over anhydrous sodium sulfate, filtered, concentrated and purified with column (petroleum ether/ethyl acetate system) to afford the target product 3-(3-(bromomethyl)-4-(4,4,5,5-tetramethyl-1,3,2-dioxaborolan-2-yl)benzyl)-2-butyl-1,3-diazaspiro[4.4]non-1-en-4-one(1.62 g, yield: 91.8%).

MS m/z (ESI): 503.2 [M+1] ⁺.

### Example 12

### 4'-(((2-butyl-4-oxo-1,3-diazospiro[4.4]non-1-en-3-yl)methyl)-N-(4,5-dimethylisoxazol-3-yl)-2' -((trifluoromethoxy)methyl)-[1,1'-biphenyl] -2-sulfonamide

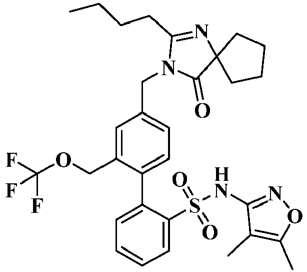

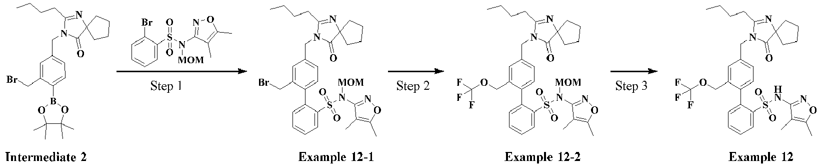

### Step 1

### Preparation of 2'-(bromomethyl)-4'-(((2-butyl-4-oxo-1,3-diazaspiro[4.4]non-1-en-3-yl)methyl)-N-(4,5-dimethylisoxazol- 3-yl)-N-(methoxymethyl)-[1,1'-biphenyl] - 2-sulfonamide

Intermediate **2** (100 mg, 0.2 mmol), (2-(N-(4,5-dimethylisoxazol-3-yl)-N-(methoxymethyl)sulfamoyl)phenyl)boronic acid (75 mg, 0.2 mmol)(referring to WO 2010135350A2 for the preparation method), [1,1'-bis(diphenylphosphino)ferrocene]palladium dichloride dichloromethane complex (16 mg, 0.02 mmol) and cesium carbonate (291 mg, 0.9 mmol) was stirred in dioxane (4 mL) and water (1 mL) at 100°C under microwave for 1 h. Water and dichloromethane (20 mL × 3) were added for extraction. The organic layers were combined, dried over anhydrous sodium sulfate, filtered, concentrated, and purified with column (petroleum ether/ethyl acetate system) to afford Example **12-1** (110 mg, yield: 82.5%).

MS m/z (ESI): 671.2 [M+1] ⁺.

### Step 2

### Preparation of 4'-((2-butyl-4-oxo-1,3-diazospiro[4.4]non-1-en-3-yl)methyl)-N-(4,5-dimethylisoxazol-3-yl)-N- (methoxymethyl)-2'-((trifluoromethoxy)methyl)-[1,1'-biphenyl] -2-sulfonamide

Trifluoromethyl trifluoromethanesulfonate (72 mg, 0.33 mmol) and silver fluoride (48 mg, 0.33 mmol) were dissolved in acetonitrile (5 mL). The reaction liquid was cooled to -30°C and reacted under stirring for 2 h. Example **12-1** (110 mg, 0.16 mmol) dissolved in 5 mL of acetonitrile was added to the reaction liquid, and the reaction liquid was reacted at room temperature for 24 h under stirring. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example 12-2 (85 mg, 77.6%).

MS m/z (ESI): 676.3 [M+1] ⁺.

### Step 3

### Preparation of 4'-(((2-butyl-4-oxo-1,3-diazospiro[4.4]non-1-en-3-yl)methyl)-N-(4,5-dimethylisoxazol-3-yl)-2' -((trifluoromethoxy)methyl)-[1,1'-biphenyl] -2-sulfonamide

Example **12-2** (85 mg, 0.13 mmol) was dissolved in dioxane (2 mL), and a solution of 6 mol/L hydrochloride in dioxane (2 mL) was added under ice bath conditions. The reaction liquid was stirred at room temperature for 1 h. The reaction liquid was concentrated, and Example **12** (32 mg, 40.4%) was prepared and isolated.

MS m/z (ESI): 633.2 [M+1] ⁺.

### Example 13

### 4'-((2-butyl-4-oxo-1,3-diazaspiro[4.4]non-1-en-3-yl)methyl)-2'-(cyclopropoxymethyl)-N-(4,5-dimethylisoxazol- 3-yl)-[1,1'-biphenyl] -2-sulfonamide

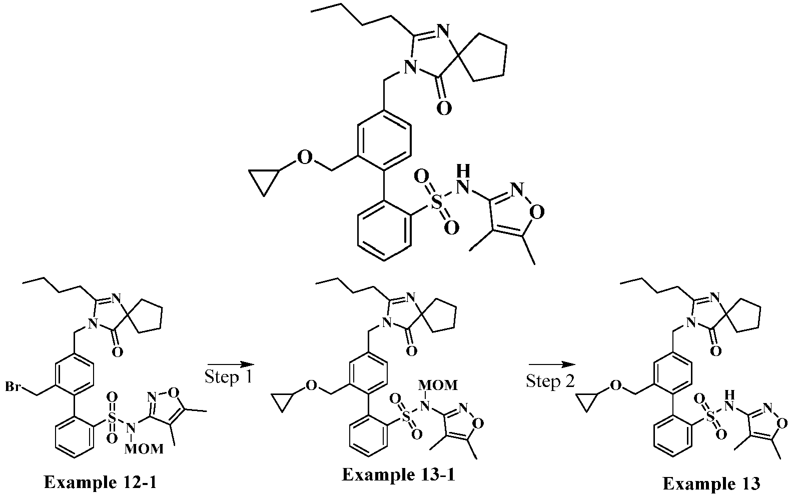

### Step 1

### Preparation of 4'-((2-butyl-4-oxo-1,3-diazaspiro[4.4]non-1-en-3-yl)methyl)-2'-(cyclopropoxymethyl)-N-(4,5-dimethylisoxazol- 3-yl)-N-(methoxymethyl)-[1,1'-biphenyl] -2-sulfonamide

Example **12-1** (100 mg, 0.15 mmol) and potassium carbonate (42 mg, 0.3 mmol) were dissolved in dichloromethane (5 mL), and then cyclopropanol (18 mg, 0.3 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **13-1** (62 mg, 78.6%).

MS m/z (ESI): 649.3 [M+1] ⁺.

### Step 2

### Preparation of 4'-((2-butyl-4-oxo-1,3-diazaspiro[4.4]non-1-en-3-yl)methyl)-2'-(cyclopropoxymethyl)-N-(4,5-dimethylisoxazol-3-yl)-[1,1'-biphenyl]-2-sulfonamide

Synthesis method of Example **13** referred to the synthesis method of Example **12**, Example **13-1** was used as raw material to afford the title compound Example **13** (22 mg, 52.7%).

MS m/z (ESI): 605.3 [M+1] ⁺.

### Example 14

### 4'-((2-butyl-4-oxo-1,3-diazaspiro[4.4]non-1-en-3-yl)methyl)-2'-((cyclopropylmethoxy)methyl)-N-(4,5 -dimethylisoxazol-3-yl)-[1,1'-biphenyl] -2-sulfonamide

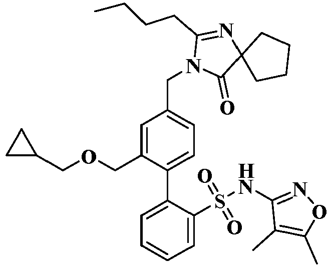

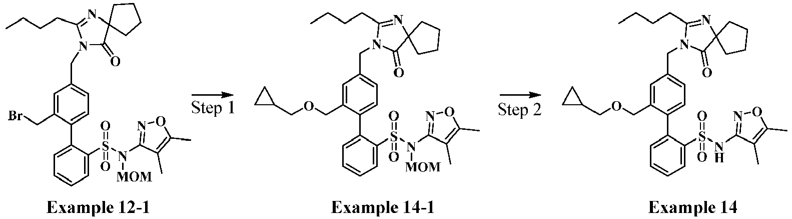

### Step 1

### Preparation of 4'-((2-butyl-4-oxo-1,3-diazaspiro[4.4]non-1-en-3-yl)methyl)-2'-((cyclopropylmethoxy)methyl)-N-(4,5 -dimethylisoxazol-3-yl)-N-(methoxymethyl)-[1,1'-biphenyl] -2-sulfonamide

Example **12-1** (100 mg, 0.15 mmol) and potassium carbonate (42 mg, 0.3 mmol) were dissolved in dichloromethane (5 mL), and then cyclopropylmethanol (22 mg, 0.3 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **14-1** (66 mg, 77.5%).

MS m/z (ESI): 663.3 [M+1] ⁺.

### Step 2

### Preparation of 4'-((2-butyl-4-oxo-1,3-diazaspiro[4.4]non-1-en-3-yl)methyl)-2'-((cyclopropylmethoxy)methyl)-N-(4,5 -dimethylisoxazol-3-yl)-[1,1'-biphenyl] -2-sulfonamide

Synthesis method of Example **14** referred to the synthesis method of Example **12**, Example **14-1** was used as raw material to afford the title compound Example **14** (28 mg, 48.8%).

MS m/z (ESI): 619.3 [M+1] ⁺.

### Example 15

### 4'-(((2-butyl-4-oxo-1,3-diazaspiro[4.4]non-1-en-3-yl)methyl)-2'-(((3,3-difluoroazetidin-1-yl)methyl)-N-(4,5-dimethylisoxazol-3-yl)-[1,1'-biphenyl] -2-sulfonamide

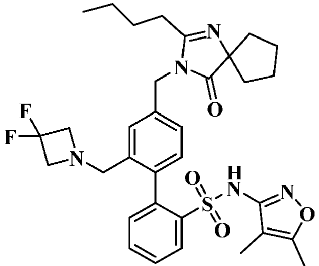

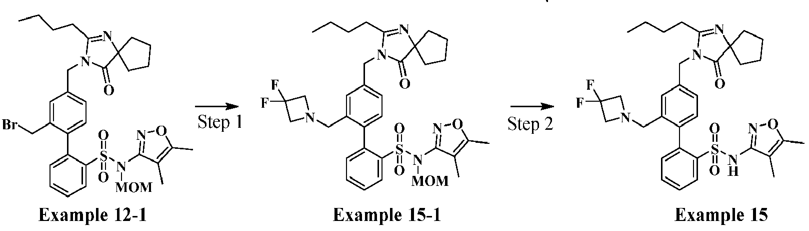

### Step 1

### Preparation of 4'-(((2-butyl-4-oxo-1,3-diazaspiro[4.4]non-1-en-3-yl)methyl)-2'-(((3,3-difluoroazetidin-1-yl)methyl)-N-(4,5-dimethylisoxazol-3-yl)-N-(methoxymethyl)-[1,1'-biphenyl]-2-sulfonamide

Example **12-1** (100 mg, 0.15 mmol) and potassium carbonate (63 mg, 0.45mmol) were dissolved in dichloromethane (5 mL), and then 3,3-difluorotrimethyleneimine hydrochloride (39 mg, 0.3 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **15-1** (66 mg, 77.5%).

MS m/z (ESI): 684.3 [M+1] ⁺.

### Step 2

### Preparation of 4'-(((2-butyl-4-oxo-1,3-diazaspiro[4.4]non-1-en-3-yl)methyl)-2'-(((3,3-difluoroazetidin-1-yl)methyl)-N-(4,5-dimethylisoxazol-3-yl)-[1,1'-biphenyl] -2-sulfonamide

Synthesis method of Example **15** referred to the synthesis method of Example **12**, Example **15-1** was used as raw material to afford the title compound Example **15** (33 mg, 62.8%).

MS m/z (ESI): 640.3 [M+1] ⁺.

### Example 16

### methyl((4-((2-butyl-4-oxo-1,3-diazaspiro[4.4]non-1-en-3-yl)methyl)-2'-(N-(4,5-dimethylisoxazol-3 -yl)sulfamoyl)-[1,1'-biphenyl] -2-yl)methyl)carbamate

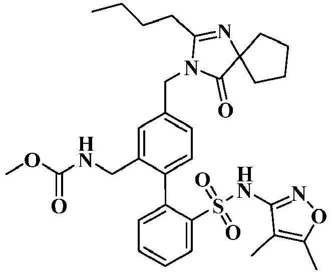

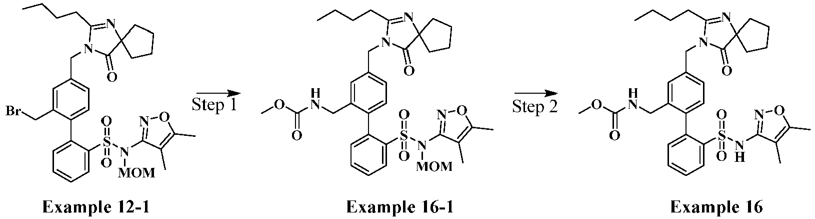

### Step 1

### Preparation of methyl((4-((2-butyl-4-oxo-1,3-diazaspiro[4.4]non-1-en-3-yl)methyl)-2'-(N-(4,5-dimethylisoxazol-3-yl)-N-(methoxymethyl)sulfamoyl)-[I, 1'-biphenyl] -2-yl)methyl)carbamate

Example **12-1** (100 mg, 0.15 mmol) and potassium carbonate (63 mg, 0.45mmol) were dissolved in dichloromethane (5 mL), and then methyl carbamate (22 mg, 0.3 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **16-1** (58 mg, 56.5%).

MS m/z (ESI): 666.3 [M+1] ⁺.

### Step 2

### Preparation of methyl((4-((2-butyl-4-oxo-1,3-diazaspiro[4.4]non-1-en-3-yl)methyl)-2'-(N-(4,5-dimethylisoxazol-3 -yl)sulfamoyl)-[1,1'-biphenyl]-2-yl)methyl)carbamate

Synthesis method of Example **16** referred to the synthesis method of Example **12**, Example **16-1** was used as raw material to afford the title compound Example **16** (26 mg, 55.8%).

MS m/z (ESI): 622.3 [M+1] ⁺.

### Example 17

### (4-((2-butyl-4-oxo-1,3-diazaspiro[4.4]non-1-en-3-yl)methyl)-2'-(N-(4,5-dimethylisoxazol-3-yl )sulfamoyl)-[1,1'-biphenyl] -2-yl)methylcarbamate

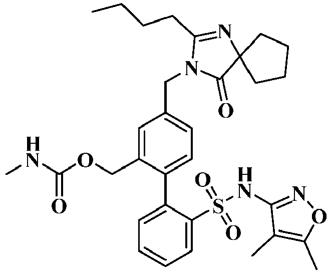

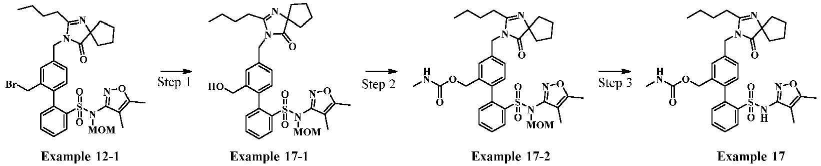

### Step 1

### Preparation of 4'-(((2-butyl-4-oxo-1,3-diazaspiro[4.4]non-1-en-3-yl)methyl)-N-(4,5-dimethylisoxazol-3-yl)-2' -(hydroxymethyl)-N-(methoxymethyl)-[1,1'-biphenyl] -2-sulfonamide

Example **12-1** (100 mg, 0.15 mmol) was dissolved in a mixed solution of 5 mL ethanol and 5 mL of water, and then sodium hydroxide (18 mg, 0.45 mmol) was added to the reaction liquid. The reaction liquid was stirred at room temperature overnight. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **17-1** (70 mg, 78.4%).

MS m/z (ESI): 609.3 [M+1] ⁺.

### Step 2

### Preparation of (4-((2-butyl-4-oxo-1,3-diazaspiro[4.4]non-1-en-3-yl)methyl)-2'-(N-(4,5-dimethylisoxazol-3-yl)-N-(methoxymethyl)sulfamoyl)-[1,1'-biphenyl] -2-yl)methylcarbamate

Example **17-1** (100 mg, 0.16 mmol) was dissolved in 5 mL of dimethyl disulfide, and then methyl isocyanate (28 mg, 0.48 mmol) was added to the reaction liquid. The reaction liquid was reacted at 55°C for 2 h under stirring. Saturated brine (10 mL) was added to the reaction liquid, and the mixture was extracted with ethyl acetate (10 mL×). The organic phases were combined, dried, concentrated, and purified with column (petroleum ether/ethyl acetate system) to afford Example **17-2** (110 mg, 62.8%).

MS m/z (ESI): 666.3 [M+1] ⁺.

### Step 3

### Preparation of (4-((2-butyl-4-oxo-1,3-diazaspiro[4.4]non-1-en-3-yl)methyl)-2'-(N-(4,5-dimethylisoxazol-3-yl )sulfamoyl)-[1,1'-biphenyl] -2-yl)methylcarbamate

Synthesis method of Example **17** referred to the synthesis method of Example **12**, Example **17-2** was used as raw material to afford the title compound Example **17** (42 mg, 68.9%).

MS m/z (ESI): 622.3 [M+1] ⁺.

### Example 18

### 2'-(azetidine-1-carbonyl)-4'-(((2-butyl-4-oxo-1,3-diazaspiro[4.4]non-1-en-3-yl)methyl)-N-(4,5-dimethylisoxazol-3-yl)-[1,1'-biphenyl] -2-sulfonamide

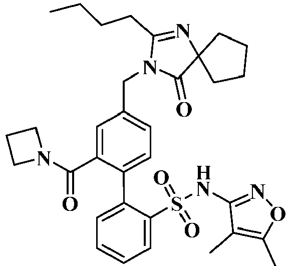

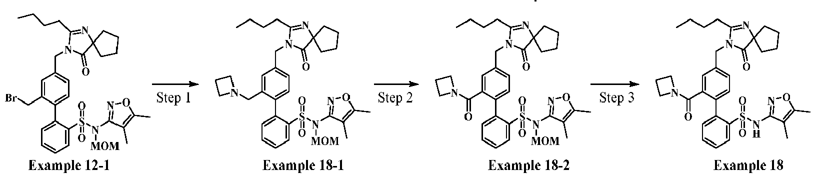

### Step 1

### Preparation of 2'-(azetidine-1-carbonyl)-4'-(((2-butyl-4-oxo-1,3-diazaspiro[4.4]non-1-en-3-yl)methyl)-N-(4, 5-dimethylisoxazol-3-yl)-N-(methoxymethyl)-[1,1'-biphenyl] -2-sulfonamide

Example **12-1** (100 mg, 0.15 mmol) and potassium carbonate (63 mg, 0.45mmol) were dissolved in dichloromethane (5 mL), and then cyclobutylamine (15 mg, 0.2 mmol) was added to the reaction liquid, and the reaction liquid was stirred and reacted at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **18-1** (65 mg, 72.6%).

MS m/z (ESI): 648.3[M+1] ⁺.

### Step 2

### Preparation of 2'-(azetidine-1-carbonyl)-4'-(((2-butyl-4-oxo-1,3-diazaspiro[4.4]non-1-en-3-yl)methyl)-N-(4, 5-dimethylisoxazol-3-yl)-N-(methoxymethyl)-[1,1'-biphenyl] -2-sulfonamide

Example **18-1** (65 mg, 0.1 mmol) was dissolved in dichloromethane (10 mL), cetyltrimethylammonium bromide (51 mg, 0.14 mmol) and KMnO₄ (22 mg, 0.14 mmol) were added to the reaction liquid respectively. The reaction liquid was reacted for 2 h under reflux conditions under stirring. The reaction mixture was cooled to room temperature, then saturated aqueous sodium sulfite solution (5 mL) was added with vigorous stirring. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **18-2** (45 mg, 73.4%).

MS m/z (ESI): 662.3[M+1] ⁺.

### Step 3

### Preparation of 2'-(azetidine-1-carbonyl)-4'-(((2-butyl-4-oxo-1,3-diazaspiro[4.4]non-1-en-3-yl)methyl)-N-(4,5-dimethylisoxazol-3-yl)-[1,1'-biphenyl] -2-sulfonamide

Synthesis method of Example **18** referred to the synthesis method of Example **12**, Example **18-2** was used as raw material to afford the title compound Example **18** (22 mg, 48.5%).

MS m/z (ESI): 618.3[M+1] ⁺.

### Example 19

### N-((4-((2-butyl-4-oxo-1,3-diazaspiro[4.4]non-1-en-3-yl)methyl)-2'-(N-(4,5-dimethylisoxazol- 3-yl)sulfamoyl)-[1,1'-biphenyl-2-yl)methyl)-N, 3,3-trimethylbutyramide

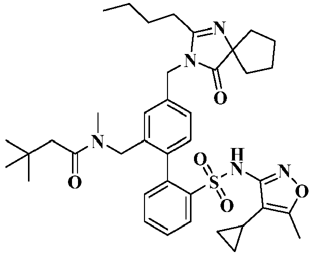

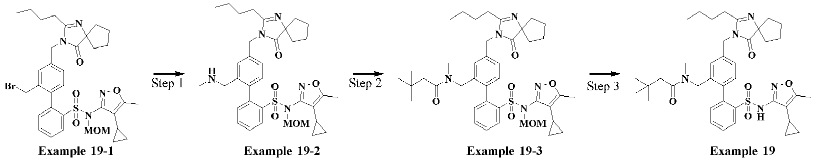

### Step 1

### Preparation of 4'-(((2-butyl-4-oxo-1,3-diazaspiro[4.4]non-1-en-3-yl)methyl)-N-(4-cyclopropyl-5-methylisoxazol-3-yl)- N-(methoxymethyl)-2'-((methylamino)methyl)-[1,1'-biphenyl] -2-sulfonamide

Example **19-1** (104 mg, 0.15 mmol) (referring to Example **12-1** for the synthesis method) and potassium carbonate (63 mg, 0.45mmol) were dissolved in dichloromethane (5 mL), and then methylamine hydrochloride (30 mg, 0.45 mmol) was added to the reaction liquid, and the reaction liquid was reacted at room temperature under stirring for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **19-2** (65 mg, 67.0%).

MS m/z (ESI): 648.3[M+1] ⁺.

### Step 2

### Preparation of N-((4-((2-butyl-4-oxo-1,3-diazaspiro[4.4]non-1-en-3-yl)methyl)-2'-(N-(4-cyclopropyl-5- methylisoxazol-3-yl)-N-(methoxymethyl)sulfamoyl)-[1,1'-biphenyl-2-yl)methyl)-N, 3,3-trimethylbutyramide

Example **19-2** (65 mg, 0.10 mmol), 3,3-dimethylbutyric acid (23 mg, 0.20 mmol), 2-(7-azobenzotriazole)-N,N,N',N'-tetramethylurea hexafluorophosphate (114 mg, 0.30 mmol) were dissolved in dichloromethane (5 mL), and triethylamine (38 mg, 0.30 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated, and purified with column (petroleum ether/ethyl acetate system) to afford Example **19-3** (55 mg, 73.7%).

MS m/z (ESI): 746.4 [M+1] ⁺.

### Step 3

### Preparation of N-((4-((2-butyl-4-oxo-1,3-diazaspiro[4.4]non-1-en-3-yl)methyl)-2'-(N-(4-cyclopropyl-5-methylisoxazol-3-yl)sulfamoyl)-[1,1'-biphenyl] -2-yl)methyl)-N,3,3-trimethylbutyramide

Synthesis method of Example **19** referred to the synthesis method of Example **12**, Example **19-2** was used as raw material to afford the title compound Example **19** (36 mg, 58.5%).

MS m/z (ESI): 702.4[M+1] ⁺.

### Example 20

### N-((4'-((2-butyl-4-oxo-1,3-diazaspiro[4.4]non-1-en-3-yl)methyl)-2'-(ethoxymethyl)-[1,1'-biphenyl]-2-yl)sulfonyl)cyclopropanecarboxamide

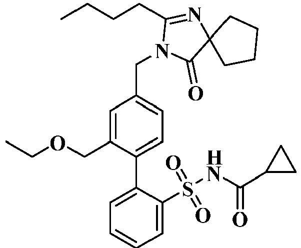

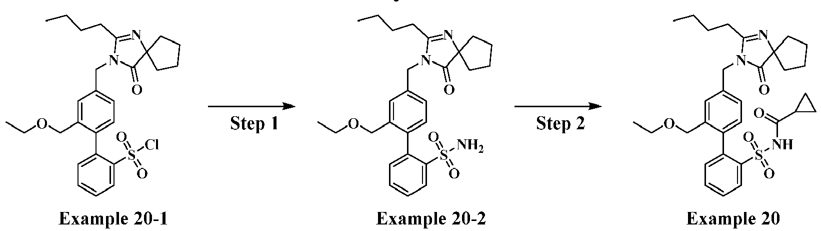

### Step 1

### Preparation of 4'-((2-butyl-4-oxo-1,3-diazaspiro[4.4]non-1-en-3-yl)methyl)-2'-(ethoxymethyl)-[1,1'-biphenyl]-2-sulfonamide

Ammonia gas was bubbled into a solution of Example **20-1** (2.35 g, 5 mmol) (referring to WO 2010135350 A2 for the preparation method) in THF (30 mL) until saturated. The mixture was stirred at 25°C for 12 h. The organic phases were combined, dried and concentrated, and the residue was purified with silica gel column chromatography (dichloromethane/methanol system) to afford Example **20-2** (2.05 g, 91%).

MS m/z (ESI): 498.2 [M+1] ⁺.

### Step 2

### Preparation of N-((4'-((2-butyl-4-oxo-1,3-diazaspiro[4.4]non-1-en-3-yl)methyl)-2'-(ethoxymethyl)-[1,1'-biphenyl]-2-yl)sulfonyl)cyclopropanecarboxamide

Cyclopropyl formyl chloride (88.2 mg, 0.84 mmol) and 1,8-diazabicycloundec-7-ene (756 mg, 3.01 mmol) were added to a solution of Example **20-2** (100 mg, 0.2 mmol) in DCM (5 mL) at 25°C, and the mixture was stirred at 50°C for 2 h. The reaction liquid was quenched by adding 5 mL of water, extracted with DCM (10 mL × 3), the organic phases were combined, dried, concentrated and purified with preparative HPLC to afford Example **20** (56 mg, 49.1%).

MS m/z (ESI): 566.3 [M+1] ⁺.

### Example 21

### N-((4'-((2-butyl-4-oxo-1,3-diazaspiro[4.4]non-1-en-3-yl)methyl)-2'-(ethoxymethyl)-[1,1'-biphenyl]-2-yl)sulfonyl)picolinamide

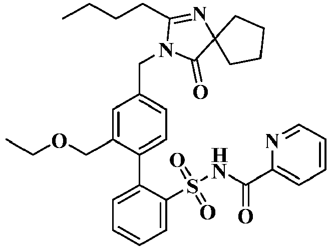

Synthesis method of Example **21** referred to the synthesis method of Example **20**, pyridyl formyl chloride was used in stead of cyclopropyl formyl chloride to afford Example **21** (65 mg, 60.4%).

MS m/z (ESI): 603.3 [M+1] ⁺.

### Example 22

### N-((4'-((2-butyl-4-oxo-1,3-diazaspiro[4.4]non-1-en-3-yl)methyl)-2'-(ethoxymethyl)-[1,1'-biphenyl]-2-yl)sulfonyl)benzamide

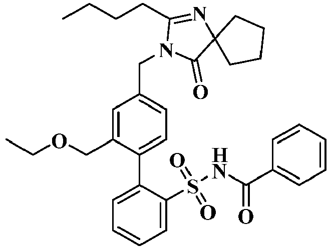

Synthesis method of Example **22** referred to the synthesis method of Example **20,** benzoyl chloride was used in stead of cyclopropanoyl chloride to afford Example **22** (57 mg, 43.2%).

MS m/z (ESI): 602.3 [M+1] ⁺.

### Example 23

### 4'-((2-butyl-4-oxo-1,3-diazaspiro[4.4]non-1-en-3-yl)methyl)-2'-(ethoxymethyl)-N-(5-oxo-2,5-dihydro-1,2,4-oxadiazol-3-yl)-[1,1'-biphenyl]-2-sulfanilamide

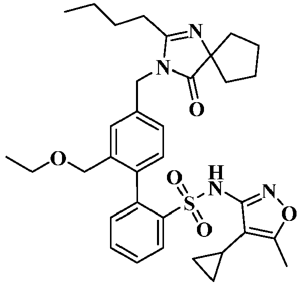

Synthesis method of Example **23** referred to the synthesis method of Example **20-2,** 4-cyclopropyl-5-methylisoxazole-3-amine was used in stead of ammonia gas to afford Example **23** (45 mg, 39.5%).

MS m/z (ESI): 619.3 [M+1] ⁺.

### Example 24

### 4'-((2-butyl-4-oxo-1,3-diazaspiro[4.4]non-1-en-3-yl)methyl)-N-(5,6-dihydro-4H-cyclopenta[d]isoxazol-3-yl)-2'-(ethoxymethyl)-[1,1'-biphenyl]-2-sulfanilamide

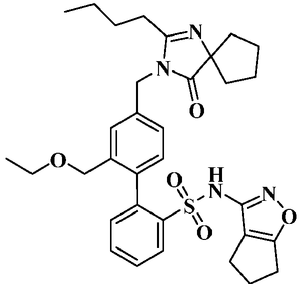

Synthesis method of Example **24** referred to the synthesis method of Example **20-2**, 5,6-dihydro-4H-cyclopentyl[d]isoxazole-3-amine was used in stead of ammonia gas to afford Example **24** (36 mg, 40.1%).

MS m/z (ESI): 605.3 [M+1] ⁺.

### Example 25

### 4'-((2-butyl-4-oxo-1,3-diazaspiro[4.4]non-1-en-3-yl)methyl)-2'-(ethoxymethyl)-N-(2H-tetrazol-5-yl)-[1,1'-biphenyl]-2-sulfonamide

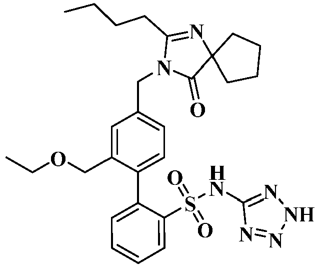

Example **20-1** (100 mg, 0.19 mmol) was added dropwise to a solution of 1H-tetrazol-5-amine (16.5 mg, 0.19 mmol) and NaOH (15.5 mg, 0.38 mmol) in water (2 mL) at 70°C. The mixture was stirred for 3 h. Under an ice bath, the mixture was acidified with concentrated hydrochloric acid, extracted with ethyl acetate (30 mL × 3), the organic phases were combined, dried and concentrated, and the residue was purified with HPLC to afford Example **25** (26 mg, 23.8%).

MS m/z (ESI): 566.7 [M+1] ⁺.

### Example 26

### 4'-((2-butyl-4-oxo-1,3-diazaspiro[4.4]non-1-en-3-yl)methyl)-2'-(ethoxymethyl)-N-(5-oxo-2,5-dihydro-1,2,4-oxadiazol-3-yl)-[1,1'-biphenyl]-2-sulfanilamide

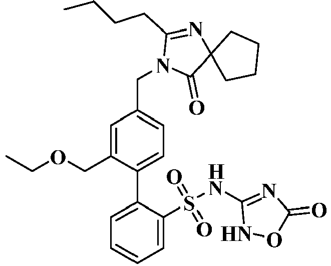

Synthesis method of Example **26** referred to the synthesis method of Example **25**, 3-amino-1,2,4-oxadiazole-5(2H)-one was used in stead of 1H-tetrazole-5-amine to afford Example **26** (36 mg, 33.1%).

MS m/z (ESI): 582.2 [M+1] ⁺.

### Example 27

### 4'-((2-butyl-4-oxo-1,3-diazaspiro[4.4]non-1-en-3-yl)methyl)-N-(cyclopropylsulfonyl)-2'-(ethoxymethyl)-[1,1'-biphenyl]-2-carboxamide

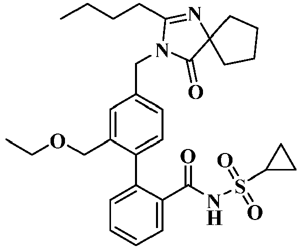

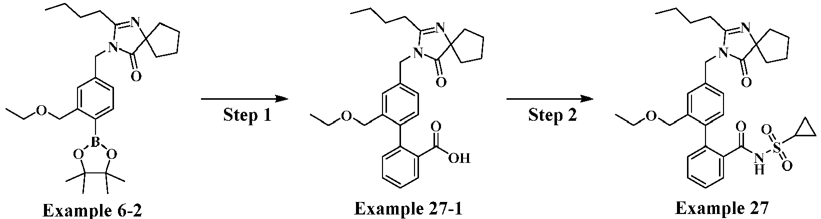

### Step 1

### Preparation of 4'-((2-butyl-4-oxo-1,3-diazaspiro[4.4]non-1-en-3-yl)methyl)-2'-(ethoxymethyl)-[1,1'-biphenyl]-2-carboxylic acid

Example **6-2** (2.35 g, 5 mmol), 2-bromobenzoic acid (0.99 g, 5 mmol), Pd(dppf)Cl₂*DCM (200 mg, 0.25 mmol), Cs₂CO₃ (3.26 g, 10 mmol), 1'4-Dioxane (25 mL) and H₂O (5 mL) were added to a round bottom flask. The mixture was stirred at 80°C for 12 h under N₂ protection. The reaction liquid was quenched by adding 20 mL of dilute hydrochloric acid (1 M), extracted with ethyl acetate (30 mL × 3), the organic phases were combined, dried and concentrated, and the residue was purified with silica gel column chromatography (petroleum ether/ethyl acetate system) to afford Example **27-1** (1.69 g, 73.3%).

MS m/z (ESI): 463.3 [M+1] ⁺.

### Step 2

### Preparation of 4'-((2-butyl-4-oxo-1,3-diazaspiro[4.4]non-1-en-3-yl)methyl)-N-(cyclopropylsulfonyl)-2'-(ethoxymethyl)-[1,1'-biphenyl]-2-carboxamide

Cyclic cyclopropyl methanesulfonamide (36 mg, 0.3 mmol) was added to a solution of Example **27-1** (100 mg, 0.22 mmol), EDCI (58 mg, 0.3 mmol) and DMAP (37 mg, 0.3 mmol) in DCM (5 mL) at 25°C, the mixture was stirred overnight at 25°C for 12 h. The reaction liquid was quenched by adding 5 mL of water, extracted with DCM (10 mL × 3), the organic phases were combined, dried, concentrated and purified with preparative HPLC to afford the title product Example **27** (48 mg, 39.2%).

MS m/z (ESI): 566.3 [M+1] ⁺.

### Example 28

### 4'-((2-butyl-4-oxo-1,3-diazaspiro[4.4]non-1-en-3-yl)methyl)-2'-(ethoxymethyl)-N-(2H-tetrazol-5-yl)-[1,1'-biphenyl]-2-carboxamide

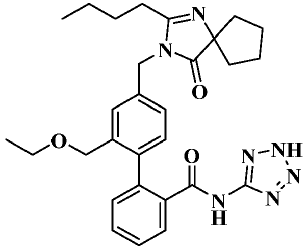

Synthesis method of Example **28** referred to the synthesis method of Example **27**, 2H-tetrazole-5-amine was used in stead of cyclopropane sulfonamide to afford Example **28** (62 mg, 58.2%).

MS m/z (ESI): 530.3 [M+1] ⁺.

### Example 29

### 4'-(2-butyl-4-oxo-1,3-diazaspiro[4.4]non-1-en-3-yl)methyl)-2'-(ethoxymethyl)-N-(benzenesulfonyl)-[1,1'-biphenyl]-2-carboxamide

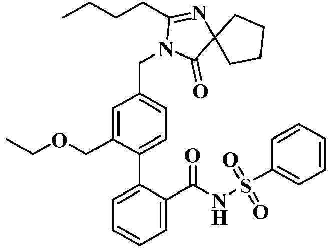

Synthesis method of Example **29** referred to the synthesis method of Example **27**, benzsulfamide was used in stead of cyclopropane sulfonamide to afford Example 29 (62 mg, 58.2%).

MS m/z (ESI): 602.3 [M+1] ⁺.

### Example 30

### methyl((4'-((2-butyl-4-oxo-1,3-diazaspiro[4.4]non-1-en-3-yl)methyl)-2'-(ethoxymethyl)-[1,1'-biphenyl]-2-yl)sulfonyl)carbamate

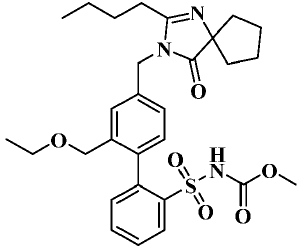

Synthesis method of Example **30** referred to the synthesis method of Example **20**, methyl chloroformate was used in stead of cyclopropanoyl chloride to afford Example **30** (66 mg, 52.2%).

MS m/z (ESI): 556.2 [M+1] ⁺.

### Example 31

### cyclopropyl((4'-((2-butyl-4-oxo-1,3-diazaspiro[4.4]non-1-en-3-yl)methyl)-2'-(ethoxymethyl)-[1,1'-biphenyl]-2-yl)sulfonyl)carbamate

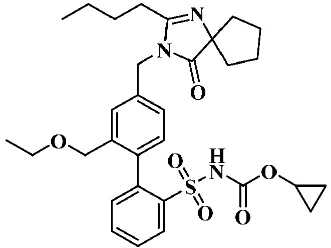

Synthesis method of Example **31** referred to the synthesis method of Example **20**, cyclopropyl chloroformate was used in stead of cyclopropanoyl chloride to afford Example **31** (45 mg, 41.3%).

MS m/z (ESI): 582.3[M+1] ⁺.

### Example 32

### benzyl((4'-((2-butyl-4-oxo-1,3-diazaspiro[4.4]non-1-en-3-yl)methyl)-2'-(ethoxymethyl)-[1,1'-biphenyl]-2-yl)sulfonyl)carbamate

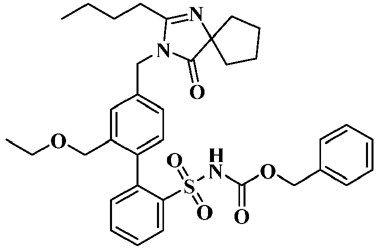

Synthesis method of Example **32** referred to the synthesis method of Example **20**, benzyl chloroformate was used in stead of cyclopropanoyl chloride to afford Example **32** (65 mg, 60.5%).

MS m/z (ESI): 632.3 [M+1] ⁺.

### Example 33

### 4'-(2-butyl-4-oxo-1,3-diazaspiro[4.4]non-1-en-3-yl)methyl)-2'-(ethoxymethyl)-N-(isopropylcarbamoyl)-[1,1'-biphenyl]-2-sulfanilamide

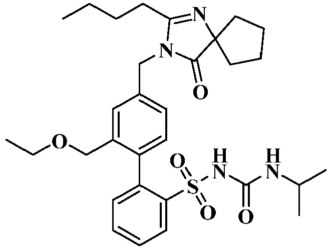

Pyridine (32 µL, 0.39 mmol) and Example **20-1** (0.1 g, 0.19 mmol) were mixed and stirred for 5 min and then added to a solution of sodium cyanate (18.9 mg, 0.29 mmol) in acetonitrile (5 mL). The mixture was stirred at room temperature for 4 h, isopropylamine (17.2 mg, 0.29 mmol) was added, and the mixture was stirred at room temperature for about 1 h. Under ice bath, the reaction liquid was acidified with dilute hydrochloric acid (pH 5-6), the aqueous layer was extracted three times with ethyl acetate, the organic phases were combined, washed with saturated brine, dried over anhydrous sodium sulfate, concentrated under reduced pressure and purified with HPLC to afford Example **33** (59 mg, 52%).

MS m/z (ESI): 583.8 [M+1] ⁺.

### Example 34

### 4'-(2-butyl-4-oxo-1,3-diazaspiro[4.4]non-1-en-3-yl)methyl)-2'-(ethoxymethyl)-N-(propylcarbamoyl)-[1,1-biphenyl]-2-sulfanilamide

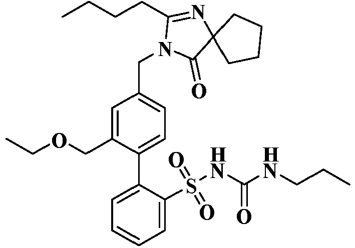

Synthesis method of Example **34** referred to the synthesis method of Example **33**, n-propylamine was used in stead of isopropylamine to afford Example **34** (68 mg, 63.1%).

MS m/z (ESI): 583.3 [M+1] ⁺.

### Example 35

### 4'-(2-butyl-4-oxo-1,3-diazaspiro[4.4]non-1-en-3-yl)methyl)-2'-(ethoxymethyl)-N-(phenylcarbamoyl)-[1,1'-biphenyl]-2-sulfanilamide

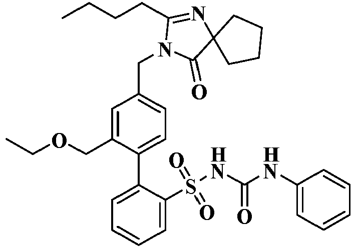

Synthesis method of Example 35 referred to the synthesis method of Example **33**, phenylamine was used in stead of isopropylamine to afford Example **35** (58 mg, 45.2%).

MS m/z (ESI): 617.3 [M+1] ⁺.

### Example 36

### 4'-(2-butyl-4-oxo-1,3-diazaspiro[4.4]non-1-en-3-yl)methyl)-2'-(ethoxymethyl)-N-(cyclopropylcarbamoyl)-[1,1'-biphenyl]-2-sulfanilamide

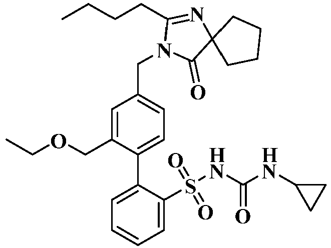

Synthesis method of Example **36** referred to the synthesis method of Example **33**, cyclopropylamine was used in stead of cycloisopropylamine to afford Example **36** (53 mg, 48.3%).

MS m/z (ESI): 581.3 [M+1] ⁺.

### Example 37

### 4'-(2-butyl-4-oxo-1,3-diazaspiro[4.4]non-1-en-3-yl)methyl)-2'-(ethoxvmethyl)-N-(ethylcarbamoyl)-[1,1'-biphenyl]-2-sulfanilamide

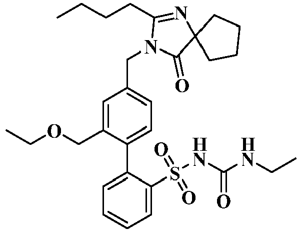

Synthesis method of Example **37** referred to the synthesis method of Example **33**, ethyl isocyanate was used in stead of cyclopropanoyl chloride to afford Example **37** (64 mg, 60.5%).

MS m/z (ESI): 569.3 [M+1] ⁺.

### Example 38

### N-(N-(4'-((2-butyl-4-oxo-1,3-diazaspiro[4.4]non-1-en-3-yl)methyl)-2'-(ethoxymethyl)-[1,1'-biphenyl]-2-yl)sulfanilamido)benzamide

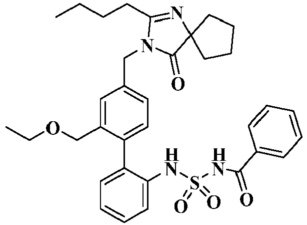

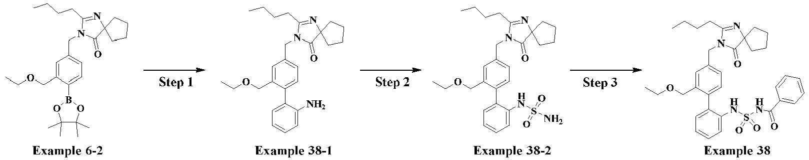

### Step 1

### Preparation of 3-((2'-amino-2-(ethoxymethyl)-[1,1'-biphenyl]-4-yl)methyl)-2-butyl-1,3-diazaspiro[4.4]non-1-en-4-one

Example **6-2** (2.35 g, 5 mmol), 2-bromoaniline (0.86 g, 5 mmol), Pd(dppf)Cl₂*DCM (200 mg, 0.25 mmol), Cs₂CO₃ (3.26 g, 10 mmol), 1'4-Dioxane (25 mL) and H₂O (5 mL) were added to a round bottom flask. The mixture was stirred at 80°C for 12 h under N₂ protection. The reaction liquid was quenched by adding 30 mL of water, extracted three times with 30 mL of ethyl acetate, the organic phases were combined, dried and concentrated, and the residue was purified by silica gel column chromatography (ethyl acetate/petroleum ether) to afford Example **38-1**(1.61 g, 75.2%).

MS m/z (ESI): 434.3 [M+1] ⁺.

### Step 2

### Preparation of 3-((2'-sulfonylureido-2-(ethoxymethyl)-[1,1'-biphenyl]-4-yl)methyl)-2-butyl-1,3 -diazaspiro[4.4]non-1 -en-4-one

Tert-butanol (0.85 g, 11.48 mmol) in anhydrous dichloromethane (10 mL) was added to a stirred solution of chlorosulfonyl isocyanate (1.62 g, 11.48 mmol) in anhydrous dichloromethane (10 mL) at 0°C. After 30 min, the resulting solution (1.75 mL, 0.91 mmol) was slowly added to a solution of Example **38-1** (0.36 g, 0.83 mmol) in anhydrous dichloromethane (5 mL) at 0°C. The reaction liquid was warmed to room temperature and stirred for 2 h. The reaction mixture was diluted with dichloromethane (30 mL). The mixture was washed with 0.1N dilute hydrochloric acid (20 mL) and water (25 mL) in sequence. The organic layer was dried over anhydrous sodium sulfate and concentrated in vacuo. The crude product was refluxed in distilled water (30 mL) for 15-30 min. The reaction mixture was extracted with ethyl acetate (3 × 30 mL), the organic phases were combined, dried over anhydrous sodium sulfate, concentrated under reduced pressure and purified with silica gel column chromatography (ethyl acetate/petroleum ether system) to afford Example **38-2** (0.31 g, 72.9%).

MS m/z (ESI): 513.3 [M+1] ⁺.

### Step 3

### Preparation of N-(N-(4'-((2-butyl-4-oxo-1,3-diazaspiro[4.4]non-1-en-3-yl)methyl)-2'-(ethoxymethyl)-[1,1'-biphenyl] -2-yl)sulfamoyl benzamide

Synthesis method of Example **38** referred to the synthesis method of Example **20**, benzoyl chloride was used in stead of cyclopropyl formyl chloride to afford Example **38** (180 mg, 48.4%).

MS m/z (ESI): 617.3 [M+1] ⁺.

### Example 39

### N-(4'-((2-butyl-4-oxo-1,3-diazaspiro[4,4]non-1-en-3-yl)methyl)-2'-(ethoxymethyl)-[1,1'-biphenyl]-2-yl)-5-methylpyridine-2-sulfanilamide

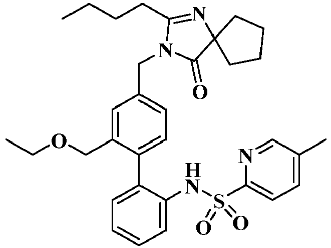

Synthesis method of Example **39** referred to the synthesis method of Example **20**, Example **38-1** was used as raw material, and 5-methylpyridine-2-sulfonyl chloride was used in stead of cyclopropyl formyl chloride to afford Example **39** (80 mg, 56.2%).

MS m/z (ESI): 589.3 [M+1] ⁺.

### Example 40

### N-(4'-((2-butyl-4-oxo-1,3-diazaspiro[4.4]non-1-en-3-yl)methyl)-2'-(ethoxymethyl)-[1,1'-biphenyl]-2-yl)-4,5-dimethylisoxazole-3-sulfonamide

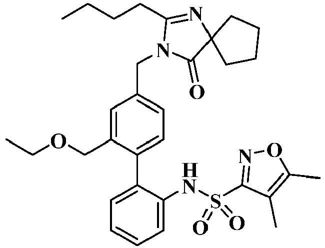

Synthesis method of Example **40** referred to the synthesis method of Example **20**, Example **38-1** was used as raw material, and 4,5-dimethylisoxazole-3-sulfonyl chloride was used in stead of cyclopropyl formyl chloride to afford Example **40** (53 mg, 62.1%).

MS m/z (ESI): 593.3 [M+1] ⁺.

### Example 41

### 2'-(benzyloxy)-4'-(2-butyl-4-oxo-1,3-diazaspiro[4.4]non-1-en-3-yl)methyl)-N-(4,5-dimethylisoxazol-3-yl)-[1,1'-biphenyl]-2-sulfonamide

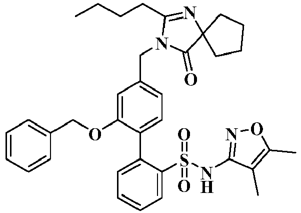

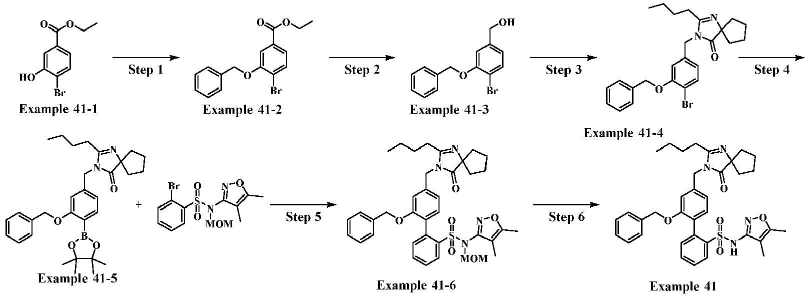

### Step 1

### Preparation of ethyl 3-(benzyloxy)-4-bromobenzoate

Benzyl bromide (3.5 g, 20.40 mmol) and potassium carbonate (5.63 g, 40.80 mmol) were slowly added to a solution of Example **41-1** (5 g, 20.40 mmol) in DMF (50 mL), and the mixture was stirred at 80°C for 2 h. After the reaction liquid was cooled, 100 mL of ethyl acetate was added to dilute the mixture, and then the mixture was washed with water (50 mL*3), the organic phases were combined, dried over anhydrous sodium sulfate, filtered and concentrated, and the residue was purified by silica gel column chromatography with eluent system B to afford the title product example **41-2** (6.50 g, colorless liquid), yield: 95.1%.

¹H NMR (400 MHz, Chloroform-d) δ 7.63 (d, J = 8.1 Hz, 2H), 7.51 (dd, J = 9.3, 7.5 Hz, 3H), 7.44 - 7.37 (m, 2H), 7.37 - 7.31 (m, 1H), 5.21 (s, 2H), 3.91 (s, 3H).

### Step 2

### Preparation of (3-(benzyloxy)-4-bromophenyl)methanol

DIBAL-H (1 M, 50.60 mL) was slowly added to a solution of Example **41-2** (6.5 g, 20.24 mmol) in DCM (60 mL) under nitrogen protection at 0°C, and the mixture was stirred at 25°C for 1 h. The reaction liquid was quenched by adding ice-cold 5% NaOH (60 mL), and extracted with dichloromethane (50 mL*3). The organic phases were combined, dried and concentrated to afford the title product Example 41-3 (6.50 g, colorless liquid) which was used directly in the next step.

### Step 3

### Preparation of 3-(3-(benzyloxy)-4-bromophenyl)-2-butyl-1,3-diazaspiro[4.4]non-1-en-4-one

Methylsulfonyl chloride (4.53 g, 39.57 mmol) and diisopropylethylamine (7.67 g, 59.35 mmol, 10.34 mL) were added to a solution of Example **41-3** (5.8 g, 19.78 mmol) in DCM (30 mL) under nitrogen protection at 0°C, the mixture was stirred at room temperature for 1 h, and then 10 mL of ice water was added to quench the reaction. The lower organic phase solution was removed and added to a mixture of tributylmethyl ammonium chloride (0.4 mL, 75% purity), 2-butyl-1,3-diazaspiro[4.4]non-1-en-4-one hydrochloride (4.57 g, 19.78 mmol), aqueous sodium hydroxide solution (10 M, 13.16 mL) and DCM (30 mL), and the resulting mixture was reacted for 2 h at room temperature. The reaction liquid was quenched by adding water (30 mL), extracted with dichloromethane (30 mL*3), the organic phases were combined, dried and concentrated, and the residue was purified by silica gel column chromatography with eluent system B to afford the title product Example **41- 4** (8.60 g, light yellow oil), yield: 92.6%.

MS m/z (ESI): 469.0 [M+1] ⁺.

¹H NMR (400 MHz, Chloroform-d) δ 7.51 (d, J = 8.0 Hz, 1H), 7.45 - 7.41 (m, 2H), 7.38 (dd, J = 8.3, 6.5 Hz, 2H), 7.34 - 7.29 (m, 1H), 6.72 (d, J = 1.9 Hz, 1H), 6.64 (dd, J = 8.0, 1.9 Hz, 1H), 5.12 (s, 2H), 4.60 (s, 2H), 2.26 (t, J = 8.1 Hz, 2H), 2.02 - 1.93 (m, 6H), 1.82 (d, J = 9.7 Hz, 2H), 1.58 - 1.51 (m, 2H), 1.31 (t, J = 7.5 Hz, 2H), 0.87 (t, J = 7.3 Hz, 3H).

### Step 4

### Preparation of 3-(3-(benzyloxy)-4-(4,4,5,5-tetramethyl-1,3,2-dioxaborolan-2-yl)benzyl)-2-butyl-1,3-diazaspiro[4.4]non-1-en-4-one

Example **41-4** (1 g, 2.13 mmol), Pd(dppf)Cl₂*DCM (174 mg, 213.03 µmol), potassium acetate (626 mg, 6.39 mmol), pinacol boronate (650 mg, 2.56 mmol), and 1'4-Dioxane (10 mL) were added to a reactor. The mixture was stirred at 90°C for 12 h under nitrogen protection. After the reaction liquid was cooled, 10 mL of water was added to quench the reaction, and the mixture was extracted with ethyl acetate (15 mL*3). The organic phases were combined, dried and concentrated to afford the title product example **41-5** (600 mg, reddish brown oil) which was used directly in the next step.

### Step 5

### Preparation of 2'-(benzyloxy)-4'-(2-butyl-4-oxo-1,3-diazaspiro[4.4]non-1-en-3-yl)methyl)-N-(4,5-dimethylisoxazol-3-yl)-N-(methoxymethyl)-[1,1'-biphenyl]-2-sulfonamide

Example **41-5** (0.6 g, 1.16 mmol), 2-bromo-N-(4,5-dimethylisoxazol-3-yl)-N-(methoxymethyl)benzsulfamide(436 mg, 1.16 mmol), Pd(dppf)Cl₂*DCM (94.80 mg, 116.17 µmol), potassium carbonate (320.63 mg, 2.32 mmol), 1'4-Dioxane (10 mL) and H2O (2 mL) were added to a reactor. The mixture was stirred at 100°C for 12 h under nitrogen protection. After the reaction liquid was cooled, 8 mL of water was added to quench the reaction, extracted with ethyl acetate (10 mL*3), the organic phases were combined, dried and concentrated, and the residue was purified by silica gel column chromatography with eluent system B to afford the title product Example **41-6** (650 mg, light brown solid), yield: 81.7%.

MS m/z (ESI): 685.1 [M+1] ⁺.

¹H NMR (400 MHz, Chloroform-d) δ 8.02 (d, J = 8.0 Hz, 1H), 7.56 (td, J = 7.6, 1.3 Hz, 1H), 7.47 - 7.43 (m, 1H), 7.25 - 7.21 (m, 4H), 7.19 - 7.16 (m, 2H), 7.15 - 7.10 (m, 1H), 6.77 - 6.73 (m, 2H), 5.01 (d, J = 6.0 Hz, 2H), 4.70 (d, J = 6.4 Hz, 2H), 4.58 (d, J = 10.9 Hz, 1H), 4.42 (d, J = 10.8 Hz, 1H), 3.31 (s, 3H), 2.34 (s, 2H), 2.28 (s, 3H), 1.96 (d, J = 10.5 Hz, 6H), 1.89 (s, 3H), 1.83 (s, 2H), 1.58 (d, J = 7.8 Hz, 2H), 1.36 (d, J = 8.3 Hz, 2H), 0.90 (s, 3H).

### Step 6

### Preparation of 2'-(benzyloxy)-4'-(2-butyl-4-oxo-1,3-diazaspiro[4.4]non-1-en-3-yl)methyl)-N-(4,5-dimethylisoxazol-3-yl)-[1,1'-biphenyl]-2-sulfonamide

Example **41-6** (50 mg, 73.01 µmol) and HCl/Dioxane (4 M, 5.00 mL) were added to a round bottom flask, and the mixture was stirred at 70°C for 1 h. The reaction liquid was cooled, concentrated, and purified by preparative HPLC to afford the title product Example **41** (14 mg, white solid), yield: 29.6%.

MS m/z (ESI): 641.0 [M+1] ⁺.

¹H NMR (400 MHz, DMSO-d6) δ 10.40 (s, 1H), 8.13 - 8.04 (m, 1H), 7.56 (d, J = 11.9 Hz, 2H), 7.22 (q, J = 4.8, 4.4 Hz, 4H), 7.15 - 7.06 (m, 2H), 6.98 (d, J = 7.7 Hz, 1H), 6.82 (s, 1H), 6.65 (d, J = 7.7 Hz, 1H), 4.96 (d, J = 12.7 Hz, 1H), 4.86 (d, J = 12.6 Hz, 1H), 4.68 (s, 2H), 2.31 (t, J = 7.5 Hz, 2H), 2.10 (s, 3H), 1.83 (d, J = 8.6 Hz, 6H), 1.66 (d, J = 7.1 Hz, 2H), 1.61 (s, 3H), 1.48 (q, J = 7.6 Hz, 2H), 1.31 - 1.26 (m, 2H), 0.82 (t, J = 7.3 Hz, 3H).

### Example 42

### 4'-(2-butyl-4-oxo-1,3-diazaspiro[4.4]non-1-en-3-yl)methyl)-N-(4,5-dimethylisoxazol-3-yl)-2'-(2,2,2-trifluoroethoxy)-[1,1'-biphenyl]-2-sulfonamide

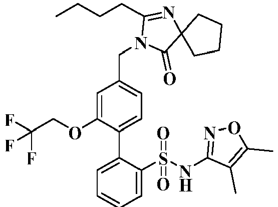

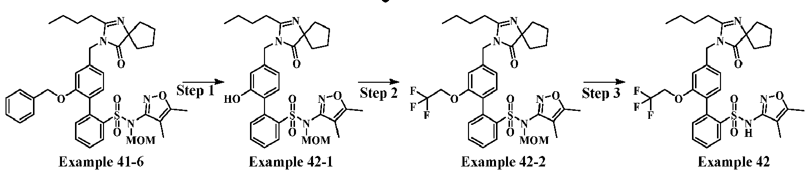

### Step 1

### Preparation of 4'-((2-butyl-4-oxo-1,3-diazaspiro[4.4]non-1-en-3-yl)methyl)-N-(4,5-dimethylisoxazol-3-yl)-2'-hydroxy-N-(methoxymethyl)-[1,1'-biphenyl]-2-sulfonamide

Example **41-6** (600 mg, 876.11 µmol), wet palladium on carbon (150 mg) and MeOH (10 mL) were added to a round bottom flask. The mixture was stirred at 20°C for 24 h under hydrogen atmosphere. The reaction liquid was filtered and concentrated to afford the title product Example **42-1** (600 mg, light yellow solid), yield: 96.0%.

MS m/z (ESI): 595.0 [M+1] ⁺.

### Step 2

### Preparation of 4'-((2-butyl-4-oxo-1,3-diazaspiro[4.4]non-1-en-3-yl)methyl)-N-(4,5-dimethylisoxazol-3-yl)-N-(methoxymethyl)-2'-(2,2,2-trifluoroethoxy)-[1,1'-biphenyl]-2-sulfonamide

Potassium carbonate (13.92 mg, 100.89 µmol) and trifluoroethyl triflate (58.51 mg, 252.22 µmol) were added to a solution of Example **42-1** (30 mg, 50.44 µmol) in DMF (1 mL), and the mixture was stirred at 80°C for 1 h. After the reaction liquid was cooled, 5 mL of water was added to quench the reaction, extracted with ethyl acetate (6 mL*3), the organic phases were combined, dried and concentrated, and the residue was purified by silica gel column chromatography with eluent system B to afford the title product Example **42-2** (26 mg, light brown solid), yield: 73.2%.

MS m/z (ESI): 677.2 [M+1] ⁺.

### Step 3

### Preparation of 4'-(2-butyl-4-oxo-1,3-diazaspiro[4.4]non-1-en-3-yl)methyl)-N-(4,5-dimethylisoxazol-3-yl)-2'-(2,2,2-trifluoroethoxy)-[1,1'-biphenyl]-2-sulfonamide

Example **42-2** (26 mg, 38.53 µmol) and HCl/Dioxane (4 M, 5 mL) were added to a round bottom flask, and the mixture was stirred at 70°C for 1 h. The reaction liquid was cooled, concentrated, and purified by preparative HPLC to afford the title product Example **42** (14 mg, white solid), yield: 39.8%.

MS m/z (ESI): 633.1 [M+1] ⁺.

¹H NMR (400 MHz, DMSO-d6) δ 10.44 (s, 1H), 8.04 (dd, J = 7.7, 1.6 Hz, 1H), 7.57 (d, J = 11.1 Hz, 2H), 7.21 - 7.16 (m, 1H), 6.99 (s, 2H), 6.71 (d, J = 7.5 Hz, 1H), 4.71 (s, 2H), 4.57 (d, J = 11.3 Hz, 1H), 4.38 (t, J = 10.4 Hz, 1H), 2.36 (t, J = 7.5 Hz, 2H), 2.18 (s, 3H), 2.00 (d, J = 7.7 Hz, 1H), 1.91 - 1.81 (m, 5H), 1.69 (d, J = 8.5 Hz, 2H), 1.63 (s, 3H), 1.53 (p, J = 7.5 Hz, 2H), 1.33 - 1.28 (m, 2H), 0.83 (t, J = 7.5 Hz, 3H).

### Example 43

### 2-(2-butyl-1-((2'-(N-(4,5-dimethylisoxazol-3-yl)sulfamoyl)-2-(ethoxymethyl)-[1,1'-biphenyl]-4-yl)methyl-d2)-4-methyl-6-oxo-1,6-dihydropyrimidin-5-yl)-N,N-dimethylethylthioamide

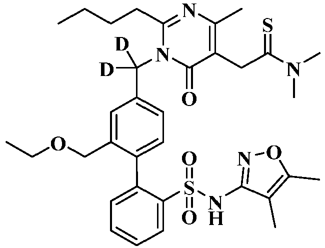

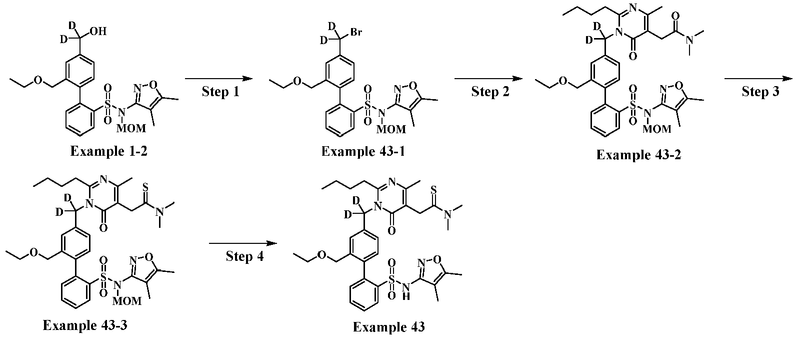

### Step 1

### Preparation of 4'-(bromomethyl-d2)-N-(4,5-dimethylisoxazol-3-yl)-2'-(ethoxymethyl)-N-(methoxymethyl)-[1,1'-biphenyl]-2-sulfonamide

Carbon tetrabromide (1.60 g, 4.86 mmol) and triphenylphosphine (1.02 g, 3.89 mmol) were added to a solution of Example **1-2** (900 mg, 1.95 mmol) in DCM (20 mL) under nitrogen protection at 0°C, the mixture was stirred at 20°C for 1 h. The reaction liquid was quenched by adding water (30 mL), extracted with dichloromethane (30 mL*3), the organic phases were combined, dried and concentrated, and the residue was purified by silica gel column chromatography with eluent system B to afford the title product Example **43-1** (1 g, white solid), yield: 97.8%.

MS m/z (ESI): 525.1 [M+1] ⁺.

### Step 2

### Preparation of 2-(2-butyl-1-((2'-(N-(4,5-dimethylisoxazol-3-yl)-N-(methoxymethyl)sulfamoyl)-2-(ethoxymethyl)-[1,1'-biphenyl]-4-yl)methyl-d2)-4-methyl-6-oxo-1,6-dihydropyrimidin-5-yl)-N,N-dimethylacetamide

Potassium carbonate (94.55 mg, 685.13 µmol) and 2-(2-butyl-4-methyl-6-oxo-1H-pyrimidin-5-yl)-N,N-dimethylacetamide (86.09 mg, 342.56 µmol) were added to a solution of Example **43-1** (180 mg, 342.56 µmol) in MeCN (6 mL), the mixture was stirred at 70°C for 1 h. The reaction liquid was quenched by adding water (20 mL), extracted with dichloromethane (20 mL*3), the organic phases were combined, dried and concentrated, and the residue was purified by silica gel column chromatography with eluent system B to afford the title product Example **43-2** (80 mg, light yellow solid), yield: 33.6%.

MS m/z (ESI): 696.3 [M+1] ⁺.

### Step 3

### Preparation of 2-(2-butyl-1-((2'-(N-(4,5-dimethylisoxazol-3-yl)-N-(methoxymethyl)sulfamoyl)-2-(ethoxymethyl)-[1,1'-biphenyl]-4-yl)methyl-d2)-4-methyl-6-oxo-1,6-dihydropyrimidin-5-yl)-N,N-dimethylethylthioamide

Lawson's reagent (139.34 mg, 344.90 µmol) was added to a solution of Example **43-2** (80 mg, 114.97 µmol) in toluene (5 mL), and the mixture was stirred at 70°C under nitrogen protection for 1 h. The reaction liquid was cooled and then quenched by adding water (10 mL), extracted with dichloromethane (10 mL*3), the organic phases were combined, dried and concentrated, and the residue was purified by silica gel column chromatography with eluent system B to afford the title product Example **43-3** (50 mg, light yellow solid), yield: 61.1%.

MS m/z (ESI): 712.0 [M+1] ⁺.

### Step 4

### Preparation of 2-(2-butyl-1-((2'-(N-(4,5-dimethylisoxazol-3-yl)sulfamoyl)-2-(ethoxymethyl)-[1,1'-biphenyl]-4-yl)methyl-d2)-4-methyl-6-oxo-1,6-dihydropyrimidin-5-yl)-N,N-dimethylethylthioamide

Example **43-3** (50 mg, 70.23 µmol) and HCl/Dioxane (4 M, 5 mL) were added to a round bottom flask, and the mixture was stirred at 70°C for 1 h. The reaction liquid was cooled, concentrated, and purified by preparative HPLC to afford the title product Example **43** (9 mg, white solid), yield: 18.8%.

MS m/z (ESI): 668.1 [M+1] ⁺.

¹H NMR (400 MHz, DMSO-d6) δ 10.51 (s, 1H), 8.04 (dd, J = 7.5, 1.8 Hz, 1H), 7.60 (d, J = 7.9 Hz, 2H), 7.25 (s, 1H), 7.17 (d, J = 7.1 Hz, 1H), 6.97 (dd, J = 8.3, 1.7 Hz, 1H), 6.89 (d, J = 7.8 Hz, 1H), 4.02 - 3.93 (m, 2H), 3.80 (s, 2H), 3.47 (s, 3H), 3.42 (s, 3H), 3.23 - 3.12 (m, 2H), 2.65 (d, J = 8.1 Hz, 2H), 2.18 (d, J = 5.1 Hz, 6H), 1.64 (s, 3H), 1.58 (q, J = 7.7 Hz, 2H), 1.30 (d, J = 7.5 Hz, 2H), 0.98 (t, J = 7.0 Hz, 3H), 0.84 (t, J = 7.4 Hz, 3H).

### Example 44

### 4'-((2-butyl-4-oxo-1,3-diazaspiro[4.4]non-1-en-3-yl)methyl-d2)-N-(4-chloro-5-methylisoxazol-3-yl)-2'-(ethoxymethyl)-[1,1'-biphenyl]-2-sulfonamide

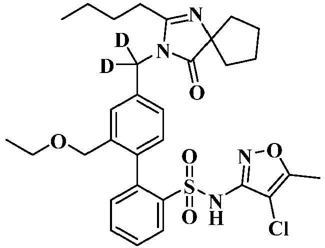

### Method I:

Synthesis method of Example **44** referred to the synthesis method of Example **1,** 4-chloro-5-methylisoxazoleamine was used in stead of 4,5-dimethylisoxazoleamine to afford Example **44** (51 mg, white solid), yield: 50.3%.

### Method II:

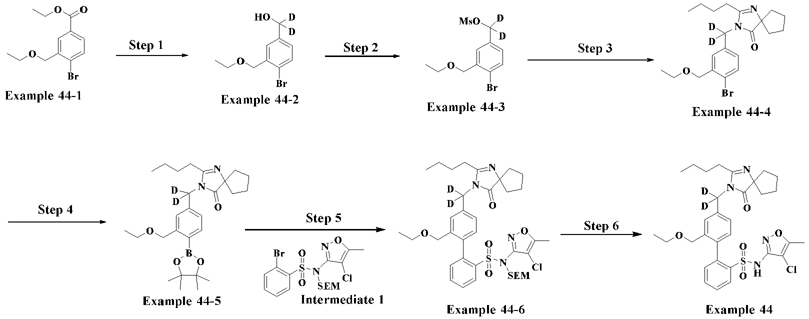

### Step 1

### Preparation of (4-bromo-3-(ethoxymethyl)phenyl)-d2methanol

Example **44-1**(1.0g, 3.48 mmol) (referring to WO 2010114801A1 for the preparation method) and deuterated lithium aluminum tetrahydrogen (219.3 mg, 5.22 mmol) were dissolved in tetrahydrofuran (20 mL), and the reaction liquid was cooled to 0°C and reacted under stirring for 2 h. Saturated brine (50 mL) was added to the reaction liquid, and the mixture was extracted with ethyl acetate (2 × 100 mL). The organic phases were combined, dried over anhydrous sodium sulfate, concentrated, and purified with silica gel chromatography column (petroleum ether/ethyl acetate system) to afford Example **44-2** (850 mg, 98%).

MS m/z (ESI): 248.1 [M+1] ⁺.

### Step 2

### Preparation of 4-bromo-3-(ethoxymethyl)benzyl-d2methanesulfonate

Under ice bath conditions, methylsulfonyl chloride (433.4 mg, 3.78 mmol) and diisopropylethylamine (1.33 g, 10.32 mmol) were added to a solution of Example **44-2** (50 mg, 3.44 mmol) in dichloromethane (20 mL), and the reaction liquid was warmed to room temperature and stirred for 1 h. The reaction liquid was concentrated to afford crude product Example **44-3** (1.1 g, 98%), which was directly used in the next reaction.

MS m/z (ESI): 326.2 [M+1] ⁺.

### Step 3

### Preparation of 3-(4-bromo-3-(ethoxymethyl)phenyl)methyl-d2)-2-butyl-1,3-diazaaspirin[4.4]non1-en-4-one

Example **44-3** (1.1 g, 3.38 mmol) was dissolved in DMF (15 mL), potassium carbonate (1.03 g, 7.44 mmol) and 2-butyl-1,3-diazaspiro-[4,4]non-1-en-4one (858.4 mg, 3.72 mmol) were added under ice bath conditions, and the reaction liquid was stirred at room temperature for 2 h. The reaction liquid was concentrated, and the crude product was purified by HPLC to afford Example **44-4** (1.2 g, 86%).

MS m/z (ESI): 424.4 [M+1] ⁺.

### Step 4

### Preparation of 2-butyl-3-((3-(ethoxymethyl)-4-(4,4,5,5-tetramethyl-1,3,2-dioxaborolan-2-yl)phenyl)methyl-d2)-1,3-diazabispiro[4.4]non-1-ene-4-one

Compound **44-4** (1.2 g, 2.86 mmol) was dissolved in 15 mL of 1,4 dioxane, and bis(pinacolato)diboron (0.87 g, 3.4 mmol), 1,1 '- bis(diphenylphosphino)ferrocene palladium dichloride (102.9 mg, 0.14 mmol) and potassium acetate (0.84 g, 8.56 mmol) were added. The mixture was heated to 80°C under nitrogen protection and reacted under stirring for 3 h. The reaction liquid was cooled to room temperature, saturated sodium chloride solution (50 mL) was added, the mixture was extracted with ethyl acetate (100 mL × 3), the organic phases were combined, dried over anhydrous sodium sulfate, filtered and concentrated. The residue was purified by silica gel chromatography with an eluent system (ethyl acetate/petroleum ether = 10-50%) to afford Example **44-5** (1.0 g, 80.0%).

MS m/z (ESI): 471.5 [M+1] ⁺.

### Step 5

### Preparation of 4'-(2-butyl-4-oxo-1,3-diazaspiro[4.4]non-1-en-3-ylmethyl-d2)-n-(4-chloro-5-methylisoxazol-3-yl)-2'-(ethoxymethyl)-n-(2-trimethylsilylethoxy) methyl)-[1,1'-biphenyl] -2-sulfonamide

Example **44-5** (0.5 g, 1.07 mmol) was dissolved in 20 mL of 1,4 dioxane and water (2 ml), and intermediate **1** (0.4 g, 1.07 mmol), 1,1'-bis(diphenylphosphino)ferrocene palladium dichloride (0.039 g, 0.053 mmol) and potassium carbonate (0.3 g, 3.2 mmol) were added. The mixture was heated to 90°C under nitrogen protection and reacted under stirring for 16 h. The reaction liquid was cooled to room temperature, saturated sodium chloride solution (50 mL) was added, the mixture was extracted with ethyl acetate (100 mL × 3), the organic phases were combined, dried over anhydrous sodium sulfate, filtered and concentrated. The residue was purified by silica gel chromatography with an eluent system (ethyl acetate/petroleum ether = 10-50%) to afford Example **44-6** (0.45 g, gray solid), yield: 66.0%.

MS m/z (ESI): 746.4[M+1] ⁺.

### Step 6

### Preparation of 4'-(2-butyl-4-oxo-1,3-diazaspiro[4.4]non-1-en-3-ylmethyl-d2)-n-(4-chloro-5-methylisoxazol-3-yl)-2'-(ethoxymethyl)-[1,1'-biphenyl]-2-sulfonamide

Example **44-6** (0.45 g, 0.7 mmol) was dissolved in 10 mL of 4M HCl/dioxane. The mixture was heated to 70°C and reacted under stirring for 2 h. The reaction liquid was cooled to room temperature, saturated sodium chloride solution (50 mL) was added, the mixture was extracted with ethyl acetate (100 mL × 3), the organic phases were combined, dried over anhydrous sodium sulfate, filtered and concentrated. The residue was purified by silica gel chromatography with an eluent system p-HPLC(FA) to afford Example 44 (0.2 g, 50.0%).

MS m/z (ESI): 615.2 [M+1] ⁺.

¹H NMR (400 MHz, DMSO-d6) δ 8.05 - 7.97 (m, 1H), 7.54 (s, 2H), 7.22 - 7.12 (m, 2H), 6.99 (s, 2H), 4.08 (d, J = 13.1 Hz, 1H), 3.99 (d, J = 13.1 Hz, 1H), 3.21 (ddd, J = 9.4, 7.0, 3.6 Hz, 2H), 2.35 (t, J = 7.5 Hz, 2H), 2.25 (s, 3H), 1.85 (d, J = 8.5 Hz, 6H), 1.69 (d, J = 8.8 Hz, 2H), 1.50 (q, J = 7.7 Hz, 2H), 1.28 (d, J = 7.6 Hz, 2H), 1.01 (t, J = 6.9 Hz, 3H), 0.82 (t, J = 7.3 Hz, 3H).

### Example 45

### 2-[4-[(2-butyl-4-oxo-1,3-diazaspiro[4.4]non-1-en-3-yl)methyl]-2-(ethoxymethyl)phenyl]-N-(4-chloro-5-methyl-isoxazol-3-yl)benzsulfamide

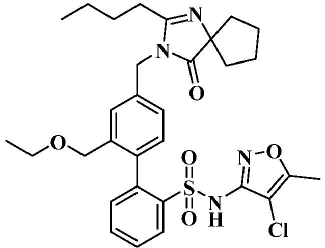

### Method I:

Synthesis method of Example **45** referred to the synthesis method of Example **1,** 4-chloro-5-methylisoxazoleamine was used in stead of 4,5-dimethylisoxazoleamine to afford Example **45** (56.6 mg, white solid), yield: 45.8%.

### Method II:

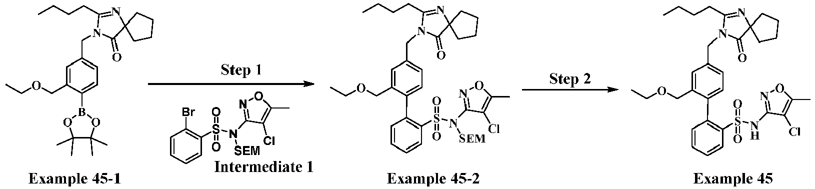

### Step 1

### Preparation of 4'-(2-butyl-4-oxo-1,3-diazaspiro[4.4]non-1-en-3-ylmethyl)-n-(4-chloro-5-methylisoxazol-3-yl)-2'-(ethoxymethyl)-n-(2-trimethylsilylethoxy)methyl)-[1,1'-biphenyl]-2-sulfonamide

Example **45-1** (0.3 g, 0.64 mmol) (referring to WO 2010114801 A1 for the preparation method) was dissolved in 10 mL of 1,4 dioxane and water (1ml), and intermediate **1** (0.24 g, 0.64 mmol), 1,1'-bis(diphenylphosphino)ferrocene palladium dichloride (0.023 g, 0.032mmol) and potassium carbonate (0.18g, 1.9 mmol) were added. The mixture was heated to 90°C under nitrogen protection and reacted under stirring for 16 h. The reaction liquid was cooled to room temperature, saturated sodium chloride solution (50 mL) was added, the mixture was extracted with ethyl acetate (100 mL × 3), the organic phases were combined, dried over anhydrous sodium sulfate, filtered and concentrated. The residue was purified by silica gel chromatography with an eluent system (ethyl acetate/petroleum ether = 10-50%) to afford Example **45-2** (0.27 g, gray solid), yield: 66.0%.

MS m/z (ESI): 744.4[M+1] ⁺.

### Step 2

### Preparation of 4'-(2-butyl-4-oxo-1,3-diazaspiro[4.4]non-1-en-3-ylmethyl)-n-(4-chloro-5-methylisoxazol-3-yl)-2'-(ethoxymethyl)-[1,1'-biphenyl]-2-sulfonamide

Example **45-2** (0.27 g, 0.7 mmol) was dissolved in 10 mL of 4M HCl/dioxane. The mixture was heated to 70°C and reacted under stirring for 2 h. The reaction liquid was cooled to room temperature, saturated sodium chloride solution (50 mL) was added, the mixture was extracted with ethyl acetate (100 mL × 3), the organic phases were combined, dried over anhydrous sodium sulfate, filtered and concentrated. The residue was purified by silica gel chromatography with an eluent system p-HPLC(FA) to afford Example **45** (0.12 g, 54.5%).

MS m/z (ESI): 612.8 [M+1] ⁺.

¹H NMR (400 MHz, DMSO-*d*₆) δ 7.97 - 7.95 (m, 1H), 7.37 - 7.31 (m, 2H), 7.09 (s, 1H), 6.97 (d, *J* = 7.6 Hz, 1H), 6.93 - 6.88 (m, 2H), 4.70 (s, 2H), 4.08 (d, *J* = 13.2 Hz, 1H), 3.93 (d, *J* = 13.2 Hz, 1H), 3.24 - 3.15 (m, 2H), 2.36 (t, *J* = 7.6 Hz, 2H), 2.12 (s, 3H), 1.88 - 1.81 (m, 6H), 1.72 - 1.65 (m, 2H), 1.57 - 1.49 (m, 2H), 1.33 - 1.27 (m, 2H), 1.01 (t, *J* = 6.8 Hz, 3H), 0.83 (t, *J* = 7.2 Hz, 3H).

### Example 46

### 2-[4-[(2-butyl-4-oxo-1,3-diazaspiro[4.4]non-1-en-3-yl)methyl]-2-(ethoxymethyl)phenyl]-N-(4-bromo-5-methyl-isoxazol-3-yl)benzsulfamide

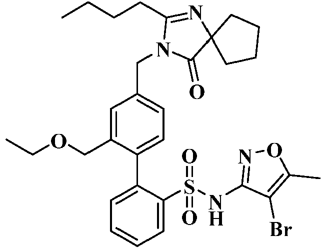

Synthesis method of Example **46** referred to the synthesis method of Example **1,** 4-bromo-5-methylisoxazoleamine was used in stead of 4,5-dimethylisoxazoleamine to afford Example **46** (40.8 mg, white solid), yield: 31.1%.

MS m/z (ESI): 656.8 [M+1] ⁺.

¹H NMR (400 MHz, DMSO-*d*₆) δ 7.98 - 7.95 (m, 1H), 7.36 - 7.31 (m, 2H), 7.09 (s, 1H), 6.98 (d, *J* = 8.0 Hz, 1H), 6.92 - 6.89 (m, 2H), 4.70 (s, 2H), 4.08 (d, *J* = 13.2 Hz, 1H), 3.93 (d, *J* = 13.2 Hz, 1H), 3.22 - 3.17 (m, 2H), 2.37 (t, *J* = 7.2 Hz, 2H), 2.12 (s, 3H), 1.90 - 1.81 (m, 6H), 1.73 - 1.65 (m, 2H), 1.57 - 1.49 (m, 2H), 1.33 - 1.27 (m, 2H), 1.01 (t, *J* = 7.2 Hz, 3H), 0.83 (t, *J* = 7.2 Hz, 3H).

### Example 47

### 4'-(2-butyl-4-chloro-5-(hydroxymethyl)-1H-imidazol-1-yl)methyl)-N-(4-chloro-5-methylisoxazol-3-yl)-2'-(ethoxymethyl)-[1,1'-biphenyl]-2-sulfonamide

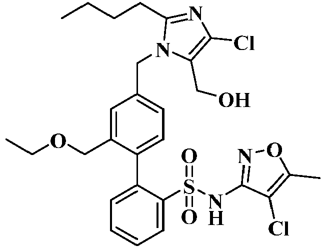

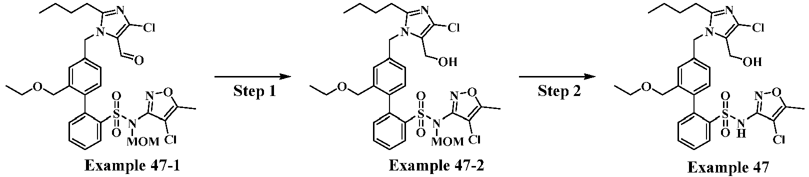

Synthesis method of Example **47-1** referred to the synthesis method of Example **43-5,** 2-bromo-N-(4-chloro-5-methylisoxazol-3-yl)-N-(methoxymethyl)benzsulfamide was used instead of 2-bromo-N-(4,5-dimethylisoxazol-3-yl)-N-(methoxymethyl)benzsulfamide, and 2-butyl-4-chloro-1H-imidazole-5-carbaldehyde was used instead of 2-(2-butyl-4-methyl-6-oxo-1H-pyrimidin-5-yl)-N,N-dimethylacetamide, to afford Example **47-1** (50 mg, white solid), yield: 44.1%.

MS m/z (ESI): 615.2 [M+1] ⁺.

### Step 1

### Preparation of 4'-(2-butyl-4-chloro-5-formyl-1H-imidazol-1-yl)methyl)-N-(4-chloro-5-methylisoxazol-3-yl)-2'-(ethoxymethyl)-N-(methoxymethyl)-[1,1'-biphenyl]-2-sulfonamide

Sodium borohydride (6 mg, 153.94 µmol) was added to a solution of Example **47-1** (50 mg, 76.97 µmol) in MeOH (6 mL) , and the mixture was stirred at 20°C for 1 h. The organic phases were combined, dried and concentrated, and the residue was purified by silica gel column chromatography with eluent system B to afford the title product **47-2** (45 mg, light yellow solid), yield: 90.3%.

MS m/z (ESI): 651.1 [M+1] ⁺.

### Step 2

### Preparation of 4'-(2-butyl-4-chloro-5-(hydroxymethyl)-1H-imidazol-1-yl)methyl)-N-(4-chloro-5-methylisoxazol-3-yl)-2'-(ethoxymethyl)-[1,1'-biphenyl]-2-sulfonamide

**47-2** (50 mg, 76.73 µmol) and hydrogen chloride in dioxane (4 M, 5 mL) were added to a round bottom flask, and the mixture was stirred at 70°C for 1 h. The reaction liquid was cooled, concentrated, and purified by preparative HPLC to afford the title Example **47** (19 mg, white solid), yield: 40.8%.

MS m/z (ESI): 607.1 [M+1] ⁺.

¹H NMR (400 MHz, DMSO-d6) δ 11.14 (s, 1H), 8.02 (dd, J = 7.5, 1.9 Hz, 1H), 7.58 (s, 2H), 7.21 (s, 1H), 7.14 (s, 1H), 6.97 (d, J = 7.9 Hz, 1H), 6.88 (d, J = 7.9 Hz, 1H), 5.29 (s, 2H), 5.27 (s, 1H), 4.34 (d, J = 4.0 Hz, 2H), 4.09 - 3.97 (m, 2H), 3.25 - 3.15 (m, 2H), 2.54 (dd, J = 8.8, 6.4 Hz, 2H), 2.27 (s, 3H), 1.51 (q, J = 7.6 Hz, 2H), 1.30 - 1.25 (m, 2H), 0.98 (t, J = 7.0 Hz, 3H), 0.83 (t, J = 7.4 Hz, 3H).

### Example 48

### 4'-((2-butyl-4-oxo-1,3-diazaspiro[4.4]non-1-en-3-yl)methyl-d2)-N-(4-fluoro-5-methylisoxazol-3-yl)-2'-(ethoxymethyl)-[1,1'-biphenyl]-2-sulfonamide

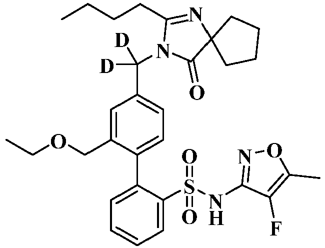

### Synthesis method of Example 48 referred to the synthesis method of Example 1, 4-fluoro-5-methylisoxazoleamine was used in stead of 4,5-dimethylisoxazoleamine to afford Example 48 (11 mg, white solid), yield: 40.3%.

MS m/z (ESI): 599.3 [M+1] ⁺.

¹H NMR (400 MHz, DMSO-d6) δ 8.05 - 7.97 (m, 1H), 7.54 (s, 2H), 7.22 - 7.12 (m, 2H), 6.99 (s, 2H), 4.08 (d, J = 13.1 Hz, 1H), 3.99 (d, J = 13.1 Hz, 1H), 3.21 (ddd, J = 9.4, 7.0, 3.6 Hz, 2H), 2.35 (t, J = 7.5 Hz, 2H), 2.25 (s, 3H), 1.85 (d, J = 8.5 Hz, 6H), 1.69 (d, J = 8.8 Hz, 2H), 1.50 (q, J = 7.7 Hz, 2H), 1.28 (d, J = 7.6 Hz, 2H), 1.01 (t, J = 6.9 Hz, 3H), 0.82 (t, J = 7.3 Hz, 3H).

### Example 49

### 4'-((2-butyl-4-oxo-1,3-diazospiro[4.4]non-1-en-3-yl)methyl)-N-(4-chloro-5-methylisoxazol-3-yl)-2'-(ethoxydeuteriummethyl)-[1,1'-biphenyl]-2-sulfonamide

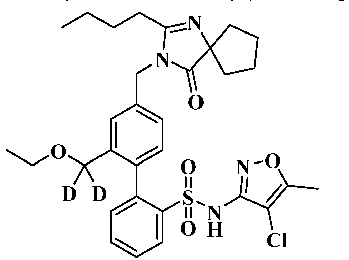

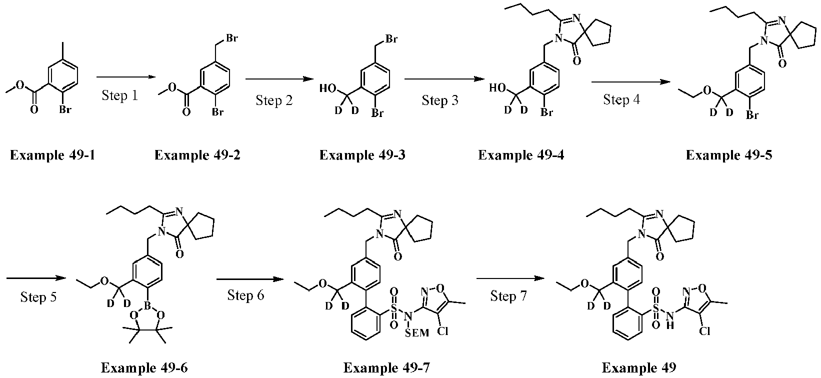

### Step 1

### Preparation of methyl 2-bromo-5-(bromomethyl)benzoate

N-bromosuccinimide (854.68 mg, 4.80 mmol) and Example **49-1** (1.0 g, 4.37 mmol) were dissolved in carbon tetrachloride (5 mL), then benzoyl oxide (105.74 mg, 436.55 µmol) was added to the reaction liquid, and the reaction liquid was reacted at 80°C for 16 h under stirring. Saturated sodium chloride (10 mL) was added to the reaction liquid, and the mixture was extracted with dichloromethane (10 mL × 3), the organic phases were combined, dried, and concentrated to afford a crude product. The crude product was purified with column (petroleum ether/ethyl acetate system) to afford the target molecule Example **49-2** (1.1 g, 3.57 mmol, 81.82% yield).

### Step 2

### Preparation of (2-bromo-5-(bromomethyl)phenyl)deuteriummethan-ol

Deuterated lithium aluminium hydride (104.72 mg, 2.44 mmol) was dissolved in tetrahydrofuran (3 mL), then Example **49-2** (500 mg, 1.62 mmol) was added, and the reaction liquid was reacted at room temperature for 1 h with stirring. Water (0.1 mL), 15% sodium hydroxide solution (0.1 mL) and water (0.3 mL) were added to the reaction liquid in sequence, the mixture was stirred for 0.5 h and then filtered. The filter cake was washed with dichloromethane (10 mL × 3), the filtrate was dried and concentrated to afford the target molecule Example **49-3** (310 mg, 1.10 mmol, 67.72% yield).

### Step 3

### Preparation of 3-(4-bromo-3-(hydroxymethyl2)benzyl)-2-butyl-1,3-diazaspiro [4.4]non-1-en-4-one

2-butyl-1,3-diazaspiro[4.4]non-1-en-4-one (62.01 mg, 319.18 µmol) and Example **49-3** were dissolved in acetonitrile (2 mL), then potassium carbonate (29.36 mg, 212.79 µmol) was added, and the reaction liquid was reacted at 80°C for 3 h under stirring. After the reaction was completed, saturated sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **49-4** (18 mg, 45.53 µmol, 21.40% yield).

MS m/z (ESI): 395.2[M+1] ⁺.

### Step 4

### Preparation of 3-(4-bromo-3-(ethoxymethylD2)benzyl)-2-butyl-1,3-diazaspiro [4.4]non-1-en-4-one

Example **49-4** (500 mg, 1.26 mmol) was dissolved in tetrahydrofuran (2 mL), then sodium hydride (151.76 mg, 3.79 mmol, 60% purity) was added, and the reaction liquid was reacted at room temperature for 0.5 h under stirring, then ethyl iodide (986.30 mg, 6.32 mmol) was added, and the reaction liquid was reacted at room temperature for 1.5 h under stirring. After the reaction was completed, saturated sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **49-5** (210 mg, 496.01 µmol, 39.22% yield).

MS m/z (ESI): 423.2[M+1] ⁺.

### Step 5

### Preparation of 2-butyl-3-(3-(ethoxymethyl-d2)-4-(4,4,5,5-tetramethyl-1,3,2-dioxaborolan-2-yl)benzyl)-1,3-diazaspiro[4.4]non-1-en-4-one

Example **49-5** (100 mg, 236.19 µmol), bis(pinacolato)diboron (71.97 mg, 283.43 µmol), [1,1'-bis(diphenylphosphino)ferrocene]palladium dichloride dichloromethane complex (19.27 mg, 23.62 µmol) and potassium acetate (45.35 mg, 472.39 µmol) were dissolved in dioxane (5 mL), and the reaction liquid was reacted at 90°C for 16 h under stirring. Saturated aqueous sodium chloride (10 mL) solution was added, the mixture was extracted with dichloromethane (10 mL × 3), the organic phases were combined, dried and concentrated to afford the target molecule crude Example **49-6** (105 mg, 223.19 µmol, 94.50% yield) . The crude product was used directly in the next step without purification.

MS m/z (ESI): 471.2[M+1] ⁺.

### Step 6

### Preparation of 4'-((2-butyl-4-oxo-1,3-diazaspiro[4.4]non-1 -en-3-yl)methyl)-N-(4-chloro-5-methylisoxazol-3-yl)- 2'-(ethoxymethyl-d2)-N-(((2-(trimethylsilyl)ethoxy)methyl)-[1,1'-biphenyl]-2-sulfonamide

Intermediate 1 (93 mg, 235.05 µmol), Example **49-6** (110.58 mg, 235.05 µmol), [1,1'-bis(diphenylphosphino)ferrocene]palladium dichloride dichloromethane complex (21.53 mg, 23.51 µmol) and cesium carbonate (229.88 mg, 705.16 µmol) were dissolved in dioxane and water (2.5 mL, 4 : 1). The reaction liquid was reacted at 100°C for 1 h under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example 49-7 (106 mg, 160.79 µmol, 68.41% yield).

MS m/z (ESI): 745.3[M+1] ⁺.

### Step 7

### 4'-((2-butyl-4-oxo-1,3-diazospiro[4.4]non-1-en-3-yl)methyl)-N-(4-chloro-5-methylisoxazol-3-yl)- 2'-(ethoxydeuteriummethyl)-[1,1'-biphenyl] -2-sulfonamide

Synthesis method of Example **49** referred to the synthesis method of Example **33,** Example **49-7** was used as raw material to afford the title compound Example **40** (32 mg, 33.5%).

MS m/z (ESI): 615.2[M+1] ⁺.

¹H NMR (400 MHz, DMSO) δ 8.01 (dd, *J* = 16.0, 14.4 Hz, 1H), 7.60 (t, *J* = 29.2 Hz, 2H), 7.15 (d, *J* = 11.1 Hz, 2H), 7.00 (s, 2H), 4.83 - 4.66 (m, 2H), 3.28 - 3.13 (m, 2H), 2.36 (t, *J* = 7.5 Hz, 2H), 2.28 (s, 3H), 1.86 (d, *J* = 6.4 Hz, 6H), 1.71 (d, *J* = 8.0 Hz, 2H), 1.52 (dt, *J=* 15.2, 7.5 Hz, 2H), 1.34 - 1.25 (m, 2H), 1.01 (t, *J* = 7.0 Hz, 3H), 0.88 - 0.75 (m, 3H).

### Example 50

### 2-(5-((2-butyl-4-oxo-1,3-diazaspiro[4.4]non-1-en-3-yl)methyl)-3-(ethoxymethyl)pyridin-2-yl)-N-(4,5-dimethylisoxazol-3-yl)benzsulfamide

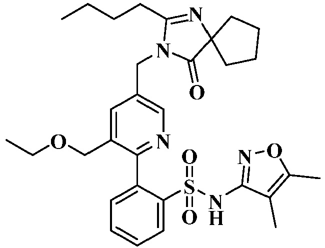

### Synthesis method of Example 50 referred to the synthesis method of Example 1 to afford Example 50 (51 mg, white solid), yield: 50.3%.

MS m/z (ESI): 594.1 [M+1] ⁺.

¹H NMR (400 MHz, DMSO-d6) δ 8.25 (s, 1H), 8.19 (s, 1H), 8.01 - 7.97 (m, 1H), 7.50 (s, 2H), 7.16 (d, J = 26.4 Hz, 2H), 4.80 (s, 2H), 4.30 (d, J = 14.9 Hz, 1H), 4.05 (d, J = 14.4 Hz, 1H), 3.25 (d, J = 7.1 Hz, 2H), 2.38 (t, J = 7.4 Hz, 2H), 2.11 (s, 3H), 1.86 (t, J = 5.2 Hz, 6H), 1.69 (d, J = 8.6 Hz, 2H), 1.60 (s, 3H), 1.52 (t, J = 7.5 Hz, 2H), 1.31 (d, J = 7.7 Hz, 2H), 1.02 (t, J = 7.0 Hz, 3H), 0.83 (t, J = 7.3 Hz, 3H).

### Example 51

### 2-(5-((2-butyl-4-oxo-1,3-diazaspiro[4.4]non-1-en-3-yl)methyl)-3-methylpyridin-2-yl)-N-(4,5-dimethylisoxazol-3-yl)benzsulfamide

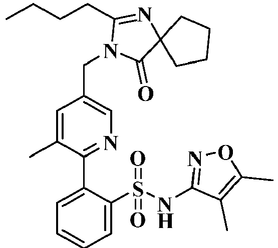

Synthesis method of Example **51** referred to the synthesis method of Example **41,** methyl 6-bromo-5-methylnicotinate was used in stead of **41b** to afford Example 51 (21 mg, white solid), yield: 50.3%.

MS m/z (ESI): 550.2 [M+1] ⁺.

¹H NMR (400 MHz, DMSO-d6) δ 8.16 (s, 1H), 8.00 (d, J = 7.8 Hz, 1H), 7.58 (dt, J = 15.1, 7.4 Hz, 2H), 7.40 (s, 1H), 7.21 (d, J = 7.6 Hz, 2H), 4.74 (s, 2H), 2.40 (t, J = 7.5 Hz, 2H), 2.16 (s, 3H), 2.01 (s, 3H), 1.86 (q, J = 6.6 Hz, 6H), 1.68 (t, J = 5.5 Hz, 2H), 1.65 (s, 3H), 1.56 - 1.50 (m, 2H), 1.33 - 1.28 (m, 2H), 0.83 (t, J = 7.3 Hz, 3H).

### Example 52

### 4'-((2-butyl-4-oxo-1,3-diazaspiro[4.4]non-1-en-3-yl)methyl)-N-(4-chloro-5-methylisoxazol-3-yl)-2'-(cyclopropoxymethyl)-[1,1'-biphenyl]-2-sulfonamide

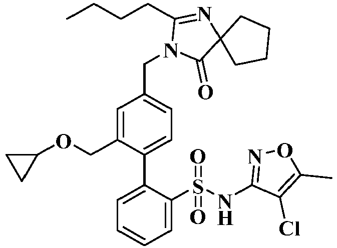

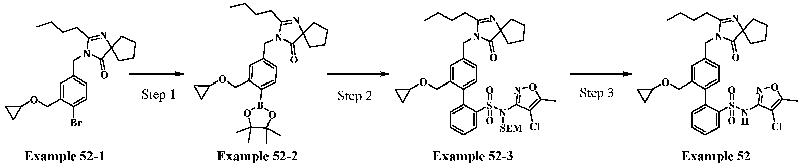

### Step 1

### Preparation of 2-butyl-3-(3-(cyclopropoxymethyl)-4-(4,4,5,5-tetramethyl-1,3,2-dioxaborolan-2-yl)benzyl)-1,3-diazaspiro[4.4]non-1-en-4-one

Example **52-1** (240 mg, 553.79 µmol) (referring to document WO 2010135350 A2 for the synthesis method), bis(pinacolato)diboron (168.8 mg, 664.54 µmol), [1,1'-bis(diphenylphosphino)ferrocene ]palladium dichloride dichloromethane complex (45.19 mg, 55.38 µmol) and potassium acetate (162.8 mg, 1.66 mmol) were dissolved in dioxane (5 mL), and the reaction liquid was reacted at 90°C for 16 h under stirring. Saturated aqueous sodium chloride (10 mL) solution was added, the mixture was extracted with dichloromethane (10 mL × 3), the organic phases were combined, dried and concentrated to afford the target molecule crude Example **52-2** (240 mg, 499.54 µmol, 90.2% yield) . The crude product was used directly in the next step without purification.

MS m/z (ESI): 481.2[M+1] ⁺.

### Step 2

### 4'-((2-butyl-4-oxo-1,3-diazaspiro[4.4]non-1-en-3-yl)methyl)-N-(4-chloro-5-methylisoxazol-3-yl)- 2'-(cyclopropoxymethyl)-N-(((2-(trimethylsilyl)ethoxy)methyl)-[1,1'-biphenyl] -2-sulfonamide

Intermediate 1 (240.71 mg, 499.54 µmol), Example **52-2** (240 mg, 499.54 µmol), [1,1'-bis(diphenylphosphino)ferrocene]palladium dichloride dichloromethane complex (40.76 mg, 49.95 µmol) and cesium carbonate (488.55 mg, 1.50 mmol) were dissolved in dioxane/water (2.5 mL, 4 : 1). The reaction liquid was reacted at 100°C for 1 h under microwave under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **52-3** (210 mg, 277.99 µmol, 55.65% yield).

MS m/z (ESI): 755.2[M+1] ⁺.

### Step 3

### 4'-((2-butyl-4-oxo-1,3-diazaspiro[4.4]non-1-en-3-yl)methyl)-N-(4-chloro-5-methylisoxazol-3-yl)- 2'-(cyclopropoxymethyl)-[1,1'-biphenyl]-2-sulfonamide

Example **52-3** was dissolved in tetrahydrofuran (2 mL), then tetrabutylamine fluoride (1 M, 2 mL) was added, the reaction liquid was reacted at 70°C for 2 h under stirring, saturated aqueous sodium chloride (10 mL) solution was added, the mixture was extracted with dichloromethane (10 mL × 3), the organic phases were combined, dried and concentrated to afford a crude product, and the crude product was purified (HCOOH) to afford Example **52** (78 mg, 124.76 µmol, 30.40% yield)

MS m/z (ESI): 625.2[M+1] ⁺.

¹H NMR (400 MHz, DMSO) δ 8.02 (d, *J* = 6.3 Hz, 1H), 7.54 (s, 2H), 7.11 (s, 2H), 6.99 (d, *J* = 7.7 Hz, 2H), 4.72 (s, 2H), 4.08 (dd, *J* = 44.2, 12.4 Hz, 2H), 3.09 (s, 1H), 2.34 (dd, *J* = 20.3, 12.7 Hz, 2H), 2.25 (s, 3H), 1.85 (d, *J =* 7.4 Hz, 6H), 1.69 (d, *J* = 6.6 Hz, 2H), 1.52 (dt, *J* = 15.2, 7.7 Hz, 2H), 1.29 (dq, *J* = 14.5, 7.3 Hz, 2H), 0.82 (t, *J* = 7.3 Hz, 3H), 0.45 - 0.11 (m, 4H).

### Example 53

### 4'-((2-butyl-4-oxo-1,3-diazaspiro[4.4]non-1-en-3-yl)methyl)-N-(4-chloro-5-methylisoxazol-3-yl)-2'-(methoxymethyl)-[1,1'-biphenyl]-2-sulfonamide

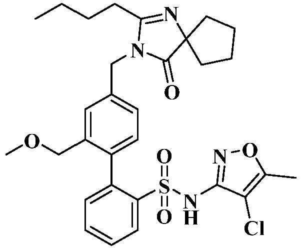

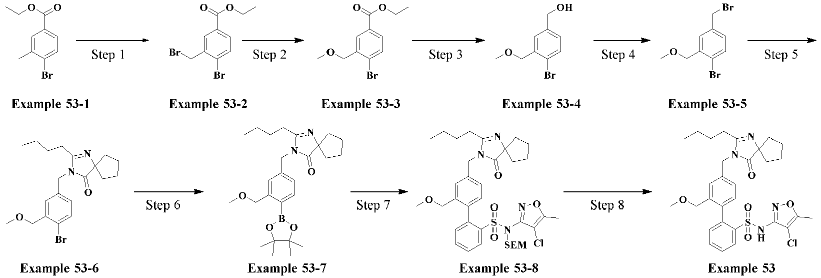

### Step 1

### Preparation of ethyl 4-bromo-3-(bromomethyl)benzoate

N-bromosuccinimide (854.68 mg, 4.80 mmol) and Example **53-1**(1.0 g, 4.37 mmol) were dissolved in carbon tetrachloride (5 mL), and benzoyl peroxide (105.74 mg, 436.55 µmol) was added, the reaction liquid was reacted at 80°C for 16 h under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **53-2** (1.1 g, 3.57 mmol, 81.82% yield).

### Step 2

### Preparation of ethyl 4-bromo-3-(methoxymethyl)benzoate

Example **53-2** (1 g, 3.11 mmol) was dissolved in N,N-dimethylformamide (2 mL) and methanol (1 mL), and sodium methoxide (335.56 mg, 6.21 mmol) was added, the reaction liquid was reacted for 16 h at 50°C under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **53-3** (610 mg, 2.35 mmol, 75.81% yield).

### Step 3

### Preparation of (4-bromo-3-(methoxymethyl)phenyl)methanol

Example **53-3** (610 mg, 2.35 mmol) was dissolved in toluene (4.76 mL), the mixture was cooled to -10°C, diisobutylaluminum hydride (1 M, 4.71 mL) was added, and the reaction liquid was reacted at -10°C for 0.5 h under stirring. 5% aqueous sodium hydroxide solution (5 mL) was added, the mixture was extracted with dichloromethane (10 mL × 3), the organic phases were combined, dried, and concentrated to afford a crude product, and the crude product was purified with column (petroleum ether/ethyl acetate system) to afford the target molecule Example **53-4** (480 mg, 2.08 mmol, 88.23% yield).

### Step 4

### Preparation of 1-bromo-4-(bromomethyl)-2-(methoxymethyl)benzene

Example **53-4** (480 mg, 2.08 mmol) and triphenylphosphine (817.21 mg, 3.12 mmol) were dissolved in dichloromethane (3 mL), then carbon tetrabromide (1.02 g, 3.12 mmol) was added, the reaction liquid was reacted at 30°C for 3 h under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **53-5** (430 mg, 1.46 mmol, 70.42% yield).

### Step 5

### Preparation of 3-(4-bromo-3-(methoxymethyl)benzyl)-2-butyl-1,3-diazaspiro[4.4]non-1-en-4-one

Example **53-5** (430 mg, 1.46 mmol) and 2-butyl-1,3-diazaspiro[4.4]non-1-en-4-one (312.57 mg, 1.61 mmol) were dissolved in acetonitrile (5 mL), then potassium carbonate (605.55 mg, 4.39 mmol) was added, and the reaction liquid was reacted at 80 °C for 16 h under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **53-6** (570 mg, 1.40 mmol, 95.67% yield).

MS m/z (ESI): 407.2[M+1] ⁺.

### Step 6

### Preparation of 2-butyl-3-(3-(methoxymethyl)-4-(4,4,5,5-tetramethyl-1,3,2-dioxaborolan-2-yl)benzyl)-1,3-diazaspiro[4.4]non-1-en-4-one

Example **53-6** (570 mg, 1.40 mmol), bis(pinacolato)diboron (426.41 mg, 1.68 mmol), potassium acetate (411.40 mg, 4.20 mmol) and [1,1'-bis(diphenylphosphino)ferrocene]palladium dichloride dichloromethane complex (114.18 mg, 139.93 µmol) were dissolved in dioxane (5 mL), and the reaction liquid was reacted at 100°C for 16 h under stirring. Saturated aqueous sodium chloride (10 mL) solution was added, the mixture was extracted with dichloromethane (10 mL × 3), the organic phases were combined, dried and concentrated to afford the target molecule crude Example **53-7** (610 mg, 1.34 mmol, 95.93% yield). The crude product was used directly in the next step without purification.

MS m/z (ESI): 455.2[M+1] ⁺.

### Step 7

### Preparation of 4'-((2-butyl-4-oxo-1,3-diazospiro[4.4]non-1-en-3-yl)methyl)-N-(4-chloro-5-methylisoxazol-3-yl)- 2'-(methoxymethyl)-N-(((2-(trimethylsilyl)ethoxy)methyl)-[1,1'-biphenyl]-2-sulfonamide

Intermediate **1** (1.23 g, 2.55 mmol), Example **53-7** (610 mg, 1.34 mmol), [1,1'-bis(diphenylphosphino)ferrocene]palladium dichloride dichloromethane complex (109.54 mg, 134.24 µmol) and cesium carbonate (1.31 g, 4.03 mmol) were dissolved in dioxane/water (2.5 mL, 4 : 1). The reaction liquid was reacted at 100°C for 1 h under microwave under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **53-8** (550 mg, 754.05 µmol, 56.17% yield).

MS m/z (ESI): 729.2[M+1] ⁺.

### Step 8

### Preparation of 4'-((2-butyl-4-oxo-1,3-diazaspiro[4.4]non-1 -en-3-yl)methyl)-N-(4-chloro-5-methylisoxazol-3-yl)- 2'-(methoxymethyl)-[1,1'-biphenyl] -2-sulfonamide

Synthesis method of Example **53** referred to the synthesis method of Example **33,** Example **53-8** was used as raw material to afford the title compound Example **53** (22 mg, 48.5%).

MS: m/z (ESI): 599.3[M+1] ⁺.

¹H NMR (400 MHz, DMSO) δ 8.24 (s, 1H), 8.05 - 7.89 (m, 1H), 7.49 - 7.23 (m, 2H), 7.10 (s, 1H), 7.00 - 6.78 (m, 3H), 4.70 (s, 2H), 4.04 (d, *J* = 12.9 Hz, 1H), 3.90 (d, *J* = 13.0 Hz, 1H), 3.06 (s, 3H), 2.37 (t, *J=* 7.5 Hz, 2H), 2.12 (s, 3H), 1.85 (d, *J* = 7.3 Hz, 6H), 1.68 (d, *J* = 7.0 Hz, 2H), 1.53 (dt, *J* = 15.0, 7.4 Hz, 2H), 1.36 - 1.17 (m, 2H), 0.83 (t, *J* = 7.3 Hz, 3H).

### Example 54

### 4'-((2-butyl-4-oxo-1,3-diazospiro[4,4]non-1-en-3-yl)methyl)-N-(4-chloro-5-methylisoxazol-3-yl)- 2'-methyl-[1,1'-biphenyl]-2-sulfonamide

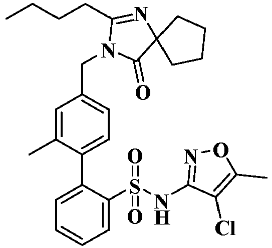

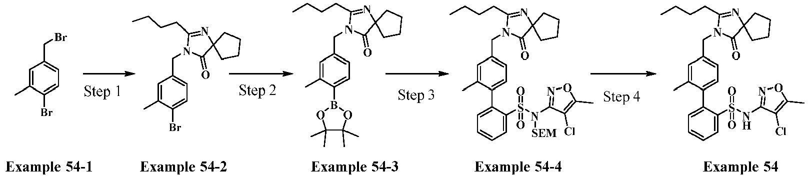

### Step 1

### Preparation of 3-(4-bromo-3-methylbenzyl)-2-butyl-1,3-diazaspiro[4.4]non-1-en-4-one

Example **54-1** (0.5 g, 1.89 mmol) and 2-butyl-1,3-diazaspiro[4.4]non-1-en-4-one (404.80 mg, 2.08 mmol) was dissolved in acetonitrile (5 mL), then potassium carbonate (784.22 mg, 5.68 mmol) was added, and the reaction liquid was reacted at 80 °C for 16 h under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **54-2** (0.59 g, 1.56 mmol, 82.55% yield).

MS m/z (ESI): 377.3[M+1] ⁺.

### Step 2

### Preparation of 2-butyl-3-(3-methyl-4-(4,4,5,5-tetramethyl-1,3,2-dioxaborolan-2-yl)benzyl)-1,3 -diazaspiro[4.4]non-1 -en-4-one

Example **54-2** (590 mg, 1.56 mmol), bis(pinacolato)diboron (476.49 mg, 1.88 mmol), potassium acetate (459.72 mg, 4.69 mmol) and [1,1'-bis(diphenylphosphino)ferrocene]palladium dichloride dichloromethane complex (127.60 mg, 156.37 µmol) were dissolved in dioxane (5 mL), and the reaction liquid was reacted at 100°C for 16 h under stirring. Saturated aqueous sodium chloride (10 mL) solution was added, the mixture was extracted with dichloromethane (10 mL × 3), the organic phases were combined, dried and concentrated to afford the target molecule crude Example **54-3** (580 mg, 1.37 mmol, 87.40% yield). The crude product was used directly in the next step without purification.

MS m/z (ESI): 425.3[M+1] ⁺.

### Step 3

### 4'-((2-butyl-4-oxo-1,3-diazospiro[4.4]non-1-en-3-yl)methyl)-N-(4-chloro-5-methylisoxazol-3-yl)- 2'-methyl-N-(((2-(trimethylsilyl)ethoxy)methyl)-[1,1'-biphenyl] -2-sulfonamide

Intermediate 1 (613.14 mg, 1.27 mmol), Example **54-3** (540 mg, 1.27 mmol), [1,1'-bis(diphenylphosphino)ferrocene]palladium dichloride dichloromethane complex (103.83 mg, 127.24 µmol) and cesium carbonate (1.24 g, 3.82 mmol) were dissolved in dioxane and water (2.5 mL, 4 : 1). The reaction liquid was reacted at 100°C for 1 h under microwave under stirring. Saturated aqueous sodium chloride (10 mL) solution was added, the mixture was extracted with dichloromethane (10 mL × 3), the organic phases were combined, dried, and concentrated to afford a crude product. The crude product was purified with column (petroleum ether/ethyl acetate system) to afford the target molecule Example **54-4** (550 mg, 754.05 µmol, 56.17% yield).

MS m/z (ESI): 699.2[M+1] ⁺.

### Step 4

### Preparation of 4'-((2-butyl-4-oxo-1,3-diazospiro[4.4]non-1-en-3-yl)methyl)-N-(4-chloro-5-methylisoxazol-3-yl)- 2'-methyl-[1,1'-biphenyl]-2-sulfonamide

Synthesis method of Example **54** referred to the synthesis method of Example **33,** Example **54-4** was used as raw material to afford the title compound Example **54** (27 mg, 33.5%).

MS m/z (ESI): 569.2[M+1] ⁺.

¹H NMR (400 MHz, DMSO) δ 7.97 (dd, *J* = 6.0, 3.3 Hz, 1H), 7.50 - 7.19 (m, 2H), 6.96 (d, *J* = 7.8 Hz, 1H), 6.92 - 6.83 (m, 2H), 6.76 (d, *J* = 7.7 Hz, 1H), 4.65 (s, 2H), 2.37 (t, *J* = 7.5 Hz, 2H), 2.09 (d, *J* = 15.8 Hz, 3H), 1.84 (d, *J* = 6.0 Hz, 9H), 1.68 (d, *J* = 6.8 Hz, 2H), 1.53 (dt, *J* = 15.1, 7.4 Hz, 2H), 1.31 (dt, J = 7.1, 6.0 Hz, 2H), 0.84 (t, *J* = 7.3 Hz, 3H).

### Example 55

### 4'-((2-butyl-4-oxo-1,3-diazaspiro[4.4]non-1-en-3-yl)methyl)-N-(4-chloro-5-methylisoxazol-3-yl)-2'-propoxy-[1,1'-biphenyl]-2-sulfonamide

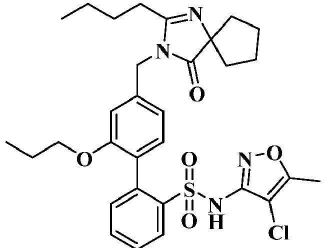

Synthesis method of Example **55** referred to the synthesis method of Example **41,** propyl iodide was used instead of benzyl bromide, and 2-bromo-N-(4-chloro-5-methylisoxazol-3-yl)-N-(methoxymethyl)benzsulfamide was used instead of 2-bromo-N-(4,5-dimethylisoxazol-3-yl)-N-(methoxymethyl)benzsulfamide to afford Example 55 (27 mg, white solid), yield: 40.9%.

MS m/z (ESI): 613.1 [M+1] ⁺.

¹H NMR (400 MHz, DMSO-d6) δ 10.90 (s, 1H), 8.04 (dd, J = 7.8, 1.5 Hz, 1H), 7.62 - 7.47 (m, 2H), 7.16 (d, J = 7.5 Hz, 1H), 7.04 (d, J = 7.7 Hz, 1H), 6.73 (s, 1H), 6.66 - 6.60 (m, 1H), 4.70 (s, 2H), 3.70 (dt, J = 12.9, 6.8 Hz, 2H), 2.38 (t, J = 7.5 Hz, 2H), 2.27 (s, 3H), 1.86 (q, J = 7.7, 6.8 Hz, 6H), 1.73 - 1.65 (m, 2H), 1.52 (p, J = 7.5 Hz, 2H), 1.39 (tt, J = 9.6, 4.7 Hz, 2H), 1.29 (q, J = 7.4 Hz, 2H), 0.83 (t, J = 7.3 Hz, 3H), 0.68 (t, J = 7.4 Hz, 3H).

### Example 56

### 2-[4-[(2-butyl-4-oxo-1,3-diazaspiro[4.4]non-1-en-3-yl)methyl]-2-(ethoxymethyl)phenyl]-N-(4-chloro-5-cyclopropyl-isoxazol-3-yl)benzsulfamide

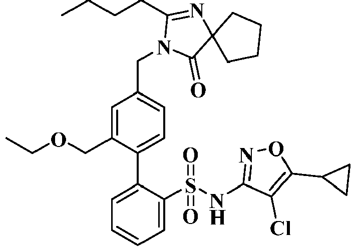

Synthesis method of Example **56** referred to the synthesis method of Example **1,** 4-chloro-5-cyclopropylisoxazoleamine was used in stead of 4,5-dimethylisoxazoleamine to afford Example **56** (29.5 mg, white solid), yield: 22.4%.

MS m/z (ESI): 638.8 [M+1] ⁺.

¹H NMR (400 MHz, DMSO-*d*₆) δ 7.96 - 7.94 (m, 1H), 7.36 - 7.31 (m, 2H), 7.08 (s, 1H), 6.97 (d, *J* = 7.6 Hz, 1H), 6.93 - 6.89 (m, 2H), 4.70 (s, 2H), 4.05 (d, *J* = 13.2 Hz, 1H), 3.91 (d, *J* = 13.2 Hz, 1H), 3.23 - 3.15 (m, 2H), 2.37 (t, *J* = 7.6 Hz, 2H), 1.91 - 1.81 (m, 7H), 1.73 - 1.64 (m, 2H), 1.57 - 1.49 (m, 2H), 1.33 - 1.27 (m, 2H), 1.01 (t, *J* = 7.2 Hz, 3H), 0.95 - 0.90 (m, 2H), 0.85 - 0.82 (m, 5H).

### Example 57

### 2-[4-[(2-butyl-4-oxo-1,3-diazaspiro[4.4]non-1-en-3-yl)methyl]-2-(ethoxymethyl)phenyl]-N-(4-chloro-3-ethyl-isoxazol-5-yl)benzsulfamide

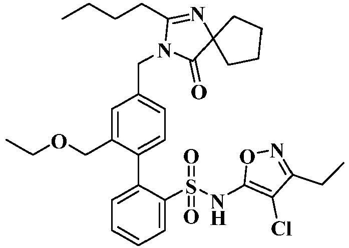

Synthesis method of Example **57** referred to the synthesis method of Example **1,** 4-chloro-3-ethylisoxazoleamine was used in stead of 4,5-dimethylisoxazoleamine to afford Example **57** (3.3 mg, white solid), yield: 2.5%.

MS m/z (ESI): 627.0 [M+1] ⁺.

¹H NMR (400 MHz, DMSO-*d*₆) δ 8.01 - 7.99 (m, 1H), 7.43 - 7.38 (m, 2H), 7.10 (s, 1H), 7.00 - 6.96 (m, 2H), 6.93 - 6.91 (m, 1H), 4.71 (s, 2H), 4.04 (d, *J* = 12.8 Hz, 1H), 3.92 (d, *J* = 13.2 Hz, 1H), 3.24 - 3.16 (m, 2H), 2.38 - 3.31 (m, 4H), 1.91 - 1.81 (m, 6H), 1.72 - 1.65 (m, 2H), 1.55 - 1.49 (m, 2H), 1.32 - 1.27 (m, 2H), 1.07 (d, *J* = 7.6 Hz, 3H), 1.00 (t, *J* = 7.2 Hz, 3H), 0.83 (t, *J* = 7.2 Hz, 3H).

### Example 58

### 2-[4-[(2-butyl-4-oxo-1,3-diazaspiro[4.4]non-1-en-3-yl)methyl]-2-(ethoxymethyl)phenyl]-N-(3-ethyl-4-methyl-isoxazol-5-yl)benzsulfamide

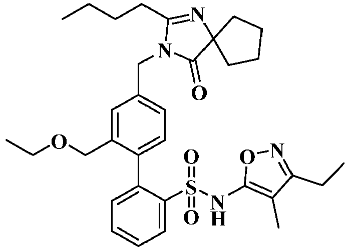

Synthesis method of Example **58** referred to the synthesis method of Example **1,** 4-chloro-3-ethylisoxazoleamine was used in stead of 4,5-dimethylisoxazoleamine to afford Example **58** (14.1 mg, white solid), yield: 11.1%.

MS m/z (ESI): 607.0 [M+1] ⁺.

¹H NMR (400 MHz, DMSO-*d*₆) δ 8.00 - 7.98 (m, 1H), 7.39 - 7.33 (m, 2H), 7.08 (s, 1H), 6.99 (d, *J* = 8.0 Hz, 1H), 6.96 - 6.90 (m, 2H), 4.71 (s, 2H), 4.01 (d, *J* = 13.2 Hz, 1H), 3.92 (d, *J* = 13.2 Hz, 1H), 3.22 - 3.15 (m, 2H), 2.36 (t, *J* = 7.2 Hz, 2H), 2.28 (q, *J=* 7.6 Hz, 2H), 1.93 - 1.80 (m, 6H), 1.73 - 1.65 (m, 2H), 1.55 - 1.51 (m, 2H), 1.43 (s, 3H), 1.32 - 1.27 (m, 2H), 1.06 - 0.99 (m, 6H), 0.83 (t, *J=* 7.2 Hz, 3H).

### Example 59

### N-(4-chloro-5-methylisoxazol-3-yl)-2'-(ethoxymethyl)-4'-(((4-ethyl-5-(hydroxymethyl)-2-propyl-1H-imidazol-1-yl)methyl)-[1,1'-biphenyl]-2-sulfonamide

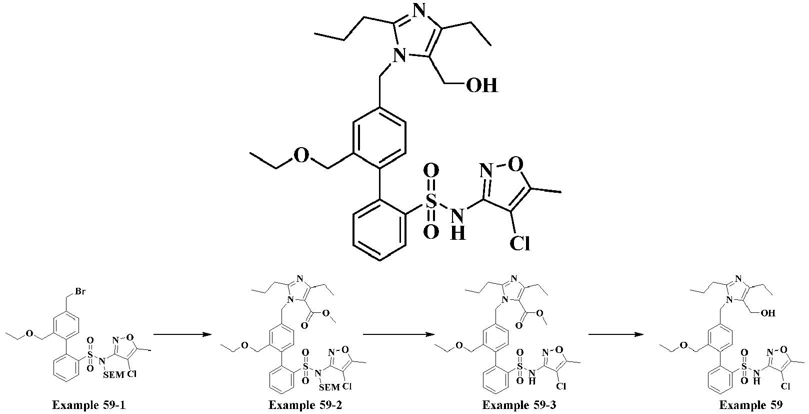

### Step 1

### Preparation of methyl 1-((2'-(N-(4-chloro-5-methylisoxazol-3-yl)-N-((2-(trimethylsilyl)ethoxy)methyl)sulfamoyl)-2-(ethoxymethyl)-[1, 1'-biphenyl] -4-yl)methyl)-4-ethyl-2-propyl-1H-imidazole-5-carboxylate

Example **59-1** (500 mg, 0.8 mmol) (referring to WO 2010114801 A1 for the preparation method) was dissolved in MeCN (15 mL), and potassium carbonate (220 mg, 1.6 mmol) and methyl 4-ethyl-2-propyl-1H-imidazole-5-carboxylate (156 mg, 0.8 mmol) were added at room temperature, the reaction liquid was reacted at 70°C for 3 h under stirring. The reaction liquid was concentrated, and the crude product was used to prepare **59-2** (500 mg, white solid), yield: 85.0%.

MS m/z (ESI): 745.2 [M+1] ⁺.

### Step 2

### Preparation of methyl 1-((2'-(N-(4-chloro-5-methylisoxazol-3-yl)sulfamoyl)-2-(ethoxymethyl)-[1,1'-biphenyl] -4-yl)methyl)-4-ethyl-2-propyl-1H-imidazole-5-carboxylate

**59-2** (500 mg, 0.67 mmol) was dissolved in HCl/dioxane (10 mL), and the reaction liquid was reacted at 70°C for 3 h under stirring, and concentrated to afford **59-3** (400 mg, white solid), yield: 97.0%.

MS m/z (ESI): 615.2 [M+1] ⁺.

### Step 3

### Preparation of 4-fluoro-5-methylisoxazol-3-amine-2-bromo-N-(4-fluoro-5-methylisoxazol-3-yl)benzsulfamide

Compound **59-3** (0.1 g, 0.16 mmol) was dissolved in 10 mL of THF and lithium borohydride (10 mg, 0.48 mmol) was added. The reaction mixture was reacted at 25°C for 4 h under stirring. The reaction liquid was extracted with ethyl acetate, and the organic phase was concentrated. The crude product was used to prepare **59** (50 mg, white solid), yield: 52.0%.

MS m/z (ESI): 587.2 [M+1] ⁺.

### Example 60

### 1-((2'-(N-(4-chloro-5-methylisoxazol-3-yl)sulfamoyl)-2-(ethoxymethyl)-[1,1'-biphenyl]-4-yl)methyl)-4-ethyl-2-propyl-1H-imidazole-5-carboxamide

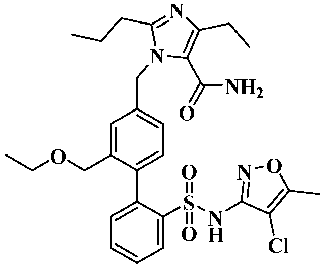

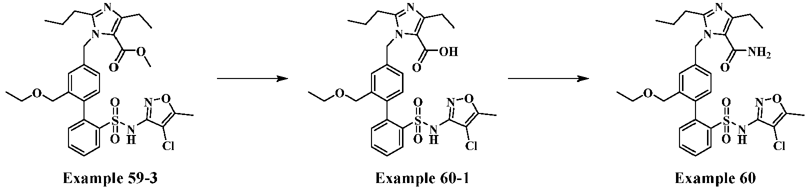

### Step 1

### Preparation of 1-((2'-(N-(4-chloro-5-methylisoxazol-3-yl)sulfamoyl)-2-(ethoxymethyl)-[1,1'-biphenyl] -4-yl)methyl)-4-ethyl-2-propyl-1H-imidazole-5-carboxylic acid

Example **59-3** (200 mg, 0.33 mmol) and sodium hydroxide (26 mg, 0.65 mmol) were dissolved in a mixed solution of THF (8 mL) and H₂O (8 mL) and the mixture was stirred at 30°C for 4 h. Aqueous hydrochloric acid solution (1M, 14 mL) was added and the mixture was extracted with dichloromethane (2 × 20 mL). The organic layers were combined, dried over anhydrous sodium sulfate, filtered, and concentrated to afford the target product **60-1** (120 mg, white solid), yield: 61.0%.

MS m/z (ESI): 601.1 [M+1] ⁺.

### Step 2

### Preparation of 1-((2'-(N-(4-chloro-5-methylisoxazol-3-yl)sulfamoyl)-2-(ethoxymethyl)-[1,1'-biphenyl] -4-yl)methyl)-4-ethyl-2-propyl-1H-imidazole-5-carboxamide

Example **60-1** (100 mg, 0.17 mmol) and ammonium chloride (18 mg, 0.34 mmol) were dissolved in DMF (5 mL), and 2-(7-azabenzotriazole)-N,N,N',N'-tetramethylurea hexafluorophosphate (129 mg, 0.34 mmol) and diisopropylethylamine (44 mg, 0.34mmol) were added, the reaction liquid was reacted at 25°C for 1 h under stirring. Saturated brine (10 mL) was added and the mixture was extracted with ethyl acetate (2 × 10 mL). The organic phases were combined, dried, concentrated, and purified with column (petroleum ether/ethyl acetate system) to afford the target product **60** (50 mg, white solid), yield: 50.0%.

MS m/z (ESI): 600.2 [M+1] ⁺.

### Example 61

### 1-((2'-(N-(4-chloro-5-methylisoxazol-3-yl)sulfamoyl)-2-(ethoxymethyl)-[1,1'-biphenyl] -4-yl)methyl)-4-ethyl-N-methyl-2-propyl-1H-imidazole-5-carboxamide

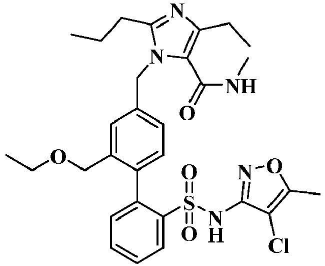

Synthesis method of Example **61** referred to the synthesis method of Example **60,** methylamine was used in stead of ammonium chloride to afford Example 61 (15 mg, 46.2%).

MS m/z (ESI): 614.2 [M+1] ⁺.

### Example 62

### N-(4-chloro-5-methylisoxazol-3-yl)-4'-((4-ethyl-5-(hydroxymethyl)-2-propyl-1H-imidazol-1-yl)methyl)-2'-(methoxymethyl)-[1,1'-biphenyl]-2-sulfonamide

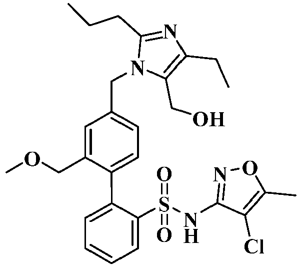

Synthesis method of Example 62 referred to the synthesis method of Example 59.

MS m/z (ESI): 573.2 [M+1] ⁺.

### Example 63

### 1-((2'-(N-(4-chloro-5-methylisoxazol-3-yl)sulfamoyl)-2-(methoxymethyl)-[1,1'-biphenyl]-4-yl)methyl)-4-ethyl-2-propyl-1H-imidazole-5-carboxamide

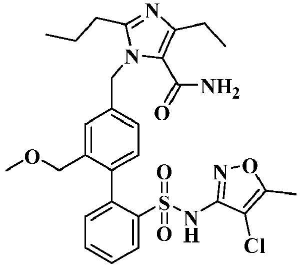

Synthesis method of Example 63 referred to the synthesis method of Example 60.

MS m/z (ESI): 586.2 [M+1] ⁺.

### Example 64

### 1-((2'-(N-(4-chloro-5-methylisoxazol-3-yl)sulfamoyl)-2-(methoxymethyl)-[1,1'-biphenyl] -4-yl)methyl)-4-ethyl-N-methyl-2-propyl-1H-imidazole-5-carboxamide

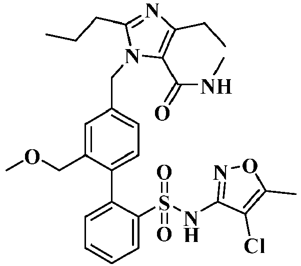

Synthesis method of Example 64 referred to the synthesis method of Example 60.

MS m/z (ESI): 600.2 [M+1] ⁺.

### Example 65

### 4'-((2'-butyl-5'-oxaspiro[bicyclo[3.1.0]hexane-3,4'-imidazole]-1'(5'H)-yl)methyl)-N-(4-chloro-5-methylisoxazol-3-yl)-2'-(ethoxymethyl)-[1,1'-biphenyl]-2-sulfonamide

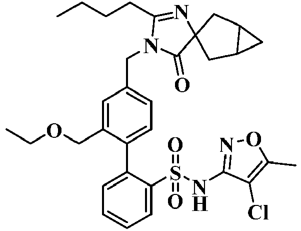

Synthesis method of Example **65** referred to the synthesis method of Example **2,** 2'-butylspiro[bicyclo[3.1.0]hexane-3,4'-imidazole]-5'(1'H)-one was used instead of 2-butyl-1,3-diazaspiro-[4,4]non-1-en-4one, to synthesize Example **65** (14 mg, yield 46%).

MS m/z (ESI): 625.22 [M+1] ⁺.

### Example 66

### N-(4-chloro-5-methylisoxazol-3-yl)-4'-((2-(3-chloropropyl)-4-oxo-1,3-diazaspiro[4.4]non-1-en-3-yl)methyl)-2'-(ethoxymethyl)-[1,1'-biphenyl]-2-sulfonamide

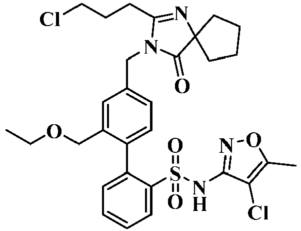

Synthesis method of Example **66** referred to the synthesis method of Example **2,** 2-chlorobutyl-1,3-diazspiro-[4,4]non-1-ene-4one was used instead of 2-butyl-1,3-diazaspiro-[4,4]non-1-en-4one, to synthesize Example **66** (6 mg, yield 15%).

MS m/z (ESI): 633.2 [M+1] ⁺.

### Example 67

### N-(4-chloro-5-methylisoxazol-3-yl)-2'-(ethoxymethyl)-4'-(((2-(2-(methylthio)ethyl)-4-oxo-1,3-diazaspiro][4.4 ]non-1-en-3-yl)methyl)-[1,1'-biphenyl]-2-sulfonamide

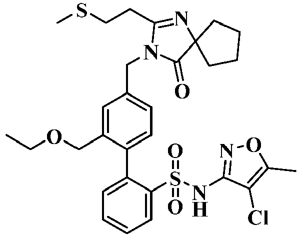

Synthesis method of Example **67** referred to the synthesis method of Example **59,** 2-(2-(methylthio)ethyl)-1,3-diazaspiro-[4,4]non-1-en-4one was used instead of 2-butyl-1,3-diazaspiro-[4,4]non-1-en-4one to synthesize Example **67** (16 mg, yield 21%).

MS m/z (ESI): 631.2 [M+1] ⁺.

### Example 68

### 4'-((2-2--4--4-oxo-1,3-diazaspiro[4.5]dec-1-en-3-yl)methyl)-N-(4-chloro-5- methylisoxazol-3-yl)- 2'-(ethoxymethyl)-[1,1'-biphenyl]-2-sulfonamide

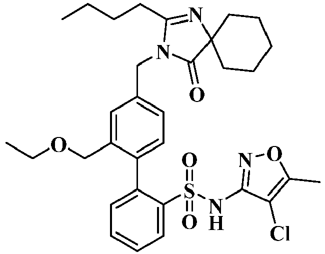

Synthesis method of Example **68** referred to the synthesis method of Example 59, 2-butyl-1,3-diazspiro[4.5]decan-1-ene-4-one was used instead of 2-butyl-1,3-diazaspiro-[4,4]non-1-en-4one, to synthesize Example **68** (21 mg, yield 52%).

MS m/z (ESI): 627.2 [M+1] ⁺.

¹H NMR (400 MHz, DMSO-d6) δ 8.03 (dd, J = 7.5, 1.8 Hz, 1H), 7.59 (t, J = 7.7 Hz, 2H), 7.16 (d, J = 9.5 Hz, 2H), 7.00 (s, 2H), 4.73 (s, 2H), 4.04 (q, J = 13.1 Hz, 2H), 3.26 - 3.16 (m, 2H), 2.35 (d, J = 7.6 Hz, 2H), 2.29 (s, 3H), 1.68 (dq, J = 16.3, 8.3, 6.4 Hz, 7H), 1.52 (q, J = 7.5 Hz, 2H), 1.40 (d, J = 12.1 Hz, 3H), 1.29 (q, J = 7.4 Hz, 2H), 1.00 (t, J = 7.0 Hz, 3H), 0.82 (t, J = 7.3 Hz, 3H).

### Example 69

### 4'-((2-butyl-4-oxo-1,3-diazaspiro[4.4]non-1-en-3-yl)methyl)-N-(4-chloro-5-methylisoxazol-3-yl)- 2'-((trifluoromethoxy)methyl)-[1,1'-biphenyl]-2-sulfonamide

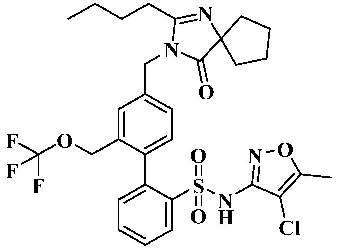

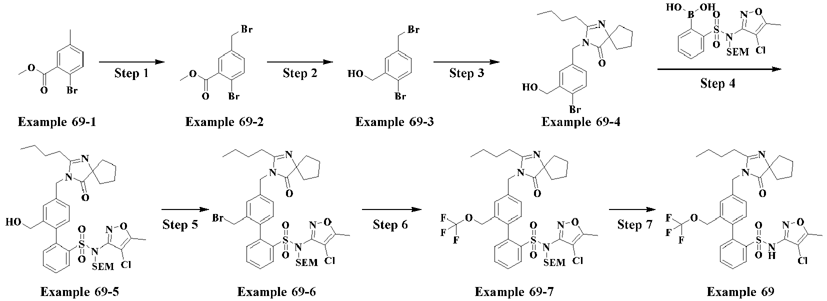

### Step 1

### Preparation of methyl 2-bromo-5-(bromomethyl)benzoate

Bromosuccinimide (8.16 g, 45.84 mmol) was added to a solution of Example **69-1** (10 g, 43.65 mmol) in MeCN (80 mL) under nitrogen protection, and the mixture was stirred at 25°C overnight, *i*.*e*., 12 h. The reaction liquid was concentrated and diluted with 100 mL of ethyl acetate, washed three times with 50 mL of water, the organic phases were combined, dried and concentrated, and the residue was purified by silica gel column chromatography with eluent system B to afford Example **69- 2**(10 g, colorless liquid), yield: 74.3%.

¹H NMR (400 MHz, Chloroform-d) δ 7.82 (d, J = 2.3 Hz, 1H), 7.64 (d, J = 8.3 Hz, 1H), 7.36 (dd, J = 8.3, 2.4 Hz, 1H), 4.44 (s, 2H), 3.94 (s, 3H).

### Step 2

### Preparation of (2-bromo-5-(bromomethyl)phenyl)methanol

Diisobutylaluminum hydride (1 M, 62.11 mL) was added to a solution of Example **69-2** (10 g, 31.06 mmol) in DCM under nitrogen protection at 0°C, and the mixture was stirred at 20°C for 1 h. The reaction liquid was quenched by adding ice water (200 mL), extracted with dichloromethane (100 mL*3), the organic phases were combined, dried and concentrated to afford Example **69-3**(6.0 g, white solid), which was directly used for the next step.

### Step 3

### Preparation of 3-(4-bromo-3-(hydroxymethyl)benzyl)-2-butyl-1,3-diazaspiro[4.4]non-1-en-4-one

Potassium carbonate (1.23 g, 8.93 mmol) was added to a solution of Example **69-3** (1.25 g, 4.46 mmol) and 2-butyl-1,3-diazaspiro[4.4]non-1-en-4-one hydrochloride (1.03 g, 4.46 mmol) in MeCN (15 mL), and the mixture was stirred at 80°C for 12 h. The reaction liquid was quenched by adding water (10 mL), extracted with dichloromethane (10 mL*3), the organic phases were combined, dried and concentrated, and the residue was purified by silica gel column chromatography with eluent system B to afford Example **69-4** (1.0 g, white solid), yield: 56.9%.

MS m/z (ESI): 493.0 [M+1] ⁺.

### Step 4

### Preparation of 4' -((2-butyl-4-oxo-1,3-diazaspiro[4.4]non-1 -en-3 -yl)methyl)-N-(4-chloro-5-methylisoxazol-3-yl)-2'-(hydroxymethyl)-N-(methoxymethyl)-[1,1'-biphenyl]-2-sulfonamide

Example **69-4** (400 mg, 1.05 mmol), (2-(N-(4-chloro-5-methylisoxazol-3-yl)-N-(methoxymethyl)sulfamoyl)phenyl)boronic acid (380 mg, 1.06 mmol), [1,1'-bis(diphenylphosphino)ferrocene]palladium dichloride dichloromethane complex(85 mg, 105.1 µmol), K₂CO₃ (285 mg, 2.10mmol), 1'4-Dioxane (5 mL) and H₂O (1 mL) were added to a reactor. The mixture was stirred at 100°C for 12 h under nitrogen protection. After the reaction liquid was cooled, 8 mL of water was added to quench the reaction, extracted with ethyl acetate (10 mL*3), the organic phases were combined, dried and concentrated, and the residue was purified by silica gel column chromatography with eluent system B to afford Example **69-5** (500 mg, light brown solid), yield: 75.3%.

MS m/z (ESI): 715.3[M+1] ⁺.

### Step 5

### Preparation of 2'-(bromomethyl)-4'-(2-butyl-4-oxo-1,3-diazaspiro[4.4]non-1-en-3-yl)methyl)-N-(4-chloro-5-methylisoxazol-3-yl)-N-(methoxymethyl)-[1,1'-biphenyl]-2-sulfonamide

Carbon tetrabromide (525 mg, 1.58 mmol) and triphenylphosphine (310 mg, 1.18 mmol) were added to a solution of Example **69-5** (500 mg, 0.79 mmol) in DCM (10 mL) under nitrogen protection at 0°C, the mixture was stirred at 20°C for 1 h. The reaction liquid was quenched by adding water (10 mL), extracted with dichloromethane (10 mL*3), the organic phases were combined, dried and concentrated, and the residue was purified by silica gel column chromatography with eluent system B to afford Example **69-6**(520 mg, light yellow solid), yield: 95.3%.

MS m/z (ESI): 778.2 [M+1] ⁺.

### Step 6

### Preparation of 4'-((2-butyl-4-oxo-1,3-diazaspiro[4.4]non-1 -en-3-yl)methyl)-N-(4-chloro-5-methylisoxazol-3-yl)-2'-(((trifluoromethoxy)methyl)-N-((2-(trimethylsilyl)ethoxy)methyl)-[1,1'-biphenyl]-2-sulfonamide

Trifluoromethyl trifluoromethanesulfonate (72 mg, 0.33 mmol) and silver fluoride (48 mg, 0.33 mmol) were dissolved in acetonitrile (5 mL). The reaction liquid was cooled to -30°C and reacted under stirring for 2 h. Example **69-6** (125 mg, 0.16 mmol) dissolved in 5 mL of acetonitrile was added to the reaction liquid, and the reaction liquid was reacted at room temperature for 24 h under stirring. Saturated brine (10 mL) was added to the reaction liquid, and the mixture was extracted with ethyl acetate (3 × 10 mL). The organic phases were combined, dried, concentrated, and purified with column (petroleum ether/ethyl acetate system) to afford Example **69-7** (85 mg, 69.1%).

MS m/z (ESI): 783.2 [M+1] ⁺.

### Step 7

### Preparation of 4'-((2-butyl-4-oxo-1,3-diazaspiro[4.4]non-1 -en-3-yl)methyl)-N-(4-chloro-5-methylisoxazol-3-yl)- 2'-((trifluoromethoxy)methyl)-[1,1'-biphenyl]-2-sulfonamide

Synthesis method of Example **69** referred to the synthesis method of Example **33,** Example **69-7** was used as raw material to afford the title compound Example **69** (27 mg, 28.5%).

MS m/z (ESI): 653.2 [M+1] ⁺.

### Example 70

### 2-[4-[(2-butyl-4-oxo-1,3-diazaspiro[4,4]non-1-en-3-yl)methyl]-2-(cyclobutoxymethyl)phenyl]-N-(4-chloro-5-methyl-isoxazol-3-yl)benzsulfamide

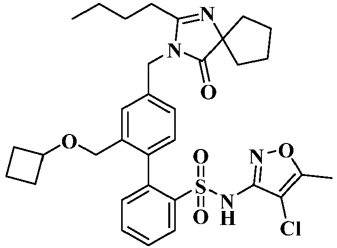

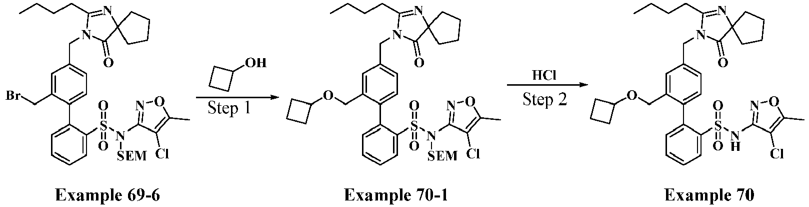

### Step 1

### Preparation of 2-[4-[(2-butyl-4-oxo-1,3-diazaspiro[4.4]non-1-en-3-yl)methyl]-2-(cyclobutoxymethyl)phenyl]-N-(4-chloro-5-methyl-isoxazol-3-yl)-N-(2-trimethylsiloxymethyl)benzsulfamide

Cyclobutanol (46 mg, 0.642 mmol) was dissolved in N,N-dimethylformamide (5 mL), sodium hydride (25 mg, 60% w.t., 0.642 mmol) was added, and the mixture was reacted at room temperature for 30 min. Example **69-6** (100 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **70-1** (90 mg, yellow solid), yield: 91.0%, which was directly used in the next reaction.

MS m/z (ESI): 769.3 [M+1] ⁺.

### Step 2

### Preparation of 2-[4-[(2-butyl-4-oxo-1,3-diazaspiro[4.4]non-1-en-3-yl)methyl]-2-(cyclobutoxymethyl)phenyl]-N-(4-chloro-5-methyl-isoxazol-3-yl)benzsulfamide

Example **70-1** (90 mg, 0.117 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **70** (35.5 mg, white solid), yield: 48.6%.

MS m/z (ESI): 639.2 [M+1] ⁺.

### Example 71

### 2-[4-[(2-butyl-4-oxo-1,3-diazaspiro[4.4]non-1-en-3-yl)methyl]-2-(oxoalk-3-oxymethyl)phenyl]-N-(4-chloro-5-methyl-isoxazol-3-yl)benzsulfamide

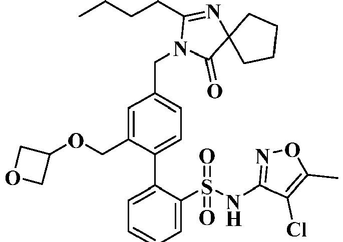

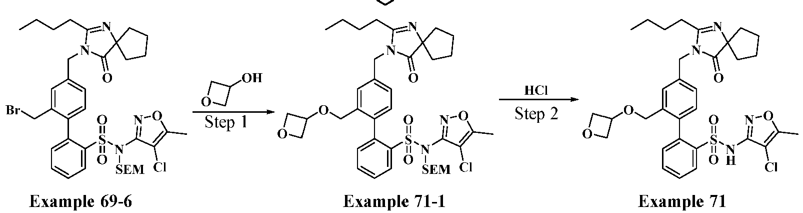

### Step 1

### 2-[4-[(2-butyl-4-oxo-1,3 -diazaspiro[4.4]non-1 -en-3 -yl)methyl]-2-(oxoalk-3 - oxymethyl)phenyl]-N-(4-chloro-5-methyl-isoxazol-3-yl)-N-(2-trimethylsiloxymethyl)benzsulfamide

Oxetan-3-ol (48 mg, 0.642 mmol) was dissolved in N,N-dimethylformamide (5 mL), sodium hydride (26 mg, 60% w.t., 0.642 mmol) was added, and the mixture was reacted at room temperature for 30 min. Example **69-6** (100 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **71-1** (75 mg, yellow solid), yield: 75.7%, which was directly used in the next reaction.

MS m/z (ESI): 771.3 [M+1] ⁺.

### Step 2

### 2-[4-[(2-butyl-4-oxo-1,3 -diazaspiro[4.4]non-1 -en-3 -yl)methyl]-2-(oxoalk-3 - oxymethyl)phenyl]-N-(4-chloro-5-methyl-isoxazol-3-yl)benzsulfamide

Example **71-1** (75 mg, 0.097 mmol) was dissolved in a solution of 1 M tetrabutylammonium fluoride in tetrahydrofuran (4 mL), and the mixture was heated to 60°C and reacted for 2 h. The reaction liquid was cooled to room temperature, concentrated under reduced pressure, and 40 mL of ethyl acetate was added. The mixture was washed with water (40 mL*2) and saturated sodium chloride solution (40 mL) in sequence, dried over anhydrous sodium sulfate and filtered, and the filtrate was concentrated under reduced pressure. The crude product obtained was subjected to reverse HPLC to afford Example **71** (12.6 mg, white solid), yield: 20.3%.

MS m/z (ESI): 641.2 [M+1] ⁺.

### Example 72

### 4'-((2-butyl-4-oxo-1,3-diazaspiro[4.4]non-1-en-3-yl)methyl)-N-(4-chloro-5-methylisoxazol-3-yl)- 2'-(isopropoxymethyl)-[1,1'-biphenyl]-2-sulfonamide

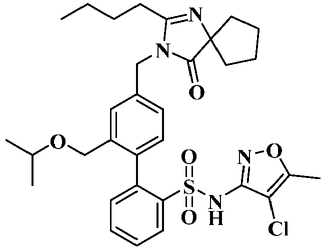

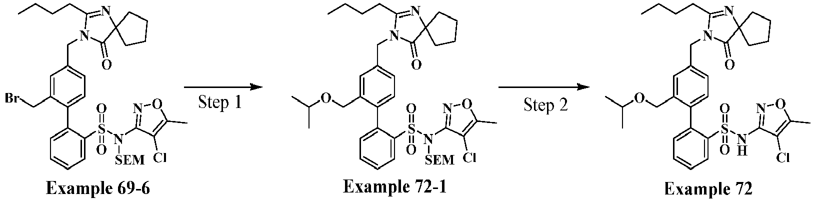

### Step 1

### Preparation of 4'-((2-butyl-4-oxo-1,3-diazospiro[4.4]non-1-en-3-yl)methyl)-N-(4-chloro-5-methylisoxazol-3-yl)- 2'-(isopropoxymethyl)-N-(((2-(trimethylsilyl)ethoxy)methyl)-[1,1'-biphenyl] -2-sulfonamide

Sodium hydride (13 mg, 0.32 mmol) was added to isopropanol (2 mL), the reaction liquid was reacted at room temperature for 1 h under stirring, and then example **69-6** (125 mg, 0.16 mmol) dissolved in N,N-dimethylformamide (2 mL) was added to the reaction liquid, and the reaction liquid was reacted at 50°C for 16 h under stirring. Saturated sodium chloride (10 mL) was added to the reaction liquid, and the mixture was extracted with dichloromethane (10 mL × 3), the organic phases were combined, dried and concentrated to afford a crude product. The crude product was purified with column (petroleum ether/ethyl acetate system) to afford Example **72-1** (610 mg, 75.81% yield).

MS m/z (ESI): 757.3 [M+1] ⁺.

### Step 2

### Preparation of 4'-((2-butyl-4-oxo-1,3-diazaspiro[4.4]non-1 -en-3-yl)methyl)-N-(4-chloro-5-methylisoxazol-3-yl)- 2'-(isopropoxymethyl)-[1,1'-biphenyl] -2-sulfonamide

Synthesis method of Example **72** referred to the synthesis method of Example **71,** Example **72-1** was used as raw material to afford the title compound Example **72** (31 mg, 36.5%).

MS m/z (ESI): 627.2 [M+1] ⁺.

### Example 73

### N-(4-chloro-5-methylisoxazol-3-yl)-4'-((4-oxo-1,3-diazaspiro[4.4]non-1-en-3-yl)methyl)-2'-((pyridine-2-oxy)methyl)-[1,1'-biphenyl]-2-sulfonamide

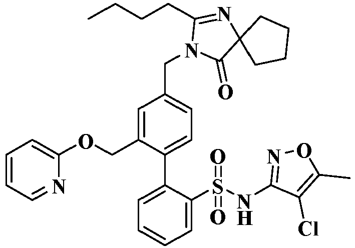

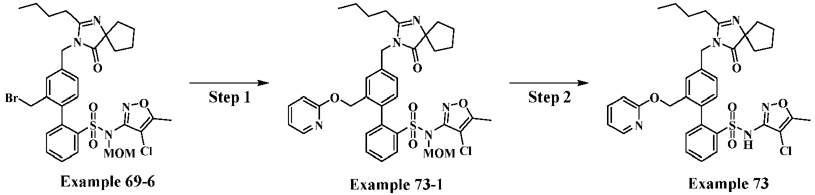

### Step 1

### Preparation of 4' -((2-butyl-4-oxo-1,3-diazaspiro[4.4]non-1 -en-3 -yl)methyl)-N-(4-chloro-5-methylisoxazol-3-yl)-N-(methoxymethyl)-2'-((pyridine-2-oxy)methyl)-[1,1'-biphenyl]-2-sulfonamide

Potassium carbonate (40 mg, 289.02 µmol) and 2-hydroxypyridine (27 mg, 289.02 µmol) were added to a solution of Example **69-6** (100 mg, 144.51 µmol) in DMF (2 mL), and the mixture was stirred at 80°C for 5 h. The reaction liquid was quenched by adding water (10 mL), extracted with dichloromethane (10 mL*3), the organic phases were combined, dried and concentrated, and the residue was purified by silica gel column chromatography with eluent system B to afford the title product **73-1** (52 mg, light yellow solid), yield: 50.8%.

MS m/z (ESI): 706.2 [M+1] ⁺.

### Step 2

### Preparation of 4'-((2-butyl-4-oxo-1,3-diazaspiro[4.4]non-1-en-3-yl)methyl)-N-(4-chloro-5-methylisoxazol-3-yl)-2'-((pyridine-2-oxy)methyl)-[1,1'-biphenyl]-2-sulfonamide

Synthesis method of Example **73** referred to the synthesis method of Example **71,** Example **73-1** was used as raw material to afford the title compound Example **73** (18 mg, white solid), yield: 36.9%.

MS m/z (ESI): 662.1 [M+1] ⁺.

### Example 74

### 2'-((1H-imidazol-1-yl)methyl)-4'-((2-butyl-4-oxo-1,3-diazaspiro[4.4]non-1-en-3-yl)methyl)-N-(4-chloro-5-methylisoxazol-3-yl)-[1,1'-biphenyl]-2-sulfonamide

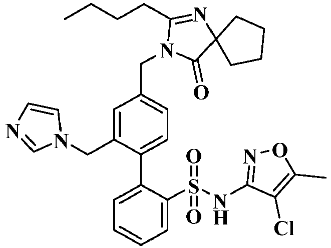

Synthesis method of Example **74** referred to the synthesis method of Example **73-1,** and imidazole was used in stead of 2-hydroxypyridine to afford Example **74** (26 mg, white solid), yield: 56.3%.

MS m/z (ESI): 635.2 [M+1] ⁺.

### Example 75

### 4'-((2-butyl-4-oxo-1,3-diazaspiro[4.4]non-1-en-3-yl)methyl)-N-(4-chloro-5-methylisoxazol-3-yl)-2'-((4-oxo-5-azaspiro[2.4]heptan-5-yl)methyl)-[1,1'-biphenyl]-2-sulfonamide

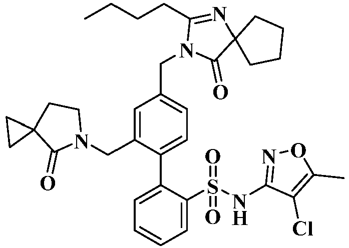

Synthesis method of Example **75** referred to the synthesis method of Example **73-1,** and imidazole was used in stead of 2-hydroxypyridine to afford Example **75** (35 mg, white solid), yield: 45.2%.

MS m/z (ESI): 678.2 [M+1] ⁺.

### Example 76

### N-[[5-[(2-butyl-4-oxo-1,3-diazaspiro[4.4]non-1-en-3-yl)methyl]-2-[(4-chloro-5-methyl-isoxazol-3-yl)sulfamoy]pheny]lphenyl]methyl]-N, 1-dimethyl-cyclopropanecarboxamide

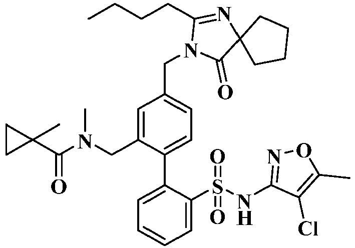

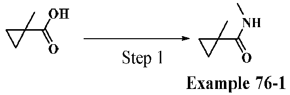

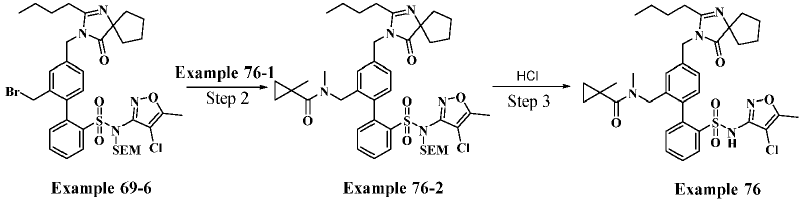

### Step 1

### Preparation of N,1-dimethylcyclopropanecarboxamide

1-methylcyclopropane-1-carboxylic acid (1.0 g, 10.0 mmol) was dissolved in anhydrous dichloromethane (20 mL), N,N-dimethylformamide (74 mg, 1.0 mmol) was added, oxalyl chloride (1.91 g, 15.0 mmol) was added dropwise and the mixture was reacted at room temperature for 2 h. The reaction liquid was concentrated under reduced pressure and redissolved in anhydrous dichloromethane (20 mL). Triethylamine (3.03 g, 30.0 mmol) and methylamine hydrochloride (1.35 g, 20.0 mmol) were added and the mixture was reacted at room temperature for 2 h. The reaction liquid was diluted with 50 mL of dichloromethane, washed with water (50 mL) and saturated sodium chloride solution (50 mL) in sequence, dried over anhydrous sodium sulfate, filtered, and the filtrate was concentrated under reduced pressure and subjected to silica gel column chromatography (methanol/dichloromethane = 0 - 10%) to afford Example **76-1** (730 mg, white solid), yield: 64.6%.

MS m/z (ESI): 114.1 [M+1] ⁺.

### Step 2

### Preparation of N-[[5-[(2-butyl-4-oxo-1,3-diazaspiro[4.4]non-1-en-3-yl)methyl]-2-[(4-chloro-5-methyl-isoxazol-3-yl)-(2-trimethylsiloxymethyl)sulfamoyl] phenyl]phenyl]methyl]-N, 1-dimethyl-cyclopropanecarboxamide

Example **76-1** (73 mg, 0.642 mmol) was dissolved in N,N-dimethylformamide (5 mL), sodium hydride (26 mg, 60% w.t., 0.642 mmol) was added, and the mixture was reacted at room temperature for 30 min. Example 69-6 (100 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **76-2** (105 mg, yellow solid), and the crude product was directly used in the next reaction.

MS m/z (ESI): 810.3 [M+1] ⁺.

### Step 3

### N-[[5-[(2-butyl-4-oxo-1,3-diazaspiro[4.4]non-1-en-3-yl)methyl]-2-[(4-chloro-5-methyl-isoxazol-3-yl)sulfamoyl]phenyl]phenyl]methyl]-N, 1-dimethyl-cyclopropanecarboxamide

Example **76-2** (105 mg, 0.130 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **76** (21.6 mg, white solid), yield: 24.4%.

MS m/z (ESI): 680.3 [M+1] ⁺.

### Example 77

### 4'-((2-butyl-4-oxo-1,3-diazospiro[4.4]non-1-en-3-yl)methyl)-N-(4-chloro-5-methylisoxazol-3-yl)-2'-(cyanomethyl)-[1,1'-biphenyl]-2-sulfonamide

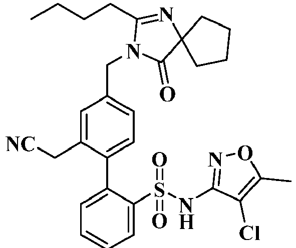

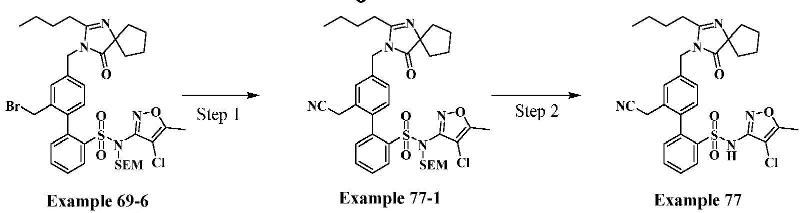

### Step 1

### Preparation of 4'-((2-butyl-4-oxo-1,3-diazospiro[4.4]non-1-en-3-yl)methyl)-N-(4-chloro-5-methylisoxazol-3-yl)-2'-(cyanomethyl)-N-(((2-(trimethylsilyl) ethoxy)methyl)-[1,1'-biphenyl] -2-sulfonamide

Trimethylsilyl cyanide (48 mg, 0.48 mmol) and Example 69-6 (125 mg, 0.16 mmol) were dissolved in CH3CN (5 mL), then tetrabutylamine fluoride (1 M, 0.48 mL) was added to the reaction liquid, and the reaction liquid was reacted at room temperature for 2 h under stirring. Saturated sodium chloride (10 mL) was added to the reaction liquid, and the mixture was extracted with dichloromethane (10 mL × 3), the organic phases were combined, dried and concentrated to afford a crude product. The crude product was purified with column (petroleum ether/ethyl acetate system) to afford the target molecule Example 77-1 (95 mg, 81.9% yield).

MS m/z (ESI): 724.2 [M+1] ⁺.

### Step 2

### Preparation of 4'-((2-butyl-4-oxo-1,3-diazospiro[4.4]non-1-en-3-yl)methyl)-N-(4-chloro-5-methylisoxazol-3-yl)- 2'-(cyanomethyl)-[1,1'-biphenyl]-2-sulfonamide

Synthesis method of Example 77 referred to the synthesis method of Example **52,** Example 77-1 was used as raw material to afford the title compound Example 77 (22 mg, 28.5%).

MS m/z (ESI): 594.2 [M+1] ⁺.

### Example 78

### 2-(4-((2-butyl-4-oxo-1,3-diazaspiro[4.4] non-1-en-3-yl)methyl)-2'-(N-(4-chloro-5-methylisoxazol-3-yl)sulfamoyl)-[1,1'-biphenyl] -2-yl)-N,N-dimethylacetamide

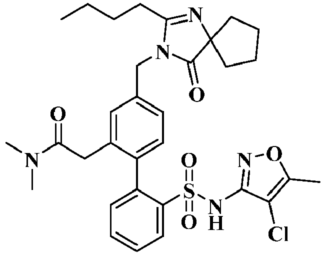

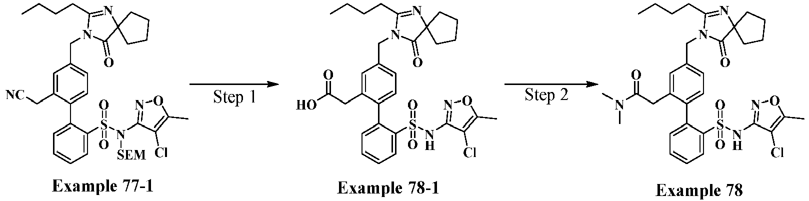

### Step 1

### Preparation of 2-(4-((2-butyl-4-oxo-1,3-diazaspiro[4.4] non-1-en-3-yl)methyl)-2'-(N-(4-chloro-5-methylisoxazol-3-yl)sulfamoyl)-[1,1'-biphenyl] -2-yl)acetic acid

Water (4 mL), concentrated sulfuric acid (4 mL) and glacial acetic acid (4 mL) were added to Example **77-1** (200 mg, 0.28 mmol), and then the reaction liquid was reacted at 100°C for 1 h under stirring. Water (10 mL) was added to the reaction liquid, the mixture was extracted with ethyl acetate (10 mL × 3), the organic phases were combined, dried and concentrated to afford a crude product. The crude product was purified with column (petroleum ether/ethyl acetate system) to afford the target molecule Example **78-1** (82 mg, 48.5% yield).

MS m/z (ESI): 613.2 [M+1] ⁺.

### Step 2

### Preparation of 2-(4-((2-butyl-4-oxo-1,3-diazaspiro[4.4]non-1-en-3-yl)methyl)-2'-(N-(4-chloro-5-methylisoxazol-3-yl)sulfamoyl)-[1,1'-biphenyl] -2-yl)-N,N-dimethylacetamide

Example **78-1** (100 mg, 0.16 mmol), dimethylamine hydrochloride (26 mg, 0.32 mmol), 2-(7-azabenzotriazole)-N,N,N',N'-tetramethylurea hexafluorophosphate (122 mg, 0.32 mmol) was dissolved in dichloromethane (5 mL), then triethylamine (33 mg, 0.32 mmol) was added to the reaction liquid, and the reaction liquid was reacted at room temperature for 1 h under stirring. Water (10 mL) was added to the reaction liquid, and the mixture was extracted with dichloromethane (10 mL × 3), the organic phases were combined, dried and concentrated to afford a crude product. The crude product was purified with column (petroleum ether/ethyl acetate system) to afford the target molecule Example **78** (32 mg, 33.5% yield).

MS m/z (ESI): 640.2 [M+1] ⁺.

### Example 79

### 2-(4-((2-butyl-4-oxo-1,3-diazaspiro[4.4] non-1-en-3-yl)methyl)-2'-(N-(4-chloro-5-methylisoxazol-3-yl)sulfamoyl)-[1,1'-biphenyl] -2-yl)-N,N-diethylacetamide

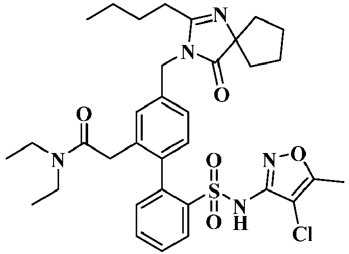

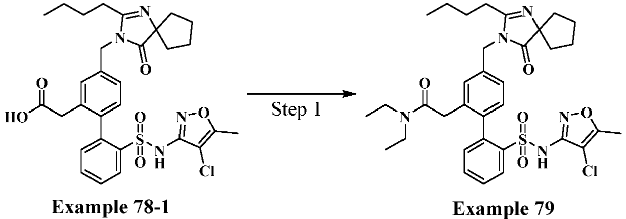

### Step 1

### Preparation of 2-(4-((2-butyl-4-oxo-1,3-diazaspiro[4.4] non-1-en-3-yl)methyl)-2'-(N-(4-chloro-5-methylisoxazol-3-yl)sulfamoyl)-[1,1'-biphenyl] -2-yl)-N,N-diethylacetamide

Example **78-1** (100 mg, 0.16 mmol), diethylamine hydrochloride (35 mg, 0.32 mmol), 2-(7-azabenzotriazole)-N,N,N',N'-tetramethylurea hexafluorophosphate (122 mg, 0.32 mmol) was dissolved in dichloromethane (5 mL), then triethylamine (33 mg, 0.32 mmol) was added to the reaction liquid, and the reaction liquid was reacted at room temperature for 1 h under stirring. Water (10 mL) was added to the reaction liquid, and the mixture was extracted with dichloromethane (10 mL × 3), the organic phases were combined, dried and concentrated to afford a crude product. The crude product was purified with column (petroleum ether/ethyl acetate system) to afford the target molecule Example **79** (32 mg, 33.5% yield).

MS m/z (ESI): 668.2 [M+1] ⁺.

### Example 82

### N-[[5-[(2-butyl-4-oxo-1,3-diazaspiro[4.4]non-1-en-3-yl)methyl]-2-[(4-chloro-5-methyl-isoxazol-3-yl)sulfamoyl]phenyl]phenyl]methyl]-N, 1-dimethyl-cyclopropanecarboxamide

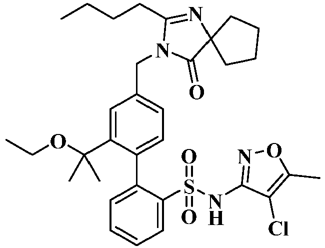

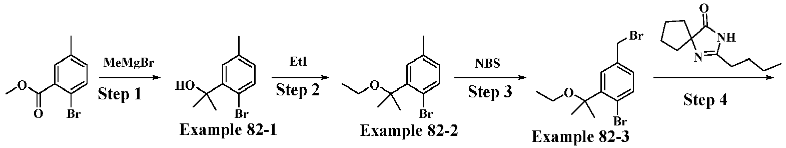

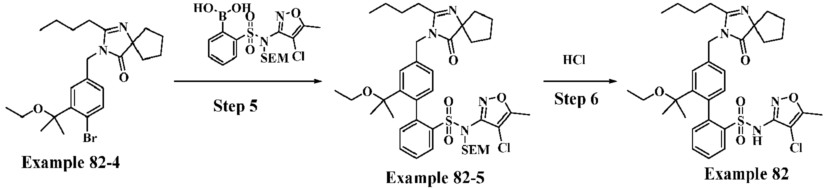

### Step 1

### Preparation of 2-(2-bromo-5-methylphenyl)propanol

Methyl 2-bromo-5-methylbenzoate (3.0 g, 13.1 mmol) was dissolved in anhydrous tetrahydrofuran (50 mL), the mixture was cooled to -78°C under nitrogen protection, and a solution of 3 M methylmagnesium bromide in tetrahydrofuran (17.5 mL, 52.4 mmol) was added dropwise, the mixture was slowly warmed to room temperature and reacted for 3 h. The reaction liquid was cooled to 0°C, saturated ammonium chloride was added dropwise to quench the reaction, and the mixture was extracted with ethyl acetate (100 mL*2). The organic phases were combined, washed with water (100 mL) and saturated sodium chloride solution (100 mL) in sequence, dried over anhydrous sodium sulfate, filtered, and the filtrate was concentrated under reduced pressure and subjected to silica gel column chromatography (ethyl acetate/petroleum ether = 0 - 30%) to afford Example **82-1** (2.2 g, white solid), yield: 73.3%.

¹H NMR (400 MHz, CDCl₃) δ 7.51 - 7.48 (m, 2H), 6.96 - 6.91 (m, 1H), 2.63 (s, 1H), 2.33 (s, 3H), 1.76 (s, 6H).

### Step 2

### Preparation of 1-bromo-2-(2-ethoxypropan-2-yl)-4-toluene

Example **82-1** (2.2 g, 9.61 mmol) was dissolved in anhydrous tetrahydrofuran (30 mL), sodium hydride (576 mg, 60% w.t., 14.4 mmol) was added, and the mixture was reacted at room temperature for 1 h. Ethyl iodide (2.25 g, 14.4 mmol) was added and the mixture was heated to 80°C and reacted for 4 h. The reaction liquid was cooled to room temperature, poured into 100 mL of ice water, and extracted with ethyl acetate (80 mL*2). The organic phases were combined, washed with water (80 mL) and saturated sodium chloride solution (80 mL) in sequence, dried over anhydrous sodium sulfate, filtered, and the filtrate was concentrated under reduced pressure and subjected to silica gel column chromatography (ethyl acetate/petroleum ether = 0 - 20%) to afford Example **82-2** (1.4 g, white solid), yield: 54.9%.

MS m/z (ESI): 257.1 [M+1] ⁺.

### Step 3

Preparation of 1-bromo-4-(bromomethyl)-2-(2-ethoxypropan-2-yl)benzene Example **82-2** (1.4 g, 5.47 mmol) was dissolved in carbon tetrachloride (30 mL), and azobisisobutyronitrile (90 mg, 0.547 mmol) and N-bromosuccinimide (1.07 g, 6.02 mmol) were added, and the mixture was reacted at reflux under nitrogen protection for 4 h. The reaction liquid was cooled to room temperature, washed with water (30 mL) and saturated sodium chloride solution (30 mL) in sequence, dried over anhydrous sodium sulfate and filtered, and the filtrate was concentrated under reduced pressure and subjected to silica gel column chromatography (ethyl acetate/petroleum ether = 0 - 30%) to afford Example **82-3** (850 mg, yellow solid), yield: 46.5%.

MS m/z (ESI): 335.0 [M+1] ⁺.

### Step 4

### Preparation of 3-(4-bromo-3-(2-ethoxypropan-2-yl)benzyl)-2-butyl-1,3-diazaspiro[4.4]non-1-en-4-one

2-butyl-1,3-diazaspiro[4.4]non-1-en-4-one (739 mg, 3.81 mmol) was dissolved in N,N-dimethylformamide (10 mL), the mixture was cooled to 0°C, sodium hydride (152 mg, 60% w.t., 3.81 mmol) was added, and the mixture was warmed to room temperature and reacted for 30 min. Example **82-3** (850 mg, 2.54 mmol) was added and the mixture was reacted at room temperature for 2 h. The reaction liquid was cooled to 0°C, water was slowly added dropwise to quench the reaction, the pH was adjusted to about 6 with dilute hydrochloric acid, and the mixture was extracted with ethyl acetate (50 mL*2). The organic phases were combined, washed with water (50 mL) and saturated sodium chloride solution (50 mL) in sequence, dried over anhydrous sodium sulfate and filtered, and the filtrate was concentrated under reduced pressure and the crude product was subjected to silica gel column chromatography (methanol/dichloromethane = 0 - 10%) to afford Example **82-4** (680 mg, yellow solid), yield: 59.7%.

MS m/z (ESI): 449.2 [M+1] ⁺.

### Step 5

### Preparation of 2-[4-[(2-butyl-4-oxo-1,3-diazaspiro[4.4]non-1-en-3-yl)methyl]-2-(1-ethoxy-1-methyl-ethyl)phenyl]-N-(4-chloro-5-methyl-isoxazol-3-yl)-N-(2-trimethylsiloxymethyl)benzsulfamide

Example **82-4**(100 mg, 0.223 mmol) was dissolved in 1,4-dioxane(2 mL) and water (0.5 mL), (2-(N-(4-chloro-5-methylisoxazol-3-yl)-N-((2-(trimethylsilyl)ethoxy) methyl)sulfamoyl)phenyl)boronic acid (149 mg, 0.334 mmol), [1,1'-bis(diphenylphosphino)ferrocene]palladium dichloride(16 mg, 0.0223 mmol) and sodium carbonate (35 mg, 0.334 mmol) were added, nitrogen replacement was performed three times, and the mixture was reacted under microwave at 100°C for 1 h. The reaction liquid was poured into 30 mL of water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure and the crude product was subjected to silica gel column chromatography (methanol/dichloromethane = 0 - 10%) to afford Example **82-5** (90 mg, yellow solid), yield: 52.4%.

MS m/z (ESI): 771.3 [M+1] ⁺.

### Step 6

### Preparation of N-[[5-[(2-butyl-4-oxo-1,3-diazaspiro[4.4]non-1-en-3-yl)methyl]-2-[(4-chloro-5-methyl-isoxazol-3-yl)sulfamoyl]phenyl]phenyl]methyl]-N, 1-dimethyl-cyclopropanecarboxamide

Example **82-5** (90 mg, 0.117 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **82** (39.5 mg, white solid), yield: 52.7%.

MS: m/z (ESI): 641.3 [M+1] ⁺.

### Example 83

### 2-[4-[(2-butyl-4-oxo-1,3-diazaspiro[4,4]non-1-en-3-yl)methyl]-2-(1-ethoxycyclopropyl)phenyl]-N-(4-chloro-5-methyl-isoxazol-3-yl)benzsulfamide

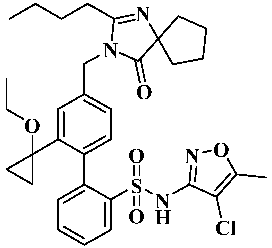

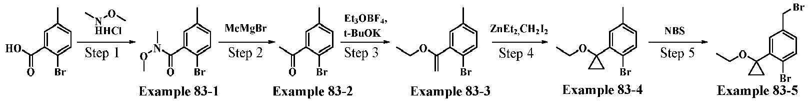

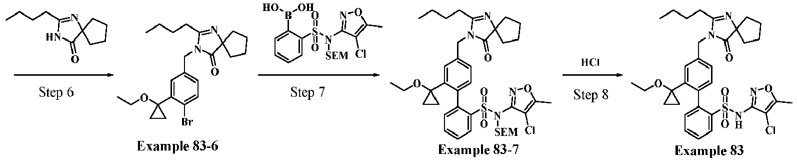

### Step 1

### Preparation of 2-bromo-N-methoxy-N,5-dimethylbenzamide

2-bromo-5-methylbenzoic acid (3.0 g, 14.0 mmol) was dissolved in anhydrous dichloromethane (50 mL), N,N-dimethylformamide (103 mg, 1.40 mmol) was added, and the mixture was cooled to 0°C, oxalyl chloride (3.54 g, 27.9 mmol) was added dropwise, and the mixture was reacted at room temperature for 3 h. The reaction liquid was concentrated under reduced pressure and redissolved in anhydrous dichloromethane (50 mL). Triethylamine (4.23 g, 41.9 mmol) and dimethylhydroxyamine hydrochloride (2.72 g, 27.9 mmol) were added and the mixture was reacted at room temperature for 2 h. The reaction liquid was poured into 100 mL of water and extracted with dichloromethane (100 mL*2). The organic phases were combined, washed with saturated sodium chloride solution (100 mL), dried over anhydrous sodium sulfate and filtered, and the filtrate was concentrated under reduced pressure and the crude product was subjected to silica gel column chromatography (ethyl acetate/petroleum ether = 0 - 60%) to afford Example **83-1** (3.2 g, white solid), yield: 88.6%.

MS m/z (ESI): 258.0 [M+1] ⁺.

### Step 2

### Preparation of 1-(2-bromo-5-methylphenyl)ethan-1-one

Example **83-1** (3.2 g, 12.4 mmol) was dissolved in anhydrous tetrahydrofuran (50 mL), the mixture was cooled to -78°C under nitrogen protection, and a solution of 3 M methylmagnesium bromide in tetrahydrofuran (6.2 mL, 18.6 mmol) was added dropwise, the mixture was warmed to room temperature and reacted for 3 h. The reaction liquid was cooled to 0°C, saturated ammonium chloride solution was added dropwise to quench the reaction, and the mixture was extracted with ethyl acetate (60 mL*2). The organic phases were combined, washed with water (60 mL) and saturated sodium chloride solution (60 mL) in sequence, dried over anhydrous sodium sulfate, filtered, and the filtrate was concentrated under reduced pressure and subjected to silica gel column chromatography (ethyl acetate/petroleum ether = 0 - 60%) to afford Example **83-2** (2.1 g, white solid), yield: 79.5%.

MS m/z (ESI): 213.0 [M+1] ⁺.

### Step 3

### Preparation of 1-bromo-2-(1-ethoxyvinyl)-4-toluene

Example **83-2** (2.1 g, 9.86 mmol) was dissolved in anhydrous tetrahydrofuran (30 mL), potassium tert-butoxide (1.66 g, 14.8 mmol) was added, and the mixture was reacted at room temperature for 1 h. A solution of 1 M triethyloxonium tetrafluoroborate in dichloromethane (14.8 mL, 14.8 mmol) was added and the mixture was reacted at room temperature for 2 h. The reaction liquid was poured into 100 mL of water and the mixture was extracted with ethyl acetate (60 mL*2). The organic phases were combined, washed with water (60 mL) and saturated sodium chloride solution (60 mL) in sequence, dried over anhydrous sodium sulfate, filtered, and the filtrate was concentrated under reduced pressure and the crude product was subjected to silica gel column chromatography (ethyl acetate/petroleum ether = 0 - 60%) to afford Example **83-3** (1.7 g, white solid), yield: 71.5%.

MS m/z (ESI): 242.1 [M+1] ⁺.

### Step 4

### Preparation of 1-bromo-2-(1-ethoxycyclopropyl)-4-toluene

Example **83-3** (1.7 g, 7.05 mmol) and diiodomethane (2.83 g, 10.6 mmol) was dissolved in dichloromethane (30 mL), the mixture was cooled to -78°C under nitrogen protection, and a solution of 1 M diethylzinc in n-hexane (10.6 mL, 10.6 mmol) was added dropwise, the mixture was reacted at -78°C for 1 h, and slowly warmed to room temperature and reacted for 4 h. The reaction liquid was slowly poured into 100 mL of ice water, and the mixture was extracted with dichloromethane (100 mL*2). The organic phases were combined, washed with water (100 mL) and saturated sodium chloride solution (100 mL) in sequence, dried over anhydrous sodium sulfate, filtered, and the filtrate was concentrated under reduced pressure and the crude product was subjected to silica gel column chromatography (ethyl acetate/petroleum ether = 0 - 30%) to afford Example **83-4** (800 mg, white solid), yield: 44.5%.

MS m/z (ESI): 255.0 [M+1] ⁺.

### Step 5

### Preparation of 1-bromo-4-(bromomethyl)-2-(1-ethoxycyclopropyl)benzene

Example **83-4** (800 mg, 3.14 mmol) was dissolved in carbon tetrachloride (15 mL), and azobisisobutyronitrile (51 mg, 0.314 mmol) and N-bromosuccinimide (670 mg, 3.76 mmol) were added, and the mixture was reacted at reflux under nitrogen protection for 4 h. The reaction liquid was cooled to room temperature, washed with water (30 mL) and saturated sodium chloride solution (30 mL) in sequence, dried over anhydrous sodium sulfate and filtered, and the filtrate was concentrated under reduced pressure and subjected to silica gel column chromatography (ethyl acetate/petroleum ether = 0 - 30%) to afford Example **83-5** (620 mg, yellow solid), yield: 59.5%.

MS m/z (ESI): 332.9 [M+1] ⁺.

### Step 6

### Preparation of 3-(4-bromo-3-(1-ethoxycyclopropyl)benzyl)-2-butyl-1,3-diazaspiro [4.4]non-1-en-4-one

2-butyl-1,3-diazaspiro[4.4]non-1-en-4-one (542 mg, 2.79 mmol) was dissolved in N,N-dimethylformamide (10 mL), the mixture was cooled to 0°C, sodium hydride (112 mg, 60% w.t., 2.79 mmol) was added, and the mixture was warmed to room temperature and reacted for 30 min. Example **83-5** (620 mg, 1.86 mmol) was added and the mixture was reacted at room temperature for 2 h. The reaction liquid was cooled to 0°C, water was slowly added dropwise to quench the reaction, the pH was adjusted to about 6 with dilute hydrochloric acid, and the mixture was extracted with ethyl acetate (50 mL*2). The organic phases were combined, washed with water (50 mL) and saturated sodium chloride solution (50 mL) in sequence, dried over anhydrous sodium sulfate and filtered, and the filtrate was concentrated under reduced pressure and the crude product was subjected to silica gel column chromatography (methanol/dichloromethane = 0 - 10%) to afford Example **83-6** (430 mg, yellow solid), yield: 51.7%.

MS m/z (ESI): 447.2 [M+1] ⁺.

### Step 7

### Preparation of 2-[4-[(2-butyl-4-oxo-1,3-diazaspiro[4.4]non-1-en-3-yl)methyl]-2-(1-ethoxycyclopropyl)phenyl]-N-(4-chloro-5-methyl-isoxazol-3-yl)-N-(2-trimethylsiloxymethyl)benzsulfamide

Example **83-6** (100 mg, 0.224 mmol) was dissolved in 1,4-dioxane (2 mL) and water (0.5 mL), (2-(N-(4-chloro-5-methylisoxazol-3-yl)-N-((2-(trimethylsilyl) ethoxy)methyl)sulfamoyl)phenyl)boronic acid**xx** (150 mg, 0.336 mmol), [1,1'-bis(diphenylphosphino)ferrocene]palladium dichloride (16 mg, 0.0224 mmol) and sodium carbonate (36 mg, 0.336 mmol) were added, nitrogen replacement was performed three times, and the mixture was reacted under microwave at 100°C for 1 h. The reaction liquid was poured into 30 mL of water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure and the crude product was subjected to silica gel column chromatography (methanol/dichloromethane = 0 - 10%) to afford Example **83-7** (70 mg, yellow solid), yield: 40.6%.

MS m/z (ESI): 769.3 [M+1] ⁺.

### Step 8

### Preparation of 2-[4-[(2-butyl-4-oxo-1,3-diazaspiro[4.4]non-1-en-3-yl)methyl]-2-(1-ethoxycyclopropyl)phenyl]-N-(4-chloro-5-methyl-isoxazol-3-yl)benzsulfamide

Example **83-7** (70 mg, 0.091 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **83** (15.0 mg, yield: 25.8%).

MS m/z (ESI): 639.3 [M+1] ⁺.

### Example 84

### 4'-((2-butyl-4-oxo-1,3-diazospiro[4.4]non-1-en-3-yl)methyl)-N-(4-chloro-5-methylisoxazol-3-yl)-2'-(((methoxy-d3)methyl)-[1,1'-biphenyl] -2-sulfonamide

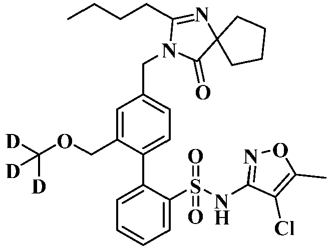

Synthesis method of Example **84** referred to the synthesis method of Example **69,** and deuterated methanol was used as raw material to afford Example 84 (26 mg, yield: 56.3%).

MS m/z (ESI): 602.2 [M+1] ⁺.

¹H NMR (400 MHz, DMSO) δ 8.09 - 7.93 (m, 1H), 7.53 (s, 2H), 7.14 (d, J = 21.2 Hz, 2H), 6.98 (dd, J = 16.8, 7.7 Hz, 2H), 4.73 (s, 2H), 4.00 (dd, J = 31.4, 13.0 Hz, 2H), 2.36 (t, J = 7.5 Hz, 2H), 2.25 (s, 3H), 1.85 (d, J = 7.1 Hz, 6H), 1.69 (d, J = 7.5 Hz, 2H), 1.52 (dt, J = 15.2, 7.6 Hz, 2H), 1.29 (dt, J = 22.4, 7.5 Hz, 2H), 0.82 (t, J = 7.3 Hz, 3H).

### Example 85

### 4'-((2-butyl-4-oxo-1,3-diazospiro[4.4]non-1-en-3-yl)methyl)-N-(4-chloro-5-methylisoxazol-3-yl)-2'-((cyclopropylmethoxy)methyl)-[1,1'-biphenyl]-2-sulfonamide

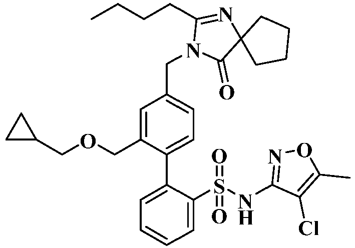

Synthesis method of Example **85** referred to the synthesis method of Example **69,** and cyclopropylmethanol was used as raw material to afford Example **85** (14 mg yield :43%).

MS m/z (ESI): 639.2 [M+1] ⁺.

### Example 86

### 4'-((2-butyl-4-oxo-1,3-diazospiro[4.4]non-1-en-3-yl)methyl)-N-(4-chloro-5-methylisoxazol-3-yl)-2'-(((2,2,2-trifluoroethoxy)methyl)-[1,1'-biphenyl]-2-sulfonamide

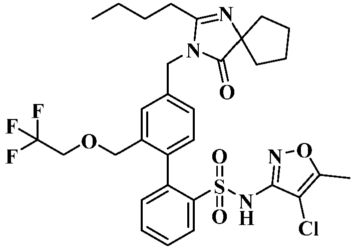

Synthesis method of Example **86** referred to the synthesis method of Example **69,** and trifluoroethanol was used as raw material to afford Example **86** (21 mg yield :46%).

MS m/z (ESI): 667.2 [M+1] ⁺.

### Example 87

### 4'-((2'-butyl-5'-oxaspiro[bicyclo[3.1.0]hexane-3,4'-imidazole]-1'(5'H)-yl)methyl)-N-(4,5-dimethylisoxazol-3-yl)-2'-(ethoxymethyl)-[1,1'-biphenyl]-2-sulfonamide

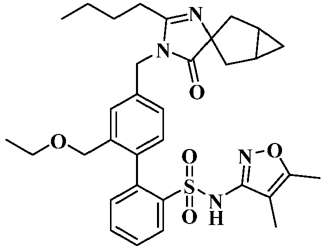

Synthesis method of Example **87** referred to the synthesis method of Example **2,** 2'-butylspiro[bicyclo[3.1.0]hexane-3,4'-imidazole]-5'(1'H)-one was used instead of 2-butyl-1,3-diazaspiro-[4,4]non-1-en-4one, to synthesize Example **87** (11 mg, yield 36%).

MS m/z (ESI): 605.2 [M+1]⁺.

¹H NMR (400 MHz, DMSO) δ 8.06 (dd, *J* = 7.4, 1.9 Hz, 1H), 7.66 - 7.57 (m, 2H), 7.21 - 7.14 (m, 2H), 7.03 - 6.92 (m, 2H), 4.68 (s, 2H), 4.00 (s, 2H), 3.21 (d, *J* = 6.8 Hz, 2H), 2.32 (d, *J* = 7.5 Hz, 2H), 2.20 (s, 4H), 1.86 (d, *J* = 13.5 Hz, 2H), 1.66 (s, 3H), 1.50 (dq, *J* = 9.2, 5.5 Hz, 5H), 1.33 - 1.26 (m, 2H), 1.08 - 0.98 (m, 4H), 0.82 (t, *J* = 7.3 Hz, 3H), 0.56 (q, *J* = 4.0 Hz, 1H).

### Example 88

### 4'-((2-2--4--4-oxo-1,3-diazaspiro[4.5]dec-1-en-3-yl)methyl)-N-(4,-5-dimethylisoxazol-3-yl)- 2'-(methoxymethyl)-[1,1'-biphenyl] -2-sulfonamide

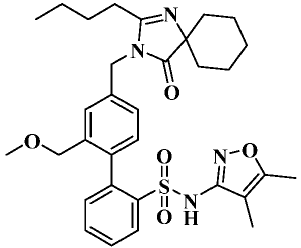

Synthesis method of Example **88** referred to the synthesis method of Example 54, 2-butyl-1,3-diazspiro[4.5]decan-1-ene-4-one was used instead of 2-butyl-1,3-diazaspiro-[4,4]non-1-en-4one, to synthesize Example **88** (29 mg, yield 52%).

MS m/z (ESI): 593.3 [M+1] ⁺.

### Example 89

### 4'-((2-2--4--4-oxo-1,3-diazaspiro[4.5] dec-1-en-3-yl)methyl)-N-(4,-5-dimethylisoxazol-3-yl)- 2'-(deuterated methoxymethyl)-[1,1'-biphenyl] -2-sulfonamide

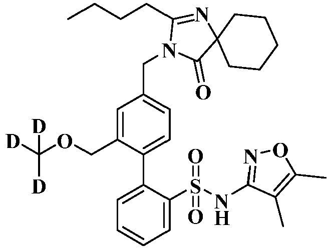

Synthesis method of Example **89** referred to the synthesis method of Example 69, 2-butyl-1,3-diazspiro[4.5]decan-1-ene-4-one was used instead of 2-butyl-1,3-diazaspiro-[4,4]non-1-en-4one, to synthesize Example **88** (31 mg, yield 42%).

MS m/z (ESI): 596.3 [M+1] ⁺.

### Example 91

### Ethyl 1-((2'-(N-(4,5-dimethylisoxazol-3-yl)sulfamoyl)-2-(ethoxymethyl)-[1,1'-biphenyl]-4-yl)methyl)-4-(2 -hydroxypropan-2-yl)-2-propyl-1H-imidazole-5-carboxylate

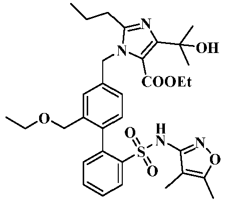

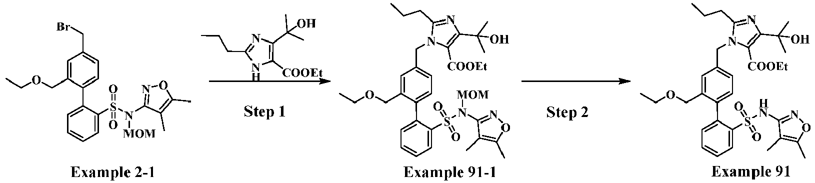

### Step 1

### Preparation of ethyl methyl-1-((2'-(N-(4,5-dimethylisoxazol-3-yl)-N-(methoxymethyl)sulfamoyl)-2-(ethoxymethyl)-[1,1'-biphenyl] -4-yl)methyl)-4-(2-hydroxyprop-2-yl)-2-propyl-1H-imidazole-5-carboxylate

Example **2-1** (100 mg, 0.19 mmol) (referring to WO 2010114801 A1 for the preparation method) was dissolved in acetonitrile (4 mL), ethyl 4-(2-hydroxyprop-2-yl)-2-propyl-1H-imidazole-5-carboxylate (53 mg, 0.23 mmol) and potassium carbonate (52.8 mg, 0.38 mmol) were added, the reaction liquid was heated to reflux for 6 h. The reaction liquid was concentrated, and the crude product was subjected to reverse phase HPLC to afford Example **91-1** (86 mg, yield: 66%).

MS m/z (ESI): 683.3 [M+1] ⁺.

### Step 2

### Preparation of ethyl 1-((2'-(N-(4,5-dimethylisoxazol-3-yl)sulfamoyl)-2-(ethoxymethyl)-[1,1'-biphenyl] -4-yl)methyl)-4-(2 -hydroxypropan-2-yl)-2-propyl-1H-imidazole-5-carboxylate

Synthesis method of Example **91** referred to the synthesis method of the step 4 in Example 1, and Example 91-**1** was used as raw material to afford Example 91 (31 mg, yield: 40%).

MS m/z (ESI): 639.3 [M+1] ⁺.

¹H NMR (400 MHz, DMSO*-*d₆) δ 8.99 (s, 1H), 8.26 (dd, J = 7.4, 1.4 Hz, 1H), 7.65 (dd, J = 7.5, 1.9 Hz, 1H), 7.63 (td, J = 7.2, 1.4 Hz, 1H), 7.46 (td, J = 7.3, 2.0 Hz, 1H), 7.30 (dq, J = 2.0, 1.0 Hz, 1H), 7.21 (d, J = 7.4 Hz, 1H), 7.00 (dq, J = 7.5, 1.1 Hz, 1H), 5.43 (t, J = 1.0 Hz, 2H), 4.97 (s, 1H), 4.70 (d, J = 1.1 Hz, 2H), 4.31 (q, J = 8.0 Hz, 2H), 3.58 (q, J = 8.0 Hz, 2H), 2.59 (t, J = 7.1 Hz, 3H), 2.28 (s, 2H), 1.81 (s, 2H), 1.73 (s, 6H), 1.76 - 1.65 (m, 2H), 1.36 (t, J = 8.0 Hz, 3H), 1.19 (t, J = 8.0 Hz, 4H), 1.01 (t, J = 8.0 Hz, 3H).

### Example 92

### 4'-(2-butyl-4-oxo-1,3-diazaspiro[4.4]non-1-en-3-yl)methyl)-N-(dimethylcarbamoyl)-2'-(ethoxymethyl)-[1,1'-biphenyl]-2-sulfonamide

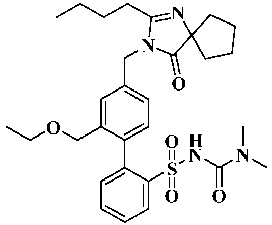

Synthesis method of Example **92** referred to the synthesis method of Example **20,** dimethyl carbamoyl chloride was used in stead of cyclopropanoyl chloride to afford Example **92** (18.8 mg, yield: 54.8%).

MS m/z (ESI): 569.3[M+1] ⁺.

¹H NMR (400 MHz, DMSO-*d*₆) δ 10.16 (s, 1H), 8.00 (d, *J* = 6.8 Hz, 1H), 7.61 - 7.48 (m, 2H), 7.20 (s, 1H), 7.16 - 7.10 (m, 1H), 7.06 - 7.00 (m, 2H), 4.73 (s, 2H), 4.10 (d, *J* = 13.2 Hz, 1H), 4.03 (d, *J* = 13.2 Hz, 1H), 3.30 - 3.21 (m, 2H), 2.61 (s, 6H), 2.37 (t, *J* = 7.6 Hz, 2H), 1.92 - 1.81 (m, 6H), 1.72 - 1.66 (m, 2H), 1.57 - 1.50 (m, 2H), 1.32 - 1.27 (m, 2H), 1.04 (t, *J* = 6.8 Hz, 3H), 0.84 (t, *J* = 7.2 Hz, 3H).

### Example 93

### 4'-(2-butyl-4-oxo-1,3-diazaspiro[4.4]non-1-en-3-yl)methyl)-2'-(ethoxymethyl)-N-(ethylcarbamoyl)-[1,1'-biphenyl]-2-sulfonamide

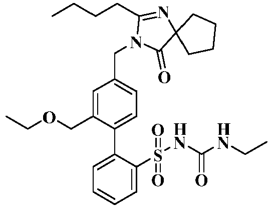

Compound **20-2** (30 mg, 0.060 mmol) was dissolved in anhydrous tetrahydrofuran (2 mL), and ethyl isocyanate (86 mg, 1.21 mmol) and N,N-diisopropylethylamine (39 mg, 0.301 mmol) were added, and the mixture was heated to 70°C and reacted for 16 h and concentrated under reduced pressure, and the obtained residue was purified by reverse pre-HPLC chromatography to afford Example **93** (9.0 mg, yield: 25.6%).

MS m/z (ESI): 569.3[M+1] ⁺.

¹H NMR (400 MHz, DMSO-*d*₆) δ 8.00 (d, *J* = 8.0 Hz, 1H), 7.56 - 7.46 (m, 2H), 7.17 (s, 1H), 7.11 - 7.00 (m, 3H), 5.94 (s, 1H), 4.74 (s, 2H), 4.15 (d, *J=* 13.2 Hz, 1H), 4.00 (d, *J* = 13.2 Hz, 1H), 3.30 - 3.21 (m, 2H), 2.91 - 2.87 (m, 2H), 2.36 (t, *J* = 7.6 Hz, 2H), 1.93 - 1.79 (m, 6H), 1.72 - 1.65 (m, 2H), 1.56 - 1.48 (m, 2H), 1.33 - 1.24 (m, 2H), 1.03 (t, *J* = 6.8 Hz, 3H), 0.91 (t, *J* = 7.2 Hz, 3H), 0.82 (t, *J*= 7.2 Hz, 3H).

### Example 94

### N-((4'-((2-butyl-4-oxo-1,3-diazaspiro[4.4]non-1-en-3-yl)methyl)-2'-(ethoxymethyl)-(1,1'-biphenyl)-2-yl)sulfonyl)pyrrolidine-1-carboxamide

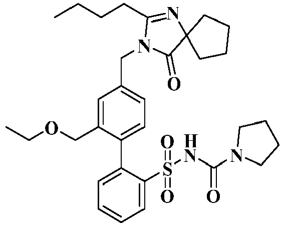

Synthesis method of Example **94** referred to the synthesis method of Example **20,** 1-pyrrolidinecarbonyl chloride was used in stead of cyclopropanoyl chloride to afford Example **94** (8.2 mg, yield: 22.3%).

MS m/z (ESI): 595.3 [M+1] ⁺.

¹H NMR (400 MHz, DMSO-*d*₆) δ 8.01 - 7.97 (m, 1H), 7.47 - 7.41 (m, 2H), 7.14 (s, 1H), 7.07 (d, *J* = 7.6 Hz, 1H), 7.01 - 7.01 (m, 1H), 6.98 - 6.93 (m, 1H), 4.72 (s, 2H), 4.10 (d, *J* = 13.2 Hz, 1H), 4.02 (d, *J* = 13.2 Hz, 1H), 3.29 - 3.25 (m, 2H), 3.11 - 2.78 (m, 4H), 2.36 (t, *J* = 7.6 Hz, 2H), 1.91 - 1.82 (m, 6H), 1.72 - 1.61 (m, 6H), 1.58 - 1.50 (m, 2H), 1.33 - 1.27 (m, 2H), 1.04 (t, *J* = 6.8 Hz, 3H), 0.84 (t, *J* = 7.2 Hz, 3H).

### Example 95

### tert-butyl((4" -((2-butyl-4-oxo-1,3-diazaspiro[4.4]non-1-en-3-yl)methyl)-2" - (ethoxymethyl) - [1,1 '-biphenyl1-2-yl)sulfonyl)carbamate

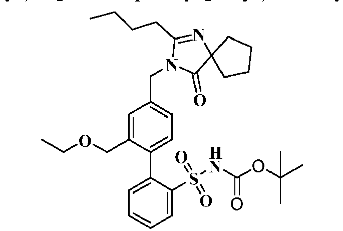

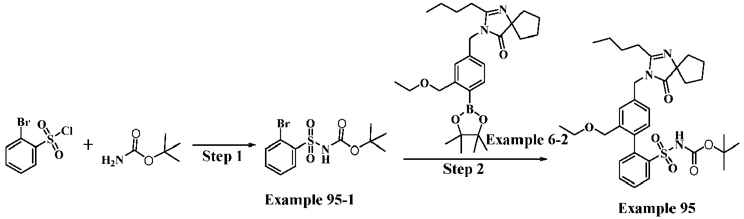

### Step 1

### Preparation of tert-butyl ((2-bromophenyl)sulfonyl) carbamate

Tert-butyl carbamate (26 mg, 2.35 mmol) was dissolved in 5 mL of dichloromethane, triethylamine (594 mg, 5.88 mmol) and 2-bromopyridine-3-sulfonyl chloride (500 mg, 1.96 mmol) were added, and the mixture was reacted at room temperature for 2 h. 50 mL of water was added and the mixture was extracted with dichloromethane (40 mL × 2). The organic phases were combined, washed with water (40 mL) and saturated sodium chloride solution (40 mL) in sequence, dried over anhydrous sodium sulfate and filtered, the filtrate was concentrated under reduced pressure, and the residue was purified with silica gel column chromatography (petroleum ether/ethyl acetate system) to afford Example **95-1** (360 mg, yield: 45.8%).

MS m/z (ESI): 336.0 [M+1] ⁺.

### Step 2

### Preparation of tert-butyl((4" -((2-butyl-4-oxo-1,3-diazaspiro[4.4]non-1-en-3-yl)methyl)-2" - (ethoxymethyl) - [1,1 '-biphenyl]-2-yl)sulfonyl)carbamate

Referring to the synthesis method of Example **6-5,** Example **95-1** and Example **6-2** were used as raw materials to afford Example **95** (18 mg, yield: 29.6%).

MS m/z (ESI): 598.3 [M+1] ⁺.

### Example 96

### 4'-((2-butyl-4-oxo-1,3-diazaspiro[4.4]non-1-en-3-yl)methyl)-2'-((cyclopropylmethoxy)methyl)-N-(3-methoxy) -5-methylpyrazin-2-yl)-[1,1'-biphenyl]-2-sulfonamide

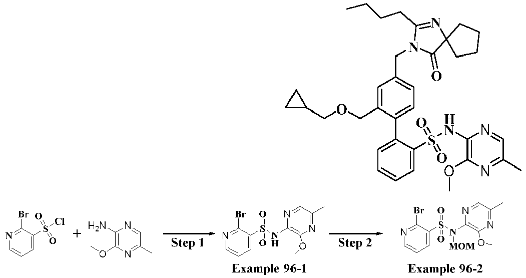

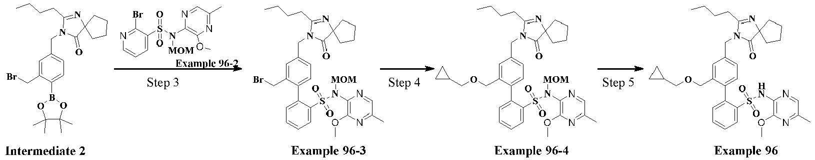

### Step 1

### Preparation of 2-bromo-N-(3-methoxy-5-methylpyrazin-2-yl)pyridine-3-sulfonamide

Referring to the synthesis method of Example 6-3, 2-bromopyridine-3-sulfonyl chloride and 3-methoxy-5-methylpyrazin-2-amine were used as raw materials to afford Example **96-1** (562 mg, yield: 55.6%).

MS m/z (ESI): 359.0 [M+1] ⁺.

### Step 2

### Preparation of 2-bromo-N-(3-methoxy-5-methylpyrazin-2-yl)-N-(methoxymethyl) pyridine-3-sulfonamide

Referring to the synthesis method of Example **6-3,** Example **96-1** and bromomethyl ether were used as raw materials to afford Example **96-2** (582 mg, yield: 85.6%).

MS m/z (ESI): 403.0 [M+1] ⁺.

### Step 3

### Preparation of 2'-(bromomethyl)-4'-((2-butyl-4-oxo-1,3-diazaspiro[4.4]non-1-en-3-yl)methyl)-N-(3-methoxy-5- methylpyrazin-2-yl)-N-(methoxymethyl)-[1,1'-biphenyl]-2-sulfonamide

Referring to the synthesis method of Example **12-1,** Example **96-2** and intermediate **2** were used as raw materials to afford Example **96-3** (380 mg, yield: 65.4%).

MS m/z (ESI): 698.2 [M+1] ⁺.

### Step 4

### Preparation of 4'-((2-butyl-4-oxo-1,3-diazaspiro[4.4]non-1-en-3-yl)methyl)-2'-((cyclopropylmethoxy)methyl)-N-(3-methoxy) -5-methylpyrazin-2-yl)-N-(methoxymethyl)-[1,1'-biphenyl]-2-sulfonamide

Referring to the synthesis method of Example **12-2,** Example **96-3** and cyclopropylmethanol were used as raw materials to afford Example **96-4** (220mg, yield: 45.4%).

MS m/z (ESI): 690.4 [M+1] ⁺.

### Step 5

### Preparation of 4'-((2-butyl-4-oxo-1,3-diazaspiro[4.4]non-1-en-3-yl)methyl)-2'-((cyclopropylmethoxy)methyl)-N-(3-methoxy) -5-methylpyrazin-2-yl)-[1,1'-biphenyl]-2-sulfonamide

Referring to the synthesis method of Example **12,** Example **96-4** was used as raw material to afford Example **96** (22 mg, yield: 15.4%).

MS m/z (ESI): 646.3 [M+1] ⁺.

### Example 97

### methyl 4-((2-butyl-4-oxo-1,3-diazaspiro[4.4]non-1-en-3-yl)methyl)-2' -(N-(4,5-dimethylisoxazol-3-yl )-N-(methoxymethyl)sulfamoyl)-[1,1'-biphenyl]-2-carboxylate

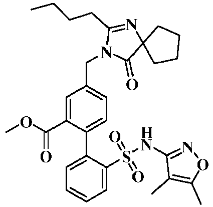

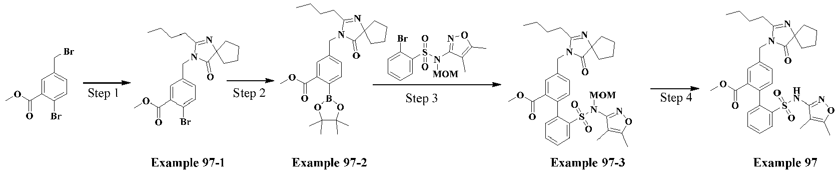

### Step 1

### Preparation of methyl 2-bromo-5-((2-butyl-4-oxo-1,3-diazaspiro[4.4]non-1-en-3-yl)methyl)benzoate

Referring to the synthesis method of intermediate **2c,** methyl 2-bromo-5-(bromomethyl)benzoate and 2-butyl-1,3-diazaspiro[4.4]non-1-en-4-one were used as raw materials to afford Example **97-1** (652 mg, yield: 55.6%).

MS m/z (ESI): 421.0 [M+1] ⁺.

### Step 2

### Preparation of methyl 5 -((2-butyl-4-oxo-1,3-diazaspiro[4.4]non-1-en-3-yl)methyl)-2-(4,4,5,5-tetramethyl-1,3-, 2-dioxaborolan-2-yl)benzoate

Referring to the synthesis method of intermediate **2d,** Example **97-1** and bis(pinacolato)diboron were used as raw materials to afford Example **97-2** (550 mg, yield: 85.6%).

MS m/z (ESI): 469.3 [M+1] ⁺.

### Step 3

### Preparation of methyl 4 -((2-butyl-4-oxo-1,3-diazaspiro[4.4]non-1-en-3-yl)methyl)-2' -(N-(4,5-dimethylisoxazol-3-yl )-N-(methoxymethyl)sulfamoyl)-[1,1'-biphenyl]-2-carboxylate

Referring to the synthesis method of Example **12-1,** Example **97-2** was used as raw material to afford Example **97-3** (220 mg, yield: 35.6%).

MS m/z (ESI): 637.3 [M+1] ⁺.

### Step 4

### Preparation of methyl 4 -((2-butyl-4-oxo-1,3-diazaspiro[4.4]non-1-en-3-yl)methyl)-2' -(N-(4,5-dimethylisoxazol-3-yl)-N-(methoxymethyl)sulfamoyl)-[1,1'-biphenyl]-2-carboxylate

Referring to the synthesis method of Example **12,** Example **97-3** was used as raw material to afford Example **97** (28 mg, yield: 22.6%).

MS m/z (ESI): 593.2 [M+1] ⁺.

¹H NMR (400 MHz, DMSO-d6) δ 8.29 (d, J = 3.7 Hz, 1H), 7.95 (dd, J = 6.7, 2.8 Hz, 1H), 7.63 (d, J = 2.1 Hz, 1H), 7.36 (d, J = 6.5 Hz, 2H), 7.18 (d, J = 8.3 Hz, 1H), 7.04 (d, J = 7.9 Hz, 1H), 6.95 (d, J = 6.4 Hz, 1H), 4.77 (s, 2H), 3.31 (d, J = 1.8 Hz, 3H), 2.36 (d, J = 7.5 Hz, 2H), 2.10 - 1.96 (m, 4H), 1.87 (q, J = 7.2 Hz, 5H), 1.70 (d, J = 8.5 Hz, 2H), 1.52 (q, J = 7.5 Hz, 2H), 1.43 (d, J = 2.9 Hz, 3H), 1.33 - 1.27 (m, 2H), 0.83 (t, J = 7.3 Hz, 3H).

### Example 98

### ethyl 1-(3-(ethoxymethyl)-4-(3-(N-(3-methoxy-5-methylpyrazin-2-yl)sulfamoyl)pyridin-2-yl)benzyl)-4-(2-hydroxypropan-2-yl)-2-propyl-1H-imidazole-5-carboxylate

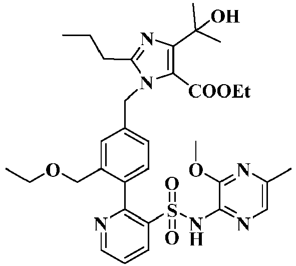

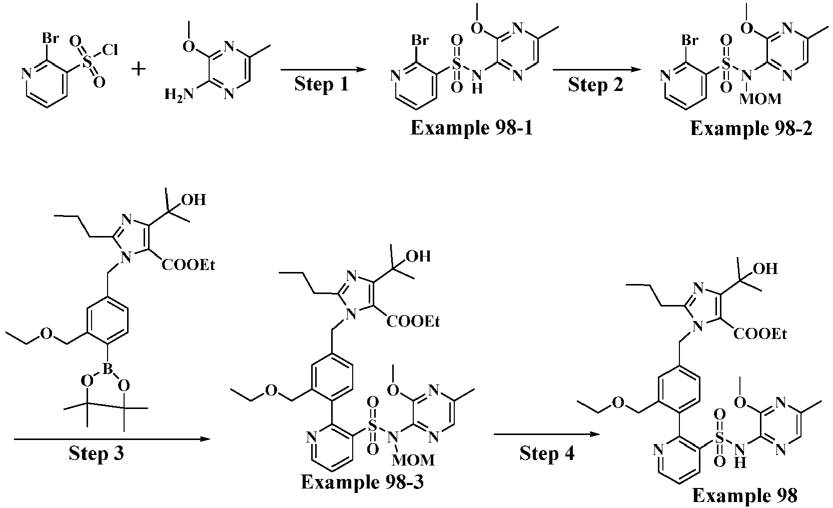

### Step 1

### Preparation of 2-bromo-N-(3-methoxy-5-methylpyrazin-2-yl)pyridine-3-sulfonamide

2-bromopyridine-3-sulfonyl chloride (1.0 g, 3.9 mmol) was dissolved in anhydrous dichloromethane (20 mL) and pyridine (5 mL), and 3-methoxy-5-methylpyrazine-2-amine (542 mg, 3.9 mmol) was added, and the mixture was reacted at room temperature for 3 h. The reaction liquid was poured into 50 mL of water, the pH was adjusted to 6 with dilute hydrochloric acid, and the mixture was extracted with dichloromethane (50 mL*2). The organic phases were combined, washed with water (50 mL) and saturated sodium chloride solution (50 mL) in sequence, dried over anhydrous sodium sulfate and filtered, and the filtrate was concentrated under reduced pressure, and the residue was purified with silica gel column chromatography to afford the title product Example 98-1 (1.0 g, yield: 71.1%).

MS m/z (ESI): 358.9 [M+1] ⁺.

### Step 2

### Preparation of 2-bromo-N-(3-methoxy-5-methylpyrazin-2-yl)-N-(methoxymethyl)pyridine-3-sulfonamide

98-1 (1g, 2.78 mmol) was dissolved in anhydrous dichloromethane (10 mL), pyridine (1 ml) was added, the mixture was cooled to 0°C, and bromomethyl methyl ether (365 mg, 2.92 mmol) was slowly added dropwise, and the mixture was warmed to room temperature and reacted for 2 h. The reaction liquid was slowly poured into 50 mL of water, and the mixture was extracted with dichloromethane (40 mL*2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford the title product Example **98-2** (1 g, yield: 89%).

MS m/z (ESI): 403.0 [M+1] ⁺.

### Step 3

### Preparation of 2-(4-((2-butyl-4-oxo-1,3-diazaspiro[4.4]non-1-en-3-yl)methyl)-2-(ethoxymethyl)phenyl)-N-(4-chloro-5-methylisoxazol-3-yl)-N-(methoxymethyl) pyridine-3-sulfonamide

Synthesis method of Example **98-3** referred to the synthesis method of Example **6-5,** and **98-2** was used instead of **6-4** to afford Example **98-3** (50 mg, yield: 39.7%).

MS m/z (ESI): 711.3 [M+1] ⁺.

### Step 4

### Preparation of 2-(4-((2-butyl-4-oxo-1,3-diazaspiro[4.4]non-1-en-3-yl)methyl)-2-(ethoxymethyl)phenyl)-N-(4-chloro-5-methylisoxazol-3-yl)pyridine-3-sulfonamide

Synthesis method of Example **98** referred to the synthesis method of Example **6,** and **98-3** was used instead of **6-5** to afford Example **98** (20 mg, yield: 55%).

MS m/z (ESI): 667.3 [M+1]⁺

¹H NMR (400 MHz, DMSO-d6) δ 8.50 (d, J = 4.8 Hz, 1H), 8.29 (dd, J = 7.9, 1.7 Hz, 1H), 7.38 (dd, J = 8.0, 4.7 Hz, 1H), 7.11 - 7.02 (m, 2H), 6.97 (s, 1H), 6.76 (d, J = 7.9 Hz, 1H), 5.49 (s, 2H), 5.43 (s, 1H), 4.20 (q, J = 7.1 Hz, 2H), 3.98 (s, 2H), 3.67 (s, 3H), 3.11 (q, J = 7.0 Hz, 2H), 2.64 (d, J = 7.7 Hz, 2H), 2.09 (s, 3H), 1.98 (d, J = 6.4 Hz, 1H), 1.68 (h, J = 7.4 Hz, 2H), 1.47 (s, 6H), 1.19 (d, J = 7.1 Hz, 3H), 0.98 - 0.92 (m, 5H).

### Example 99

### ethyl ethyl-1-((2'-(N-(4,5-dimethylisoxazol-3-yl)sulfamoyl)-2-(ethoxymethyl)-(1,1'-biphenyl]-4-yl)methyl-d2)-4-(2-hydroxypropan-2-yl)-2-propyl-1H-imidazole-5-carboxylate

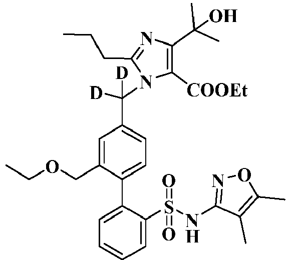

Synthesis method of compound 99 referred to the synthesis method of Example 1 to afford **99** (30 mg, yield: 66%).

MS m/z (ESI): 641.3 [M+1]⁺

¹H NMR (400 MHz, DMSO-d6) δ 8.99 (s, 1H), 8.26 (dd, J = 7.4, 1.4 Hz, 1H), 7.65 (dd, J = 7.5, 1.9 Hz, 1H), 7.63 (td, J = 7.2, 1.4 Hz, 1H), 7.46 (td, J = 7.3, 2.0 Hz, 1H), 7.30 (q, J = 1.1 Hz, 1H), 7.24 - 7.17 (m, 2H), 4.97 (s, 1H), 4.70 (d, J = 1.1 Hz, 2H), 4.31 (q, J = 8.0 Hz, 2H), 3.58 (q, J = 8.0 Hz, 2H), 2.59 (t, J = 7.1 Hz, 3H), 2.28 (s, 3H), 1.81 (s, 2H), 1.73 (s, 6H), 1.71 (dtd, J = 15.1, 8.0, 7.1 Hz, 2H), 1.36 (t, J = 8.0 Hz, 3H), 1.19 (t, J = 8.0 Hz, 3H), 1.01 (t, J = 8.0 Hz, 3H).

### Example 100

### 4'-(2-butyl-4-oxo-1,3-diazaspiro[4.4]non-1-en-3-yl)methyl)-2'-(ethoxymethyl)-N-(5-methyl-4-(methyl-d₃)isoxazol-3-yl)-[1,1'-biphenyl]-2-sulfonamide

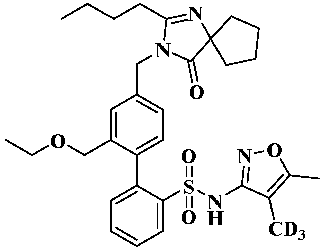

Synthesis method of Example **100** referred to the synthesis method of Example **1,** 5-methyl-4-(deuterated methyl)isoxazol-3-amine was used instead of 4,5-dimethylisoxazolamine to afford Example **100** (6.8 mg, yield: 14.0%).

MS m/z (ESI): 596.3 [M+1] ⁺.

### Example 101

### 4'-(2-butyl-4-oxo-1,3-diazaspiro[4.4]non-1-en-3-yl)methyl)-2'-(ethoxymethyl)-N-(5-methyl-1,2,4-oxadiazol-3-yl)-[1,1'-biphenyl]-2-sulfonamide

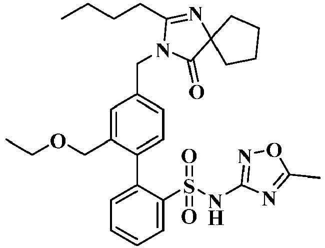

Synthesis method of Example **101** referred to the synthesis method of Example **1,** 5-methyl-3-amino-4-azaisoxazole was used instead of 4,5-dimethylisoxazoleamine to afford Example **101** (7.0 mg, yield: 15.0%).

MS m/z (ESI): 580.3 [M+1] ⁺.

¹H NMR (400 MHz, DMSO-*d*₆) δ 7.96 - 7.94 (m, 1H), 7.36 - 7.31 (m, 2H), 7.08 (s, 1H), 7.01 (d, *J* = 7.6 Hz, 1H), 6.94 - 6.91 (m, 2H), 4.71 (s, 2H), 4.14 (d, *J* = 13.2 Hz, 1H), 3.95 (d, *J* = 13.2 Hz, 1H), 3.23 - 3.19 (m, 2H), 2.36 (t, *J* = 7.6 Hz, 2H), 2.22 (s, 3H), 1.92 - 1.79 (m, 6H), 1.74 - 1.65 (m, 2H), 1.56 - 1.48 (m, 2H), 1.32 - 1.26 (m, 2H), 1.01 (t, *J* = 6.8 Hz, 3H), 0.82 (t, *J* = 7.2 Hz, 3H).

### Example 102

### 1-(3-(ethoxymethyl)-4-(3-(N-(3-methoxy-5-methylpyrazin-2-yl)sulfamoyl)pyridin-2-yl)benzyl)-4-(2-hydroxypropan-2-yl)-2-propyl-1H-imidazole-5-carboxylic acid

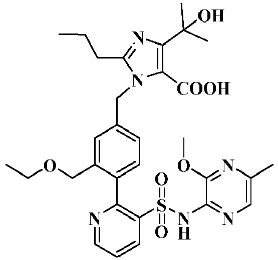

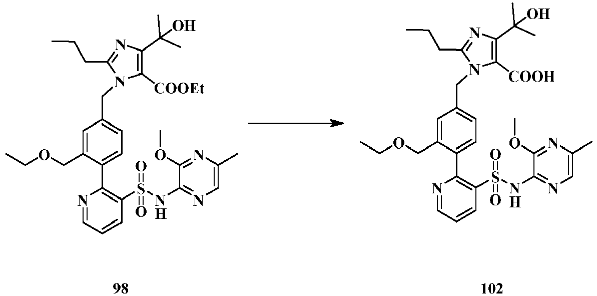

Compound **98** (100 mg, 0.15 mmol) and NaOH (2 M, 1.5 mL) were added to tetrahydrofuran (10 mL). The reaction liquid was stirred at 25°C for 4 h, 1M HCl (10 ml) was added, the mixture was extracted with dichloromethane (30ml*2), and the combined extracts were dried over Na2SO4, spined to dryness, and the obtained crude product was purified with silica gel column chromatography to afford the title compound **102** (60 mg, yield: 63%).

MS m/z (ESI): 639.3 [M+1]⁺

¹H NMR (400 MHz, DMSO-d6) δ 8.94 (s, 1H), 8.79 (dd, J = 7.5, 1.5 Hz, 1H), 8.29 (dd, J = 7.5, 1.5 Hz, 1H), 7.96 (d, J = 7.5 Hz, 1H), 7.51 - 7.43 (m, 2H), 7.36 (dq, J = 2.1, 1.1 Hz, 1H), 7.05 (dq, J = 7.5, 1.1 Hz, 1H), 5.42 (t, J = 1.0 Hz, 2H), 4.97 (s, 1H), 4.77 (d, J = 1.1 Hz, 2H), 4.09 (s, 2H), 3.58 (q, J = 8.0 Hz, 2H), 2.59 (t, J = 7.1 Hz, 2H), 2.51 (d, J = 0.7 Hz, 3H), 1.73 (s, 6H), 1.76 - 1.65 (m, 2H), 1.19 (t, J = 8.0 Hz, 4H), 1.01 (t, J = 8.0 Hz, 3H).

### Example 103

### 4'-(2-butyl-4-oxo-1,3-diazaspiro[4.4]non-1-en-3-yl)methyl)-2'-(ethoxymethyl)-N-(5-methylisoxazol-3-yl)-[1,1'-biphenyl]-2-sulfonamide

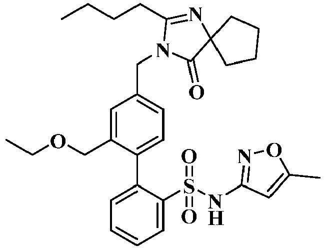

Synthesis method of Example **103** referred to the synthesis method of Example **1,** 3-amino-5-methylisoxazole was used instead of 4,5-dimethylisoxazoleamine to afford Example **103** (18.6 mg, yield: 15.2%).

MS m/z (ESI): 579.3 [M+1] ⁺.

¹H NMR (400 MHz, DMSO-*d*₆) δ 11.22 (br s, 1H), 7.98 (d, *J* = 7.6 Hz, 1H), 7.65 - 7.57 (m, 2H), 7.24 - 7.17 (m, 2H), 7.07 - 7.02 (m, 2H), 5.76 (s, 1H), 4.76 (s, 2H), 4.07 (d, *J* = 13.2 Hz, 1H), 4.00 (d, *J* = 13.2 Hz, 1H), 3.28 - 3.16 (m, 2H), 2.35 (t, *J* = 7.6 Hz, 2H), 2.25 (s, 3H), 1.92 - 1.79 (m, 6H), 1.76 - 1.65 (m, 2H), 1.54 - 1.47 (m, 2H), 1.32 - 1.24 (m, 2H), 1.01 (t, *J* = 6.8 Hz, 3H), 0.81 (t, *J* = 7.2 Hz, 3H).

### Example 104

### 4'-(2-butyl-4-oxo-1,3-diazaspiro[4.4]non-1-en-3-yl)methyl)-N-(3-cyclopropylisoxazol-5-yl)-2'-(ethoxymethyl)-[1,1'-biphenyl]-2-sulfonamide

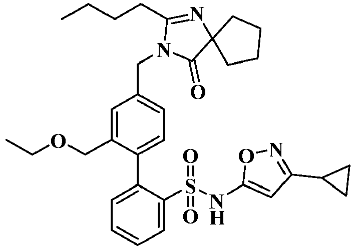

Synthesis method of Example **104** referred to the synthesis method of Example **1,** (3-cyclopropylisoxazol-5-yl)amine was used instead of 4,5-dimethylisoxazoleamine to afford Example **104** (18.4 mg, yield: 15.2%).

MS m/z (ESI): 605.3 [M+1] ⁺.

¹H NMR (400 MHz, DMSO-*d*₆) δ 8.00 (d, *J* = 8.0 Hz, 1H), 7.68 - 7.60 (m, 2H), 7.22 (d, *J* = 7.6 Hz, 1H), 7.20 (s, 1H), 7.06 (d, *J* = 8.4 Hz, 1H), 7.01 (d, *J* = 7.6 Hz, 1H), 5.19 (s, 1H), 4.77 (s, 2H), 4.03 (d, *J* = 13.2 Hz, 1H), 3.97 (d, *J* = 13.2 Hz, 1H), 3.27 - 3.21 (m, 2H), 2.38 (t, *J* = 7.6 Hz, 2H), 1.93 - 1.84 (m, 6H), 1.83 - 1.79 (m, 1H), 1.75 - 1.68 (m, 2H), 1.55 - 1.47 (m, 2H), 1.31 - 1.24 (m, 2H), 1.01 (t, *J* = 6.8 Hz, 3H), 0.95 - 0.90 (m, 2H), 0.81 (t, *J* = 7.2 Hz, 3H), 0.66 - 0.60 (m, 2H).

### Example 105

### 4'-(2-butyl-4-oxo-1,3-diazaspiro[4.4]non-1-en-3-yl)methyl)-N-(2-chloro-5-methoxypyrimidin-4-yl)-2'-(ethoxymethyl)-[1,1'-biphenyl]-2-sulfonamide

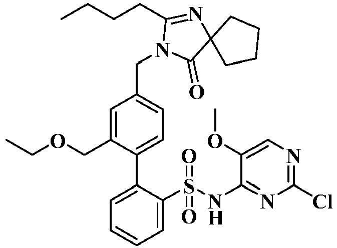

Synthesis method of Example **105** referred to the synthesis method of Example **1,** 2-chloro-4-amino-5-methoxypyrimidine was used instead of 4,5-dimethylisoxazoleamine to afford Example **105** (26.6 mg, yield: 21.4%).

MS m/z (ESI): 640.2 [M+1] ⁺.

¹H NMR (400 MHz, DMSO-*d*₆) δ 8.08 (d, *J* = 7.6 Hz, 1H), 8.05 - 7.98 (m, 1H), 7.66 - 7.56 (m, 2H), 7.23 - 7.13 (m, 2H), 7.02 (s, 2H), 4.75 (s, 2H), 4.01 - 3.93 (m, 2H), 3.78 (s, 3H), 3.24 - 3.16 (m, 3H), 2.36 (t, *J* = 7.6 Hz, 2H), 1.92 - 1.82 (s, 6H), 1.76 - 1.66 (m, 2H), 1.55 - 1.47 (m, 2H), 1.32 - 1.24 (m, 2H), 1.00 (t, *J* = 6.8 Hz, 3H), 0.81 (t, *J* = 7.2 Hz, 3H).

### Example 106

### N-(5-(tert-butyl)isoxazol-3-yl)-4'-(2-butyl-4-oxo-1,3-diazaspiro[4.4]non-1-en-3-yl)methyl)-2'-(ethoxymethyl)-(1,1'-biphenyl)-2-sulfonamide

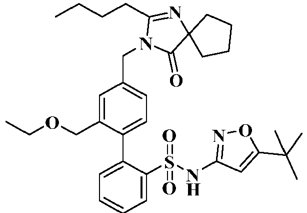

Synthesis method of compound 106 referred to the synthesis method of Example 1 to afford **106** (62 mg, yield: 66%).

MS m/z (ESI): 621.3 [M+1]⁺

¹H NMR (400 MHz, DMSO-d6) δ 11.23 (s, 1H), 8.02 (dd, J = 7.6, 1.8 Hz, 1H), 7.69 - 7.59 (m, 2H), 7.33 - 7.11 (m, 3H), 7.03 (t, J = 6.5 Hz, 2H), 5.68 (s, 2H), 4.76 (s, 2H), 4.09 - 3.96 (m, 2H), 3.25 - 3.16 (m, 2H), 2.34 (t, J = 7.5 Hz, 2H), 1.85 (d, J = 7.6 Hz, 4H), 1.70 (d, J = 8.9 Hz, 2H), 1.50 (p, J = 7.5 Hz, 2H), 1.31 - 1.25 (m, 2H), 1.20 (s, 9H), 1.00 (t, J = 7.0 Hz, 3H), 0.80 (t, J = 7.3 Hz, 3H).

### Example 107

### 4'-((2-butyl-4-oxo-1,3-diazaspiro[4.4]non-1-en-3-yl)methyl)-N-(4,5-dimethylisoxazol-3-yl)-2' -hydroxy-[1,1'-biphenyl]-2-sulfonamide

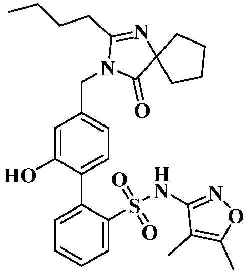

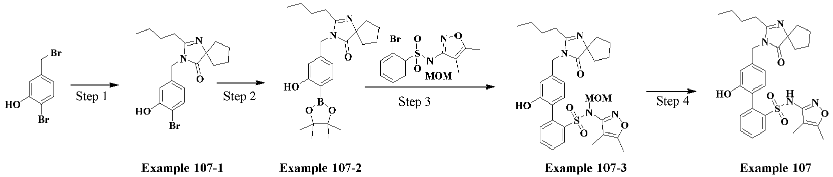

### Step 1

### Preparation of 3-(4-bromo-3-hydroxybenzyl)-2-butyl-1,3-diazaspiro[4.4]non-1-en-4-one

Referring to the synthesis method of intermediate **2c,** 2-bromo-5-(bromomethyl)phenol and 2-butyl-1,3-diazaspiro[4.4]non-1-en-4-one were used as raw materials to afford Example **107-1** (343 mg, yield: 45.3%).

MS m/z (ESI): 379.1 [M+1] ⁺.

### Step 2

### Preparation of 2-butyl-3-(3-hydroxy-4-(4,4,5,5-tetramethyl-1,3,2-dioxaborolan-2-yl)benzyl)-1,3 -diazaspiro[4.4]non-1 -en-4-one

Referring to the synthesis method of intermediate **2d,** Example **107-1** and bis(pinacolato)diboron were used as raw materials to afford Example **107-2** (252 mg, yield: 65.6%).

MS m/z (ESI): 427.3 [M+1] ⁺.

### Step 3

### Preparation of 4'-((2-butyl-4-oxo-1,3-diazaspiro[4.4]non-1-en-3-yl)methyl)-N-(4,5-dimethylisoxazol-3-yl)-2' -hydroxy-N-(methoxymethyl)-[1,1'-biphenyl]-2-sulfonamide

Referring to the synthesis method of Example **12-1,** Example **107-2** was used as raw material to afford Example **107-3** (120 mg, yield: 45.6%).

MS m/z (ESI): 595.3 [M+1] ⁺.

### Step 4

### Preparation of 4'-((2-butyl-4-oxo-1,3-diazaspiro[4.4]non-1-en-3-yl)methyl)-N-(4,5-dimethylisoxazol-3-yl)-2' -hydroxy-[1,1'-biphenyl]-2-sulfonamide

Referring to the synthesis method of Example **12,** Example **107-3** was used as raw material to afford Example **107** (21 mg, yield: 24.6%).

MS m/z (ESI): 551.2 [M+1] ⁺.

¹H NMR (400 MHz, DMSO) δ 10.24 (s, 1H), 9.37 (s, 1H), 7.95 (d, J = 7.4 Hz, 1H), 7.49 (dt, J = 26.6, 7.1 Hz, 2H), 7.12 (d, J = 7.3 Hz, 1H), 6.73 (t, J = 39.8 Hz, 1H), 6.49 (d, J = 10.6 Hz, 2H), 4.56 (s, 2H), 2.28 (t, J = 7.5 Hz, 2H), 2.13 (s, 3H), 1.79 (d, J = 6.9 Hz, 5H), 1.71 - 1.54 (m, 5H), 1.46 (dt, J = 15.2, 7.6 Hz, 2H), 1.29 - 1.17 (m, 3H), 0.77 (t, J = 7.4 Hz, 3H).

### Example 108

### 4'-(2-butyl-4-oxo-1,3-diazaspiro[4.4]non-1-en-3-yl)methyl)-2'-(ethoxymethyl)-N-(4-isopropyl-5-methylisoxazol-3-yl)-[1,1'-biphenyl]-2-sulfonamide

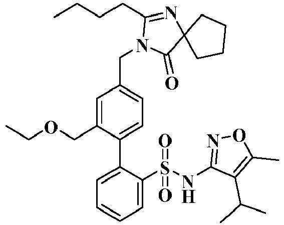

Synthesis method of Example **108** referred to the synthesis method of Example **1,** 4-isopropyl-5-methylisoxazole-3-amine was used instead of 4,5-dimethylisoxazoleamine to afford Example **108** (3.5 mg, yield: 5.4%).

MS m/z (ESI): 621.3[M+1] ⁺.

¹H NMR (400 MHz, DMSO-*d*₆) δ 8.00 (d, *J* = 8.8 Hz, 1H), 7.38 - 7.30 (m, 2H), 7.09 (s, 1H), 6.98 (d, *J* = 8.0 Hz, 1H), 6.91 - 6.86 (m, 2H), 4.70 (s, 2H), 4.03 (d, *J* = 13.2 Hz, 1H), 3.97 (d, *J* = 13.2 Hz, 1H), 3.22 - 3.17 (m, 2H), 2.37 - 2.33 (m, 2H),2.09 (s, 3H), 2.03 - 1.97 (m, 1H), 1.89 - 1.81 (m, 6H), 1.72 - 1.65 (m, 2H), 1.55 - 1.49 (m, 2H), 1.33 - 1.27 (m, 2H), 1.07 - 1.00 (m, 9H), 0.83 (t, *J* = 7.2 Hz, 3H).

### Example 109

### 2-(4-((2-butyl-4-oxo-1,3-diazaspiro[4.4]non-1-en-3-yl)methyl)-2-(ethoxymethyl)phenyl)-N-(4-chloro-5-methylisoxazol-3-yl)pyridine-3-sulfonamide

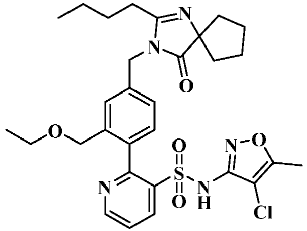

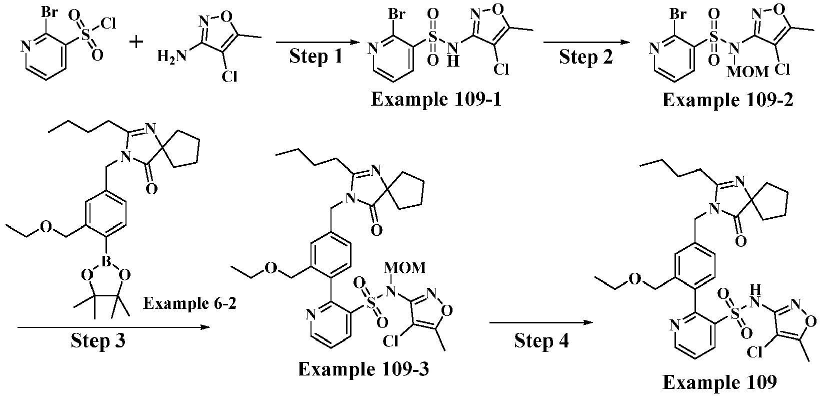

### Step 1

### Preparation of 2-bromo-N-(4-chloro-5-methylisoxazol-3-yl)pyridine-3-sulfonamide

2-bromopyridine-3-sulfonyl chloride (1.0 g, 4.72 mmol) was dissolved in anhydrous dichloromethane (20 mL) and pyridine (5 mL), and 4-chloro-5-methylisoxazole-3-amine (625 mg, 4.72 mmol) was added, and the mixture was reacted at room temperature for 3 h. The reaction liquid was poured into 50 mL of water, the pH was adjusted to 6 with dilute hydrochloric acid, and the mixture was extracted with dichloromethane (50 mL*2). The organic phases were combined, washed with water (50 mL) and saturated sodium chloride solution (50 mL) in sequence, dried over anhydrous sodium sulfate and filtered, and the filtrate was concentrated under reduced pressure, and the residue was purified by silica gel column chromatography with eluent system B to afford the title product Example **109-1** (1.0 g, yield: 55.1%).

MS m/z (ESI): 351.9 [M+1] ⁺.

### Step 2

### Preparation of 2-bromo-N-(4-chloro-5-methylisoxazol-3-yl)-N-(methoxymethyl) pyridine-3-sulfonamide

2-bromo-N-(4-chloro-5-methylisoxazol-3-yl)pyridine-3-sulfonamide(600 mg, 1.95 mmol) was dissolved in anhydrous dichloromethane (10 mL), pyridine (770 mg, 9.74 mmol) was added, the mixture was cooled to 0°C, bromomethyl methyl ether (365 mg, 2.92 mmol) was slowly added dropwise, and the mixture was warmed to room temperature and reacted for 2 h. The reaction liquid was slowly poured into 50 mL of water, and the mixture was extracted with dichloromethane (40 mL*2). The organic phases were combined, washed with water (40 mL) and saturated sodium chloride solution (40 mL) in sequence, dried over anhydrous sodium sulfate and filtered, and the filtrate was concentrated under reduced pressure, and the residue was purified with silica gel column chromatography to afford the title product Example **109-2** (500 mg, yield: 72.9%).

MS m/z (ESI): 395.9 [M+1] ⁺.

### Step 3

### Preparation of 2-(4-((2-butyl-4-oxo-1,3-diazaspiro[4.4]non-1-en-3-yl)methyl)-2-(ethoxymethyl)phenyl)-N-(4-chloro-5-methylisoxazol-3-yl)-N-(methoxymethyl) pyridine-3-sulfonamide

Synthesis method of Example **109-3** referred to the synthesis method of Example **6-5,** and **109-2** was used instead of **6-4** to afford Example **109-3** (40 mg, yield: 35.7%).

MS m/z (ESI): 658.2 [M+1] ⁺.

### Step 4

### Preparation of 2-(4-((2-butyl-4-oxo-1,3-diazaspiro[4.4]non-1-en-3-yl)methyl)-2-(ethoxymethyl)phenyl)-N-(4-chloro-5-methylisoxazol-3-yl)pyridine-3-sulfonamide

Synthesis method of Example **109** referred to the synthesis method of Example **6,** and **109-3** was used instead of **6-5** to afford Example **109** (18.1 mg, yield: 48.2%).

MS m/z (ESI): 614.2 [M+1] ⁺.

¹H NMR (400 MHz, DMSO-*d*₆) δ 8.72 - 8.66 (m, 1H), 8.37 (d, *J* = 8.0 Hz, 1H), 7.58 - 7.55 (m, 1H), 7.19 (s, 1H), 7.09 (d, *J* = 7.6 Hz, 1H), 7.01 (d, *J* = 8.0 Hz, 1H), 4.75 (s, 2H), 4.07 (s, 2H), 3.25 - 3.19 (m, 2H), 2.37 (t, *J* = 7.6 Hz, 2H), 2.24 (s, 3H), 1.93 - 1.79 (m, 6H), 1.74 - 1.67 (m, 2H), 1.57 - 1.50 (m, 2H), 1.33 - 1.27 (m, 2H), 1.00 (t, *J* = 6.8 Hz, 3H), 0.84 (t, *J* = 7.2 Hz, 3H).

### Example 110

### 2-(4-((2-butyl-4-oxo-1,3-diazaspiro[4.4]non-1-en-3-yl)methyl-d2)-2-(ethoxymethyl)phenyl)-N-(4-fluoro-5-methylisoxazol-3-yl)pyridine-3-sulfonamide

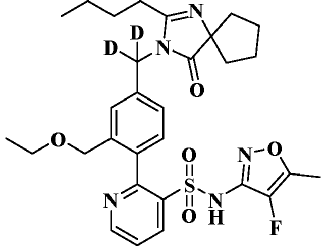

Synthesis method of Example 110 referred to the synthesis method of Example 1, 4-fluoro-5-methylisoxazoleamine was used instead of 4,5-dimethylisoxazoleamine to afford Example 110 (19 mg, yield: 50.3%).
MS m/z (ESI): 600.3 [M+1]
¹H NMR (400 MHz, DMSO) δ 8.79 (t, *J* = 9.8 Hz, 1H), 8.41 (d, *J* = 8.1 Hz, 1H), 7.64 (dd, *J* = 8.0, 4.8 Hz, 1H), 7.22 (s, 1H), 7.05 (dt, *J* = 42.2, 21.0 Hz, 3H), 4.09 (s, 2H), 3.22 (dd, *J* = 13.9, 7.0 Hz, 2H), 2.36 (t, *J* = 7.5 Hz, 2H), 2.28 (s, 3H), 1.87 (s, 6H), 1.71 (s, 2H), 1.52 (dt, *J* = 15.2, 7.5 Hz, 2H), 1.30 (dt, *J* = 14.7, 7.4 Hz, 2H), 0.99 (t, *J* = 7.0 Hz, 3H), 0.83 (t, *J* = 7.3 Hz, 3H).

### Example 111

### (2-(4-((2-butyl-4-oxo-1,3-diazaspiro[4.4]non-1-en-3-yl)methyl)-2-(ethoxymethyl)phenyl)-N-(4-fluoro-5-methylisoxazol-3-yl)pyridine-3-sulfonamide

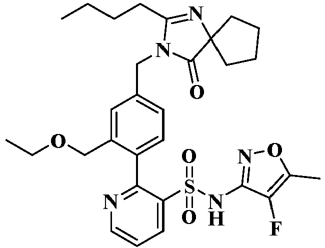

Synthesis method of Example 111 referred to the synthesis method of Example 1, 4-fluoro-5-methylisoxazoleamine was used instead of 4,5-dimethylisoxazoleamine to afford Example 111 (29 mg, yield: 55.6%).
MS m/z (ESI): 598.3 [M+1]
¹H NMR (400 MHz, DMSO-d6) δ 9.13 (s, 1H), 8.79 (dd, J = 7.5, 1.5 Hz, 1H), 8.29 (dd, J = 7.5, 1.5 Hz, 1H), 8.03 (d, J = 7.4 Hz, 1H), 7.54 (q, J = 1.1 Hz, 1H), 7.50 - 7.43 (m, 2H), 5.00 (t, J = 1.0 Hz, 2H), 4.77 (d, J = 1.1 Hz, 2H), 3.58 (q, J = 8.0 Hz, 2H), 2.55 (t, J = 7.1 Hz, 2H), 2.30 (s, 2H), 2.11 - 2.00 (m, 6H), 1.99 - 1.89 (m, 2H), 1.60 (p, J = 7.1 Hz, 2H), 1.40 (dtd, J = 15.1, 7.9, 6.9 Hz, 2H), 1.19 (t, J = 8.0 Hz, 3H), 0.91 (t, J = 8.0 Hz, 3H).

### Example 112

### 4'-(2-butyl-4-chloro-5-(hydroxymethyl)-1H-imidazol-1-yl)methyl)-2'-(ethoxymethyl)-N-(4-fluoro-5-methylisoxazol-3-yl)-(1,1'-biphenyl)-2-sulfonamide

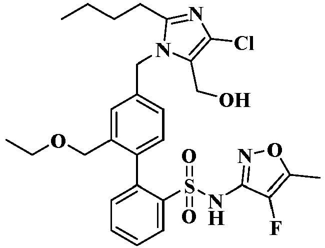

Synthesis method of Example 112 referred to the synthesis method of Example 1, 4-fluoro-5-methylisoxazoleamine was used instead of 4,5-dimethylisoxazoleamine to afford Example 112 (25 mg, yield: 52.6%).
MS m/z (ESI): 591.2 [M+1]
¹H NMR (400 MHz, DMSO-d6) δ 9.10 (s, 1H), 8.26 (dd, J = 7.4, 1.4 Hz, 1H), 7.65 (dd, J = 7.5, 1.9 Hz, 1H), 7.63 (td, J = 7.2, 1.4 Hz, 1H), 7.46 (td, J = 7.3, 2.0 Hz, 1H), 7.31 (dq, J = 1.8, 1.1 Hz, 1H), 7.21 (d, J = 7.4 Hz, 1H), 7.00 (dt, J = 7.6, 1.3 Hz, 1H), 5.09 (t, J = 1.0 Hz, 2H), 4.77 (d, J = 7.5 Hz, 2H), 4.70 (d, J = 1.1 Hz, 2H), 3.71 (t, J = 7.7 Hz, 1H), 3.58 (q, J = 8.0 Hz, 2H), 2.57 (t, J = 7.1 Hz, 2H), 2.30 (s, 2H), 1.73 - 1.63 (m, 2H), 1.57 - 1.46 (m, 2H), 1.19 (t, J = 8.0 Hz, 3H), 0.94 (t, J = 8.0 Hz, 3H).

### Example 113

### 4'-((2-butyl-4-oxo-1,3-diazaspiro[4.4]non-1-en-3-yl)methyl)-N-(4,5-dimethylisoxazol-3-yl)-2' -propoxy-[1,1'-biphenyl]-2-sulfonamide

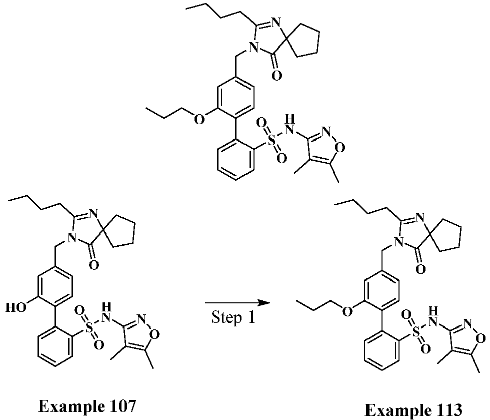

Example 107 (100 mg, 0.18 mmol) and potassium carbonate (45 mg, 0.36mmol) were dissolved in dichloromethane (5 mL), and then bromopropane (44 mg, 0.36 mmol) was added to the reaction liquid, and the reaction liquid was stirred at room temperature for 2 h. Saturated brine (10 mL) was added to the reaction liquid, and the mixture was extracted with ethyl acetate (10 mL × 3). The organic phases were combined, dried, concentrated and purified to afford Example 113 (52 mg, yield: 49.1%).

MS m/z (ESI): 593.3 [M+1] ⁺.

¹H NMR (400 MHz, DMSO) δ 10.86 (s, 1H), 7.96 (d, J = 7.1 Hz, 2H), 7.63 - 7.15 (m, 2H), 7.20 - 6.91 (m, 2H), 6.75 - 6.45 (m, 2H), 4.63 (s, 2H), 3.75 (dd, J = 36.1, 7.0 Hz, 2H), 3.31 (s, 3H), 2.29 (dd, J = 25.0, 17.5 Hz, 2H), 2.18 (s, 3H), 1.78 (d, J = 7.2 Hz, 6H), 1.62 (d, J = 7.3 Hz, 2H), 1.46 (dt, J = 15.2, 7.5 Hz, 2H), 1.22 (ddd, J = 20.5, 14.1, 6.6 Hz, 3H), 0.94 (t, J = 6.9 Hz, 3H), 0.76 (t, J = 7.3 Hz, 3H).

### Example 114

### 4'-(2-butyl-4-oxo-1,3-diazaspiro[4.4]non-1-en-3-yl)methyl)-2'-(ethoxymethyl)-N-(5-methyl-4-(trifluoromethyl)isoxazol-3-yl)-[1,1'-biphenyl]-2-sulfonamide

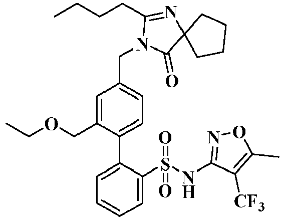

Synthesis method of Example **114** referred to the synthesis method of Example **1,** 5-methyl-4-(trifluoromethyl)isoxazol-3-amine was used instead of 4,5-dimethylisoxazolamine to afford Example **114** (12.5 mg, yield: 8.6%).

MS m/z (ESI): 647.2[M+1] ⁺.

¹H NMR (400 MHz, DMSO-*d*₆) δ 8.00 - 7.98 (m, 1H), 7.38 - 7.32 (m, 2H), 7.10 (s, 1H), 6.99 (d, *J* = 7.6 Hz, 1H), 6.94 - 6.91 (m, 1H), 6.88 (d, *J* = 8.0 Hz, 1H), 4.70 (s, 2H), 4.08 (d, *J* = 13.2 Hz, 1H), 3.98 (d, *J* = 13.2 Hz, 1H), 3.23 - 3.18 (m, 2H), 2.37 (t, *J* = 7.6 Hz, 2H), 2.26 (s, 3H), 1.91 - 1.82 (m, 6H), 1.72 - 1.66 (m, 2H), 1.57 - 1.50 (m, 2H), 1.34 - 1.28 (m, 2H), 1.02 (t, *J* = 6.8 Hz, 3H), 0.84 (t, *J* = 7.2 Hz, 3H).

### Example 115

### 4'-(2-butyl-4-oxo-1,3-diazaspiro[4.4]non-1-en-3-yl)methyl)-N-(4-cyano-3-methylisoxazol-5-yl)-2'-(ethoxymethyl)-[1,1'-biphenyl]-2-sulfonamide

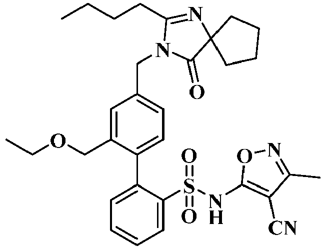

Synthesis method of Example **115** referred to the synthesis method of Example 1, 5-amino-3-methylisoxazole-4-carbonitrile was used instead of 4,5-dimethylisoxazoleamine to afford Example **115** (5.1 mg, yield: 6.6%).

MS m/z (ESI): 604.2 [M+1] ⁺.

¹H NMR (400 MHz, DMSO-*d*₆) δ 8.02 - 7.99 (m, 1H), 7.49 - 7.45 (m, 2H), 7.13 (s, 1H), 7.07 - 7.05 (m, 1H), 7.02 (d, *J* = 7.6 Hz, 1H), 6.95 (d, *J* = 8.0 Hz, 1H), 4.72 (s, 2H), 4.08 (d, *J* = 13.2 Hz, 1H), 3.96 (d, *J* = 13.2 Hz, 1H), 3.26 - 3.18 (m, 2H), 2.35 (t, *J* = 7.6 Hz, 2H), 2.01 (s, 3H), 1.91 - 1.82 (m, 6H), 1.73 - 1.66 (m, 2H), 1.55 - 1.48 (m, 2H), 1.32 - 1.24 (m, 2H), 1.02 (t, *J* = 6.8 Hz, 3H), 0.83 (t, *J* = 7.2 Hz, 3H).

### Example 116

### 4'-(2-butyl-4-oxo-1,3-diazaspiro[4.4]non-1-en-3-yl)methyl)-N-(4-chloro-5-methylisoxazol-3-yl)-2'-ethoxy-[1,1'-biphenyl]-2-sulfonamide

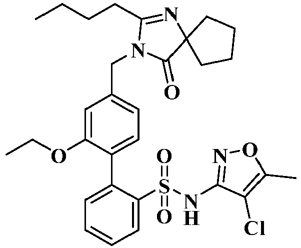

Synthesis method of Example **116** referred to the synthesis method of Example **113,** and ethyl iodide was used instead of propyl iodide to afford Example **116** (27.0 mg, yield: 34.5%).

MS m/z (ESI): 599.0 [M+1] ⁺.

¹H NMR (400 MHz, DMSO-*d*₆) δ 10.91 (br s, 1H), 8.03 (d, *J* = 8.0 Hz, 1H), 7.58 - 7.47 (m, 2H), 7.20 - 6.97 (m, 2H), 6.72 (s, 1H), 6.63 (d, *J* = 8.0 Hz, 1H), 4.70 (s, 2H), 3.91 - 3.83 (m, 1H), 3.79 - 3.72 (m, 1H), 2.38 (t, *J* = 7.6 Hz, 2H), 2.25 (s, 3H), 1.92 - 1.82 (m, 6H), 1.71 - 1.65 (m, 2H), 1.56 - 1.49 (m, 2H), 1.33 - 1.27 (m, 2H), 1.01 (t, *J* = 6.8 Hz, 3H), 0.83 (t, *J* = 7.2 Hz, 3H).

### Example 117

### Methyl 1-((2'-(N-(4-chloro-5-methylisoxazol-3-yl)-N-(methoxymethyl)sulfamoyl)-2-(ethoxymethyl)-[1,1'-biphenyl]-4-yl)methyl)-4-ethyl-2-propyl-1H-imidazole-5-carboxylate

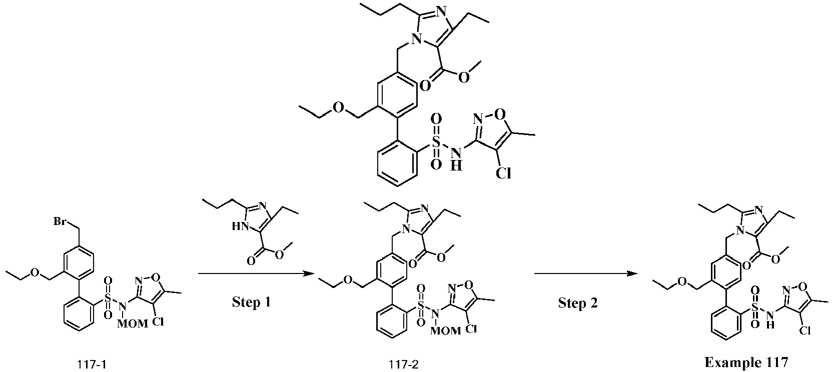

Example 117-1 (300 mg, 0.55 mmol) (referring to WO 2010114801 A1 for the preparation method) was dissolved in acetonitrile (15 mL), and methyl 4-ethyl-2-propyl-1H-imidazole-5-carboxylate (108 mg, 0.55 mmol) and potassium carbonate (160 mg, 1.1 mmol) were added, and the reaction liquid was heated to reflux for 6 h. The reaction liquid was concentrated, and the crude product was subjected to reverse phase HPLC to afford Example 117-2 (300 mg, yield: 82.5%).

MS m/z (ESI): 659.2 [M+1] +.

### Step 2

### Preparation of methyl 1-((2'-(N-(4-chloro-5-methylisoxazol-3-yl)-N-(methoxymethyl)sulfamoyl)-2-(ethoxymethyl)-[1,1'-biphenyl]-4-yl)methyl)-4-ethyl-2-propyl-1H-imidazole-5-carboxylate

Example 117-2 (300 mg, 0.46 mmol) was dissolved in 4M HCl/dioxane (5 mL), and the reaction liquid was heated to 70 degrees for 2 h. The reaction liquid was concentrated, and the crude product was subjected to reverse phase HPLC to afford Example 117 (200 mg, yield: 71.4%).

MS m/z (ESI): 615.2 [M+1] ⁺.

¹H NMR (400 MHz, DMSO-d6) δ 9.07 (s, 1H), 8.26 (dd, J = 7.4, 1.4 Hz, 1H), 7.68 - 7.59 (m, 2H), 7.46 (td, J = 7.3, 2.0 Hz, 1H), 7.30 (dq, J = 2.0, 1.0 Hz, 1H), 7.21 (d, J = 7.4 Hz, 1H), 7.00 (dq, J = 7.5, 1.1 Hz, 1H), 5.43 (t, J = 1.0 Hz, 2H), 4.70 (d, J = 1.1 Hz, 2H), 3.94 (s, 3H), 3.58 (q, J = 8.0 Hz, 2H), 2.99 (q, J = 8.0 Hz, 2H), 2.58 (s, 3H), 2.29 (s, 2H), 1.71 (dtd, J = 15.1, 8.0, 7.1 Hz, 2H), 1.37 (t, J = 8.0 Hz, 3H), 1.19 (t, J = 8.0 Hz, 3H), 1.01 (t, J = 8.0 Hz, 3H).

### Example 118

### 4'-(2-butyl-4-oxo-1,3-diazaspiro[4.4]non-1-en-3-yl)methyl)-2'-(ethoxymethyl)-N-(5-methoxy-4-methylisoxazol-3-yl)-(1,1'-biphenyl)-2-sulfonamide

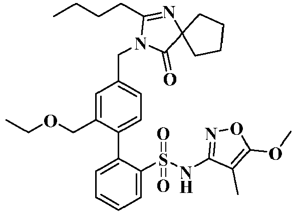

Synthesis method of Example **118** referred to the synthesis method of Example **1,** 5-methoxy-4-methylisoxazole-3-amine was used instead of 4,5-dimethylisoxazoleamine to afford Example **118** (11.5 mg, yield: 8.3%).

MS m/z (ESI): 609.3 [M+1] ⁺.

¹H NMR (400 MHz, DMSO-*d*₆) δ 7.98 - 7.96 (m, 1H), 7.38 - 7.36 (m, 2H), 7.09 (s, 1H), 7.01 (d, *J* = 7.6 Hz, 1H), 6.97 - 6.91 (m, 2H), 4.71 (s, 2H), 4.03 (d, *J* = 13.2 Hz, 1H), 3.96 (d, *J* = 13.2 Hz, 1H), 3.67 (s, 3H), 3.25 - 3.17 (m, 2H), 2.35 (t, *J* = 7.6 Hz, 2H), 1.90 - 1.81 (m, 6H), 1.72 - 1.66 (m, 2H), 1.54 - 1.48 (m, 2H), 1.35 (s, 3H), 1.32 - 1.26 (m, 2H), 1.01 (t, *J=* 6.8 Hz, 3H), 0.83 (t, *J* = 7.2 Hz, 3H).

### Example 119

### 4'-(2-butyl-4-oxo-1,3-diazaspiro[4.4]non-1-en-3-yl)methyl)-N-(5-cyclopropyl-4-methylisoxazol-3-yl)-2'-(ethoxymethyl)-[1,1'-biphenyl]-2-sulfonamide

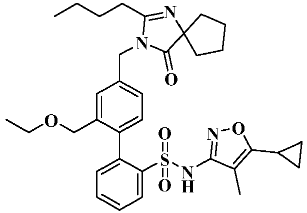

Synthesis method of Example **119** referred to the synthesis method of Example **1,** 5-cyclopropyl-4-methylisoxazole-3-amine was used instead of 4,5-dimethylisoxazoleamine to afford Example **119** (13.2 mg, yield: 9.6%).

MS m/z (ESI): 619.3 [M+1] ⁺.

¹H NMR (400 MHz, DMSO-*d*₆) δ 8.02 - 7.99 (m, 1H), 7.50 - 7.42 (m, 2H), 7.12 (s, 1H), 7.06 - 7.00 (m, 1H), 6.95 (s, 2H), 4.72 (s, 2H), 4.00 - 3.91 (m, 2H), 3.22 - 3.17 (m, 2H), 2.36 (t, *J* = 7.6 Hz, 2H), 1.92 - 1.81 (m, 8H), 1.74 - 1.65 (m, 2H), 1.59 (s, 3H), 1.55 - 1.51 (m, 2H), 1.32 - 1.27 (m, 2H), 1.01 (t, *J* = 6.8 Hz, 3H), 0.89 - 0.86 (m, 2H), 0.83 (t, *J* = 7.6 Hz, 3H), 0.77 - 0.74 (m, 2H).

### Example 120

### methyl 1-((2'-(N-(4-chloro-5-methylisoxazol-3-yl)-N-(methoxymethyl)sulfamoyl)-2-(ethoxymethyl)-[1,1'-biphenyl]-4-yl)methyl)-4-ethyl-2-propyl-1H-imidazole-5 carboxylic acid

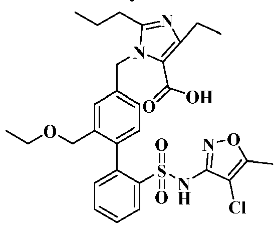

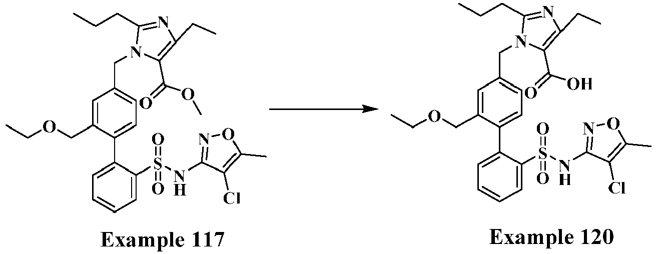

Compound 117 (100 mg, 0.16 mmol) and NaOH (2 M, 1.5 mL) were added to tetrahydrofuran (10 mL). The reaction liquid was stirred at 25°C for 4 h, 1M HCl (10 ml) was added, the mixture was extracted with dichloromethane (30ml*2), and the combined extracts were dried over Na₂SO₄, spined to dryness, and the obtained crude product was purified with silica gel column chromatography to afford the title compound **120** (60 mg, yield: 61%).
MS m/z (ESI): 601.2 [M+1]⁺
¹H NMR (400 MHz, DMSO-d6) δ 11.07 (s, 1H), 9.07 (s, 1H), 8.26 (dd, J = 7.4, 1.4 Hz, 1H), 7.65 (dd, J = 7.5, 1.9 Hz, 1H), 7.63 (td, J = 7.2, 1.4 Hz, 1H), 7.46 (td, J = 7.3, 2.0 Hz, 1H), 7.31 (dq, J = 1.8, 1.1 Hz, 1H), 7.21 (d, J = 7.4 Hz, 1H), 7.00 (dt, J = 7.6, 1.2 Hz, 1H), 5.42 (t, J = 1.0 Hz, 2H), 4.70 (d, J = 1.1 Hz, 2H), 3.58 (q, J = 8.0 Hz, 2H), 2.96 (q, J = 8.0 Hz, 2H), 2.58 (t, J = 7.1 Hz, 3H), 2.29 (s, 2H), 1.71 (dtd, J = 15.1, 8.0, 7.1 Hz, 2H), 1.37 (t, J = 8.0 Hz, 3H), 1.19 (t, J = 8.0 Hz, 3H), 1.01 (t, J = 8.0 Hz, 3H).

### Example 121

### N-(4-chloro-5-methylisoxazol-3-yl)-2'-(ethoxymethyl)-4'-(4-oxy-2-(4,4,4-trifluorobutyl)-1,3-diazaspiro[4.4]non-1-en-3-yl)methyl)-[1,1'-biphenyl]-2-sulfonamide

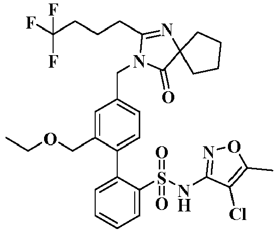

Synthesis method of Example 121 referred to the synthesis method of Example 1, 4-chloro-5-methylisoxazoleamine was used instead of 4,5-dimethylisoxazoleamine to afford Example 121 (27 mg, yield: 51.5%).
MS: m/z (ESI): 667.2 [M+1]⁺
¹H NMR (400 MHz, DMSO-d6) δ 9.07 (s, 1H), 8.26 (dd, J = 7.4, 1.4 Hz, 1H), 7.65 (dd, J = 7.5, 1.9 Hz, 1H), 7.63 (td, J = 7.2, 1.4 Hz, 1H), 7.51 - 7.43 (m, 2H), 7.33 (dq, J = 7.5, 1.1 Hz, 1H), 7.28 (d, J = 7.5 Hz, 1H), 5.00 (t, J = 1.0 Hz, 2H), 4.70 (d, J = 1.1 Hz, 2H), 3.58 (q, J = 8.0 Hz, 2H), 2.70 (t, J = 7.1 Hz, 2H), 2.34 (qt, J = 9.0, 7.1 Hz, 2H), 2.29 (s, 3H), 2.11 - 2.00 (m, 6H), 1.99 - 1.89 (m, 2H), 1.74 (p, J = 7.1 Hz, 2H), 1.19 (t, J = 8.0 Hz, 3H).

### Example 122

### 4'-(2-butyl-4-oxo-1,3-diazaspiro[4.4]non-1-en-3-yl)methyl)-N-(4-chloro-5-methylisoxazol-3-yl)-2'-(phenoxymethyl)-[1,1'-biphenyl]-2-sulfonamide

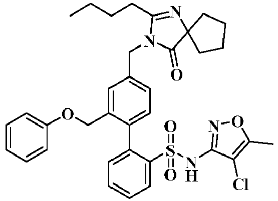

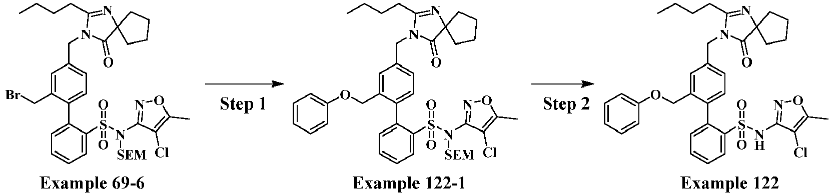

### Step 1

### Preparation of 4'-(2-butyl-4-oxo-1,3-diazaspiro[4.4]non-1-en-3-yl)methyl)-N-(4-chloro-5-methylisoxazol-3-yl)-2'-(phenoxymethyl)-N-((2-(trimethylsilyl)ethoxy) methyl)-[1,1'-biphenyl]-2-sulfonamide

Compound 69-6 (60 mg, 0.077 mmol) was dissolved in anhydrous acetonitrile (3 mL), phenol (22 mg, 0.231 mmol) and cesium carbonate (75 mg, 0.231 mmol) were added, and the mixture was heated to 50°C for 2 h. The reaction liquid was poured into 30 mL of water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure, and the residue was purified with silica gel column chromatography to afford the title product **122-1** (60 mg, yield: 98.3%).
MS: m/z (ESI): 791.3 [M+1]

### Step 2

### Preparation of 4'-(2-butyl-4-oxo-1,3-diazaspiro[4.4]non-1-en-3-yl)methyl)-N-(4-chloro-5-methylisoxazol-3-yl)-2'-(phenoxymethyl)-[1,1'-biphenyl]-2-sulfonamide

Synthesis method of compound **122** referred to the synthesis method of Example **12,** compound **122-1** was used as raw material to afford the title compound Example **122** (30.3 mg, yield: 60.1%).

MS m/z (ESI): 661.2[M+1] ⁺.

¹H NMR (400 MHz, DMSO-*d*₆) δ 12.70 (br s, 1H), 8.01 - 7.99 (m, 1H), 7.47 - 7.37 (m, 2H), 7.20 - 7.15 (m, 3H), 7.09 - 7.00 (m, 3H), 6.88 - 6.84 (m, 1H), 6.70 (d, *J* = 8.0 Hz, 2H), 4.73 (d, *J* = 13.2 Hz, 1H), 4.71 (s, 2H), 4.51 (d, *J* = 13.2 Hz, 1H), 2.31 (t, *J* = 7.6 Hz, 2H), 2.13 (s, 3H), 1.90 - 1.79 (m, 6H), 1.68 - 1.61 (m, 2H), 1.52 - 1.45 (m, 2H), 1.29 - 1.24 (m, 2H), 0.82 (t, *J* = 7.2 Hz, 3H).

### Intermediate 3

### 2'-(bromomethyl)-4'-(2-butyl-4-oxo-1,3-diazaspiro[4.5]dec-1-en-3-yl)methyl)-N-(4,5-dimethylisoxazol-3-yl)-N-(methoxymethyl)-[1,1'-biphenyl]-2-sulfonamide

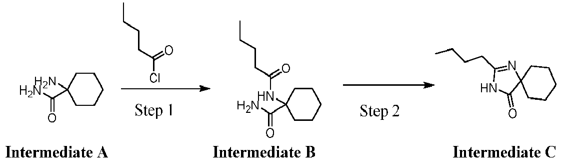

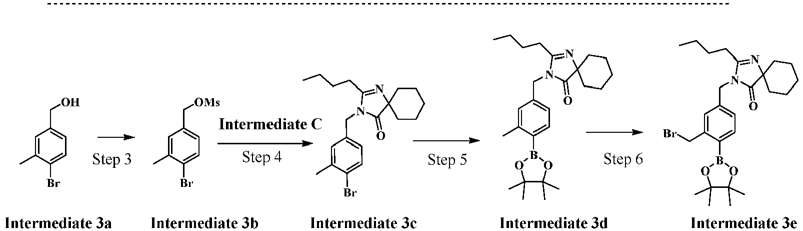

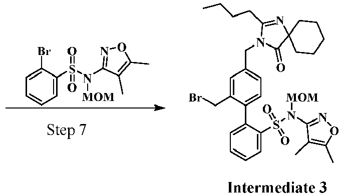

### Step 1: Preparation of 1-pentamidocyclohexane-1-carboxamide

Valeryl chloride (7.2 g, 60 mmol) and triethylamine (17 mL, 120 mmol) were added to a solution of 1-amino-cyclohexanecarboxamide (8.0 g, 56 mmol) in dichloromethane (200 mL) under an ice bath, and the reaction was continued for 1 h under ice bath under stirring. After the reaction was completed, 50 mL of water was added and the mixture was extracted with DCM (80 mL × 3). The organic layer was dried over MgSO4. After filtration, the solvent was removed under reduced pressure. The target product 1-pentamidocyclohexane-1-carboxamide (12 g, yield: 94.3%) was obtained.

MS m/z (ESI): 227.1 [M+1] ⁺.

### Step 2: Preparation of 2-butyl-1,3-diazaspiro-[4,5]dec-1-en-4one

Sodium hydroxide (10 M, 50 ml) was slowly added to a solution of intermediate B (12.0 g, 53 mmol) in methanol (80 mL), and the reaction was stirred at 60°C for 3 h. After the reaction was completed, water (30 mL) and dichloromethane (60 mL × 3) were added for extraction. The organic layers were combined, dried over anhydrous sodium sulfate, filtered, concentrated, and purified with column (petroleum ether/ethyl acetate system) to afford the target product intermediate C (8 g, yield: 70.2%).

MS m/z (ESI): 209.1 [M+1] ⁺.

### Step 3: Preparation of 4-bromo-3-methylbenzylmethanesulfonate

(4-bromo-3-methylphenyl)methanol (2.0 g, 10.0 mmol) and triethylamine (1.4 g, 11.0 mmol) were dissolved in dichloromethane (5 mL), and methylsulfonyl chloride was added (1.24 g, 11.0 mmol) under ice bath, and the mixture was stirred at room temperature for 1 h. Water and dichloromethane (20 mL × 3) were added for extraction. The organic layers were combined, dried over anhydrous sodium sulfate, filtered, concentrated, and purified with column (petroleum ether/ethyl acetate system) to afford the target product 4-bromo-3-methylbenzylmethanesulfonate (2.6 g, yield: 93.5%).

MS m/z (ESI): 278.9 [M+1] ⁺.

### Step 4 Preparation of 3-(4-bromo-3-methylbenzyl)-2-butyl-1,3-diazaspiro[4.5]decan-1-en-4-one

Sodium hydride (0.34 g, 8.6 mmol) was added to a solution of intermediate 3b (2.0 g, 7.2 mmol) in N,N-dimethylformamide (5 mL) under ice bath, and the reaction was stirred under ice bath for 1 h. 2-butyl-1,3-diazaspiro[4.5]dec-1-en-4-one (1.7 g, 8.**2**mmol) was added, and the reaction liquid was stirred at room temperature for 1 h. Water and dichloromethane (20 mL × 3) were added for extraction. The organic layers were combined, dried over anhydrous sodium sulfate, filtered, concentrated and purified with column (petroleum ether/ethyl acetate system) to afford the target product 3-(4-bromo-3-methylbenzyl)-2-butyl-1,3-diazaspiro[4.5]decan-1-en-4-one(2.3 g, yield: 82.1%).

MS m/z (ESI): 391.1 [M+1] ⁺.

### Step 5 Preparation of 2-butyl-3-(3-methyl-4-(4,4,5,5-tetramethyl-1,3,2-dioxaborolan-2-yl)benzyl)-1,3-diazaspiro[4.5]decan-1-en-4-one

Intermediate 3c (2.07 g, 5.3 mmol), bis(pinacolato)diboron (1.6 g, 6.4 mmol), [1,1'-bis(diphenylphosphino)ferrocene]palladium dichloride dichloromethane complex (42 mg, 0.05 mmol) and potassium acetate (1.1 g, 10.6 mmol) were dissolved in dioxane (35 mL), and the reaction liquid was stirred at 80°C under nitrogen protection for 16 h. Water and dichloromethane (50 mL × 2) were added for extraction. The organic layers were combined, dried over anhydrous sodium sulfate, filtered, concentrated and purified with column (petroleum ether/ethyl acetate system) to afford the target product 2-butyl-3-(3-methyl-4-(4,4,5,5-tetramethyl-1,3,2-dioxaborolan-2-yl)benzyl)-1,3-diazaspiro[4.5]decan-1-en-4-one (1.8 g, yield: 77.6%).

MS m/z (ESI): 439.3 [M+1] ⁺.

### Step 6 Preparation of 3-(3-(bromomethyl)-4-(4,4,5,5-tetramethyl-1,3,2-dioxaborolan-2-yl)benzyl)-2-butyl-1,3-diazaspiro[4.5]decan-1-en-4-one

N-bromosuccinimide (0.75 g, 4.2 mmol) was added to a solution of intermediate 3d (1.55 g, 3.5 mmol) in acetonitrile (5 mL) under ice bath, and the reaction liquid was stirred at room temperature for 1 h. Water and dichloromethane (3 × 20 mL) were added for extraction. The organic layers were combined, dried over anhydrous sodium sulfate, filtered, concentrated and purified with column (petroleum ether/ethyl acetate system) to afford the target product 3-(3-(bromomethyl)-4-(4,4,5,5-tetramethyl-1,3,2-dioxaborolan-2-yl)benzyl)-2-butyl-1,3-diazaspiro[4.5]decan-1-en-4-one(1.62 g, yield: 88.5%).

MS m/z (ESI): 517.2 [M+1] ⁺.

### Step 7 Preparation of 2'-(bromomethyl)-4'-(((2-butyl-4-oxo-1,3-diazaspiro[4.5]decan-1-en-3-yl)methyl)-N-(4,5-dimethylisoxazol- 3-yl)-N-(methoxymethyl)-[1,1'-biphenyl] -2-sulfonamide

Intermediate 3e (100 mg, 0.19 mmol), (2-(N-(4,5-dimethylisoxazol-3-yl)-N-(methoxymethyl)sulfamoyl)phenyl)boronic acid (75 mg, 0.2 mmol)(referring to WO 2010135350 A2 for the preparation method), [1,1'-bis(diphenylphosphino)ferrocene]palladium dichloride dichloromethane complex (16 mg, 0.02 mmol) and cesium carbonate (291 mg, 0.9 mmol) were dissolved indioxane(4 mL) and water (1 mL), the reaction liquid was stirred at 100°C under microwave for 1 h. Water and dichloromethane (20 mL × 3) were added for extraction. The organic layers were combined, dried over anhydrous sodium sulfate, filtered, concentrated, and purified with column (petroleum ether/ethyl acetate system) to afford intermediate 3 (110 mg, yield: 83%).

MS m/z (ESI): 685.2 [M+1] ⁺.

### Intermediate 4

### 2'-butylspiro[bicyclo[3.1.0]hexane-3,4'-imidazole]-5'(1'H)-one

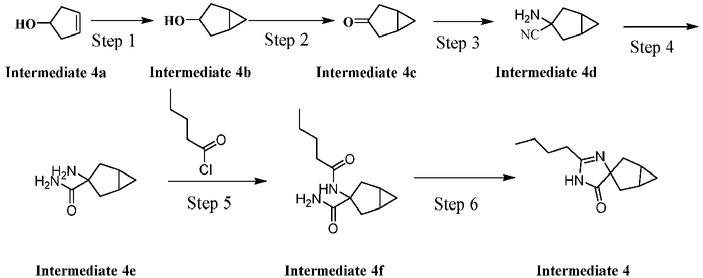

### Step 1: Preparation of bicyclo[3.1.0]hexan-3-ol

Cyclopent-3-en-1-ol (2 g, 24 mmol) was dissolved in 200 mL of dichloromethane under nitrogen protection under an ice bath, diethylzinc (5.9 g, 48 mmol) and diiodomethane (12.7 mL, 48 mmol) were added and the reaction was stirred under an ice bath for 3 h. After the reaction was completed, 50 mL of water was added and the mixture was extracted with DCM (80 mL × 3). The organic layer was dried over MgSO₄. After filtration, the solvent was removed under reduced pressure. The target product intermediate 4b (2 g, yield: 86%) was obtained.

MS m/z (ESI): 99.1 [M+1] ⁺.

### Step 2: Preparation of bicyclo[3.1.0]hexan-3-one

Intermediate 4b (2.0 g, 20 mmol) was dissolved in dichloromethane (40 mL), Dess-Martin (10 g, 24 mmol) was slowly added, and the reaction was stirred at 20°C for 3 h. Water (30 mL) and dichloromethane (30 mL × 3) were added for extraction. The organic layers were combined, dried over anhydrous sodium sulfate, filtered, concentrated, and purified with column (petroleum ether/ethyl acetate system) to afford the target product intermediate 4c (1.7 g, yield: 87%).

MS m/z (ESI): 97.1 [M+1] ⁺.

### Step 3: Preparation of 3-aminobicyclo[3.1.0]hexane-3-carbonitrile

Intermediate 4c (1.7 g, 17.7 mmol) and ammonium chloride (1.42 g, 26.5 mmol) were dissolved in DMF (5 mL) and water (25 mL), and potassium cyanide (1.73 g, 26.5 mmol) was added under ice bath, and the reaction liquid was stirred at room temperature for 2 h. Water and dichloromethane (20 mL × 3) were added for extraction. The organic layers were combined, dried over anhydrous sodium sulfate, filtered, concentrated, and purified with column (petroleum ether/ethyl acetate system) to afford the target product intermediate 4d (1.5 g, yield: 69.4%).

MS m/z (ESI): 123.1 [M+1] ⁺.

### Step 4: Preparation of 3-aminobicyclo[3.1.0]hexane-3-carboxamide

Sulfuric acid (5 mL) was added to a solution of intermediate 4d (1.5 g, 12.3 mmol) in dichloromethane (15 mL) under ice bath, and the reaction liquid was stirred under ice bath for 3 h. Water and dichloromethane (20 mL × 3) were added for extraction. The organic layers were combined, dried over anhydrous sodium sulfate, filtered, concentrated, and purified with column (petroleum ether/ethyl acetate system) to afford the target product intermediate 4e (1.6 g, yield: 93%).

MS m/z (ESI): 141.1 [M+1] ⁺.

### Step 5: Preparation of 3-pentaamido bicyclo[3.1.0]hexane-3-carboxamide

Valeryl chloride (1.5 g, 12.55 mmol) and triethylamine (2.8 mL, 22.8 mmol) were added to a solution of intermediate 4e (1.6 g, 11.4 mmol) in 30 mL of dichloromethane under ice bath, and the reaction liquid was stirred under ice bath for 1 h. After the reaction was completed, 50 mL of water was added and the mixture was extracted with DCM (80 mL × 3). The organic layer was dried over MgSO4. After filtration, the solvent was removed under reduced pressure. The target product intermediate 4f (2.3 g, yield: 89.8%) was obtained.

MS m/z (ESI): 225.1 [M+1] ⁺.

### Step 6: Preparation of 2'-butylspiro[bicyclo[3.1.0]hexane-3,4'-imidazole]-5'(1'H)-one

Sodium hydroxide (10 M, 30 ml) was slowly added to a solution of intermediate 4f (2.3 g, 10.25 mmol) in methanol (40 mL), and the reaction liquid was stirred at 60°C for 3 h. Water (30 mL) and dichloromethane (60 mL × 3) were added for extraction. The organic layers were combined, dried over anhydrous sodium sulfate, filtered, concentrated, and purified with column (petroleum ether/ethyl acetate system) to afford the target product intermediate 4 (1.3 g, yield: 61.3%).

MS m/z (ESI): 207.1 [M+1] ⁺.

¹H NMR (400 MHz, DMSO-d6) δ 13.30 (s, 1H), 2.71 (t, J = 7.7 Hz, 2H), 2.37 (dd, J = 14.7, 4.0 Hz, 2H), 2.09 (d, J = 14.4 Hz, 2H), 1.68 (p, J = 7.6 Hz, 2H), 1.53 (dt, J = 8.5, 4.1 Hz, 2H), 1.33 (dt, J = 14.6, 7.4 Hz, 2H), 0.99 - 0.81 (m, 4H), 0.60 (td, J = 8.3, 4.8 Hz, 1H).

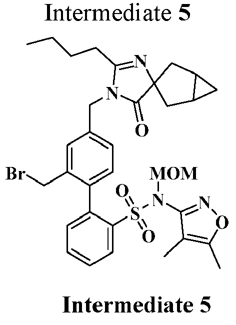

Synthesis method of **intermediate 5** referred to the synthesis method of steps 4 to 7 of **intermediate 3,** 2'-butylspiro[bicyclo[3.1.0 ]hexane-3,4'-imidazole]-5'(1'H)-one was used instead of 2-butyl-1,3-diazaspiro-[4,5]dec-1-en-4one to afford **intermediate 5**(2g), yield: 65.8%.

MS m/z (ESI): **683.2** [M+1] ⁺.

### Example 123

### 4'-((2'-butyl-5'-oxopyrrolo[bicyclo[3.1.0]hexane-3,4'-imidazole]-1'(5'H)-yl)methyl)-2'-((cyclopropylmethoxy)methyl)-N-(4,5-dimethylisoxazol-3-yl)-[1,1'-biphenyl]-2-sulfonamide

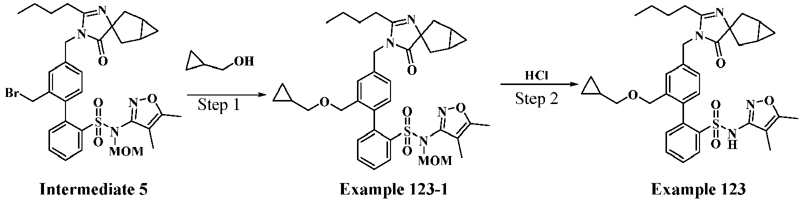

### Step 1

### Preparation of 4'-((2'-butyl-5'-oxopyrrolo[bicyclo[3.1.0]hexane-3,4'-imidazole]-1'(5'H)-yl)methyl)-2'-((cyclopropylmethoxy)methyl)-N-(4,5-dimethylisoxazol-3-yl)-N-(methoxymethyl)-[1,1'-biphenyl]-2-sulfonamide

Cyclopropylmethanol (46 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 5** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **123-1** (70 mg,), yield: 80.6%, which was directly used in the next reaction.

MS m/z (ESI): 675.3 [M+1] ⁺.

### Step 2

### Preparation of 4'-((2'-butyl-5'-oxopyrrolo[bicyclo[3.1.0]hexane-3,4'-imidazole]-1'(5'H)-yl)methyl)-2'-((cyclopropylmethoxy)methyl)-N-(4,5-dimethylisoxazol-3-yl)-[1,1'-biphenyl]-2-sulfonamide

Example **123-1** (70 mg, 0.104mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **123** (30 mg), yield: 45.8%.

MS m/z (ESI): 631.2 [M+1] ⁺.

### Example 124

### 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(methoxymethyl)-[1,1'-biphenyl]-2-sulfonamide

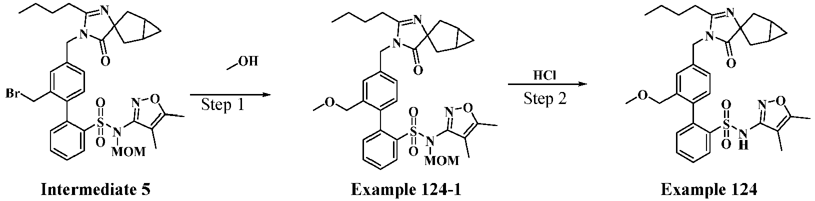

### Step 1

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-N, 2'-bis(methoxymethyl)-[1,1'-biphenyl]-2-sulfonamide

Methanol (20.5 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 5** (88 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **124-1** (65 mg,), yield: 79.6%, which was directly used in the next reaction.

MS m/z (ESI): 635.3 [M+1] ⁺.

### Step 2

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(methoxymethyl)-[1,1'-biphenyl]-2-sulfonamide

Example **124-1** (65 mg, 0.102 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **124** (38 mg), yield: 62.8%.

MS m/z (ESI): 591.2 [M+1] ⁺.

### Example 125

### 4'-((2'-butyl-5'-oxopyrrolo[bicyclo[3.1.0]hexane-3,4'-imidazolel-1'(5'H)-yl)methyl)-2'-(cyclobutoxymethyl)-N-(4,5-dimethylisoxazol-3-yl)-[1,1'-biphenyl]-2-sulfonamide

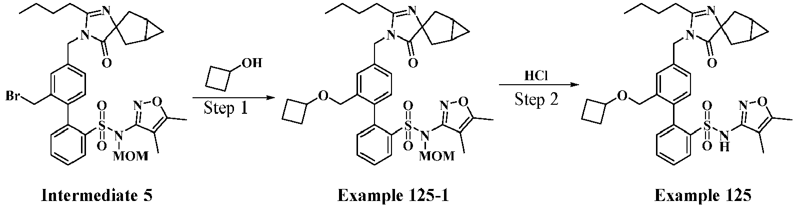

### Step 1

### Preparation of 4'-(2'-butyl-5'-oxopyrrolo[bicyclo[3.1.0]hexane-3,4'-imidazole]-1'(5'H)-yl)methyl)-2'-(cyclobutoxymethyl)-N-(4,5-dimethylisoxazol-3-yl)-N-(methoxymethyl)-[1,1'-biphenyl]-2-sulfonamide

Cyclobutanol (46 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 5** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **125-1** (70 mg,), yield: 80.6%, which was directly used in the next reaction.

MS m/z (ESI): 675.3 [M+1] ⁺.

### Step 2

### Preparation of 4'-((2'-butyl-5'-oxopyrrolo[bicyclo[3.1.0]hexane-3,4'-imidazole]-1'(5'H)-yl)methyl)-2'-(cyclobutoxymethyl)-N-(4,5-dimethylisoxazol-3-yl)-[1,1'-biphenyl]-2-sulfonamide

Example **125-1** (70 mg, 0.104 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **125** (40 mg), yield: 61.2%.

MS m/z (ESI): 631.2 [M+1] ⁺.

### Example 126

### 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(deuterated methoxymethyl)-[1,1'-biphenyl]-2-sulfonamide

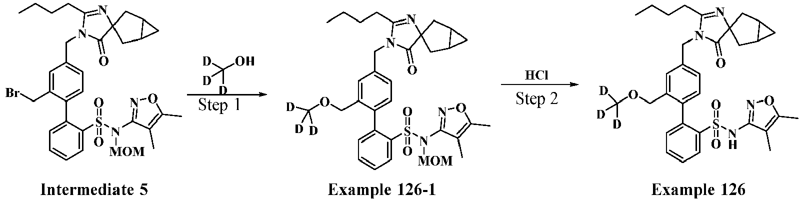

### Step 1

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-((methoxy-d3)methyl)-N-(methoxymethyl)-[1,1'-biphenyl]-2-sulfonamide

Deuterated methanol (22.5 mg, 0.644 mmol) was dissolved in N,N-dimethylformamide (5 mL), sodium hydride (26 mg, 60% w.t., 0.644 mmol) was added, and the mixture was reacted at room temperature for 30 min. **Intermediate 5** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **126-1** (65 mg,), yield: 79.3%, which was directly used in the next reaction.

MS m/z (ESI): 638.3 [M+1] ⁺.

### Step 2

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(deuterated methoxymethyl)-[1,1'-biphenyl]-2-sulfonamide

Example **126-1** (65 mg, 0.102 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **126** (42 mg), yield: 69.4%.

MS m/z (ESI): 594.2 [M+1] ⁺.

### Example 127

### 4'-(2'-butyl-5'-oxopyrrolo[bicyclo[3.1.0]hexane-3,4'-imidazole]-1'(5'H)-yl)methyl)-2'-(cyclopropoxymethyl)-N-(4,5-dimethylisoxazol-3-yl)-[1,1'-biphenyl]-2-sulfonamide

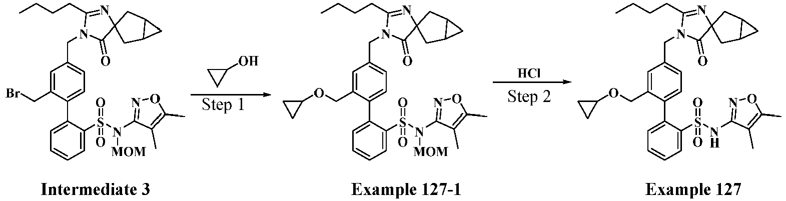

### Step 1

### Preparation of 4'-(2'-butyl-5'-oxopyrrolo[bicyclo[3.1.0]hexane-3,4'-imidazole]-1'(5'H)-yl)methyl)-2'-(cyclopropoxymethyl)-N-(4,5-dimethylisoxazol-3-yl)-N-(methoxymethyl)-[1,1'-biphenyl]-2-sulfonamide

Cyclopropanol (36 mg, 0.64 mmol) was dissolved in N,N-dimethylformamide (5 mL), sodium hydride (24 mg, 60% w.t., 0.64 mmol) was added, and the mixture was reacted at room temperature for 30 min. **Intermediate 5** (87.5 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **127-1** (60 mg,), yield: 70.9%, which was directly used in the next reaction.

MS m/z (ESI): 661.3 [M+1] ⁺.

### Step 2

### Preparation of 4'-(2'-butyl-5'-oxopyrrolo[bicyclo[3.1.0]hexane-3,4'-imidazole]-1'(5'H)-yl)methyl)-2'-(cyclopropoxymethyl)-N-(4,5-dimethylisoxazol-3-yl)-[1,1'-biphenyl]-2-sulfonamide

Example **127-1** (60 mg, 0.91 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **127** (37 mg), yield: 66.1%.

MS m/z (ESI): 617.2 [M+1] ⁺.

### Example 128

### 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(isopropoxymethyl)-[1,1'-biphenyl]-2-sulfonamide

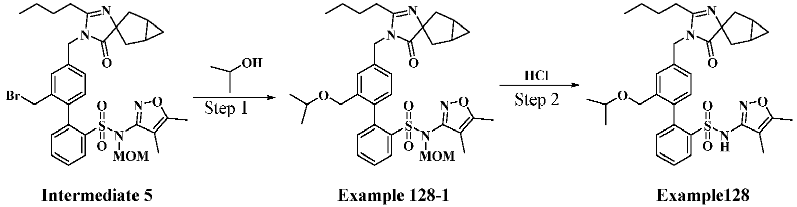

### Step 1

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(isopropoxymethyl)-N-(methoxymethyl)-[1,1'-biphenyl]-2-sulfonamide

Isopropanol (38.5 mg, 0.64 mmol) was dissolved in N,N-dimethylformamide (5 mL), sodium hydride (24 mg, 60% w.t., 0.642 mmol) was added, and the mixture was reacted at room temperature for 30 min. **Intermediate 5** (87.5 mg, 0.128 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **128-1** (65 mg,), yield: 76.6%, which was directly used in the next reaction.

MS m/z (ESI): 663.3 [M+1] ⁺.

### Step 2

### Preparation of 4'-((2'-butyl-5'-oxopyrrolo[bicyclo[3.1.0]hexane-3,4'-imidazole]-1'(5'H)-yl)methyl)-N-(4,5-dimethylisoxazol-3-yl)-2'-(isopropoxymethyl)-[1,1'-biphenyl]-2-sulfonamide

Example **128-1** (65 mg, 0.098 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **128** (43 mg), yield: 70.8%.

MS m/z (ESI): 619.2 [M+1] ⁺.

### Example 129

### 4'-((2-butyl-4-oxo-1,3-diazaspiro[4.5]decan-1-en-3-yl)methyl)-2'-((cyclopropylmethoxy)methyl)-N-(4,5-dimethylisoxazol-3-yl)-[1,1'-biphenyl]-2-sulfonamide

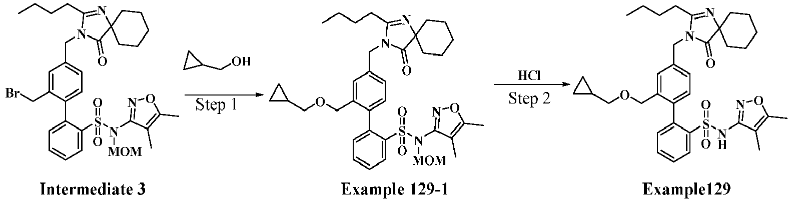

### Step 1

### Preparation of 4'-(2-butyl-4-oxo-1,3-diazaspiro[4.5]decan-1-en-3-yl)methyl)-2'-(cyclopropylmethoxy)methyl)-N-(4,5-dimethylisoxazol-3-yl)-N-(methoxymethyl)-[1,1'-biphenyl]-2-sulfonamide

Cyclopropylmethanol (46 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 3** (88 mg, 0.129mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **129-1** (70 mg,), yield: 80.6%, which was directly used in the next reaction.

MS m/z (ESI): 677.3 [M+1] ⁺.

### Step 2

### Preparation of 4'-((2-butyl-4-oxo-1,3-diazaspiro[4.5]decan-1-en-3-yl)methyl)-2'-((cyclopropylmethoxy)methyl)-N-(4,5-dimethylisoxazol-3-yl)-[1,1'-biphenyl]-2-sulfonamide

Example **129-1** (70 mg, 0.103 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **129** (35.5 mg), yield: 54.2%.

MS m/z (ESI): 633.3 [M+1] ⁺.

### Example 130

### 4'-(2-butyl-4-oxo-1,3-diazaspiro[4.5]decan-1-en-3-yl)methyl)-2'-(cyclobutoxymethyl)-N-(4,5-dimethylisoxazol-3-yl)-[1,1'-biphenyl]-2-sulfonamide

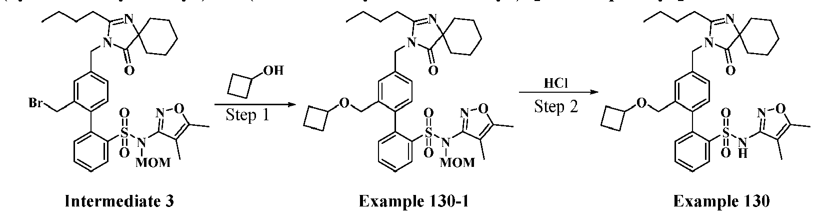

### Step 1

### Preparation of 4'-(2-butyl-4-oxo-1,3-diazaspiro[4.5]decan-1-en-3-yl)methyl)-2'-(cyclobutoxymethyl)-N-(4,5-dimethylisoxazol-3-yl)-N-(methoxymethyl)-[1,1'-biphenyl]-2-sulfonamide

Cyclobutanol (46 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 3** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **130-1** (70 mg,), yield: 80.6%, which was directly used in the next reaction.

MS m/z (ESI): 677.3 [M+1] ⁺.

### Step 2

### Preparation of 4'-(2-butyl-4-oxo-1,3-diazaspiro[4.5]decan-1-en-3-yl)methyl)-2'-(cyclobutoxymethyl)-N-(4,5-dimethylisoxazol-3-yl)-[1,1'-biphenyl]-2-sulfonamide

Example **130-1** (70 mg, 0.103 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **130** (35.5 mg), yield: 54.3%.

MS m/z (ESI): 633.3 [M+1] ⁺.

### Example 131

### 4'-(2-butyl-4-oxo-1,3-diazaspiro[4.5]decan-1-en-3-yl)methyl)-2'-(cyclopropoxymethyl)-N-(4,5-dimethylisoxazol-3-yl)-[1,1'-biphenyl]-2-sulfonamide

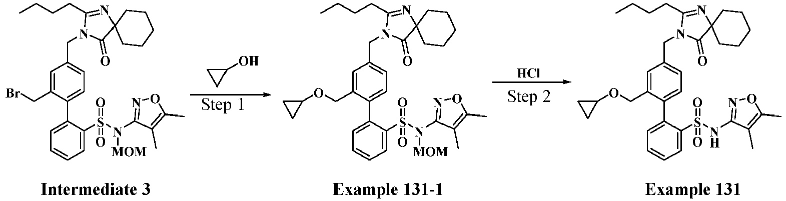

### Step 1

### Preparation of 4'-(2-butyl-4-oxo-1,3-diazaspiro[4.5]decan-1-en-3-yl)methyl)-2'-(cyclopropoxymethyl)-N-(4,5-dimethylisoxazol-3-yl)-N-(methoxymethyl)-[1,1'-biphenyl]-2-sulfonamide

Cyclopropanol (37 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 3** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **131-1** (65 mg,), yield: 75.8%, which was directly used in the next reaction.

MS m/z (ESI): 663.3 [M+1] ⁺.

### Step 2

### Preparation of 4'-(2-butyl-4-oxo-1,3-diazaspiro[4.5]decan-1-en-3-yl)methyl)-2'-(cyclopropoxymethyl)-N-(4,5-dimethylisoxazol-3-yl)-[1,1'-biphenyl]-2-sulfonamide

Example **131-1** (65 mg, 0.1 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **131** (35 mg), yield: 57.7%.

MS m/z (ESI): 619.3 [M+1] ⁺.

### Example 132

### 4'-(2-bulyl-4-oxo-1,3-diazaspiro[4.5]decan-1-en-3-yl)methyl)-2'-(isopropoxymethyl)-N-(4,5-dimethylisoxazol-3-yl)-[1,1'-biphenyl]-2-sulfonamide

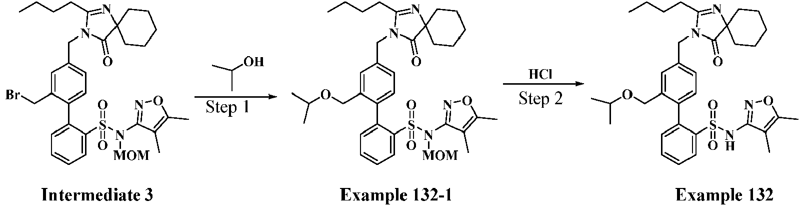

### Step 1

### Preparation of 4'-(2-butyl-4-oxo-1,3-diazaspiro[4.5]decan-1-en-3-yl)methyl)-2'-(isopropoxymethyl)-N-(4,5-dimethylisoxazol-3-yl)-N-(methoxymethyl)-[1,1'-biphenyl]-2-sulfonamide

Isopropanol (39 mg, 0.642 mmol) was dissolved in N,N-dimethylformamide (5 mL), sodium hydride (25 mg, 60% w.t., 0.642 mmol) was added, and the mixture was reacted at room temperature for 30 min. **Intermediate 3** (88 mg, 0.129 mmol) was added and the mixture was reacted at room temperature for 1 h. The reaction liquid was poured into 30 mL of ice water and the mixture was extracted with ethyl acetate (30 mL*2). The organic phases were combined, washed with saturated sodium chloride solution (30 mL), dried over anhydrous sodium sulfate and filtered, and the filtrate was concentrated under reduced pressure to afford Example **132-1** (65 mg,), yield: 76.2%, which was directly used in the next reaction.

MS m/z (ESI): 665.3 [M+1] ⁺.

### Step 2

### Preparation of 4'-(2-butyl-4-oxo-1,3-diazaspiro[4.5]decan-1-en-3-yl)methyl)-2'-(isopropoxymethyl)-N-(4,5-dimethylisoxazol-3-yl)-[1,1'-biphenyl]-2-sulfonamide

Example **132-1** (65 mg, 0.1 mmol) was dissolved in a solution of 4 M hydrochloride in dioxane (4 mL), heated to 60°C and reacted for 4 h. The reaction liquid was cooled to room temperature and concentrated under reduced pressure. The crude product was subjected to reverse HPLC to afford Example **132** (40 mg), yield: 65.9%.

MS m/z (ESI): 621.3 [M+1] ⁺.

### Biological test and evaluation

The invention will be further described and explained below in conjunction with test examples, but these examples are not meant to limit the scope of the present invention.

### 1. Cell function experiment

### Test example 1. Determination of the effect of the compounds of the present invention on calcium current in cells stably expressing AT1 receptors

1. Experiment purpose:
Antagonistic effect of test compounds on HEK293-AT1 cell activity
2. Experimental instruments and reagents:
2.1 Instruments:
   384-well test plate (Corning: 3764);
   384-well Echo compound plate (Labcyte: LP-0200);
   384-well compound plate (PE: 6008590);
   Bravo Tip (Agilent: 10734-202);
   FLIPR Tip (Molecular Device: 9000-0764);
   Plate reader FLIPR Tetra (Molecular Device);
   Pipetting workstation Bravo (Agilent);
   ECHO 550 (LABCYTE);
   Liquid injector Multidrop Combi (ThermoFisher).
2.2 Reagents:
   DMEM, high glucose (Gibco: 12100);
   Fetal bovine serum (Biosera: FB-1058/500);
   P/S (Biosera: XC-A4122);
   5X Matrigel (Corning: 354230);
   HBSS (Sigma: H1387);
   HEPES (Invitrogen: 15630080);
   Fluo-8 AM (AAT Bioquest: 21080);
   Probenecid (Sigma: P8761);
   Pluronic F-127 (Sigma: P2443-250G);
   Angiotensin III TFA (MCE: HY-113035A);
   Irbesartan (MCE: HY-B0202);
   1000X Fluo-8 AM (2 mM): Fluo-8 AM was dissolved in DMSO, the mixture was shaken for 1-2 min, sub-packaged and stored at -20°C;
   Complete culture medium: DMEM + 10% FBS + 1X P/S;
   Cell seeding medium: DMEM + 10% FBS + 1X PS;
   Experimental buffer 1: 1X HBSS + 20 mM HEPES +1 mM Probenecid + 0.025% Pluronic F-127;
   Experimental buffer 2: 1X HBSS + 20 mM HEPES + 0.075% Pluronic F-127; 1X Matrigel: 5X Matrigel was diluted with DMEM;
   Cell line: HDB HEK293-AT1.

3. Experimental method:
1) HEK293-AT1 cell line was cultured in complete culture medium at 37°C, 5% CO₂ to 70%-90% confluence.
2) the 384-well cell plate was coated with 1X Matrigel with 5 µL/well at room temperature for 10 to 30 min.
3) the cells were digested and resuspended in cell seeding medium, inoculation was performed at 8,000 cells/well/20 µL in a 384-well cell culture plate, and the cells were cultured at 37°C, 5% CO₂ for 24 h.
4) the cell culture plate was taken out of the CO₂ incubator and equilibrated at room temperature for 10 min.
5) 1000X Fluo-8 AM was taken and diluted to 1X Fluo-8 AM with experimental buffer 1 that has been equilibrated to room temperature, with a concentration of 2 µM.
6) the culture medium was removed from the cell culture plate, 20 µL of 1× Fluo-8 AM was added to each well, the mixture was centrifuged at 300 rpm for 60 seconds at room temperature, and incubated at room temperature in the dark for 1 h.
7) positive control compound and test compound working solution (3X) were formulated:
   ① instrument Bravo was used to dilute the compound to 11 concentration points on the 384-well Echo compound plate (LABCYTE: LP-0200);
   ② instrument ECHO was used to transfer 90 nL of compound per well (compound storage concentration, such as the highest concentration point 10 mM) to the 384-well compound plate (PE: 6008590);
   ③ Multidrop Combi was used to add 30 uL of experimental buffer 2 to the 384-well compound plate (PE: 6008590), the positive control compound and test compound were diluted to 30 µM (3X), and placed at room temperature until use.
8) FLIPR Tetra was used to add 10 µL of diluted 3X compound into the experimental well of the corresponding 384-well cell plate, the compound and cells were incubated for 10 min at room temperature, then 10 µL of diluted 4X agonist was added, and the data was read and collected at the same time.

4. Experimental data processing method:
FLIPR Tetra was used to read and collect the fluorescence signal value (RFU). The maximum RFU value was used to calculate the percent activation data based on the readings of the Low control (DMSO control) and High control (100 nM positive compound) experimental groups {% activation rate = (RFUsample - RFUlow control) / (RFUhigh control - RFUlow control) × 100}. The concentration of the test compound was 11 concentrations after diluting the reaction system 3 times, ranging from 10 uM to 0.17 nM. XLFit was used to fit the percentage activation rate and 11-point concentration data to a parametric nonlinear logic formula to calculate the IC₅₀ value of the compound.
5. Experimental results:

**Table 1 IC₅₀ values of compounds on calcium current in cells stably expressing AT1 receptors**

| Number | IC₅₀ (nM) |
|---|---|
| Example 13 | 10.0 |
| Example 14 | 6.9 |
| Example 44 | 6.2 |
| Example 45 | 3.2 |
| Example 46 | 3.9 |
| Example 49 | 7.2 |
| Example 52 | 8.6 |
| Example 53 | 9.1 |
| Example 55 | 3.7 |
| Example 65 | 4.4 |
| Example 68 | 8.8 |
| Example 72 | 6.1 |
| Example 85 | 5.2 |
| Example 86 | 2.2 |
| Example 87 | 9.6 |

6. Experimental conclusion:
It can be seen from the data in the table that the example compounds shown in the present invention show good antagonistic effects in the experiment on the effect on calcium current in cells stably expressing AT1 receptors.

### Test example 2. Determination of the effect of the compounds of the present invention on calcium current in cells stably expressing ETA receptors

1. Experiment purpose:
Antagonistic effect of test compounds on HEK293-ETA cell activity.
2. Experimental instruments and reagents:
2.1 Instruments:
   384-well test plate (Corning: 3764);
   384-well Echo compound plate (Labcyte: LP-0200);
   384-well compound plate (PE: 6008590);
   Bravo Tip (Agilent: 10734-202);
   FLIPR Tip (Molecular Device: 9000-0764)
   Plate reader FLIPR Tetra (Molecular Device);
   Pipetting workstations Bravo (Agilent) and ECHO 550 (LABCYTE);
   Liquid injector Multidrop Combi (ThermoFisher).
2.2 Reagents:
   DMEM, high glucose (Gibco: 12100);
   Fetal bovine serum (Biosera: FB-1058/500);
   P/S (Biosera: XC-A4122);
   5X Matrigel (Corning: 354230);
   HBSS (Sigma: H1387);
   HEPES (Invitrogen: 15630080);
   Fluo-8 AM (AAT Bioquest: 21080);
   Probenecid (Sigma: P8761);
   Pluronic F-127 (Sigma: P2443-250G);
   Endothelin 1 (MCE: HY-P0202);
   Zibotentan (MCE: HY-10088);
   1000X Fluo-8 AM (2 mM): Fluo-8 AM was dissolved in DMSO, the mixture was shaken for 1-2 min, sub-packaged and stored at -20°C;
   Complete culture medium: DMEM + 10% FBS + 1X P/S;
   Cell seeding medium: DMEM + 10% FBS + 1X PS;
   Experimental buffer 1: 1× HBSS + 20 mM HEPES +1 mM Probenecid + 0.025% Pluronic F-127;
   Experimental buffer 2: 1X HBSS + 20 mM HEPES + 0.075% Pluronic F-127; 1X Matrigel: 5X Matrigel was diluted with DMEM;
   Cell line: HDB HEK293-ETA.

3. Experimental method:
1) HEK293-ETA cell line was cultured in complete culture medium at 37°C, 5% CO₂ to 70%-90% confluence.
2) the 384-well cell plate was coated with 1X Matrigel with 5 µL/well at room temperature for 10 to 30 min.
3) the cells were digested and resuspended in cell seeding medium, inoculation was performed at 8,000 cells/well/20 µL in a 384-well cell culture plate, and the cells were cultured at 37°C, 5% CO₂ for 24 h.
4) the cell culture plate was taken out of the CO₂ incubator and equilibrated at room temperature for 10 min.
5) 1000X Fluo-8 AM was taken and diluted to 1× Fluo-8 AM with experimental buffer 1 that has been equilibrated to room temperature, with a concentration of 2 µM.
6) the culture medium was removed from the cell culture plate, 20 µL of 1× Fluo-8 AM was added to each well, the mixture was centrifuged at 300 rpm for 60 seconds at room temperature, and incubated at room temperature in the dark for 1 h.
7) positive control compound and test compound working solution (3X) were formulated:
   ① instrument Bravo was used to dilute the compound to 11 concentration points on the 384-well Echo compound plate (LABCYTE: LP-0200);
   ② instrument ECHO was used to transfer 90 nL of compound per well (compound storage concentration, such as the highest concentration point 10 mM) to the 384-well compound plate (PE: 6008590);
   (3) Multidrop Combi was used to add 30 µL of experimental buffer 2 to the 384-well compound plate (PE: 6008590), the positive control compound and test compound were diluted to 30 µM (3X), and placed at room temperature until use.
8) FLIPR Tetra was used to add 10 µL of diluted 3X compound into the experimental well of the corresponding 384-well cell plate, the compound and cells were incubated for 10 min at room temperature, then 10 µL of diluted 4X agonist was added, and the data was read and collected at the same time.

4. Experimental data processing method:
FLIPR Tetra was used to read and collect the fluorescence signal value (RFU). The maximum RFU value was used to calculate the percent activation data based on the readings of the Low control (DMSO control) and High control (100 nM positive compound) experimental groups {% activation rate = (RFUsample - RFUlow control) / (RFUhigh control - RFUlow control) × 100}. The concentration of the test compound was 11 concentrations after diluting the reaction system 3 times, ranging from 10 µM to 0.17 nM. XLFit was used to fit the percentage activation rate and 11-point concentration data to a parametric nonlinear logic formula to calculate the IC₅₀ value of the compound.
5. Experimental results:

**Table 2 IC₅₀ values of compounds on calcium current in cells stably expressing ETA receptors**

| Number | IC₅₀ (nM) |
|---|---|
| Example 44 | 15.1 |
| Example 45 | 14.9 |
| Example 49 | 12.9 |
| Example 65 | 7.2 |
| Example 68 | 18.7 |
| Example 86 | 13.9 |
| Example 87 | 8.2 |

6. Experimental conclusion:
It can be seen from the data in the table that the example compounds shown in the present invention show good antagonistic effects in the experiment on the effect on calcium current in cells stably expressing ETA receptors.

### 2. Rat pharmacokinetic evaluation test

1. Study purpose:
SD rats were used as test animals to study the pharmacokinetic behavior of the compounds of the present invention in the rat body (plasma) after oral administration at a dose of 5 mg/kg.
2. Experimental scheme:
2.1 Experimental drugs:
the example compounds of the present invention, made in house.
2.2 Experimental animals:
3 SD rats/group, male, from Shanghai Jiesijie Experimental Animal Co., Ltd., animal production license number (SCXK (Shanghai) 2013-0006 N0.311620400001794).
2.3 Formulation prescription:
0.5% CMC-Na (1% Tween80) was dissolved with ultrasound, and formulated into a clear solution or homogeneous suspension.
2.4 Administration:
p.o. respectively after the rats were fasted overnight;
the p.o. dose was 5 mg/kg and the administration volume was 10 mL/kg.

2.5 Sample collection:
After oral administration of rats, 0.2 mL of blood was collected from the jugular vein at 0.25 h, 0.5 h, 1.0 h, 2.0 h, and 4.0 h, placed in an EDTA-2K test tube, centrifuged at 6000 rpm for 6 min at 4°C to separate plasma which was stored at -80°C; the rats can eat 4 h after administration.
2.6 Sample processing:
1) 160 µL of acetonitrile was added to 40 µL of plasma sample for precipitation, mixed and centrifuged at 3500 × g for 5 to 20 min.
2) the treated supernatant solution was taken and subjected to LC/MS/MS to analyze the concentrations of the test compounds. LC/MS/MS analysis instrument: AB Sciex API 4000 Qtrap.

2.7 Liquid phase analysis:
• Liquid phase conditions: Shimadzu LC-20AD pump
• chromatographic column: Agilent ZORBAX XDB-C18 (50 × 2.1 mm, 3.5 µm) mobile phase: liquid A was 0.1% aqueous formic acid solution, liquid B was acetonitrile
• flow rate: 0.4 mL/min
• Elution time: 0-4.0 min, the eluent was as follows:

| time/min | liquid A | liquid B |
|---|---|---|
| 0.01 | 90% | 10% |
| 0.5 | 90% | 10% |
| 0.8 | 5% | 95% |
| 2.4 | 5% | 95% |
| 2.5 | 90% | 10% |
| 4.0 | Stop | |

3. Test results and analysis

The main pharmacokinetic parameters were calculated using WinNonlin 6.1. The results of pharmacokinetic experiments in rats are shown in Table 3 below:

**Table 3: Pharmacokinetic parameters of oral administration to rats of the compounds of the present invention**

| Number | AUC_{0-∞} (ng/mL*h) | Cₘₐₓ (ng/mL) | Tₘₐₓ (h) | MRT _{0-∞} (h) |
|---|---|---|---|---|
| Example 14 | 11312 | 2320 | 0.5 | 5.3 |
| Example 44 | 10223 | 1693 | 0.5 | 8.4 |
| Example 45 | 16024 | 2140 | 0.5 | 8.6 |
| Example 46 | 13822 | 2303 | 0.5 | 6.6 |
| Example 87 | 9763 | 2627 | 0.5 | 3.8 |

### 3.4 Experimental conclusion:

The data in the table show that in the rat pharmacokinetic evaluation experiment, the example compounds of the present invention show high exposure after oral administration.

### 3. In vitro ADMET evaluation test

### Test example 1. Liver microsomal metabolism stability test

### 1. Experiment purpose:

The purpose of this experiment was to detect the stability of the example compounds in liver microsomes of rats, dogs and humans.

### 2. Experimental steps:

### 2.1 Formulation of working solution of compounds

Formulation of working solution of compounds: compound stock solution was added to phosphate buffer to a final concentration of 20 µM.

### 2.2 Formulation of working solution of liver microsome

Dilution was performed with 100 mM phosphate buffer to a final concentration of 0.625 mg/mL.

### 2.3 Preparation of NADPH and UDPGA

NADPH (reduced nicotinamide adenine dinucleotide phosphate) and UDPGA (uridine diphosphate glucuronic acid) were weighed, 100 mM phosphate buffer was added, and the final concentration was 20 mM.

### 2.4 Preparation of perforation agent

1 mg of Alamethicin was weighed, and 200 µL of DMSO was added to formulate a 5 mg/mL solution. Then dilution was performed with phosphate buffer to a final concentration of 50 µg/mL.

### 2.5 Formulation of reaction stop solution

Stop solution: the stop solution contains 100 ng/mL labetalol hydrochloride and 400 ng/mL tolbutamide and cold acetonitrile as internal standard.

### 2.6 Incubation process

400 µL of formulated liver microsomes, 25 µL of compound working solution and 25 µL of Alamethicin were added in sequence to the 96-well plate, and pre-incubated at 37°C for 10 min. Then 50 µL of formulated NADPH/UDPGA was added to start the reaction and the mixture was incubated at 37°C. The total volume of the reaction system was 500 µL. The final contents of each component are as follows:

| Component | Content |
|---|---|
| Liver microsomes | 0.5 mg/mL |
| Compound | 1 µM |
| NADPH | 2 mM |
| UDPGA | 2 mM |
| Alamethicin | 2.5 µg/mL |

### 2.7 Sample analysis

### 2.7.1 Chromatographic conditions:

Instrument: Shimadzu LC-30 AD;
Chromatographic column: XBridge^{®} C18 (50*4.6 mm, 5 µm particle size);
Mobile phase: A: 0.1% formic acid solution, B: methanol
Wash gradient: 0.2-1.6 min 5% A to 95% A, 3.0-3.1 min 95% A to 5% A
Run time: 4.0 min.

### 2.7.2 Mass spectrometry conditions:

Instrument: API5500 liquid chromatography mass spectrometer, AB Sciex;
Ion source: electrospray ionization source (ESI);
Drying gas: N₂, temperature 500°C;
Electrospray voltage: 5000 V;
Detection mode: positive ion detection;

### Scanning mode: reaction monitoring (MRM) mode.

### 3. Experimental results:

**Table 4 Results of liver microsomal metabolism stability of example compounds**

| Example Number | Rat | | Dog | | Human | |
|---|---|---|---|---|---|---|
| | *t*_{1/2} (min) | Remaining rate (%, 60 min) | *t*_{1/2} (min) | Remaining rate (%, 60 min) | *t*_{1/2} (min) | Remaining rate (%, 60 min) |
| 44 | 26.3 | 29.4 | 29.9 | 24.6 | 38.5 | 33.0 |
| 45 | 29.9 | 23.3 | 28.5 | 23.6 | 36.5 | 32.0 |
| 46 | 24.3 | 17.3 | 22.2 | 15.4 | 28.9 | 23.8 |
| 49 | 24.0 | 17.1 | 29.5 | 23.9 | 26.2 | 30.7 |

### 4. Experimental conclusion:

The above data show that the example compounds of the present invention have good metabolism stability in liver microsomes of rats, dogs and humans.

### Test example 2. CYP enzyme single point inhibition test

### 1. Experiment purpose

Human liver microsome incubation system was used, and the single point method was used to quickly predict the inhibition on CYP450 enzyme subtypes (1A2, 2C19, 2D6, 3A4-M, and 3A4-T) by compounds.

### 2. Experimental steps

### 2.1 Solution formulation

For 2.5 mM NADPH, 4.165 mg of NADPH (reduced nicotinamide adenine dinucleotide phosphate) was weighed and 100 mM phosphate buffer was added to 2 mL. For 0.25 mg/mL microsomes, 50 µL of 20 mg/mL microsomes was added to 4 mL of 100 mM phosphate buffer and mixed evenly.

### Formulation of the reaction liquid of the test compound:

The test example compound was weighed, diluted to 10 mM with DMSO, and then diluted to 100 µM with 100 mM phosphate buffer.

### 2.2 Experimental process:

1. 40 µL of liver microsomes, 10 µL of substrate, and 10 µL of test compound were added in a 96-well plate and the mixture was pre-incubated for 3 min.
2. 40 µL of NADPH was added.
3. At 20 min, 300 µL of acetonitrile stop solution containing internal standard was added.
4. The mixture was centrifuged and the sample was loaded.

### 3. Experimental results:

Table 5 Results of CYP enzyme single point inhibition of example compounds

| **Compound** | **IC₅₀ (µM)** | | | | |
|---|---|---|---|---|---|
| | **1A2** | **2C19** | **2D6** | **3A4-M** | **3A4-T** |
| 44 | >100 | >100 | >100 | 14.7 | >100 |
| 45 | >100 | >100 | >100 | 24.9 | >100 |
| 46 | >100 | >100 | >100 | 16.7 | >100 |

| | | | | | |
|---|---|---|---|---|---|
| Notes: Strong inhibition: IC₅₀ < 1 µM; Medium inhibition: 1 µM < IC₅₀ < 10 µM; Weak inhibition: IC₅₀ > 10 µM | | | | | |

### 4. Experimental conclusion:

The above data show that the example compounds of the present invention do not have strong inhibition, but all have weak inhibition on each CYP enzyme subtype, and the risk of DDI is small.

### 4. Pharmacodynamic evaluation of compounds on blood pressure in spontaneously hypertensive rats

### 1. Experiment purpose:

This experiment used a spontaneously hypertensive rat (SHR) model to evaluate the pharmacodynamic effect of the test compounds on the blood pressure and heart rate of the model.

### 2. Main experimental instrument and material

Kent Scientific CODA non-invasive blood pressure system.

### 3. Experimental animals:

Spontaneously hypertensive rats (SHR) were provided by Beijing Vital River Laboratory Animal Technology Co., Ltd., male, 150-200 g, 13-15 weeks old, 50 rats.

### 4. Experimental method:

4.1 Adaptation period: After animals arrived at the experimental facility, the animals were adapted to the animal facility for 5-7 days. 4.2 After the adaptation period, the animals were adapted to the tail oversleeve restrain for 3 days, and the tail oversleeve restrain was performed twice a day. After the last restrain adaptation, the basal blood pressure was measured, and the animals were randomly divided into groups according to the basal blood pressure. There was no significant difference between the average systolic blood pressure of animals in each group, and the number of animals in each group met the requirements of statistical testing and pharmacodynamic guidelines. 4.3 On the second day after grouping, the animals were administered according to experimental design and grouping (P.O, 5 mL/kg), and the systolic blood pressure (SBP), diastolic blood pressure (DBP), mean arterial pressure (MAP) and heart rate (HR) of the animals were measured before administration and at 1, 2, 4, 6, 8 and 24 h after administration using tail oversleeve method.

### 5. Data processing and analysis

Statistical differences between groups were analyzed by Two-way ANOVA test. Among them, P < 0.05 indicated a significant difference, and P < 0.01 indicated an extremely significant difference.

### 6. Experimental results

The results show that in the spontaneously hypertensive model, compared with the vehicle group, the example compounds 44, 45 and 49 of the present invention significantly reduced blood pressure (systolic blood pressure, diastolic blood pressure, mean arterial pressure) 1-8 h after administration in the 30 mpk administration group (P < 0.05); in addition, no significant effect on heart rate was found in each administration group, and the safety was good.

## Claims

1. A compound of general formula (I), a stereoisomer or a pharmaceutically acceptable salt thereof: 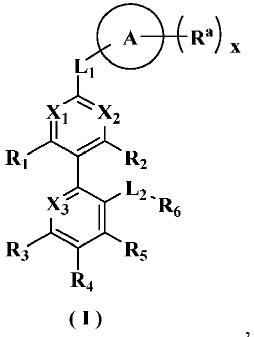
X₁ is N or CR¹;
X₂ is N or CR²;
X₃ is N or CR³;
R¹, R² and R³ are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-;
L₂ is selected from the group consisting of -(CH₂)ₙ₂-, -(CH₂)ₙ₂NR_{c}-, - (CH₂)ₙ₂C(O)NR_{c}-, -(CH₂)ₙ₂C(O)NR_{c}S(O)ₘ₂-, -(CH₂)ₙ₂NR_{c}C(O)-, -(CH₂)ₙ₂S(O)ₘ₂-, -(CH₂)ₙ₂S(O)ₘ₂NR_{c}-, -(CH₂)ₙ₂S(O)ₘ₂NR_{c}C(O)-, -(CH₂)ₙ₂S(O)ₘ₂NR_{c}C(O)NR_{d}-, - (CH₂)ₙ₂S(O)ₘ₂NR_{c}C(O)O(CH₂)ₙ₃-, -(CH₂)ₙ₂NR_{c}S(O)ₘ₂- and-(CH₂)ₙ₂NR_{c}S(O)ₘ₂NR_{d}C(O)-;
the ring A is selected from the group consisting of 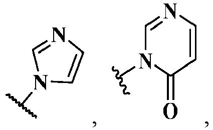 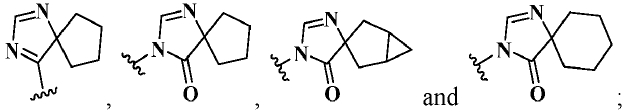
R^{a} is independently selected from the group consisting of hydrogen, deuterium, halogen, amino, nitro, hydroxy, cyano, oxo, thio, alkyl, deuteroalkyl, haloalkyl, hydroxyalkyl, alkoxy, haloalkoxy, alkenyl, alkynyl, cycloalkyl, heterocyclyl, aryl, heteroaryl, -(CH₂)ₙ₄C(O)R_{A1}, -(CH₂)ₙ₄C(O)OR_{A1}, - (CH₂)ₙ₄C(O)NR_{A1}R_{B1} and -(CH₂)ₙ₄C(=S)NR_{A1}R_{B1}, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
R₁ is selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl, heteroaryl, - (CH₂)ₙ₅R_{A2}-, -(CH₂)ₙ₅O(CH₂)ₙ₆R_{A2}-, -(CH₂)ₙ₅C(O)R_{A2}, -(CH₂)ₙ₅NR_{A2}C(O)R_{B2}, - (CH₂)ₙ₅C(O)NR_{A2}R_{B2}, -(CH₂)ₙ₅OC(O)NR_{A2}R_{B2} and -(CH₂)ₙ₅NR_{A2}C(O)OR_{B2}, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
or, R₁ and R^{a}, together with the atoms to which they are attached, form a cycloalkyl, heterocyclyl, aryl or heteroaryl, and the cycloalkyl, heterocyclyl, aryl or heteroaryl may be optionally further substituted;
R₂, R₃, R₄ and R₅ are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
or, R₂ and R₅, together with the atoms to which they are attached, form a heterocyclyl, and the heterocyclyl may be optionally further substituted;
R₆ is selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
Rₐ, R_{b}, R_{c}, R_{d}, R_{A1}, R_{A2}, R_{B1} and R_{B2} are each independently selected from the group consisting of hydrogen, deuterium, halogen, amino, nitro, hydroxy, cyano, alkyl, deuteroalkyl, haloalkyl, hydroxyalkyl, alkoxy, haloalkoxy, alkenyl, alkynyl, cycloalkyl, heterocyclyl, aryl and heteroaryl, wherein the amino, alkyl, deuteroalkyl, haloalkyl, hydroxyalkyl, alkoxy, haloalkoxy, alkenyl, alkynyl, cycloalkyl, heterocyclyl, aryl or heteroaryl may be optionally further substituted;
x is 0, 1, 2, 3, 4 or 5;
n1-n6 are 0, 1, 2, 3, 4 or 5; and
m1 and m2 are 0, 1 or 2.

2. The compound, or the stereoisomer or the pharmaceutically acceptable salt thereof according to claim 1, **characterized in that** the compound is a compound of general formula (II): 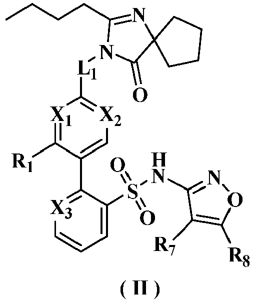
X₁ is N or CR¹;
X₂ is N or CR²;
X₃ is N or CR³;
R¹, R² and R³ are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3 to 12membered heterocyclyl, C₆₋₁₄ aryl and 5 to 14 membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3 to 12 membered heterocyclyl, C₆₋₁₄ aryl or 5 to 14 membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
L₁ is selected from the group consisting of -(CRₐR_{b})ₙ₁-, -(CRₐR_{b})ₙ₁O-, - O(CRₐR_{b})ₙ₁-, -(CRₐR_{b})ₙ₁S-, -S(CRₐR_{b})ₙ₁-, -(CH₂)ₙ₁C(O)NRₐ-, -(CH₂)ₙ₁NRₐC(O)-, - (CH₂)ₙ₁S(O)ₘ₁-, -(CH₂)ₙ₁S(O)ₘ₁NRₐ-, -(CH₂)ₙ₁NRₐS(O)ₘ₁- and -(CH₂)ₙ₁NRₐ-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R₇ and R₈ are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
or, R₇ and R₈ are bond to form a C₃₋₈ cycloalkyl, 5- to 8-membered heterocyclyl, C₆₋₁₄ aryl or 5- to 14-membered heteroaryl, wherein the C₃₋₈ cycloalkyl, 5- to 8-membered heterocyclyl, C₆₋₁₄ aryl or 5- to 14-membered heteroaryl may be optionally further substituted;
Rₐ and R_{b} are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R_{A2} and R_{B2} are each independently selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
n1, n5 and n6 are 0, 1, 2 or 3; and
m1 is 0, 1 or 2;
when R₇ and R₈ are both methyl and R₁ is not 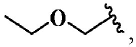 R₁ is selected from the group consisting of 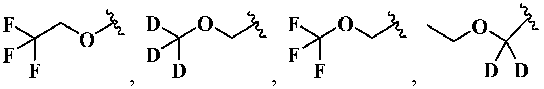 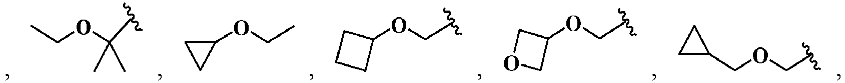 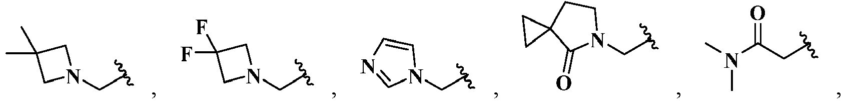 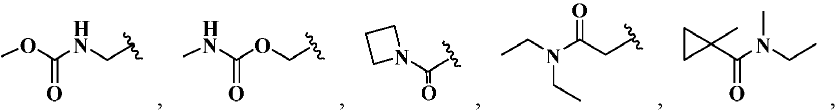 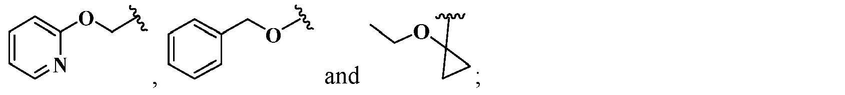
when R₇ and R₈ are both methyl and R₁ is 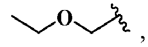 , L₁ contains deuterium, or at least one of X₁, X₂ or X₃ is N, or R³ is not hydrogen.

3. The compound, or the stereoisomer or the pharmaceutically acceptable salt thereof according to claim 2, **characterized in that** the compound is a compound of formula (II-1): 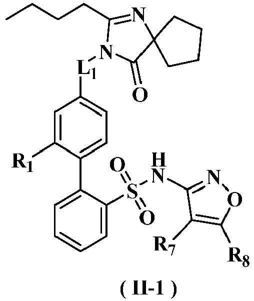
L₁ is selected from the group consisting of -CH₂- and -CD₂-;
R₁ is selected from the group consisting of deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl;
R₇ is selected from the group consisting of halogen, preferably fluorine, chlorine and bromine;
R₇ and R₈ are each independently selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, preferably methyl, ethyl and cyclopropyl.

4. The compound, or the stereoisomer or the pharmaceutically acceptable salt thereof according to claim 1, **characterized in that** the compound is a compound of general formula (VIII-1) or a compound of general formula (VIII-2): 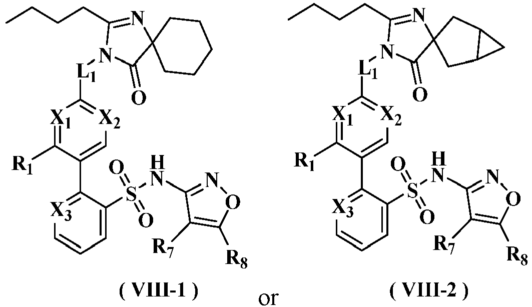
wherein L₁, X₁, X₂, X₃, R₇ and R₈ are as defined in claim 2;
for general formula (VIII-1), when L₁ is CH₂, R₇ and R₈ are both methyl, and
R₁ is 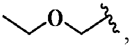 at least one of X₁, X₂ and X₃ is not CH.

5. The compound, or the stereoisomer or the pharmaceutically acceptable salt thereof according to any one of claims 1 to 4, **characterized in that**,
L₁ is selected from the group consisting of -CRₐR_{b}-, -CRₐR_{b}O-, - OCRₐR_{b}-, - CRₐR_{b}S- and -SCRₐR_{b}-,
Rₐ and R_{b} are each independently selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl, wherein the C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl or 5- to 10-membered heteroaryl is optionally further substituted with one or more substituents selected from the group consisting of deuterium, halogen, amino, hydroxy, cyano, oxo, thioxo, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl;
preferably, L₁ is selected from the group consisting of -CH₂-, -CD₂- and - CH₂O-;
and more preferably, L₁ is -CH₂-.

6. The compound, or the stereoisomer or the pharmaceutically acceptable salt thereof according to any one of claims 1 to 5, **characterized in that**,
X₁, X₂ and X₃ are all CH;
L₁ is selected from the group consisting of -CH₂- and -CD₂-;
R₁ is selected from the group consisting of hydrogen, deuterium, halogen, amino, hydroxy, cyano, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl, 5- to 14-membered heteroaryl, -(CH₂)ₙ₅R_{A2}, -(CH₂)ₙ₅O(CH₂)ₙ₆R_{A2}, -(CH₂)ₙ₅C(O)R_{A2}, - (CH₂)ₙ₅NR_{A2}C(O)R_{B2}, -(CH₂)ₙ₅C(O)NR_{A2}R_{B2}, -(CH₂)ₙ₅OC(O)NR_{A2}R_{B2} and - (CH₂)ₙ₅NR_{A2}C(O)OR_{B2}, wherein the C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl or 5- to 14-membered heteroaryl is optionally further substituted with one or more substituents selected from the group consisting of deuterium, halogen, amino, hydroxy, cyano, oxo, thioxo, C₁₋₆ alkyl, C₂₋₆ alkenyl, C₂₋₆ alkynyl, C₁₋₆ deuteroalkyl, C₁₋₆ haloalkyl, C₁₋₆ hydroxyalkyl, C₁₋₆ alkoxy, C₁₋₆ alkylthio, C₁₋₆ haloalkoxy, C₃₋₁₂ cycloalkyl, 3- to 12-membered heterocyclyl, C₆₋₁₄ aryl and 5- to 14-membered heteroaryl,
R₇ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl;
R₈ is selected from the group consisting of hydrogen, deuterium, halogen, amino, hydroxy, cyano, C₁₋₃ alkyl, C₂₋₃ alkenyl, C₂₋₃ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, C₃₋₈ cycloalkyl, 3- to 8-membered heterocyclyl, C₆₋₁₀ aryl and 5- to 10-membered heteroaryl.

7. The compound, or the stereoisomer or the pharmaceutically acceptable salt thereof according to claim 6, **characterized in that**,
L₁ is -CH₂;
R₁ is selected from the group consisting of C₁₋₃ alkyl and - (CH₂)ₙ₅O(CH₂)ₙ₆R_{A2}, wherein the C₁₋₃ alkyl is optionally further substituted with one or more substituents selected from the group consisting of deuterium, halogen, amino, hydroxy, cyano, oxo, thioxo, C₁₋₃ alkyl, C₂₋₄ alkenyl, C₂₋₄ alkynyl, C₁₋₃ deuteroalkyl, C₁₋₃ haloalkyl, C₁₋₃ hydroxyalkyl, C₁₋₃ alkoxy, C₁₋₃ alkylthio, C₁₋₃ haloalkoxy, and C₃₋₆ cycloalkyl;
R_{A2} is selected from the group consisting of C₁₋₃ alkyl, C₁₋₃ deuteroalkyl, C₁₋₃ haloalkyl, C₃₋₆ cycloalkyl, 4- to 7-membered heterocyclyl, C₃₋₆ cycloalkyl C₁₋₃ alkyl and 4- to 7-membered heterocyclyl C₁₋₃ alkyl;
R₇ is selected from the group consisting of deuterium, fluorine, chlorine, bromine and C₁₋₃ alkyl;
R₈ is C₁₋₃ alkyl;
n5 is 0, 1, 2 or 3; and
n6 is 0, 1 or 2.

8. The compound, or the stereoisomer or the pharmaceutically acceptable salt thereof according to claim 7, **characterized in that**,
R₁ is selected from the group consisting of hydrogen, -CH₃, -CH₂CH₃, 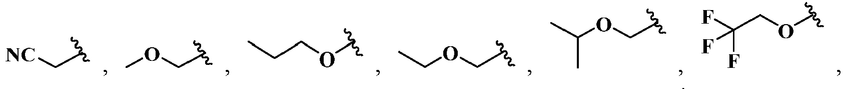 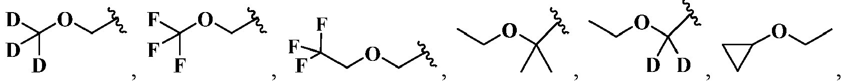 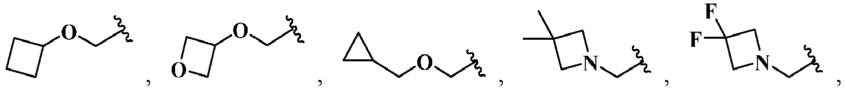 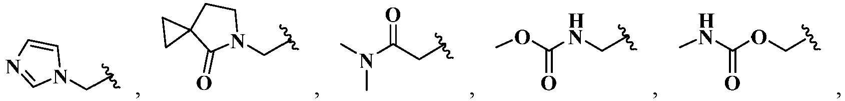 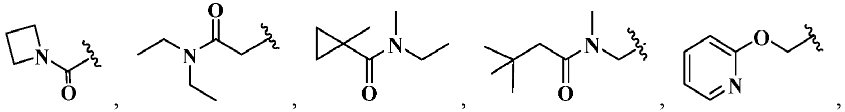 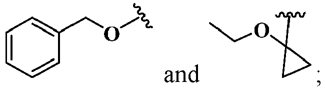
R₇ is selected from the group consisting of fluorine, chlorine, bromine and methyl; and
Rs is methyl.

9. A compound, or a stereoisomer or a pharmaceutically acceptable salt thereof, which is: 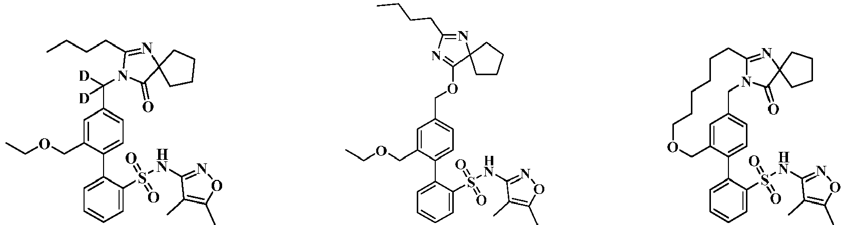 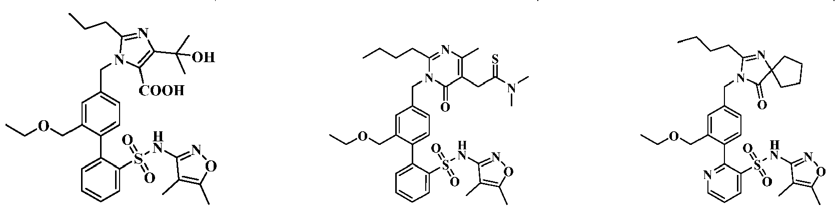 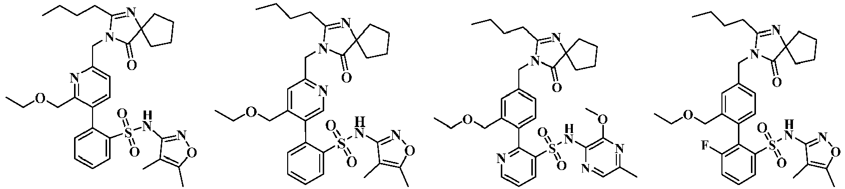 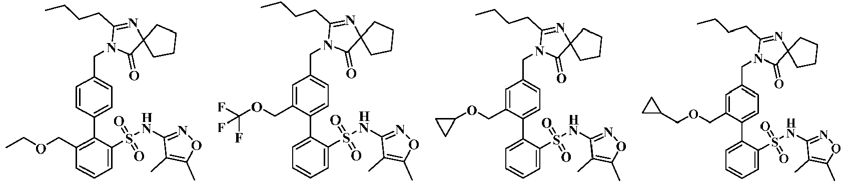 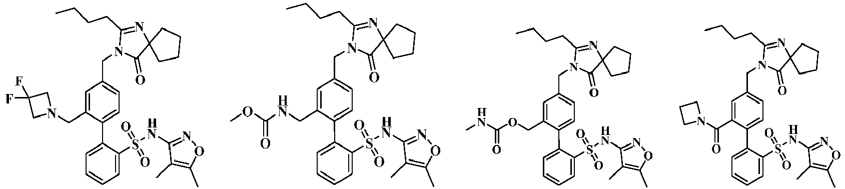 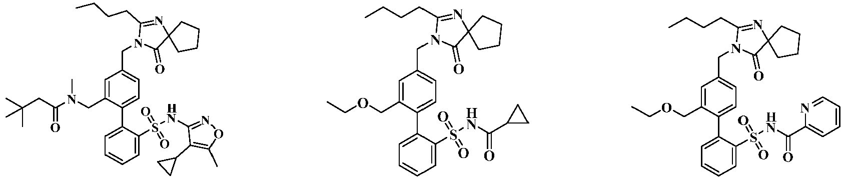 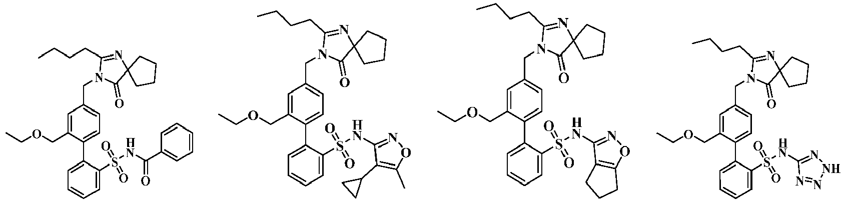 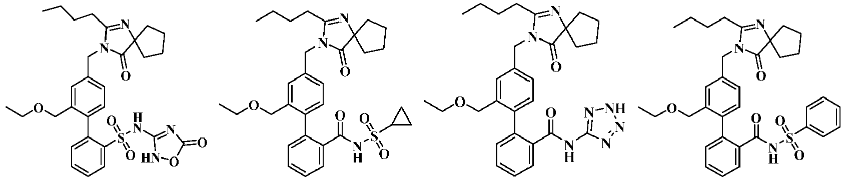 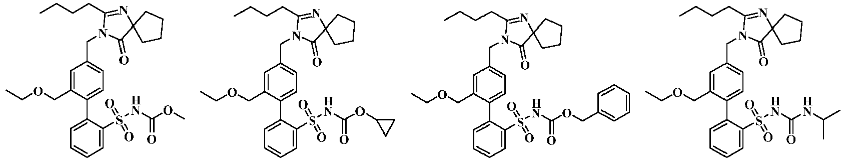 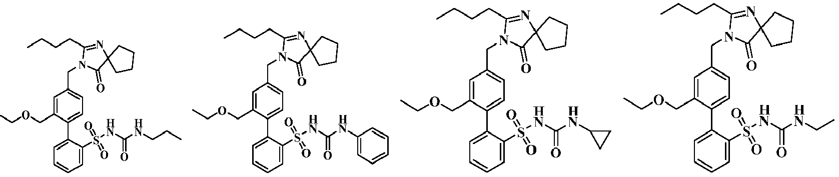 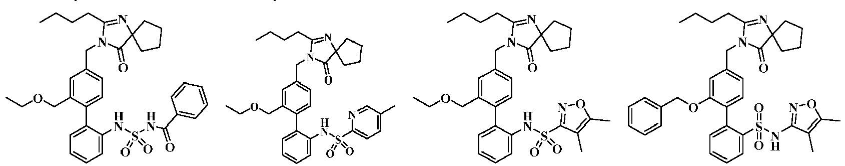 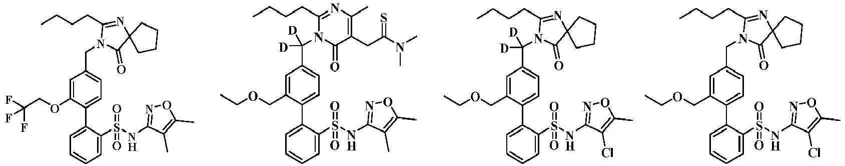 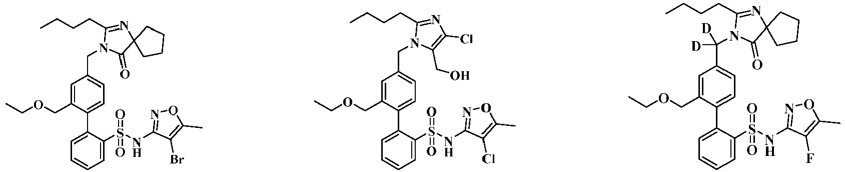 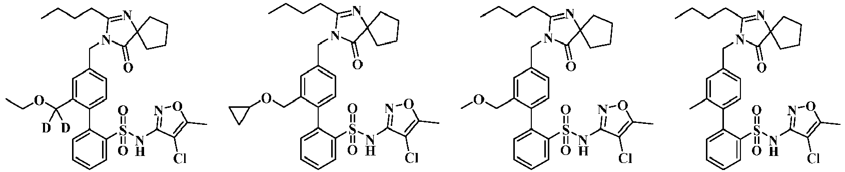 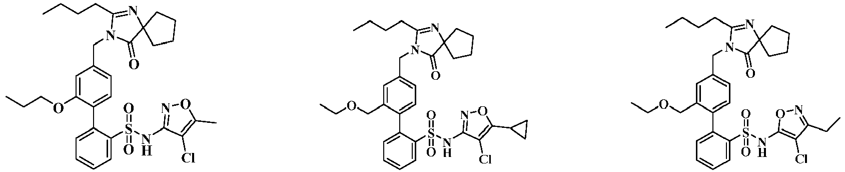 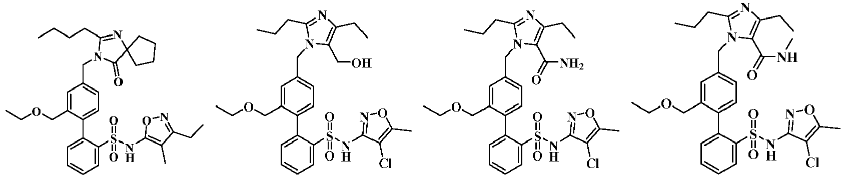 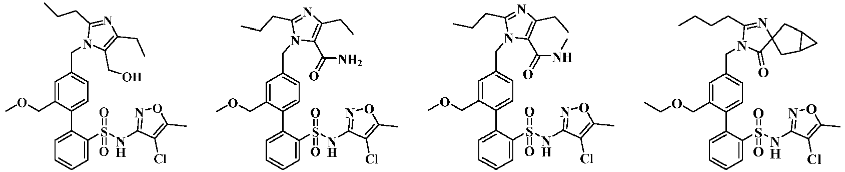 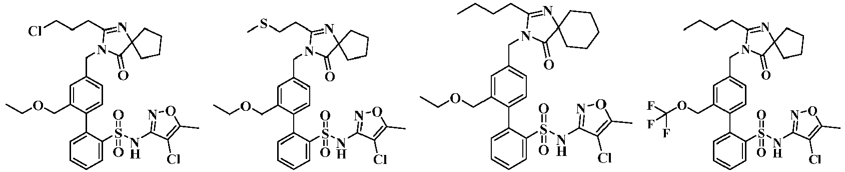 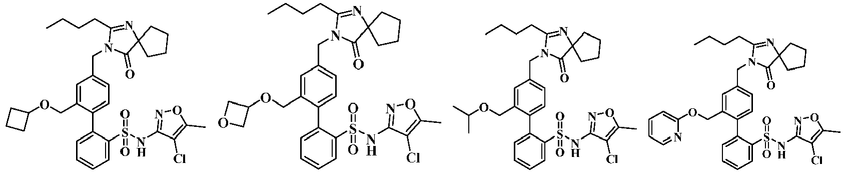 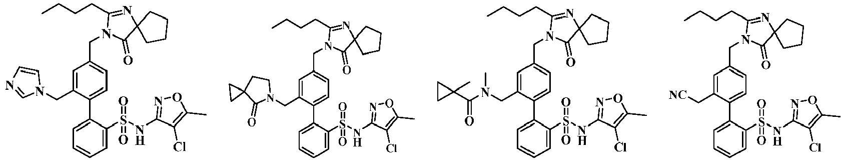 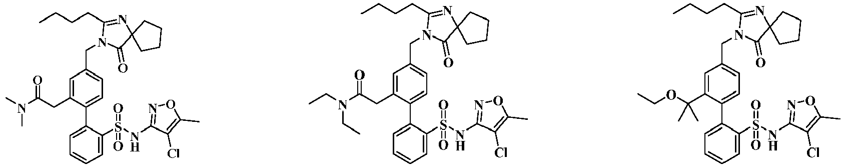 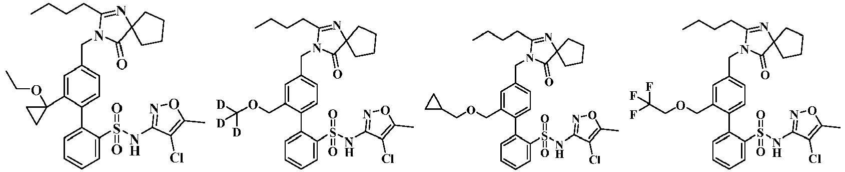 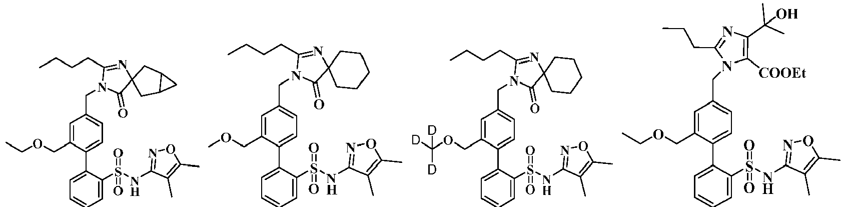 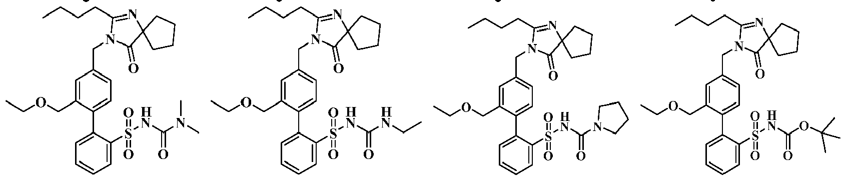 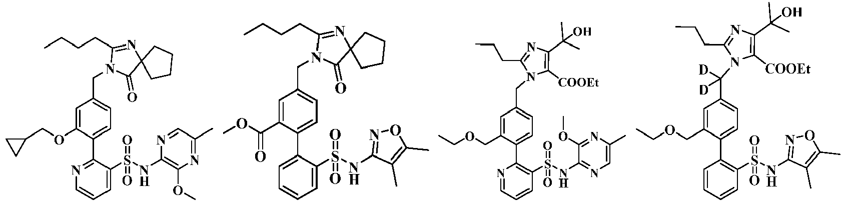 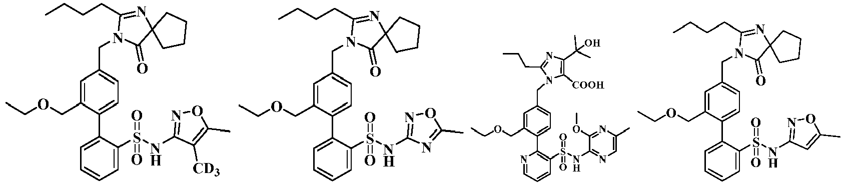 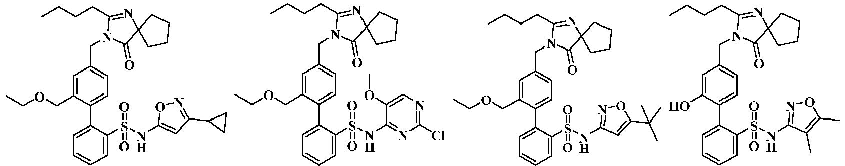 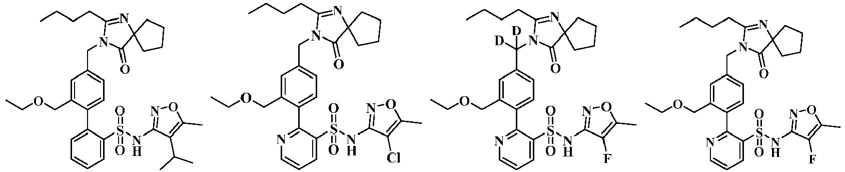 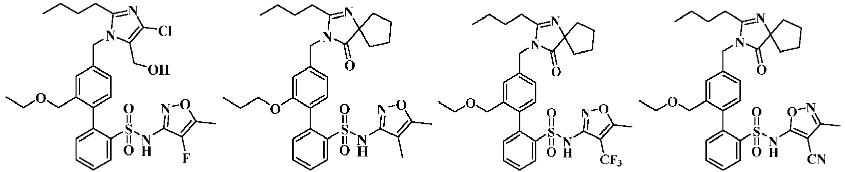 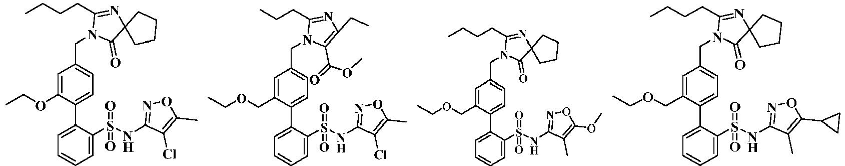 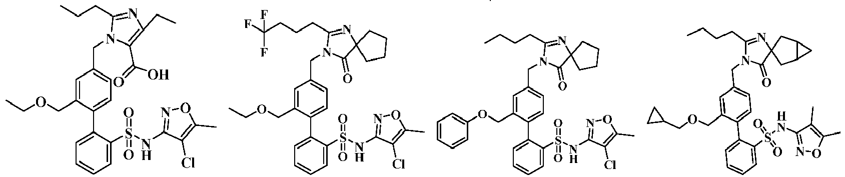 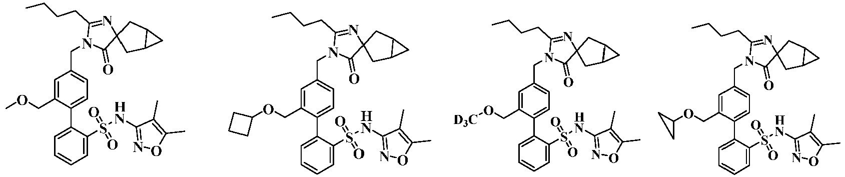 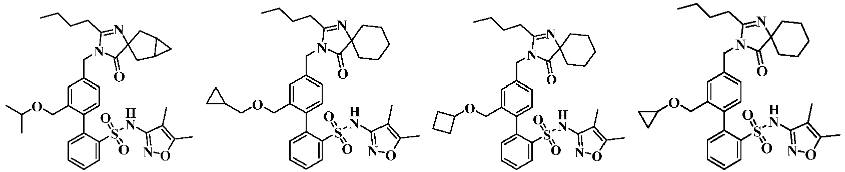 or

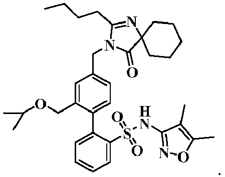

10. A compound of general formula (M-1) or (M-2), a stereoisomer or a pharmaceutically acceptable salt thereof: 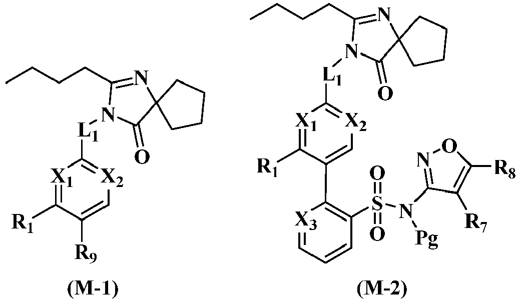
wherein L₁, X₁, X₂, X₃, R₁, R₇, and Rs are as defined in claim 2;
R₉ is selected from the group consisting of halogen and 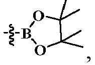 preferably bromine, chlorine or 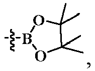 more preferably 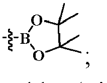
Pg is an amino protecting group, preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl, more preferably (trimethylsilyl)ethoxymethyl or methoxymethylether.

11. A method for preparing the compound, or the stereoisomer or the pharmaceutically acceptable salt thereof according to claim 2, **characterized in that** the method comprises the following steps: 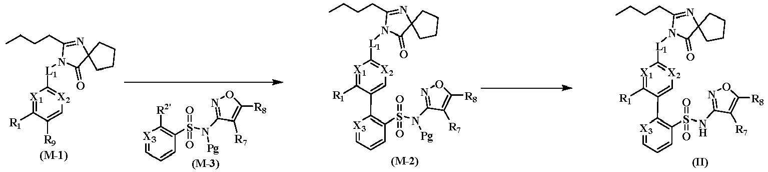
reacting a compound of general formula (M-1) with a compound of general formula (M-3) to obtain a compound of general formula (M-2), and deprotecting the compound of general formula (M-2) to obtain a compound of general formula (II),
wherein L₁, X₁, X₂, X₃, R₁, R₇, and Rs are as defined in claim 2;
R^{2'} is selected from the group consisting of 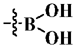 and halogen, preferably 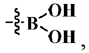 chlorine or bromine;
R₉ is selected from the group consisting of 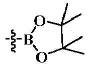 and halogen, preferably 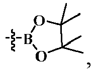 chlorine or bromine;
Pg is an amino protecting group, preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl, more preferably (trimethylsilyl)ethoxymethyl or methoxymethylether;
or 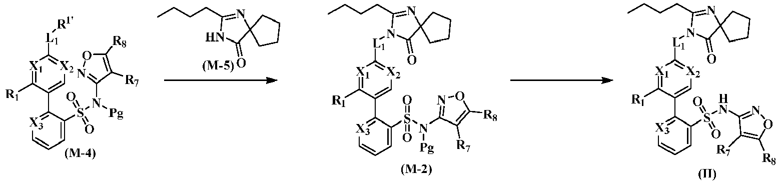
reacting a compound of general formula (M-4) with a compound of general formula (M-5) to obtain a compound of general formula (M-2), and deprotecting the compound of general formula (M-2) to obtain a compound of general formula (II),
wherein L₁, X₁, X₂, X₃, R₁, R₇, and Rs are as defined in claim 2;
R^{1'} is selected from the group consisting of methanesulfonyloxy and halogen, preferably methanesulfonyloxy or bromine;
Pg is an protecting group for amino, preferably (trimethylsilyl)ethoxymethyl, methoxymethylether, allyloxycarbonyl, trifluoroacetyl, 2,4-dimethoxybenzyl, nitrobenzenesulfonyl, trityl, fluorenylmethoxycarbonyl, p-toluenesulfonyl, formate, acetyl, benzyloxycarbonyl, tert-butoxycarbonyl, benzyl or p-methoxyphenyl, more preferably (trimethylsilyl)ethoxymethyl or methoxymethylether.

12. A pharmaceutical composition comprising a therapeutically effective dose of the compound, the stereoisomer or the pharmaceutically acceptable salt thereof according to any one of claims 1 to 9, and one or more pharmaceutically acceptable carriers or excipients.

13. Use of the compound, the stereoisomer or the pharmaceutically acceptable salt thereof according to any one of claims 1 to 9, and the pharmaceutical composition according to claim 12 in the preparation of a drug for treating an angiotensin II-dependent or endothelin-dependent disease; particularly, in the preparation of a drug for treating a dual-acting angiotensin-dependent and endothelin-dependent disease.

14. Use of the compound, the stereoisomer or the pharmaceutically acceptable salt thereof according to any one of claims 1 to 9, and the pharmaceutical composition according to claim 12 in the preparation of a drug for treating pain, sexual dysfunction, hypoxia and an ischemic disease, dementia, a neurological disease, a liver disease, a cancer, hypertension, diabetes or a kidney disease, wherein the kidney disease is a disease or a condition related to kidney, glomerular or glomerular mesangial cell function.

15. The use according to claim 14, **characterized in that** the kidney disease is selected from the group consisting of focal segmental glomerulosclerosis and IgA nephropathy.
